# EUROPÄISCHE PATENTSCHRIFT

(11) **EP 4 078 692 B1**
(45) Veröffentlichungstag und Bekanntmachung des Hinweises auf die Patenterteilung: **18.03.2026**
(21) Anmeldenummer: 20824566.2
(22) Anmeldetag: 17.12.2020
(51) Int. Cl.: C07D 405/04, C07D 405/10, C07D 405/14, C07D 409/04, C07D 409/10, C07D 409/14, C07D 495/04, H10K 71/12, H10K 85/10, H10K 85/60, H10K 50/11, H10K 50/165, H10K 50/16

(54) **VERBINDUNGEN FÜR ELEKTRONISCHE VORRICHTUNGEN**
COMPOUNDS FOR ELECTRONIC DEVICES
COMPOSÉS POUR DISPOSITIFS ÉLECTRONIQUES

(30) Priorität: 19.12.2019 EP 19218239
(43) Veröffentlichungstag der Anmeldung: 26.10.2022
(73) Patentinhaber: Merck Patent GmbH, 64293 Darmstadt (DE)
(72) Erfinder: PARHAM, Amir Hossain, 64293 Darmstadt (DE); MONTENEGRO, Elvira, 64293 Darmstadt (DE)
(74) Vertreter: Merck Patent Association
(86) Internationale Anmeldenummer: PCT/EP2020/086602
(87) Internationale Veröffentlichungsnummer: WO 2021/122868

(56) Entgegenhaltungen:
- KR-A- 20140 109 058

## Beschreibung

Die vorliegende Anmeldung betrifft Fluoren-Derivate sowie Spirobifluoren-Derivate, in denen einer der Benzolringe gegen einen Heteroarylring ausgetauscht ist. Die Verbindungen eignen sich zur Verwendung in elektronischen Vorrichtungen.

Unter elektronischen Vorrichtungen im Sinne dieser Anmeldung werden sogenannte organische elektronische Vorrichtungen verstanden (organic electronic devices), welche organische Halbleitermaterialien als Funktionsmaterialien enthalten. Insbesondere werden darunter OLEDs (organische Elektrolumineszenzvorrichtungen) verstanden. Unter der Bezeichnung OLEDs werden elektronische Vorrichtungen verstanden, welche eine oder mehrere Schichten enthaltend organische Verbindungen aufweisen und unter Anlegen von elektrischer Spannung Licht emittieren. Der Aufbau und das allgemeine Funktionsprinzip von OLEDs sind dem Fachmann bekannt.

Bei elektronischen Vorrichtungen, insbesondere OLEDs, besteht großes Interesse an einer Verbesserung der Leistungsdaten. In diesen Punkten konnte noch keine vollständig zufriedenstellende Lösung gefunden werden.

Einen großen Einfluss auf die Leistungsdaten von elektronischen Vorrichtungen haben Emissionsschichten und Schichten mit elektronentransportierender Funktion. Zur Verwendung in diesen Schichten werden weiterhin neue Verbindungen gesucht, insbesondere elektronentransportierende Verbindungen und Verbindungen, die als elektronentransportierendes Matrixmaterial, insbesondere für phosphoreszierende Emitter, in einer emittierenden Schicht dienen können und in dieser Verwendung zu elektronischen Vorrichtung führen, die gleichzeitig eine hohe Effizienz, lange Lebensdauer undund geringe Betribsspannung aufweisen. Hierzu werden insbesondere Verbindungen gesucht, die eine hohe Glasübergangstemperatur, eine hohe Stabilität, und eine hohe Elektronenleitfähigkeit aufweisen. Eine hohe Stabilität der Verbindung ist eine Voraussetzung, um eine lange Lebensdauer der elektronischen Vorrichtung zu erreichen.

Im Stand der Technik sind insbesondere Hetero-Fluoren- und Hetero-Spirobifluoren-Derivate mit ankondensierten Phenyl-Gruppen sowie bipolare Hetero-Fluoren- und Hetero-Spirobifluoren-Derivate, die insbesondere mit N-Carbazol substituiert sind, als Elektronentransportmaterialien und elektronentransportierende Matrixmaterialien für elektronische Vorrichtungen bekannt.

KR 2014/0109058 A beschreibt u.a.Fluoren- und Spirobifluoren-Derivate, bei welchen ein Benzolring durch einen Pyrrol-Ring ausgetauscht ist und welche eine Diarylamingruppe als einen Substituenten aufweisen. Diese Verbindungen mit lochtransportierenden Eigenschaften können in organischen elektronischen Vorrichtungen eingesetzt werden.

Es besteht jedoch weiterhin Bedarf an alternativen Verbindungen, die zur Verwendung in elektronischen Vorrichtungen geeignet sind, insbesondere an Verbindungen, die eine oder mehrere der oben genannten vorteilhaften Eigenschaften aufweisen. Es besteht weiterhin Verbesserungsbedarf bei den erzielten Leistungsdaten bei der Verwendung der Verbindungen in elektronischen Vorrichtungen, insbesondere bei Lebensdauer, Betriebsspannung und Effizienz der Vorrichtungen.

Es wurde nun gefunden, dass sich bestimmte Fluoren-Derivate sowie Spirobifluoren-Derivate, in denen einer der Benzolringe gegen einen Heteroarylring ausgetauscht ist, hervorragend zur Verwendung in elektronischen Vorrichtungen eignen. Sie eignen sich insbesondere zur Verwendung in OLEDs, und darin insbesondere zur Verwendung als Elektronentransportmaterialien und zur Verwendung als eletronentransportierende Matrixmaterialien, insbesondere für phosphoreszierende Emitter. Die gefundenen Verbindungen führen zu hoher Lebensdauer, hoher Effizienz und geringer Betriebsspannung der elektronischen Vorrichtungen. Weiterhin bevorzugt weisen die gefundenen Verbindungen eine hohe Glasübergangstemperatur, eine hohe Stabilität und eine hohe Leitfähigkeit für Elektronen auf.

Gegenstand der vorliegenden Anmeldung sind damit Verbindungen gemäß der folgenden Formel (I) 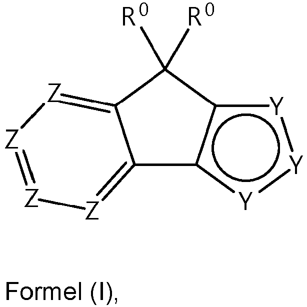 wobei gilt:
Y ist bei jedem Auftreten gleich oder verschieden gewählt aus O, S, NAr° und CAr¹, wobei mindestens ein Y gewählt ist aus O, S und NAr⁰;
Z ist bei jedem Auftreten gleich oder verschieden gewählt aus N und CR¹;
Ar⁰ ist bei jedem Auftreten gleich oder verschieden gewählt aus aromatischen Ringsystemen mit 6 bis 40 aromatischen Ringatomen, die mit Resten R² substituiert sind, und heteroaromatischen Ringsystemen mit 5 bis 40 aromatischen Ringatomen, die mit Resten R² substituiert sind;
Ar¹ ist bei jedem Auftreten gleich oder verschieden gewählt aus H, D, F, Cl, Br, I, C(=O)R², CN, Si(R²)₃, P(=O)(R²)₂, OR², S(=O)R², S(=O)₂R², geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl-, Alkoxy-, Alkenyl- und Alkinylgruppen und die genannten aromatischen Ringsysteme und heteroaromatischen Ringsysteme jeweils mit Resten R² substituiert sind; und wobei eine oder mehrere CH₂-Gruppen in den genannten Alkyl-, Alkoxy-, Alkenyl- und Alkinylgruppen durch - R²C=CR²-, -C≡C-, Si(R²)₂, C=O, C=NR², -C(=O)O-, -C(=O)NR²-, NR², P(=O)(R²), -O-, -S-, SO oder SO₂ ersetzt sein können; wobei ausgeschlossen ist, dass zwei Gruppen Ar¹ miteinander verknüpft sind, um ein mono- oder polycyclisches, aliphatisches oder aromatisches Ringsystem zu bilden;
R⁰ ist bei jedem Auftreten gleich oder verschieden gewählt aus H, D, F, Cl, Br, I, C(=O)R¹, CN, Si(R¹)₃, N(R¹)₂, P(=O)(R¹)₂, OR¹, S(=O)R¹, S(=O)₂R¹, geradkettigen Alkyl-, Alkoxy- oder Thioalkylgruppen mit 1 bis 20 C-Atomen, verzweigten oder cyclischen Alkyl-, Alkoxy- oder Thioalkylgruppen mit 3 bis 20 C-Atomen, Alkenyl- oder Alkinylgruppen mit 2 bis 20 C-Atomen, aromatischen Ringsystemen mit 6 bis 40 aromatischen Ringatomen, heteroaromatischen Ringsystemen mit 5 bis 40 aromatischen Ringatomen, Aryloxy- oder Heteroaryloxygruppen mit 5 bis 40 aromatischen Ringatomen, und Aralkylgruppen mit 5 bis 40 aromatischen Ringatomen; wobei die beiden Reste R⁰ miteinander verknüpft sein können und einen aliphatischen oder heteroaliphatischen Ring bilden können, so dass eine Spiroverbindung in Position 8 des Fluoren-Derivats entsteht, insbesondere ein Spirobifluoren-Derivat; wobei die genannten Alkyl-, Alkoxy-, Thioalkyl-, Alkenyl-, Alkinyl-, Aryloxy-, Heteroaryloxy- und Aralkylgruppen und die genannten aromatischen Ringsysteme und heteroaromatischen Ringsysteme jeweils mit Resten R¹ substituiert sind; und wobei eine oder mehrere CH₂-Gruppen in den genannten Alkyl-, Alkoxy-, Thioalkyl-, Alkenyl-, Alkinyl-, Aryloxy-, Heteroaryloxy- und Aralkylgruppen durch - R¹C=CR¹-, -C≡C-, Si(R¹)₂, C=O, C=NR¹, -C(=O)O-, -C(=O)NR¹-, NR¹, P(=O)(R¹), -O-, -S-, SO oder SO₂ ersetzt sein können;
R¹ ist bei jedem Auftreten gleich oder verschieden gewählt aus H, D, F, Cl, Br, I, C(=O)R⁵, CN, Si(R⁵)₃, P(=O)(R⁵)₂, OR⁵, S(=O)R⁵, S(=O)₂R⁵, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹ miteinander verknüpft sein können und einen aliphatischen oder heteroaliphatischen Ring bilden können; wobei die genannten Alkyl-, Alkoxy-, Alkenyl- und Alkinylgruppen und die genannten aromatischen Ringsysteme und heteroaromatischen Ringsysteme jeweils mit Resten R⁵ substituiert sind; und wobei eine oder mehrere CH₂-Gruppen in den genannten Alkyl-, Alkoxy-, Alkenyl- und Alkinylgruppen durch -R⁵C=CR⁵-, -C≡C-, Si(R⁵)₂, C=O, C=NR⁵, -C(=O)O-, -C(=O)NR⁵-, NR⁵, P(=O)(R⁵), -O-, -S-, SO oder SO₂ ersetzt sein können;
R² ist bei jedem Auftreten gleich oder verschieden gewählt aus H, D, F, Cl, Br, I, C(=O)R⁵, CN, Si(R⁵)₃, P(=O)(R⁵)₂, OR⁵, S(=O)R⁵, S(=O)₂R⁵, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R² miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit Resten R⁵ substituiert sind; und wobei eine oder mehrere CH₂-Gruppen in den genannten Alkyl-, Alkoxy-, Alkenyl- und Alkinylgruppen durch -R⁵C=CR⁵-, -C=C-, Si(R⁵)₂, C=O, C=NR⁵, -C(=O)O-, -C(=O)NR⁵-, NR⁵, P(=O)(R⁵), -O-, -S-, SO oder SO₂ ersetzt sein können;
R⁵ ist bei jedem Auftreten gleich oder verschieden gewählt aus H, D, F, Cl, Br, I, C(=O)R⁶, CN, Si(R⁶)₃, P(=O)(R⁶)₂, OR⁶, S(=O)R⁶, S(=O)₂R⁶, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R⁵ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit Resten R⁶ substituiert sind; und wobei eine oder mehrere CH₂-Gruppen in den genannten Alkyl-, Alkoxy-, Alkenyl- und Alkinylgruppen durch -R⁶C=CR⁶-, -C=C-, Si(R⁶)₂, C=O, C=NR⁶, -C(=O)O-, -C(=O)NR⁶-, NR⁶, P(=O)(R⁶), -O-, -S-, SO oder SO₂ ersetzt sein können;
R⁶ ist bei jedem Auftreten gleich oder verschieden gewählt aus H, D, F, Cl, Br, I, CN, Alkyl- oder Alkoxygruppen mit 1 bis 20 C-Atomen, Alkenyl- oder Alkinylgruppen mit 2 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R⁶ miteinander verknüpft sein können und einen Ring bilden können; und wobei die genannten Alkyl-, Alkoxy-, Alkenyl- und Alkinylgruppen, aromatischen Ringsysteme und heteroaromatischen Ringsysteme mit einem oder mehreren Resten gewählt aus F und CN substituiert sein können; und
in der Formel (I) ist mindestens ein Rest R¹, eine Gruppe Ar⁰ oder eine Gruppe Ar¹ durch eine Gruppe A substituiert, die einer Formel (A) entspricht: 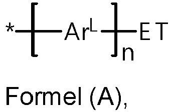 wobei gilt:
   * bezeichnet die Bindung an die Struktur der Formel (I);
   Ar^{L} ist bei jedem Auftreten gleich oder verschieden gewählt aus aromatischen Ringsystemen mit 6 bis 40 aromatischen Ringatomen, die mit Resten R² substituiert sind, und heteroaromatischen Ringsystemen mit 5 bis 40 aromatischen Ringatomen, die mit Resten R² substituiert sind;
   ET ist bei jedem Auftreten gleich oder verschieden eine elektronentransportierende Gruppe, die gewählt ist aus elektronenarmen heteroaromatischen Gruppen, an die eine oder mehrere Gruppen Ar² gebunden sind;
   Ar² ist bei jedem Auftreten gleich oder verschieden gewählt aus ist bei jedem Auftreten gleich oder verschieden gewählt aus H, D, F, Cl, Br, I, C(=O)R³, CN, Si(R³)₃, P(=O)(R³)₂, OR³, S(=O)R³, S(=O)₂R³, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl-, Alkoxy-, Alkenyl- und Alkinylgruppen und die genannten aromatischen Ringsysteme und heteroaromatischen Ringsysteme jeweils mit Resten R³ substituiert sind; wobei eine oder mehrere CH₂-Gruppen in den genannten Alkyl-, Alkoxy-, Alkenyl- und Alkinylgruppen durch -R³C=CR³-, -C=C-, Si(R³)₂, C=O, C=NR³, -C(=O)O-, -C(=O)NR³-, NR³, P(=O)(R³), -O-, -S-, SO oder SO₂ ersetzt sein können; und wobei zwei oder mehr benachbarte Gruppen Ar² miteinander ein mono- oder polycyclisches, aliphatisches oder aromatisches Ringsystem bilden können;
   R³ ist bei jedem Auftreten gleich oder verschieden gewählt ist aus H, D, F, Cl, Br, I, C(=O)R⁵, CN, Si(R⁵)₃, N(R⁵)₂, P(=O)(R⁵)₂, OR⁵, S(=O)R⁵, S(=O)₂R⁵, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R³ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit Resten R⁵ substituiert sind; und wobei eine oder mehrere CH₂-Gruppen in den genannten Alkyl-, Alkoxy-, Alkenyl- und Alkinylgruppen durch -R⁵C=CR⁵-, -C=C-, Si(R⁵)₂, C=O, C=NR⁵, -C(=O)O-, -C(=O)NR⁵-, NR⁵, P(=O)(R⁵), -O-, -S-, SO oder SO₂ ersetzt sein können; und
   n ist gleich 0 oder 1, wobei im Fall n=0 die Gruppe Ar^{L} wegfällt und die Gruppe FG über eine Einfachbindung direkt an die Struktur der Formel (I) gebunden ist.

Folglich enthalten die Verbindungen der Formel (I) definitionsgemäß immer wenigstens einen Substituenten der Formel (A) mit einer elektronentransportierenden Gruppe ET.

Die Zählweise an den Hetero-Fluoren-Derivaten gemäß der vorliegenden Anmeldung ist wie folgt festgelegt:

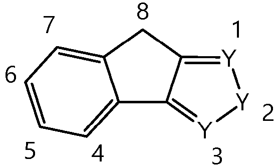

Die Zählweise an den Hetero-Spirobifluoren-Derivaten gemäß der vorliegenden Anmeldung ist wie folgt festgelegt:

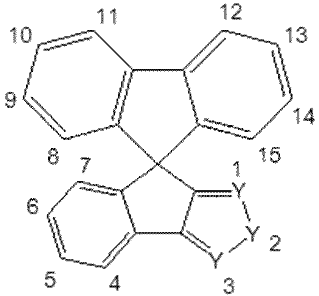

Die folgenden Definitionen gelten für die chemischen Gruppen, die in der vorliegenden Anmeldung verwendet werden. Sie gelten, soweit keine spezielleren Definitionen angegeben sind.

Unter einer Arylgruppe im Sinne dieser Erfindung wird entweder ein einzelner aromatischer Cyclus, also Benzol, oder ein kondensierter aromatischer Polycyclus, beispielsweise Naphthalin, Phenanthren oder Anthracen, verstanden. Ein kondensierter aromatischer Polycyclus besteht im Sinne der vorliegenden Anmeldung aus zwei oder mehr miteinander kondensierten einzelnen aromatischen Cyclen. Unter Kondensation zwischen Cyclen ist dabei zu verstehen, dass die Cyclen mindestens eine Kante miteinander teilen. Eine Arylgruppe im Sinne dieser Erfindung enthält 6 bis 40 aromatische Ringatome. Weiterhin enthält eine Arylgruppe kein Heteroatom als aromatisches Ringatom, sondern nur Kohlenstoffatome.

Unter einer Heteroarylgruppe im Sinne dieser Erfindung wird entweder ein einzelner heteroaromatischer Cyclus, beispielsweise Pyridin, Pyrimidin oder Thiophen, oder ein kondensierter heteroaromatischer Polycyclus, beispielsweise Chinolin oder Carbazol, verstanden. Ein kondensierter heteroaromatischer Polycyclus besteht im Sinne der vorliegenden Anmeldung aus zwei oder mehr miteinander kondensierten einzelnen aromatischen oder heteroaromatischen Cyclen, wobei wenigstens einer der aromatischen und heteroaromatischen Cyclen ein heteroaromatischer Cyclus ist. Unter Kondensation zwischen Cyclen ist dabei zu verstehen, dass die Cyclen mindestens eine Kante miteinander teilen. Eine Heteroarylgruppe im Sinne dieser Erfindung enthält 5 bis 40 aromatische Ringatome, von denen mindestens eines ein Heteroatom darstellt. Die Heteroatome der Heteroarylgruppe sind bevorzugt ausgewählt aus N, O und S.

Unter einer Aryl- oder Heteroarylgruppe, die jeweils mit den oben genannten Resten substituiert sein kann werden insbesondere Gruppen verstanden, welche abgeleitet sind von Benzol, Naphthalin, Anthracen, Phenanthren, Pyren, Dihydropyren, Chrysen, Perylen, Triphenylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Benzimidazolo[1,2-a]benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Unter einer Aryloxygruppe gemäß der Definition der vorliegenden Erfindung wird eine Arylgruppe, wie oben definiert, verstanden, welche über ein Sauerstoffatom gebunden ist. Eine analoge Definition gilt für Heteroaryloxygruppen.

Ein aromatisches Ringsystem im Sinne dieser Erfindung ist ein System, welches nicht notwendigerweise nur Arylgruppen enthält, sondern welches zusätzlich einen oder mehrere nicht-aromatische Ringe enthalten kann, die mit wenigstens einer Arylgruppe kondensiert sind. Diese nicht-aromatischen Ringe enthalten ausschließlich Kohlenstoffatome als Ringatome. Beispiele für Gruppen, die von dieser Definition umfasst sind, sind Tetrahydronaphthalin, Fluoren und Spirobifluoren. Weiterhin umfasst der Begriff aromatisches Ringsystem Systeme, die aus zwei oder mehr aromatischen Ringsystemen bestehen, die über Einfachbindungen miteinander verbunden sind, beispielsweise Biphenyl, Terphenyl, 7-Phenyl-2-fluorenyl, Quaterphenyl und 3,5-Diphenyl-1-phenyl. Ein aromatisches Ringsystem im Sinne dieser Erfindung enthält 6 bis 40 C-Atome und keine Heteroatome im Ringsystem. Die Definition von "aromatisches Ringsystem" umfasst nicht Heteroarylgruppen.

Ein heteroaromatisches Ringsystem entspricht der oben genannten Definition eines aromatischen Ringsystems, mit dem Unterschied dass es mindestens ein Heteroatom als Ringatom enthalten muss. Wie es beim aromatischen Ringsystem der Fall ist, muss das heteroaromatische Ringsystem nicht ausschließlich Arylgruppen und Heteroarylgruppen enthalten, sondern es kann zusätzlich einen oder mehrere nicht-aromatische Ringe enthalten, die mit wenigstens einer Aryl- oder Heteroarylgruppe kondensiert sind. Die nicht-aromatischen Ringe können ausschließlich C-Atome als Ringatome enthalten, oder sie können zusätzlich ein oder mehrere Heteroatome enthalten, wobei die Heteroatome bevorzugt gewählt sind aus N, O und S. Ein Beispiel für ein derartiges heteroaromatisches Ringsystem ist Benzopyranyl. Weiterhin werden unter dem Begriff "heteroaromatisches Ringsystem" Systeme verstanden, die aus zwei oder mehr aromatischen oder heteroaromatischen Ringsystemen bestehen, die miteinander über Einfachbindungen verbunden sind, wie beispielsweise 4,6-Diphenyl-2-triazinyl. Ein heteroaromatisches Ringsystem im Sinne dieser Erfindung enthält 5 bis 40 Ringatome, die gewählt sind aus Kohlenstoff und Heteroatomen, wobei mindestens eines der Ringatome ein Heteroatom ist. Die Heteroatome des heteroaromatischen Ringsystems sind bevorzugt ausgewählt aus N, O und S.

Die Begriffe "heteroaromatisches Ringsystem" und "aromatisches Ringsystem" gemäß der Definition der vorliegenden Anmeldung unterscheiden sich damit dadurch voneinander, dass ein aromatisches Ringsystem kein Heteroatom als Ringatom aufweisen kann, während ein heteroaromatisches Ringsystem mindestens ein Heteroatom als Ringatom aufweisen muss. Dieses Heteroatom kann als Ringatom eines nicht-aromatischen heterocyclischen Rings oder als Ringatom eines aromatischen heterocyclischen Rings vorliegen.

Entsprechend der obenstehenden Definitionen ist jede Arylgruppe vom Begriff "aromatisches Ringsystem" umfasst, und jede Heteroarylgruppe ist vom Begriff "heteroaromatisches Ringsystem" umfasst.

Unter einem aromatischen Ringsystem mit 6 bis 40 aromatischen Ringatomen oder einem heteroaromatischen Ringsystem mit 5 bis 40 aromatischen Ringatomen, werden insbesondere Gruppen verstanden, die abgeleitet sind von den oben unter Arylgruppen und Heteroarylgruppen genannten Gruppen sowie von Biphenyl, Terphenyl, Quaterphenyl, Fluoren, Spirobifluoren, Dihydrophenanthren, Dihydropyren, Tetrahydropyren, Indenofluoren, Truxen, Isotruxen, Spirotruxen, Spiroisotruxen, Indenocarbazol, oder von Kombinationen dieser Gruppen.

Im Rahmen der vorliegenden Erfindung werden unter einer geradkettigen Alkylgruppe mit 1 bis 20 C-Atomen bzw. einer verzweigten oder cyclischen Alkylgruppe mit 3 bis 20 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden.

Unter einer Alkoxy- oder Thioalkylgruppe mit 1 bis 20 C-Atomen, in der auch einzelne H-Atome oder CH₂-Gruppen durch die oben bei der Definition der Reste genannten Gruppen substituiert sein können, werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet.

Gemäß einer bevorzugten Ausführungsform der Erfindung sind in Formel (I) genau zwei Y, mehr bevorzugt ist genau ein Y gewählt aus NAr⁰, O und S, und die anderen beiden Y sind gleich CAr¹. Besonders bevorzugt ist genau ein Y gewählt aus O und S, ganz besonders bevorzugt aus S, und die anderen beiden Y sind gleich CAr¹.

In einer bevorzugten Ausführungsform der Erfindung ist die Verbindung der Formel (I) demnach ausgewählt aus den Verbindungen der folgenden Formeln (II), (III) und (IV): 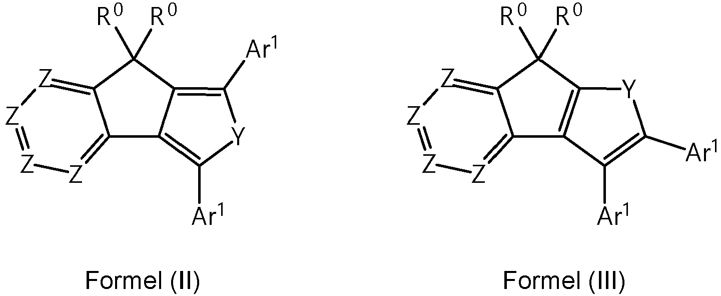 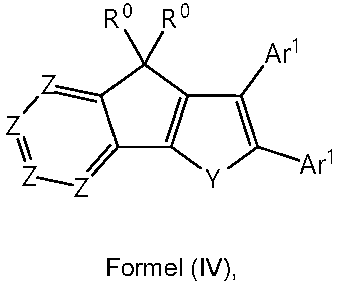 wobei mindestens eine Gruppe A je Formel vorliegt.

Bevorzugt unter den oben genannten Formeln sind die Formeln (II) und (IV), insbesondere die Formel (II), wobei Y bevorzugt gleich O oder S ist, besonders bevorzugt ist Y gleich S.

Bevorzugt sind höchstens zwei Gruppen Z in Formel (I) gleich N, besonders bevorzugt ist höchstens eine Gruppe Z in Formel (I) gleich N, ganz besonders ist keine Gruppe Z gleich N. Die verbleibenden Gruppen Z sind entsprechend gleich CR¹. Weiterhin ist es bevorzugt, dass benachbarte Gruppen Z in einem Ring nicht gleich N sind.

Ar⁰ ist bevorzugt bei jedem Auftreten gleich oder verschieden gewählt aus aromatischen Ringsystemen mit 6 bis 40 aromatischen Ringatomen, die jeweils mit Resten R² substituiert sind. Besonders bevorzugt ist Ar⁰ bei jedem Auftreten gleich oder verschieden gewählt aus Phenyl, Biphenyl, Terphenyl, Quaterphenyl, Naphthyl, Fluorenyl, insbesondere 9,9'-Dimethylfluorenyl und 9,9'-Diphenylfluorenyl, Benzofluorenyl, Spirobifluorenyl, Indenofluorenyl, Indenocarbazolyl, Dibenzofuranyl, Dibenzothiophenyl, Benzofuranyl, Benzothiophenyl, benzokondensiertem Dibenzofuranyl, benzokondensiertem Dibenzothiophenyl, Naphthyl-substituiertem Phenyl, Fluorenyl-substituiertem Phenyl, Spirobifluorenyl-substituiertem Phenyl, Dibenzofuranyl-substituiertem Phenyl, Dibenzothiophenyl-substituiertem Phenyl, Carbazolyl-substituiertem Phenyl, Pyridyl-substituiertem Phenyl, Pyrimidyl-substituiertem Phenyl, und Triazinyl-substituiertem Phenyl, wobei die genannten Gruppen jeweils mit Resten R² substituiert sind. Ganz besonders bevorzugt ist Phenyl, Biphenyl, Terphenyl, Quaterphenyl, Naphthyl, Fluorenyl, insbesondere 9,9'-Dimethylfluorenyl und 9,9'-Diphenylfluorenyl, Naphthyl-substituiertes Phenyl, Fluorenyl-substituiertes Phenyl, Spirobifluorenyl-substituiertes Phenyl, Dibenzofuranyl-substituiertes Phenyl, Dibenzothiophenyl-substituiertes Phenyl, Carbazolyl-substituiertes Phenyl, Pyridyl-substituiertes Phenyl, Pyrimidyl-substituiertes Phenyl, und Triazinylsubstituiertes Phenyl, wobei die genannten Gruppen jeweils mit Resten R² substituiert sind. Am stärksten bevorzugt ist Ar⁰ gleich Phenyl, das mit Resten R² substituiert ist, wobei R² bevorzugt gleich H ist.

Ar¹ ist bevorzugt bei jedem Auftreten gleich oder verschieden gewählt aus H, D, geradkettigen Alkyl- oder Alkoxygruppen mit 1 bis 20 C-Atomen, verzweigten oder cyclischen Alkyl- oder Alkoxygruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen, wobei die Alkylgruppen, Alkoxygruppen, aromatischen Ringsysteme und heteroaromatischen Ringsysteme jeweils mit Resten R² substituiert sind, wobei ausgeschlossen ist, dass zwei Gruppen Ar¹ miteinander verknüpft sind, um einen aliphatischen oder heteroaliphatischen Ring zu bilden ; Ar¹ ist besonders bevorzugt bei jedem Auftreten gleich oder verschieden gewählt aus H und aromatischen Ringsystemen mit 6 bis 40 aromatischen Ringatomen, die mit Resten R² substituiert sind. Ganz besonders bevorzugt ist Ar¹ bei jedem Auftreten gleich oder verschieden gewählt aus Phenyl, Biphenyl, Terphenyl, Quaterphenyl, Naphthyl, Fluorenyl, insbesondere 9,9'-Dimethylfluorenyl und 9,9'-Diphenylfluorenyl, Benzofluorenyl, Spirobifluorenyl, Indenofluorenyl, Indenocarbazolyl, Dibenzofuranyl, Dibenzothiophenyl, Benzofuranyl, Benzothiophenyl, benzokondensiertem Dibenzofuranyl, benzokondensiertem Dibenzothiophenyl, Naphthyl-substituiertem Phenyl, Fluorenyl-substituiertem Phenyl, Spirobifluorenyl-substituiertem Phenyl, Dibenzofuranyl-substituiertem Phenyl, Dibenzothiophenyl-substituiertem Phenyl, Carbazolyl-substituiertem Phenyl, Pyridyl-substituiertem Phenyl, Pyrimidyl-substituiertem Phenyl, Triazinyl-substituiertem Phenyl, wobei die genannten Gruppen jeweils mit Resten R² substituiert sind, und H. Noch stärker bevorzugt ist Phenyl, Biphenyl, Terphenyl, Quaterphenyl, Naphthyl, Fluorenyl, insbesondere 9,9'-Dimethylfluorenyl und 9,9'-Diphenylfluorenyl, Naphthyl-substituiertes Phenyl, Fluorenyl-substituiertes Phenyl, Spirobifluorenyl-substituiertes Phenyl, Dibenzofuranyl-substituiertes Phenyl, Dibenzothiophenyl-substituiertes Phenyl, Carbazolyl-substituiertes Phenyl, Pyridyl-substituiertes Phenyl, Pyrimidyl-substituiertes Phenyl, Triazinylsubstituiertes Phenyl, wobei die genannten Gruppen jeweils mit Resten R² substituiert sind, wobei R² bevorzugt gleich H ist, und H.

Bevorzugte Gruppen Ar¹ sind in der folgenden Tabelle gezeigt:

| | | |
|---|---|---|
| 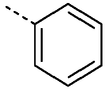 | 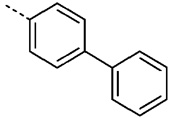 | 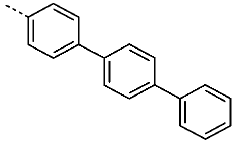 |
| Ar¹-1 | Ar¹-2 | Ar¹-3 |
| 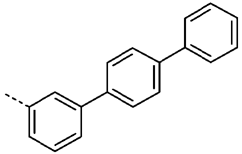 | 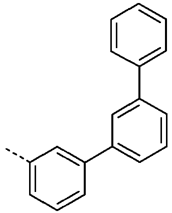 | 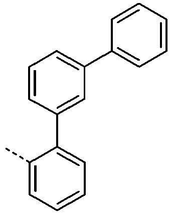 |
| Ar¹-4 | Ar¹-5 | Ar¹-6 |
| 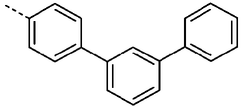 | 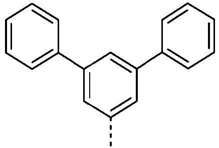 | 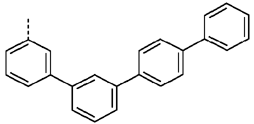 |
| Ar¹-7 | Ar¹-8 | Ar¹-9 |
| 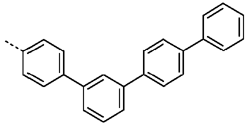 | 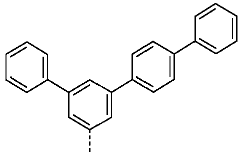 | 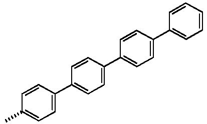 |
| Ar¹-10 | Ar¹-11 | Ar¹-12 |
| 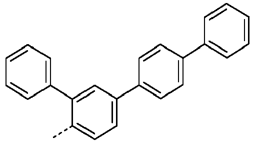 | 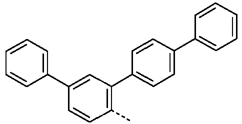 | 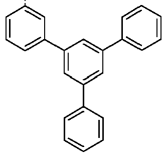 |
| Ar¹-13 | Ar¹-14 | Ar¹-15 |
| 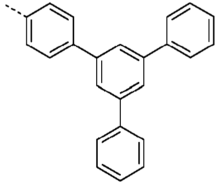 | 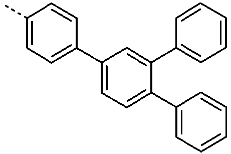 | 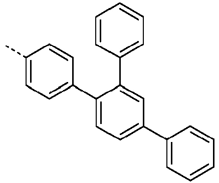 |
| Ar¹-16 | Ar¹-17 | Ar¹-18 |
| 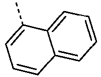 | 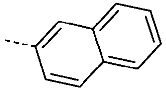 | 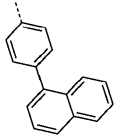 |
| Ar¹-19 | Ar¹-20 | Ar¹-21 |
| 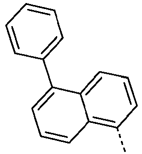 | 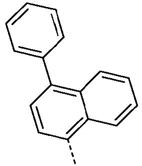 | 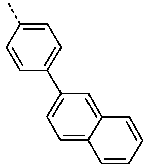 |
| Ar¹-22 | Ar¹-23 | Ar¹-24 |
| 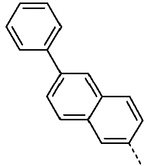 | 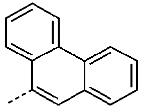 | 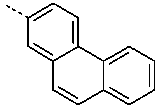 |
| Ar¹-25 | Ar¹-26 | Ar¹-27 |
| 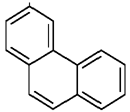 | 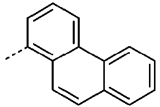 | 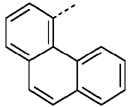 |
| Ar¹-28 | Ar¹-29 | Ar¹-30 |
| 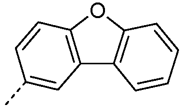 | 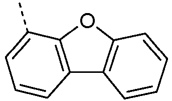 | 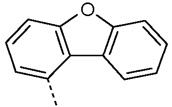 |
| Ar¹-31 | Ar¹-32 | Ar¹-33 |
| 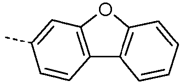 | 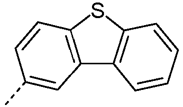 | 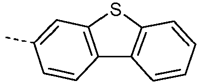 |
| Ar¹-34 | Ar¹-35 | Ar¹-36 |
| 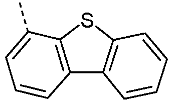 | 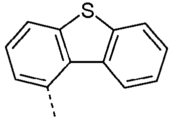 | 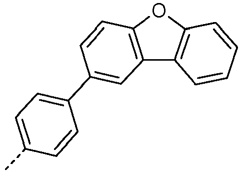 |
| Ar¹-37 | Ar¹-38 | Ar¹-39 |
| 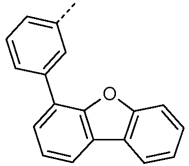 | 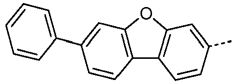 | 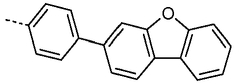 |
| Ar¹-40 | Ar¹-41 | Ar¹-42 |
| 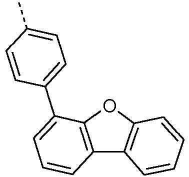 | 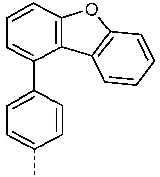 | 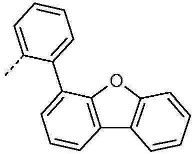 |
| Ar¹-43 | Ar¹-44 | Ar¹-45 |
| 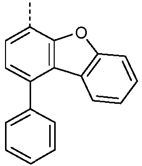 | 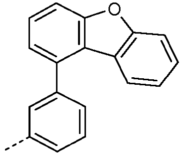 | 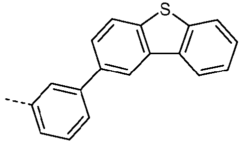 |
| Ar¹-46 | Ar¹-47 | Ar¹-48 |
| 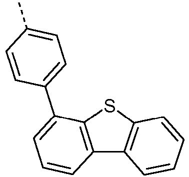 | 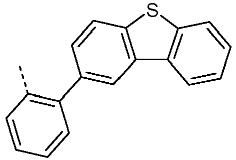 | 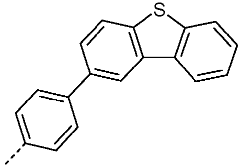 |
| Ar¹-49 | Ar¹-50 | Ar¹-51 |
| 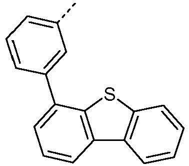 | 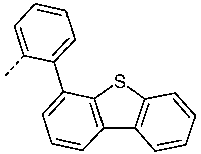 | 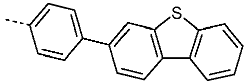 |
| Ar¹-52 | Ar¹-53 | Ar¹-54 |
| 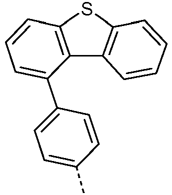 | 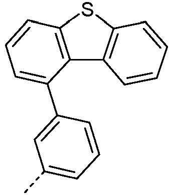 | 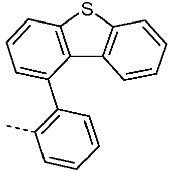 |
| Ar¹-55 | Ar¹-56 | Ar¹-57 |
| 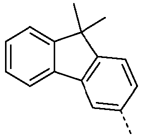 | 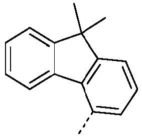 | 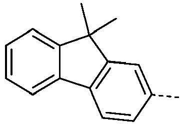 |
| Ar¹-82 | Ar¹-83 | Ar¹-84 |
| 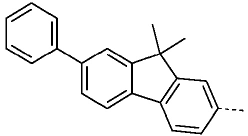 | 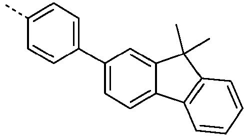 | 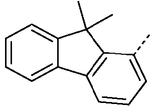 |
| Ar¹-85 | Ar¹-86 | Ar¹-87 |
| 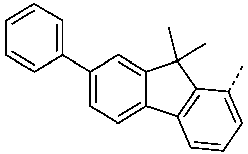 | 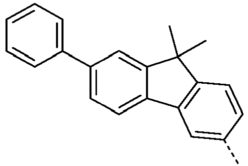 | 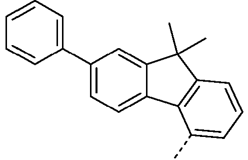 |
| Ar¹-88 | Ar¹-89 | Ar¹-90 |
| 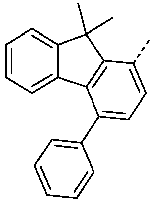 | 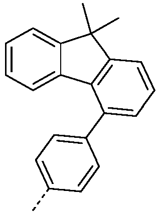 | 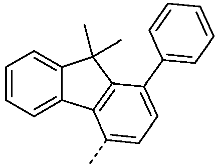 |
| Ar¹-91 | Ar¹-92 | Ar¹-93 |
| 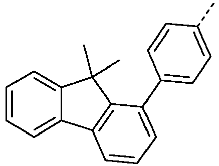 | 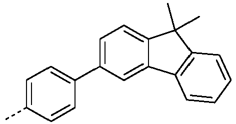 | 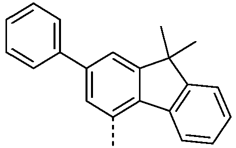 |
| Ar¹-94 | Ar¹-95 | Ar¹-96 |
| 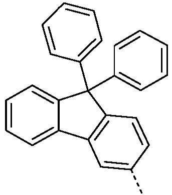 | 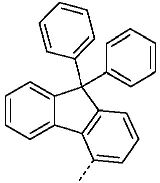 | 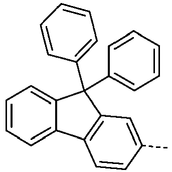 |
| Ar¹-97 | Ar¹-98 | Ar¹-99 |
| 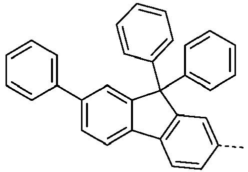 | 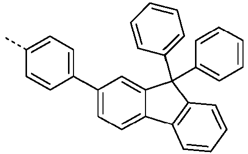 | 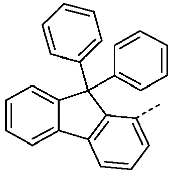 |
| Ar¹-100 | Ar¹-101 | Ar¹-102 |
| 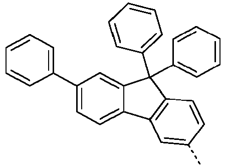 | 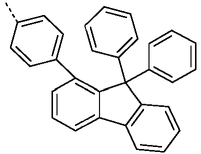 | 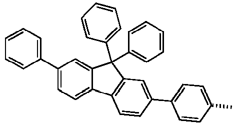 |
| Ar¹-103 | Ar¹-104 | Ar¹-105 |
| 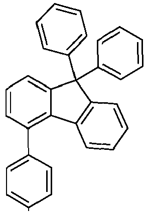 | 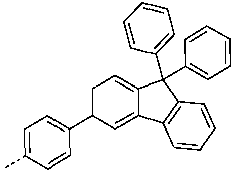 | 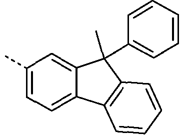 |
| Ar¹-106 | Ar¹-107 | Ar¹-108 |
| 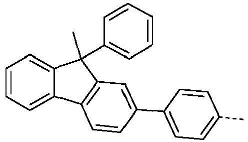 | 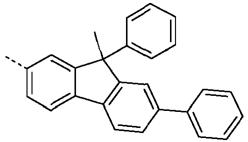 | 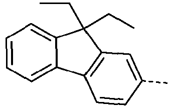 |
| Ar¹-109 | Ar¹-110 | Ar¹-111 |
| 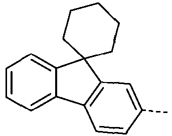 | 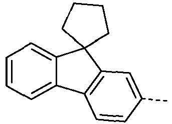 | 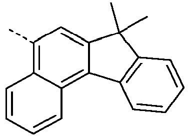 |
| Ar¹-112 | Ar¹-113 | Ar¹-114 |
| 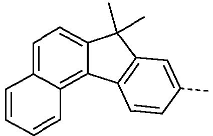 | 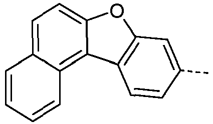 | 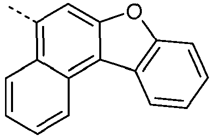 |
| Ar¹-115 | Ar¹-116 | Ar¹-117 |
| 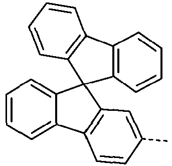 | 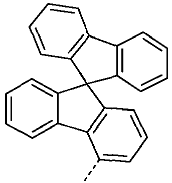 | 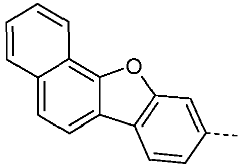 |
| Ar¹-118 | Ar¹-119 | Ar¹-120 |
| 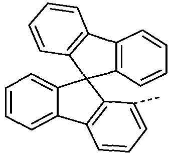 | 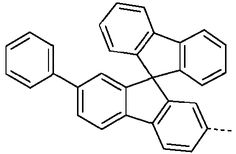 | 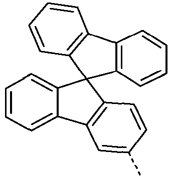 |
| Ar¹-121 | Ar¹-122 | Ar¹-123 |
| 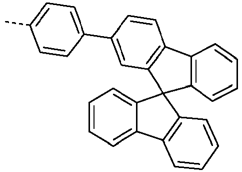 | 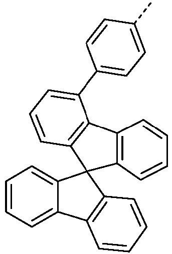 | 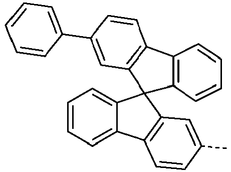 |
| Ar¹-124 | Ar¹-125 | Ar¹-126 |
| 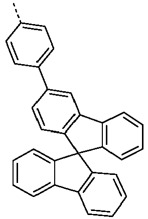 | 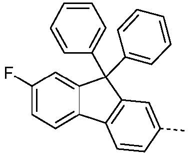 | 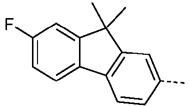 |
| Ar¹-127 | Ar¹-128 | Ar¹-129 |
| 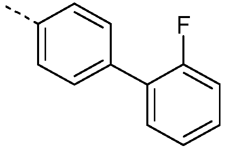 | 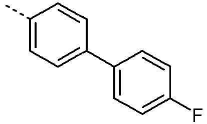 | 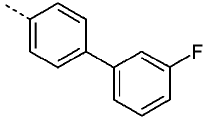 |
| Ar¹-130 | Ar¹-131 | Ar¹-132 |
| 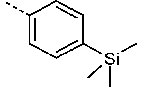 | 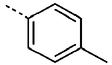 | 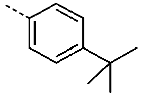 |
| Ar¹-133 | Ar¹-134 | Ar¹-135 |
| 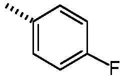 | 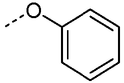 | 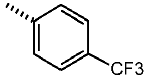 |
| Ar¹-136 | Ar¹-137 | Ar¹-138 |
| 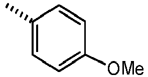 | 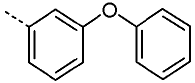 | 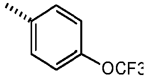 |
| Ar¹-139 | Ar¹-140 | Ar¹-141 |
| 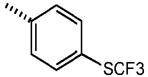 | -F | -Cl |
| Ar¹-142 | Ar¹-143 | Ar¹-144 |
| -Br | -I | -H |
| Ar¹-145 | Ar¹-146 | Ar¹-147 |
| -CH₃ | 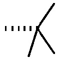 | -CH₂CH₃ |
| Ar¹-148 | Ar¹-149 | Ar¹-150 |
| 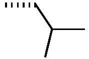 | -CF₃ | -CF₂CF₃ |
| Ar¹-151 | Ar¹-152 | Ar¹-153 |
| -OCF₃ | -SCF₃ | -SF₅ |
| Ar¹-154 | Ar¹-155 | Ar¹-156 |
| -OCF₂CF₃ | -SCF₂CF₃ | 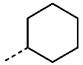 |
| Ar¹-157 | Ar¹-158 | Ar¹-159 |
| 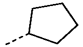 | 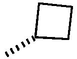 | 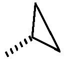 |
| Ar¹-160 | Ar¹-161 | Ar¹-162 |
| -CN | -SCN | -OCH₂CH₃ |
| Ar¹-163 | Ar¹-164 | Ar¹-165 |
| -OCH₃ | -SCH₃ | -Si(CH₃)₃ |
| Ar¹-166 | Ar¹-167 | Ar¹-168 |
| -Si(CH₃)₂t-Bu | -Si(iPr)₃ | -Si(CH₃)₂Ph |
| Ar¹-169 | Ar¹-170 | Ar¹-171 |
| Si-(Ph)₃ | C-(Ph)₃ | -D |
| Ar¹-172 | Ar¹-173 | Ar¹-174 |
| -CD₃ | -CD₂-CD₃ | -C(CD₃)₃ |
| Ar¹-175 | Ar¹-176 | Ar¹-177 |
| -CD₂-(CD₃)₂ | -OCD₃ | -SCD₃ |
| Ar¹-178 | Ar¹-179 | Ar¹-180 |
| -Si(CD₃)₃ | 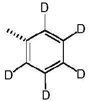 | 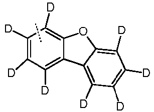 |
| Ar¹-181 | Ar¹-182 | Ar¹-183 |
| 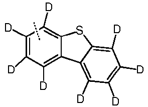 | 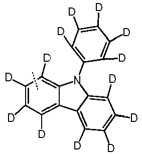 | -CD₂(CH₃)₂ |
| Ar¹-184 | Ar¹-185 | Ar¹-186 |
| -CD₂(CH₃)₃ | 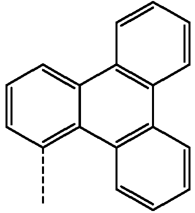 | 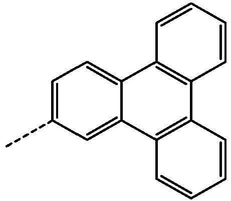 |
| Ar¹-187 | Ar¹-188 | Ar¹-189 |
| 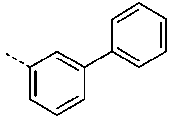 | 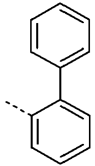 | |
| Ar¹-190 | Ar'-191, | |

wobei die gestrichelten Linien die Bindungen an den Rest der Formel (I) darstellen. Dabei sind die Gruppen (Ar¹-1), (Ar¹-2), (Ar¹-8), (Ar¹-28), (Ar¹-30), (Ar¹-31), (Ar¹-34), (Ar¹-34), (Ar¹-43), (Ar¹-44), (Ar¹-144), (Ar¹-163), (Ar¹-189) und (Ar¹-190) besonders bevorzugt.

R¹ ist bevorzugt bei jedem Auftreten gleich oder verschieden gewählt aus H, D, F, CN, Si(R⁵)₃, geradkettigen Alkyl- oder Alkoxygruppen mit 1 bis 20 C-Atomen, verzweigten oder cyclischen Alkyl- oder Alkoxygruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl- und Alkoxygruppen, die genannten aromatischen Ringsysteme und die genannten heteroaromatischen Ringsysteme jeweils mit Resten R⁵ substituiert sind; und wobei in den genannten Alkyl- oder Alkoxygruppen eine oder mehrere CH₂-Gruppen durch -C≡C-, -R⁵C=CR⁵-, Si(R⁵)₂, C=O, C=NR⁵, -NR⁵-, -O-, - S-, -C(=O)O- oder -C(=O)NR⁵- ersetzt sein können. Besonders bevorzugt ist R¹ bei jedem Auftreten gleich oder verschieden gewählt aus H, D, Si(R⁵)₃, geradkettigen Alkylgruppen mit 1 bis 20 C-Atomen, die deuteriert sein können, verzweigten oder cyclischen Alkylgruppen mit 3 bis 20 C-Atomen, die deuteriert sein können, aromatischen Ringsystemen mit 6 bis 40 aromatischen Ringatomen, die deuteriert sein können, und heteroaromatischen Ringsystemen mit 5 bis 40 aromatischen Ringatomen, die deuteriert sein können, wobei die genannten Alkylgruppen, die genannten aromatischen Ringsysteme und die genannten heteroaromatischen Ringsysteme jeweils mit Resten R⁵ substituiert sind, die bevorzugt gleich H sind. Ganz besonders bevorzugt ist R¹ gleich H.

Dabei vorzugsweise ist augeschlossen, dass R¹ sowie Ar⁰ und Ar¹ eine Carbazolyl-Gruppe bedeuten, die über ihr N-Atom an den Rest der Formel (I) gebunden ist.

Bevorzugte Gruppen R¹ sind in der folgenden Tabelle gezeigt:

| | | |
|---|---|---|
| 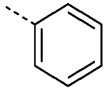 | 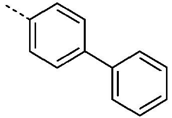 | 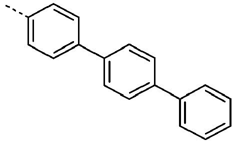 |
| R¹-1 | R¹-2 | R¹-3 |
| 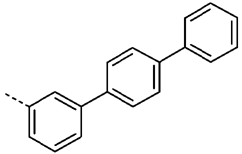 | 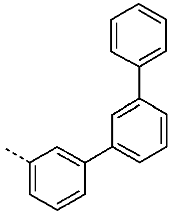 | 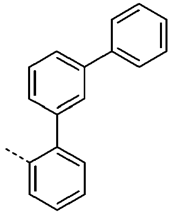 |
| R¹-4 | R¹-5 | R¹-6 |
| 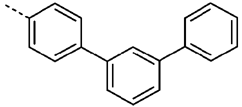 | 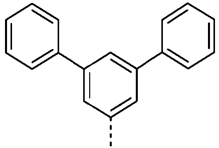 | 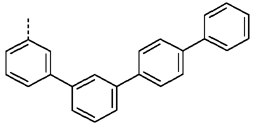 |
| R¹-7 | R¹-8 | R¹-9 |
| 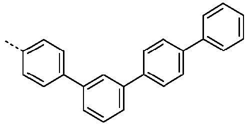 | 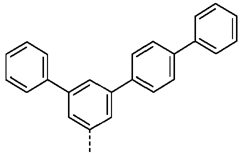 | 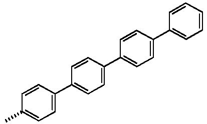 |
| R¹-10 | R¹-11 | R¹-12 |
| 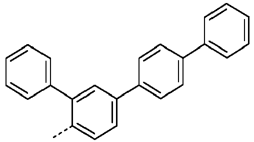 | 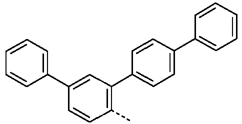 | 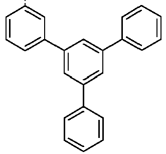 |
| R¹-13 | R¹-14 | R¹-15 |
| 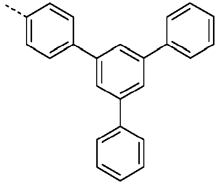 | 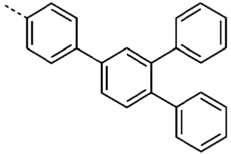 | 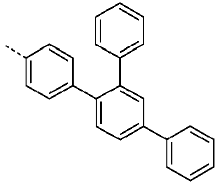 |
| R¹-16 | R¹-17 | R¹-18 |
| 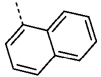 | 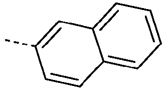 | 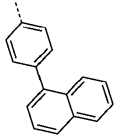 |
| R¹-19 | R¹-20 | R¹-21 |
| 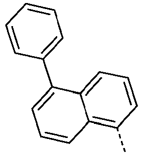 | 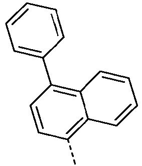 | 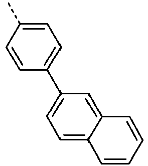 |
| R¹-22 | R¹-23 | R¹-24 |
| 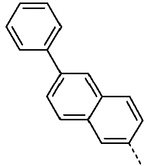 | 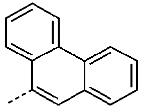 | 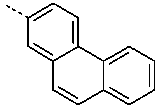 |
| R¹-25 | R¹-26 | R¹-27 |
| 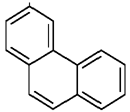 | 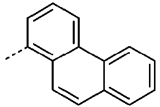 | 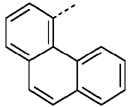 |
| R¹-28 | R¹-29 | R¹-30 |
| 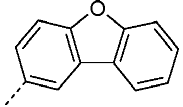 | 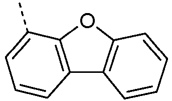 | 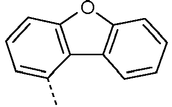 |
| R¹-31 | R¹-32 | R¹-33 |
| 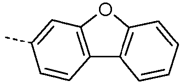 | 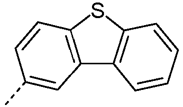 | 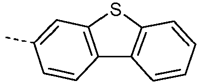 |
| R¹-34 | R¹-35 | R¹-36 |
| 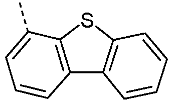 | 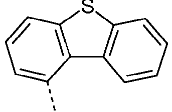 | 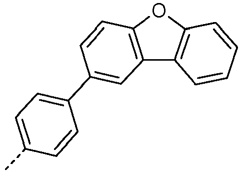 |
| R¹-37 | R¹-38 | R¹-39 |
| 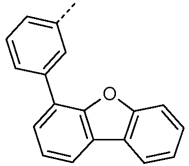 | 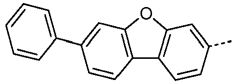 | 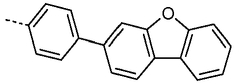 |
| R¹-40 | R¹-41 | R¹-42 |
| 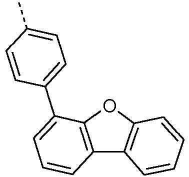 | 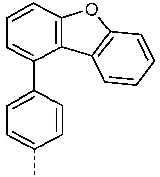 | 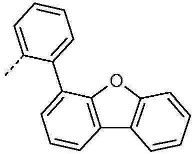 |
| R¹-43 | R¹-44 | R¹-45 |
| 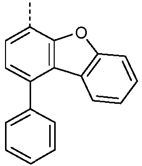 | 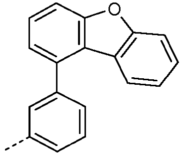 | 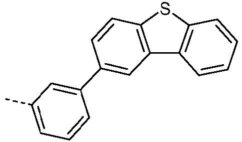 |
| R¹-46 | R¹-47 | R¹-48 |
| 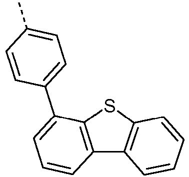 | 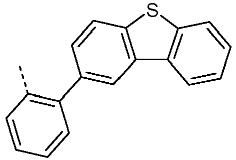 | 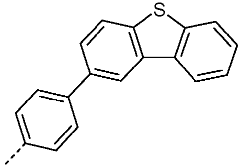 |
| R¹-49 | R¹-50 | R¹-51 |
| 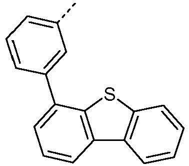 | 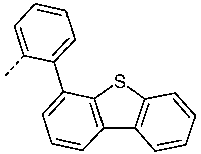 | 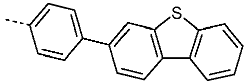 |
| R¹-52 | R¹-53 | R¹-54 |
| 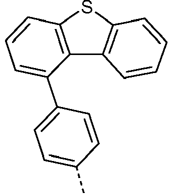 | 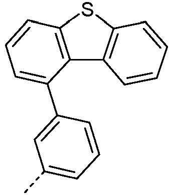 | 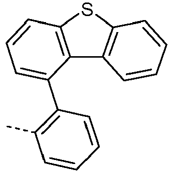 |
| R¹-55 | R¹-56 | R¹-57 |
| 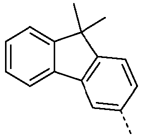 | 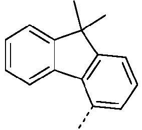 | 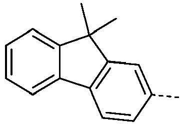 |
| R¹-82 | R¹-83 | R¹-84 |
| 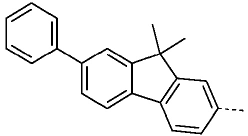 | 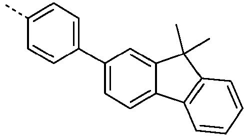 | 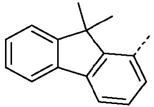 |
| R¹-85 | R¹-86 | R¹-87 |
| 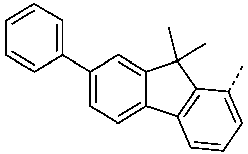 | 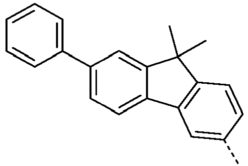 | 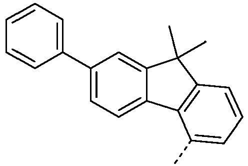 |
| R¹-88 | R¹-89 | R¹-90 |
| 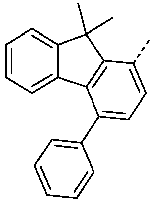 | 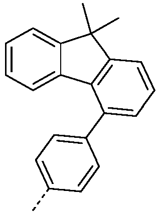 | 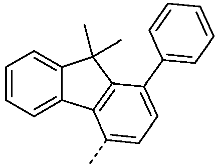 |
| R¹-91 | R¹-92 | R¹-93 |
| 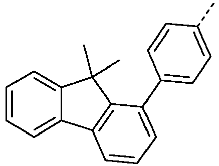 | 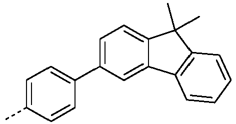 | 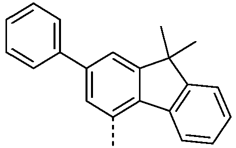 |
| R¹-94 | R¹-95 | R¹-96 |
| 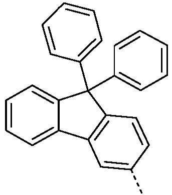 | 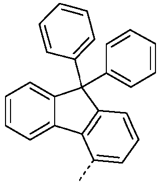 | 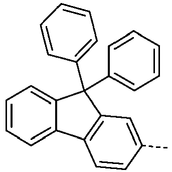 |
| R¹-97 | R¹-98 | R¹-99 |
| 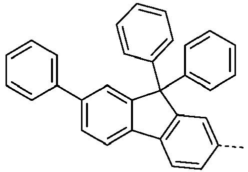 | 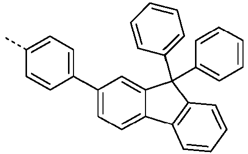 | 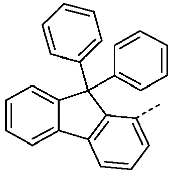 |
| R¹-100 | R¹-101 | R¹-102 |
| 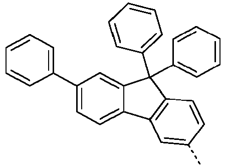 | 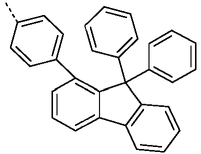 | 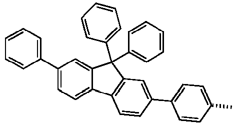 |
| R¹-103 | R¹-104 | R¹-105 |
| 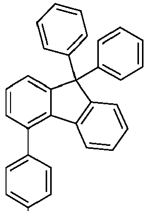 | 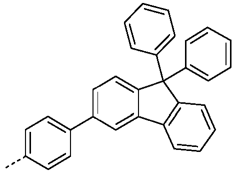 | 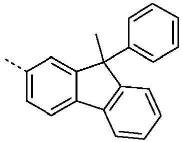 |
| R¹-106 | R¹-107 | R¹-108 |
| 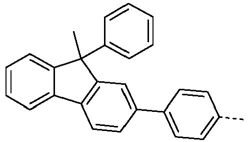 | 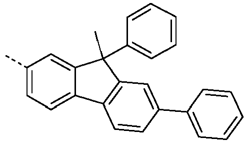 | 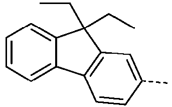 |
| R¹-109 | R¹-110 | R¹-111 |
| 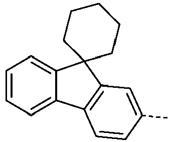 | 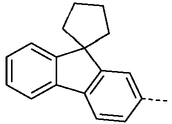 | 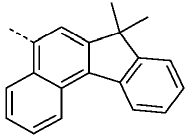 |
| R¹-112 | R¹-113 | R¹-114 |
| 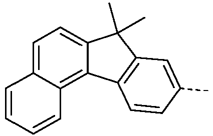 | 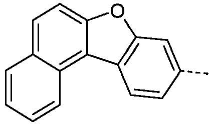 | 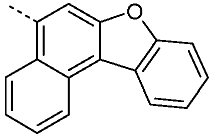 |
| R¹-115 | R¹-116 | R¹-117 |
| 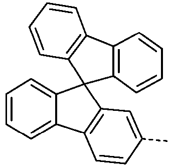 | 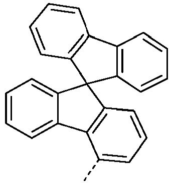 | 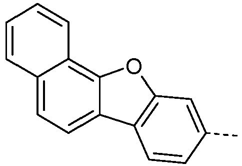 |
| R¹-118 | R¹-119 | R¹-120 |
| 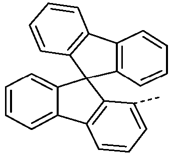 | 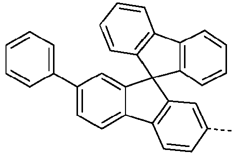 | 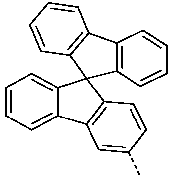 |
| R¹-121 | R¹-122 | R¹-123 |
| 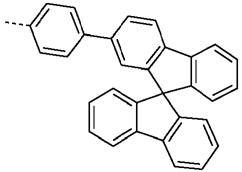 | 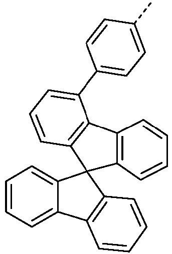 | 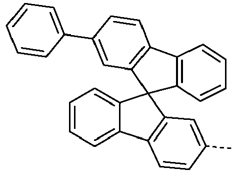 |
| R¹-124 | R¹-125 | R¹-126 |
| 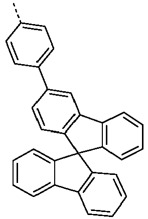 | 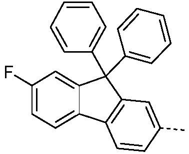 | 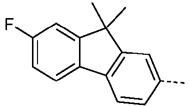 |
| R¹-127 | R¹-128 | R¹-129 |
| 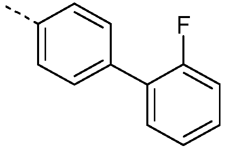 | 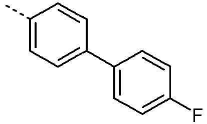 | 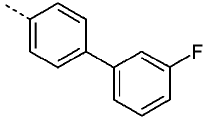 |
| R¹-130 | R¹-131 | R¹-132 |
| 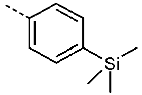 | 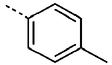 | 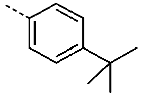 |
| R¹-133 | R¹-134 | R¹-135 |
| 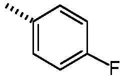 | 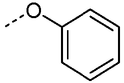 | 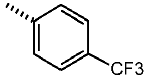 |
| R¹-136 | R¹-137 | R¹-138 |
| 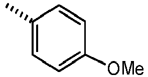 | 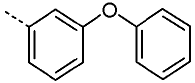 | 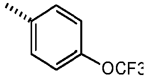 |
| R¹-139 | R¹-140 | R¹-141 |
| 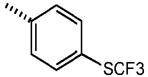 | -F | -Cl |
| R¹-142 | R¹-143 | R¹-144 |
| -Br | -I | -H |
| R¹-145 | R¹-146 | R¹-147 |
| -CH₃ | 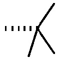 | -CH₂CH₃ |
| R¹-148 | R¹-149 | R¹-150 |
| 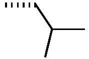 | -CF₃ | -CF₂CF₃ |
| R¹-151 | R¹-152 | R¹-153 |
| -OCF₃ | -SCF₃ | -SF₅ |
| R¹-154 | R¹-155 | R¹-156 |
| -OCF₂CF₃ | -SCF₂CF₃ | 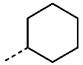 |
| R¹-157 | R¹-158 | R¹-159 |
| 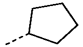 | 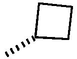 | 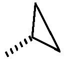 |
| R¹-160 | R¹-161 | R¹-162 |
| -CN | -SCN | -OCH₂CH₃ |
| R¹-163 | R¹-164 | R¹-165 |
| -OCH₃ | -SCH₃ | -Si(CH₃)₃ |
| R¹-166 | R¹-167 | R¹-168 |
| -Si(CH₃)₂t-Bu | -Si(iPr)₃ | -Si(CH₃)₂Ph |
| R¹-169 | R¹-170 | R¹-171 |
| Si-(Ph)₃ | C-(Ph)₃ | -D |
| R¹-172 | R¹-173 | R¹-174 |
| -CD₃ | -CD₂-CD₃ | -C(CD₃)₃ |
| R¹-175 | R¹-176 | R¹-177 |
| -CD₂-(CD₃)₂ | -OCD₃ | -SCD₃ |
| R¹-178 | R¹-179 | R¹-180 |
| -Si(CD₃)₃ | 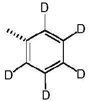 | 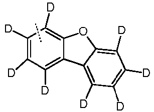 |
| R¹-181 | R¹-182 | R¹-183 |
| 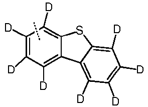 | 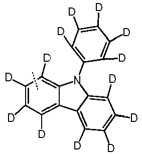 | -CD₂(CH₃)₂ |
| R¹-184 | R¹-185 | R¹-186 |
| -CD₂(CH₃)₃ | 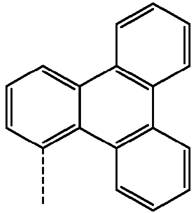 | 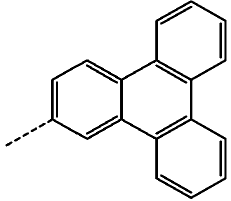 |
| R¹-187 | R¹-188 | R¹-189 |
| 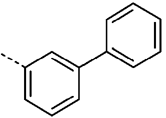 | 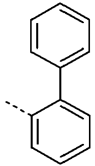 | |
| R¹-190 | R¹-191, | |

wobei die gestrichelten Linien die Bindungen an den Rest der Formel (I) darstellen. Dabei sind die Gruppen (Ar¹-1), (Ar¹-2), (Ar¹-8), (Ar¹-28), (Ar¹-30), (Ar¹-31), (Ar¹-34), (Ar¹-34), (Ar¹-43), (Ar¹-44), (Ar¹-144), (Ar¹-163), (Ar¹-189) und (Ar¹-190) besonders bevorzugt.

R² ist bevorzugt bei jedem Auftreten gleich oder verschieden gewählt aus H, D, F, CN, Si(R⁵)₃, geradkettigen Alkyl- oder Alkoxygruppen mit 1 bis 20 C-Atomen, verzweigten oder cyclischen Alkyl- oder Alkoxygruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl- und Alkoxygruppen, die genannten aromatischen Ringsysteme und die genannten heteroaromatischen Ringsysteme jeweils mit Resten R⁵ substituiert sind; und wobei in den genannten Alkyl- oder Alkoxygruppen eine oder mehrere CH₂-Gruppen durch -C≡C-, -R⁵C=CR⁵-, Si(R⁵)₂, C=O, C=NR⁵, -NR⁵-, -O-, - S-, -C(=O)O- oder -C(=O)NR⁵- ersetzt sein können. Besonders bevorzugt ist R² bei jedem Auftreten gleich oder verschieden gewählt aus H, D, Si(R⁵)₃, geradkettigen Alkylgruppen mit 1 bis 20 C-Atomen, die deuteriert sein können, verzweigten oder cyclischen Alkylgruppen mit 3 bis 20 C-Atomen, die deuteriert sein können, aromatischen Ringsystemen mit 6 bis 40 aromatischen Ringatomen, die deuteriert sein können, und heteroaromatischen Ringsystemen mit 5 bis 40 aromatischen Ringatomen, die deuteriert sein können, wobei die genannten Alkylgruppen, die genannten aromatischen Ringsysteme und die genannten heteroaromatischen Ringsysteme jeweils mit Resten R⁵ substituiert sind, die bevorzugt gleich H sind. Ganz besonders bevorzugt ist R² gleich H.

Bevorzugt sind nur eine oder zwei Gruppen A in Formel (I) vorhanden, besonders bevorzugt ist genau eine Gruppe A in Formel (I) vorhanden.

Wenn zwei Gruppen A in Formel (I) vorhanden sind, so sind diese bevorzugt beide an dem heteroaromatischen Fünfring der Formel (I) gebunden.

Die verbrückende Gruppe Ar^{L} ist bevorzugt bei jedem Auftreten gleich oder verschieden gewählt aus aromatischen Ringsystemen mit 6 bis 20 aromatischen Ringatomen, die mit Resten R² substituiert sind, und heteroaromatischen Ringsystemen mit 5 bis 20 aromatischen Ringatomen, die Resten R² substituiert sind. Besonders bevorzugte Gruppen Ar^{L} sind bei jedem Auftreten gleich oder verschieden gewählt aus divalenten Gruppen abgeleitet von Benzol, Biphenyl, Terphenyl, Naphthalin, Fluoren, Indenofluoren, Indenocarbazol, Spirobifluoren, Dibenzofuran und Dibenzothiophen, die jeweils mit Resten R² substituiert sind. Ganz besonders bevorzugt ist Ar^{L} eine divalente Gruppe abgeleitet von Benzol, Biphenyl, Dibenzofuran oder Dibenzothiophen, das jeweils mit einem oder mehreren Resten R² substituiert ist, wobei die Reste R² in diesem Fall bevorzugt gleich H sind.

Bevorzugt ist n gleich 0.

Bevorzugte Gruppen -(Ar^{L})ₙ- für den Fall n=1 entsprechen den folgenden Formeln:

| | | |
|---|---|---|
| 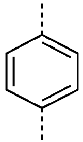 | 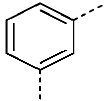 | 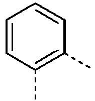 |
| Ar^{L}-1 | Ar^{L}-2 | Ar^{L}-3 |
| 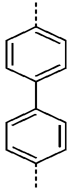 | 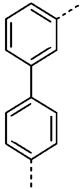 | 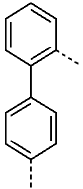 |
| Ar^{L}-4 | Ar^{L}-5 | Ar^{L}-6 |
| 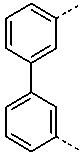 | 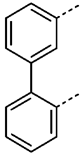 | 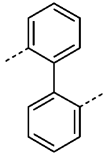 |
| Ar^{L}-7 | Ar^{L}-8 | Ar^{L}-9 |
| 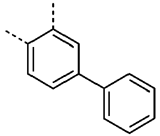 | 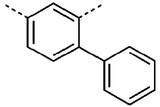 | 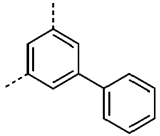 |
| Ar^{L}-10 | Ar^{L}-11 | Ar^{L}-12 |
| 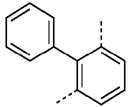 | 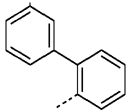 | 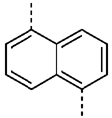 |
| Ar^{L}-13 | Ar^{L}-14 | Ar^{L}-15 |
| 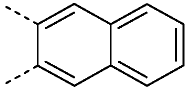 | 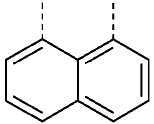 | 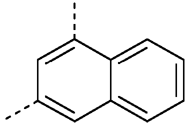 |
| Ar^{L-}-16 | Ar^{L}-17 | Ar^{L}-18 |
| 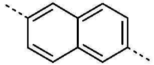 | 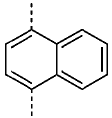 | 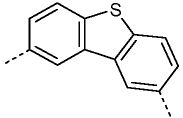 |
| Ar^{L}-19 | Ar^{L}-20 | Ar^{L}-21 |
| 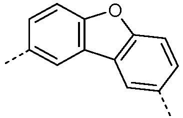 | 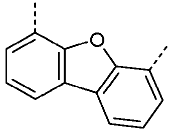 | 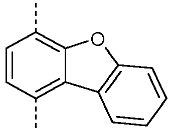 |
| Ar^{L}-22 | Ar^{L}-23 | Ar^{L}-24 |
| 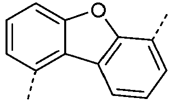 | 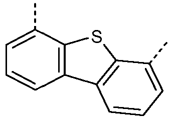 | 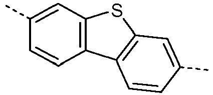 |
| Ar^{L}-25 | Ar^{L}-26 | Ar^{L}-27 |
| 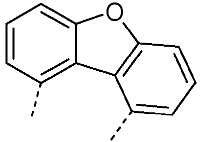 | 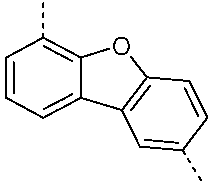 | 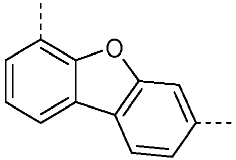 |
| Ar^{L}-28 | Ar^{L}-29 | Ar^{L}-30 |
| 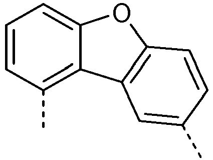 | 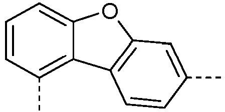 | 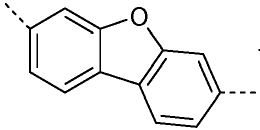 |
| Ar^{L}-31 | Ar^{L}-32 | Ar^{L}-33 |
| 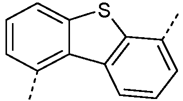 | 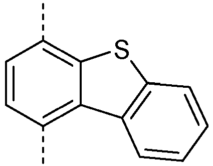 | 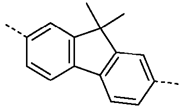 |
| Ar^{L}-34 | Ar^{L}-35 | Ar^{L}-36 |
| 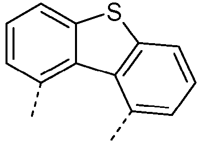 | 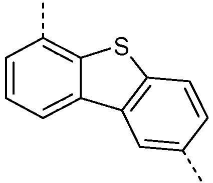 | 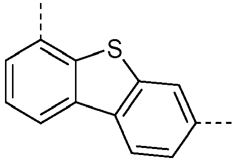 |
| Ar^{L}-37 | Ar^{L}-38 | Ar^{L}-39 |
| 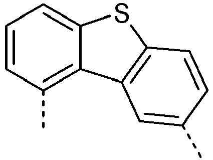 | 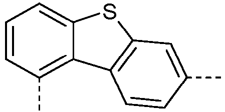 | 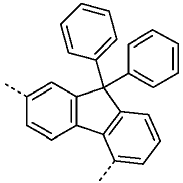 |
| Ar^{L}-40 | Ar^{L}-41 | Ar^{L}-42 |
| 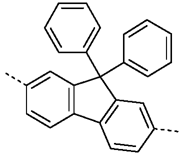 | 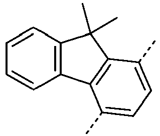 | 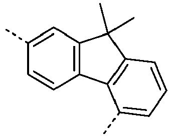 |
| Ar^{L}-43 | Ar^{L}-44 | Ar^{L}-45 |
| 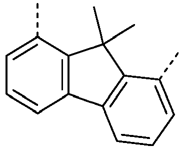 | 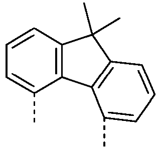 | 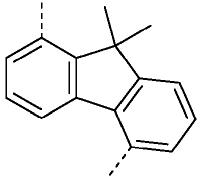 |
| Ar^{L}-46 | Ar^{L}-47 | Ar^{L}-48 |
| 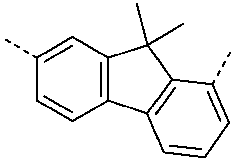 | 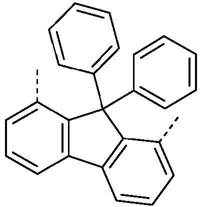 | 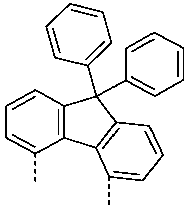 |
| Ar^{L}-49 | Ar^{L}-50 | Ar^{L}-51 |
| 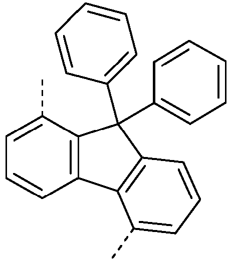 | 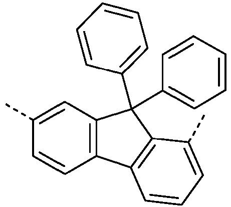 | 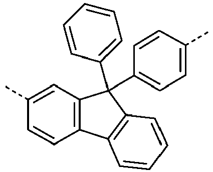 |
| Ar^{L}-52 | Ar^{L}-53 | Ar^{L}-54 |
| 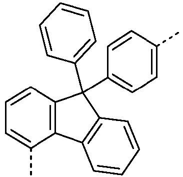 | 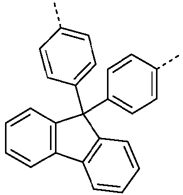 | 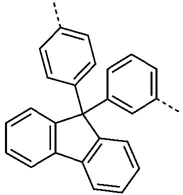 |
| Ar^{L}-55 | Ar^{L}-56 | Ar^{L}-57 |
| 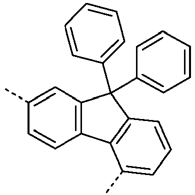 | 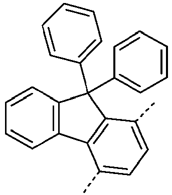 | 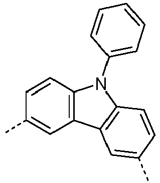 |
| Ar^{L}-58 | Ar^{L}-59 | Ar^{L}-60 |
| 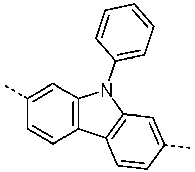 | 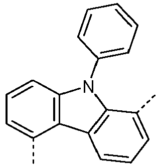 | 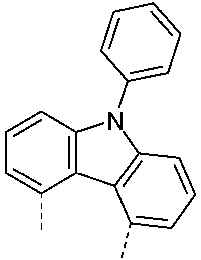 |
| Ar^{L}-61 | Ar^{L}-62 | Ar^{L}-63 |
| 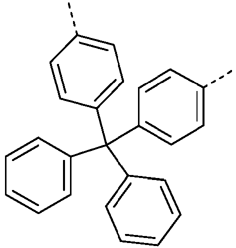 | 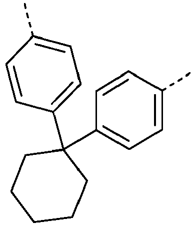 | 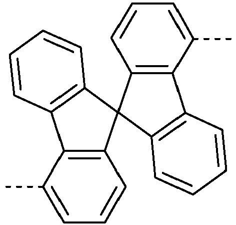 |
| Ar^{L}-64 | Ar^{L}-65 | Ar^{L}-72 |
| 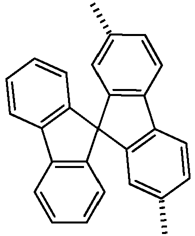 | 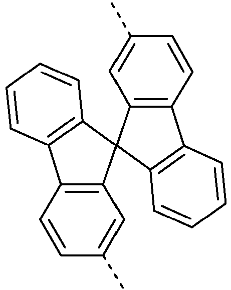 | 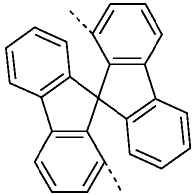 |
| Ar^{L}-73 | Ar^{L}-74 | Ar^{L}-75 |
| 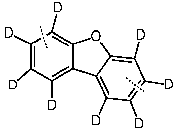 | 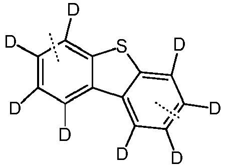 | 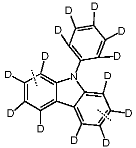 |
| Ar^{L}-76 | Ar^{L}-77 | Ar^{L}-78 |
| 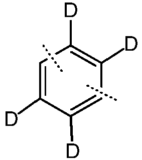 | 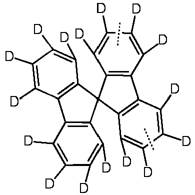 | 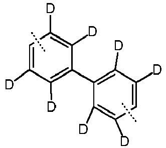 |
| Ar^{L}-79 | Ar^{L}-80 | Ar^{L}-81 |
| 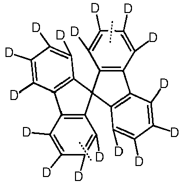 | | |
| Ar^{L}-82 | | |

wobei die gestrichelten Linien die Bindungen an den Rest der Formel (I) darstellen, und wobei die Gruppen an den unsubstituiert gezeichneten Positionen jeweils mit Resten R² substituiert sind, wobei die Reste R² in diesen Positionen bevorzugt H sind.

Unter den oben genannten Formeln sind die Formeln (Ar^{L}-1), (Ar^{L}-2), (Ar^{L}-7), (Ar^{L}-12), (Ar^{L}-20), (Ar^{L}-31) besonders bevorzugt.

Die elektronentransportierende Gruppe ET ist bevorzugt bei jedem Auftreten gleich oder verschieden gewählt aus Heteroarylgruppen mit 5 bis 40 aromatischen Ringatomen, von denen mindestens eines, bevorzugt mindestens zwei und ganz bevorzugt mindestens drei Heteroatome sind, die bevorzugt ausgewählt sind aus N, O und/oder S, und die ganz besonders bevorzugt gleich N sind, wobei die Heteroarylgruppen jeweils mit Gruppen Ar² substituiert sind.

Es ist bevorzugt, dass jede Gruppe ET durch mindestens eine Gruppe Ar², die ungleich H ist, mehr bevorzugt mindestens zwei Gruppen Ar², die ungleich H sind, substituiert ist.

Weiter bevorzugt ist die Gruppe ET gleich oder verschieden bei jedem Auftreten gewählt ist aus den Gruppen der Formeln (ET-1) bis (ET-11) und (ET-11a) bis (ET-11c): 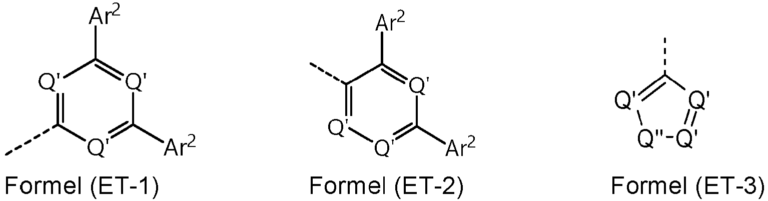 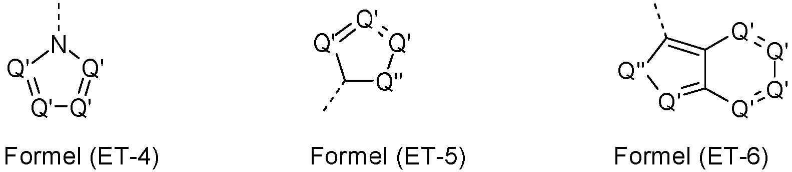 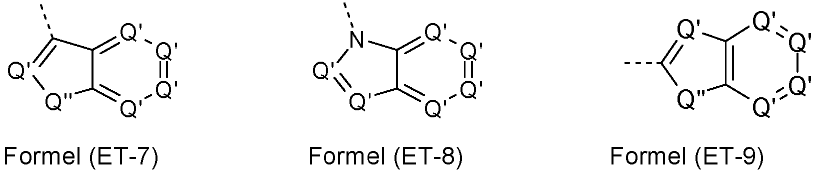 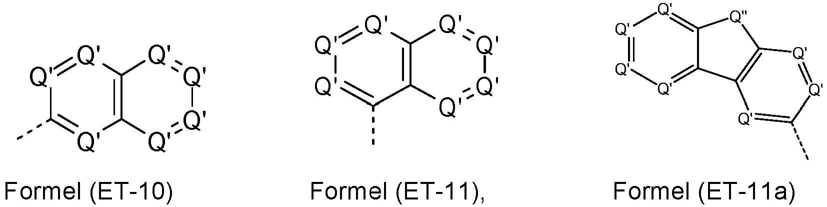 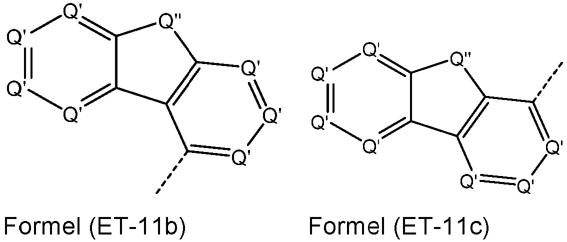 wobei
Q' bei jedem Auftreten gleich oder verschieden gewählt ist aus CAr² oder N;
Q" bei jedem Auftreten gleich oder verschieden gewählt ist aus NR², O oder S;
die gestrichelte Line jeweils die Anbindungsposition an den Rest der Formel (I) markiert und R² und Ar² wie vorstehend definiert sind; und
wobei wenigstens ein Q` gleich N ist.

Besonders bevorzugte Gruppen ET sind bei jedem Auftreten gleich oder verschieden gewählt aus Gruppen abgeleitet von Pyridin, Pyrazin, Pyrimidin, Pyridazin, 1,2,4-Triazin, 1,3,5-Triazin, Chinolin, Isochinolin, Chinoxalin, Chinazolin, Pyrazol, Imidazol, Benzimidazol, Thiazol, Benzothiazol, Oxazol, Oxadiazol und Benzooxazol, die jeweils mit Gruppen Ar² substituiert sind. Ganz besonders bevorzugt ist die Gruppe ET ein mit einem oder mehreren Gruppen Ar² substituiertes Pyridin, Pyrazin, Pyrimidin, Pyridazin, 1,3,5-Triazin, Benzimidazol und Chinazolin.

Ganz besonders bevorzugte heteroaromatische elektronentransportierende Gruppen ET sind ausgewählt aus den Formeln (ET-12) bis (ET-35) 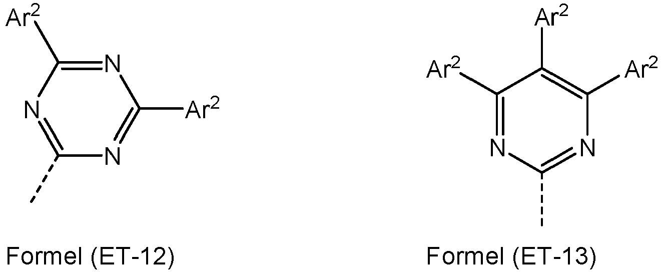 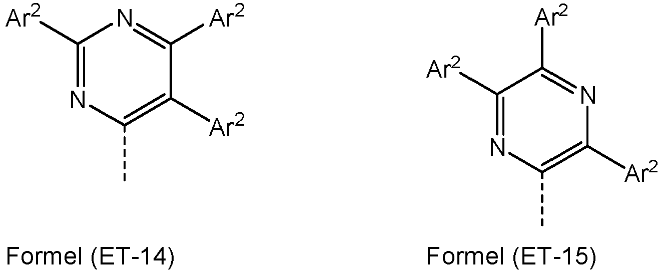 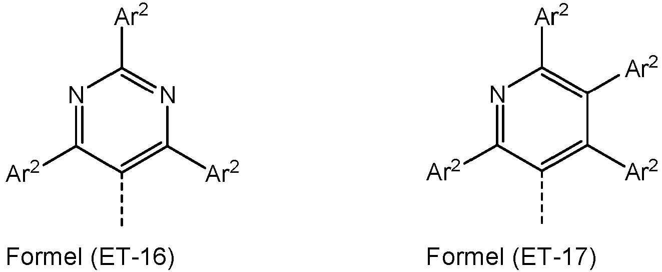 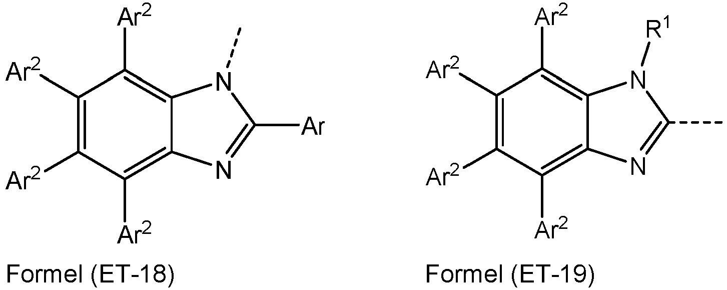 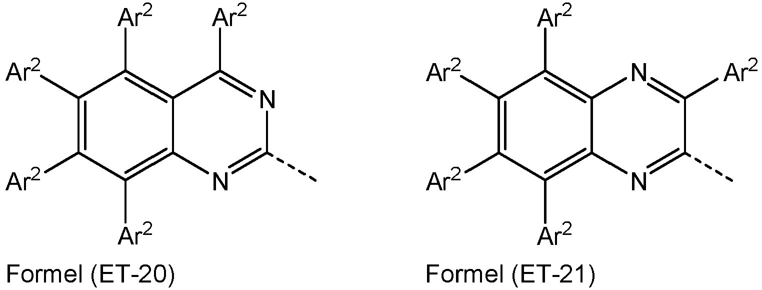 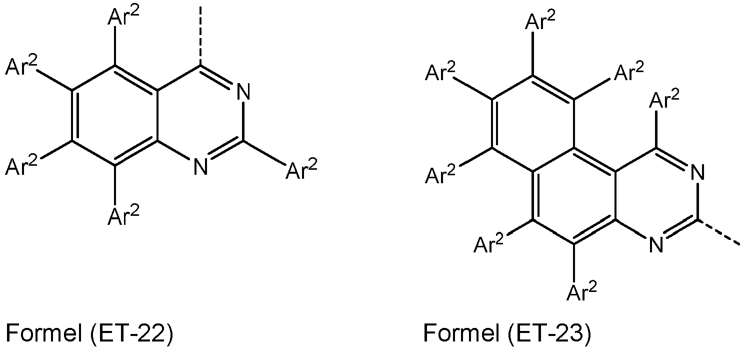 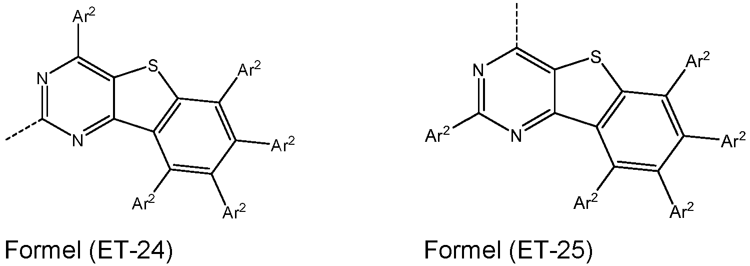 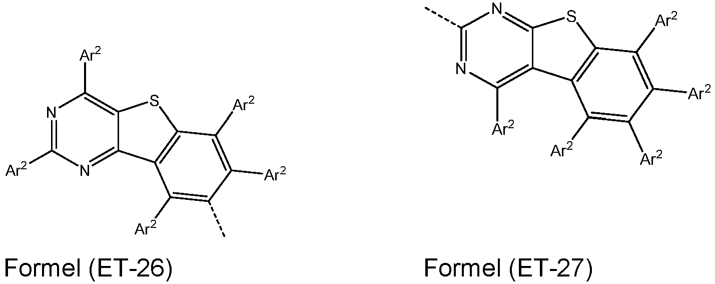 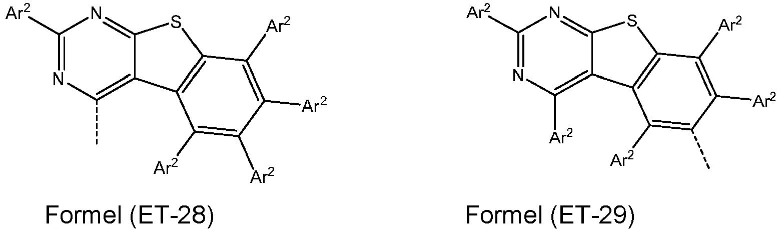 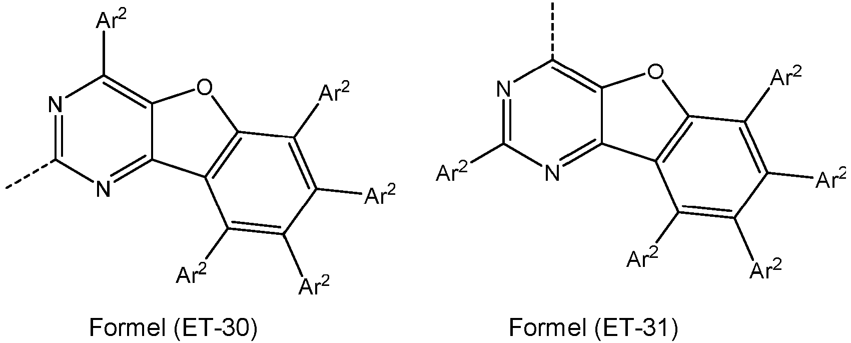 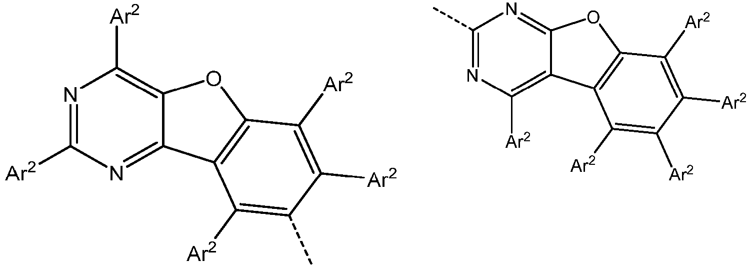 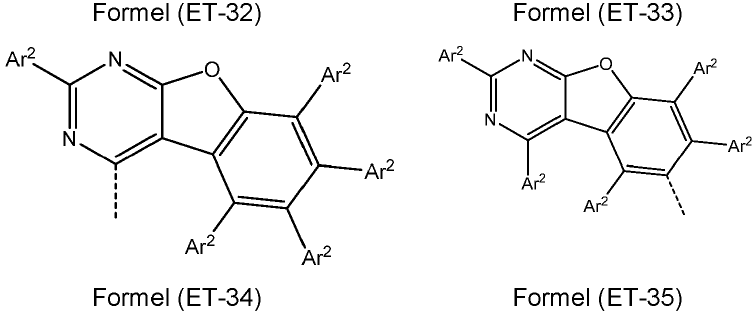 wobei die gestrichelten Linien die Bindungen an den Rest der Formel (I) darstellen, und wobei Gruppen der Formeln (ET-12), (ET-13), (ET-14), (ET-20), (ET-21) und (ET-22) ganz besonders bevorzugt sind, und Gruppen der Formel (ET-12) am meisten bevorzugt sind.

Bevorzugt ist Ar² bei jedem Auftreten gleich oder verschieden gewählt aus H, geradkettigen Alkyl- oder Alkoxygruppen mit 1 bis 20 C-Atomen, verzweigten oder cyclischen Alkyl- oder Alkoxygruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen, wobei die Alkylgruppen, Alkoxygruppen, aromatischen Ringsysteme und heteroaromatischen Ringsysteme jeweils mit Resten R³ substituiert sind, und wobei zwei oder mehr benachbarte Gruppen Ar² miteinander ein mono- oder polycyclisches, aliphatisches oder aromatisches Ringsystem bilden können.

Ar² ist besonders bevorzugt bei jedem Auftreten gleich oder verschieden gewählt aus H und aromatischen Ringsystemen mit 6 bis 40 aromatischen Ringatomen, die mit Resten R³ substituiert sind. Weiterhin besonders bevorzugt ist Ar² bei jedem Auftreten gleich oder verschieden gewählt aus Phenyl, Biphenyl, Terphenyl, Quaterphenyl, Naphthyl, Fluorenyl, insbesondere 9,9'-Dimethylfluorenyl und 9,9'-Diphenylfluorenyl, Benzofluorenyl, Spirobifluorenyl, Indenofluorenyl, Indenocarbazolyl, Dibenzofuranyl, Dibenzothiophenyl, Carbazolyl, Benzofuranyl, Benzothiophenyl, benzokondensiertem Dibenzofuranyl, benzokondensiertem Dibenzothiophenyl, Naphthyl-substituiertem Phenyl, Fluorenyl-substituiertem Phenyl, Spirobifluorenyl-substituiertem Phenyl, Dibenzofuranyl-substituiertem Phenyl, Dibenzothiophenyl-substituiertem Phenyl, Carbazolyl-substituiertem Phenyl, Pyridyl-substituiertem Phenyl, Pyrimidyl-substituiertem Phenyl, Triazinyl-substituiertem Phenyl, wobei die genannten Gruppen jeweils mit Resten R³ substituiert sind, und H. Ganz besonders bevorzugte Gruppen Ar² sind bei jedem Auftreten gleich oder verschieden gewählt aus Phenyl, Biphenyl, Terphenyl, Quaterphenyl, Naphthyl, Fluorenyl, insbesondere 9,9'-Dimethylfluorenyl und 9,9'-Diphenylfluorenyl, Benzofluorenyl, Spirobifluorenyl, Indenofluorenyl, Indenocarbazolyl, Dibenzofuranyl, Dibenzothiophenyl, Carbazolyl, Benzofuranyl, Benzothiophenyl, benzokondensiertem Dibenzofuranyl, benzokondensiertem Dibenzothiophenyl, Naphthyl-substituiertem Phenyl, Fluorenyl-substituiertem Phenyl, Spirobifluorenyl-substituiertem Phenyl, Dibenzofuranyl-substituiertem Phenyl, Dibenzothiophenyl-substituiertem Phenyl, Carbazolyl-substituiertem Phenyl, Pyridyl-substituiertem Phenyl, Pyrimidyl-substituiertem Phenyl, Triazinyl-substituiertem Phenyl, wobei die genannten Gruppen jeweils mit Resten R³ substituiert sind, und H.

Ganz besonders bevorzugte Gruppen Ar² sind gleich oder verschieden gewählt aus H und den folgenden Formeln:

| | | |
|---|---|---|
| 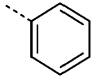 | 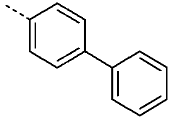 | 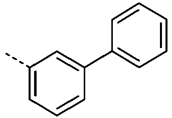 |
| Ar-1 | Ar-2 | Ar-3 |
| 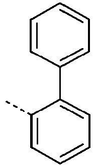 | 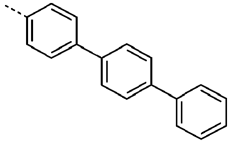 | 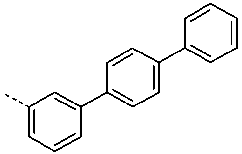 |
| Ar-4 | Ar-5 | Ar-6 |
| 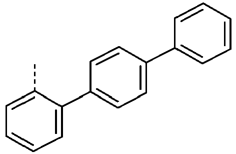 | 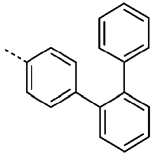 | 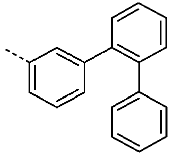 |
| Ar-7 | Ar-8 | Ar-9 |
| 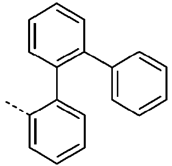 | 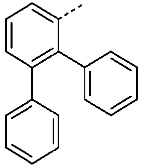 | 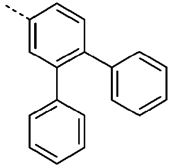 |
| Ar-10 | Ar-11 | Ar-12 |
| 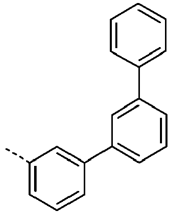 | 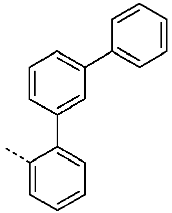 | 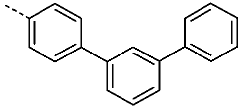 |
| Ar-13 | Ar-14 | Ar-15 |
| 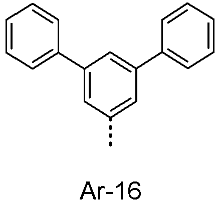 | 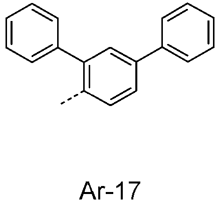 | 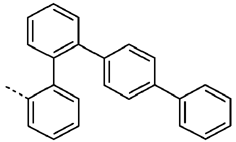 |
| | | Ar-18 |
| 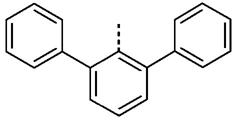 | | |
| Ar-19 | | |
| 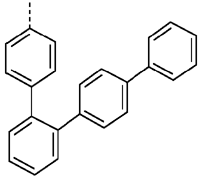 | 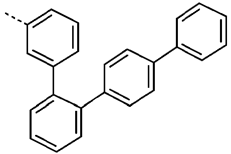 | 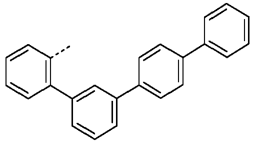 |
| Ar-20 | Ar-21 | Ar-22 |
| 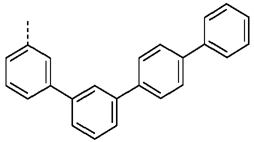 | 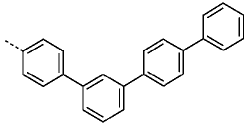 | 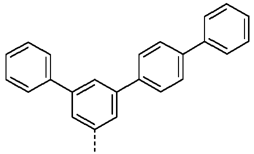 |
| Ar-23 | Ar-24 | Ar-25 |
| 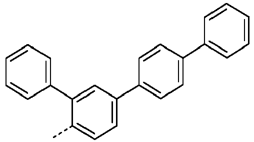 | 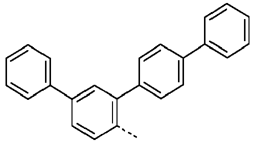 | 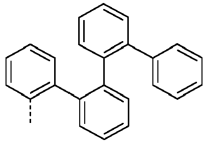 |
| Ar-26 | Ar-27 | Ar-28 |
| 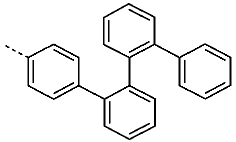 | 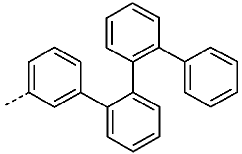 | 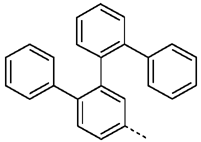 |
| Ar-29 | Ar-30 | Ar-31 |
| 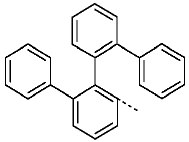 | 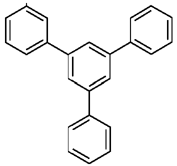 | 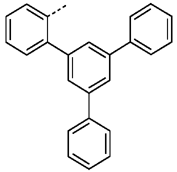 |
| Ar-32 | Ar-33 | Ar-34 |
| 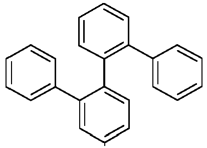 | 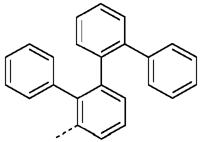 | 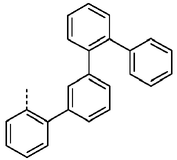 |
| Ar-35 | Ar-36 | Ar-37 |
| 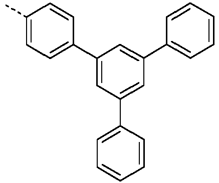 | 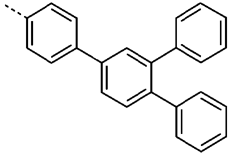 | 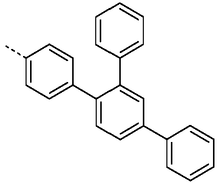 |
| Ar-38 | Ar-39 | Ar-40 |
| 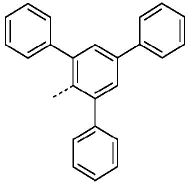 | 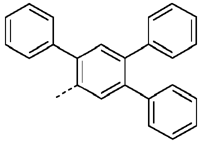 | 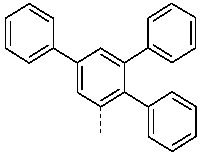 |
| Ar-41 | Ar-42 | Ar-43 |
| 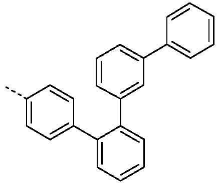 | 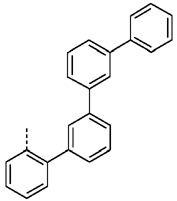 | 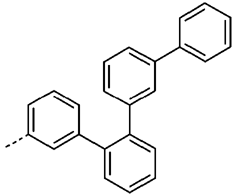 |
| Ar-44 | Ar-45 | Ar-46 |
| 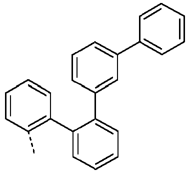 | | |
| Ar-47 | | |
| 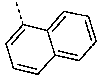 | 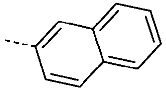 | 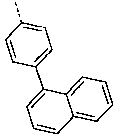 |
| Ar-48 | Ar-49 | Ar-50 |
| 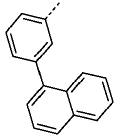 | 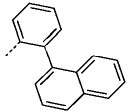 | 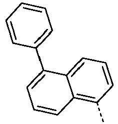 |
| Ar-51 | Ar-52 | Ar-53 |
| 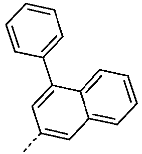 | 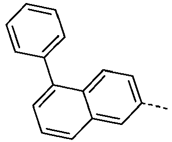 | 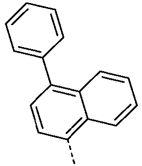 |
| Ar-54 | Ar-55 | Ar-56 |
| 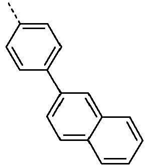 | 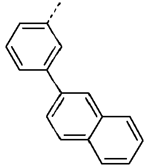 | 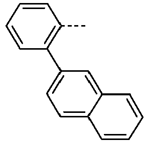 |
| Ar-57 | Ar-58 | Ar-59 |
| 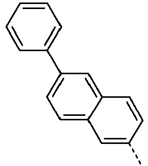 | 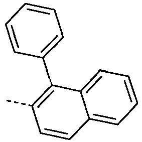 | 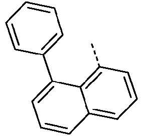 |
| Ar-60 | Ar-61 | Ar-62 |
| 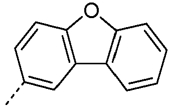 | 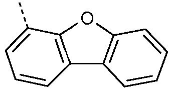 | 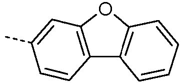 |
| Ar-63 | Ar-64 | Ar-65 |
| 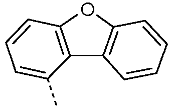 | 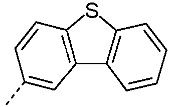 | 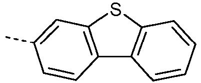 |
| Ar-66 | Ar-67 | Ar-68 |
| 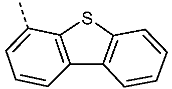 | 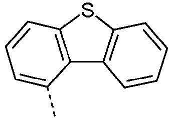 | 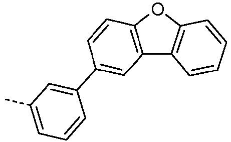 |
| Ar-69 | Ar-70 | Ar-71 |
| 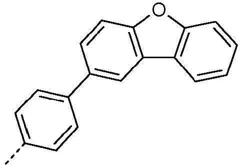 | 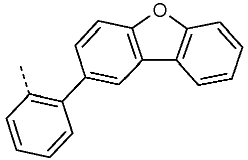 | 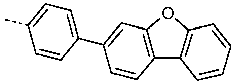 |
| Ar-72 | Ar-73 | Ar-74 |
| 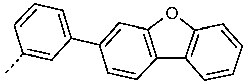 | 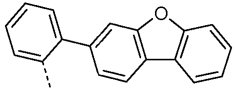 | 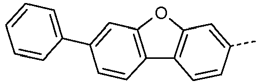 |
| Ar-75 | Ar-76 | Ar-77 |
| 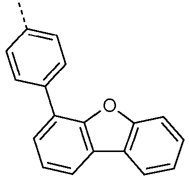 | 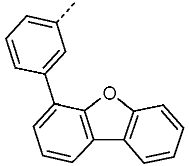 | 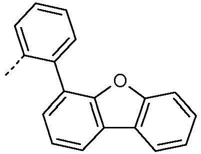 |
| Ar-78 | Ar-79 | Ar-80 |
| 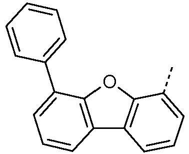 | 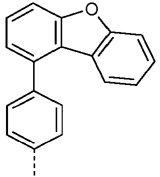 | 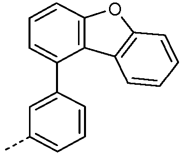 |
| Ar-81 | Ar-82 | Ar-83 |
| 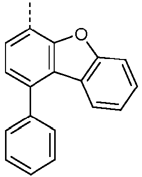 | 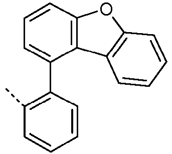 | 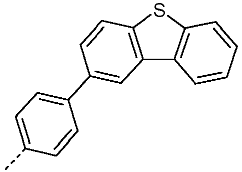 |
| Ar-84 | Ar-85 | Ar-86 |
| 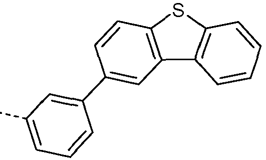 | 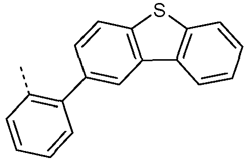 | 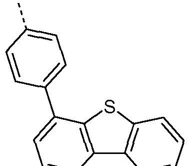 |
| Ar-87 | Ar-88 | Ar-89 |
| 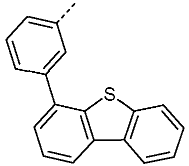 | 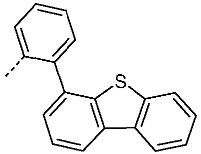 | 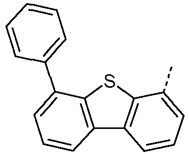 |
| Ar-90 | Ar-91 | Ar-92 |
| 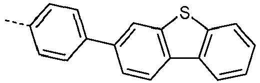 | 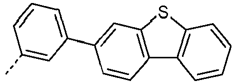 | 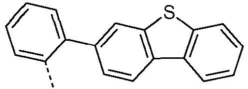 |
| Ar-93 | Ar-94 | Ar-95 |
| 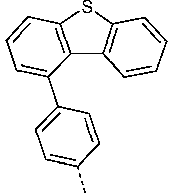 | 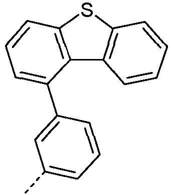 | 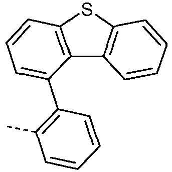 |
| Ar-96 | Ar-97 | Ar-98 |
| 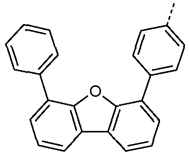 | 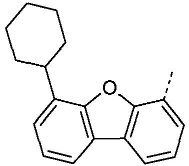 | 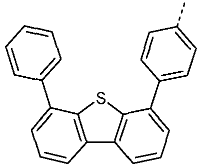 |
| Ar-99 | Ar-100 | Ar-101 |
| 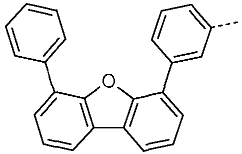 | 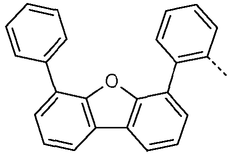 | 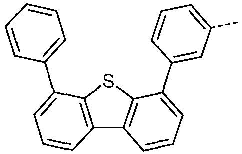 |
| Ar-102 | Ar-103 | Ar-104 |
| 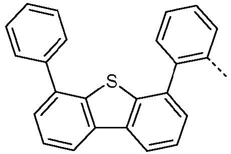 | | |
| Ar-105 | | |
| 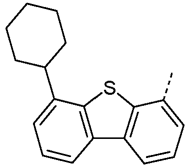 | 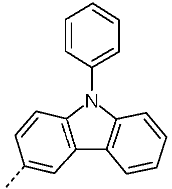 | 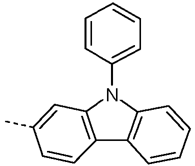 |
| Ar-106 | Ar-107 | Ar-108 |
| 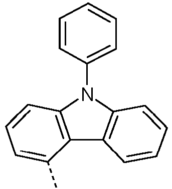 | 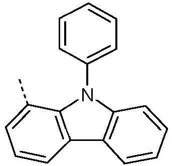 | 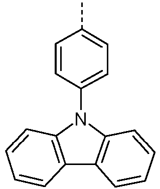 |
| Ar-109 | Ar-110 | Ar-111 |
| 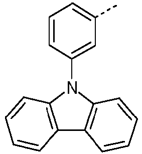 | 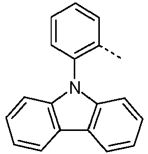 | |
| Ar-112 | Ar-113 | |
| 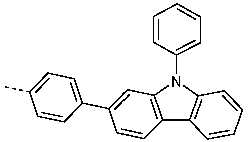 | 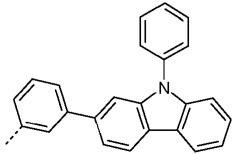 | 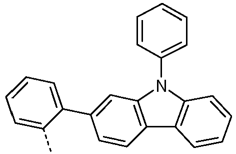 |
| Ar-114 | Ar-115 | Ar-116 |
| 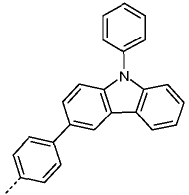 | 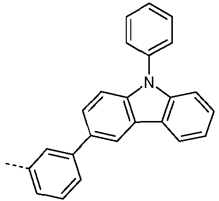 | 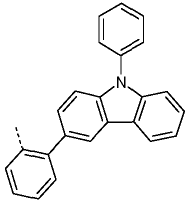 |
| Ar-117 | Ar-118 | Ar-119 |
| 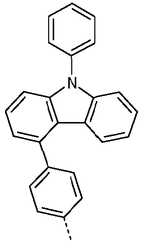 | 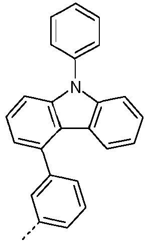 | 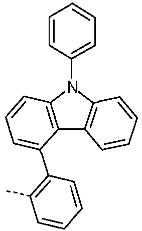 |
| Ar-120 | Ar-121 | Ar-122 |
| 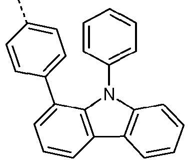 | 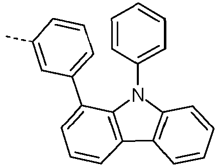 | 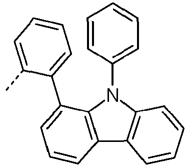 |
| Ar-123 | Ar-124 | Ar-125 |
| 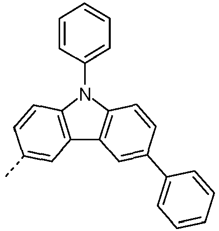 | 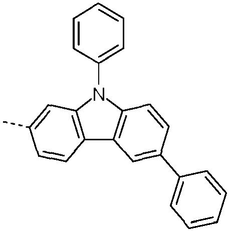 | 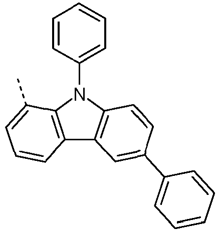 |
| Ar-126 | Ar-127 | Ar-128 |
| 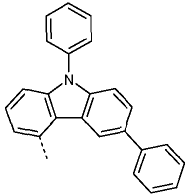 | 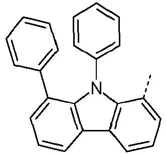 | 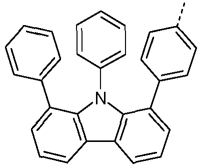 |
| Ar-129 | Ar-130 | Ar-131 |
| 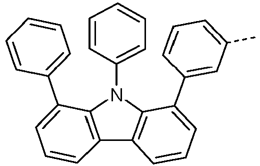 | 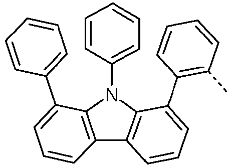 | |
| Ar-132 | Ar-133 | |
| 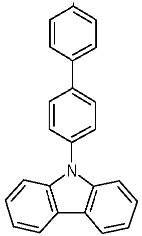 | 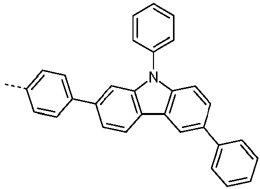 | 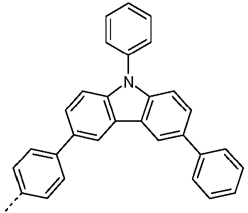 |
| Ar-134 | Ar-135 | Ar-136 |
| 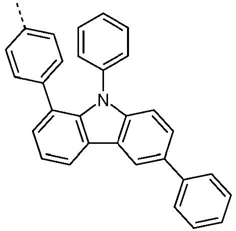 | 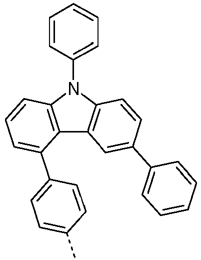 | 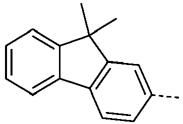 |
| Ar-137 | Ar-138 | Ar-139 |
| 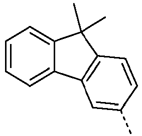 | 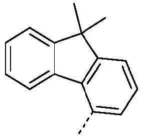 | 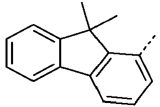 |
| Ar-140 | Ar-141 | Ar-142 |
| 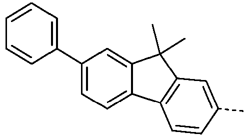 | 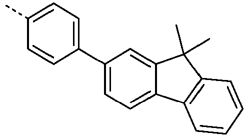 | 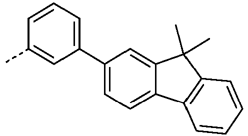 |
| Ar-143 | Ar-144 | Ar-145 |
| 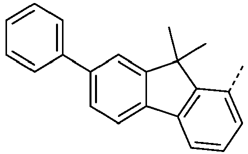 | 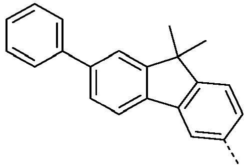 | 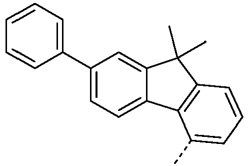 |
| Ar-146 | Ar-147 | Ar-148 |
| 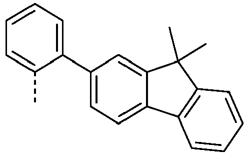 | 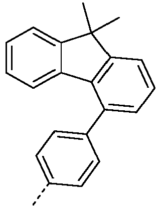 | 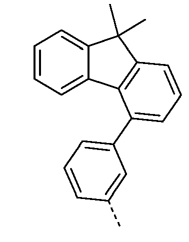 |
| Ar-149 | Ar-150 | Ar-151 |
| 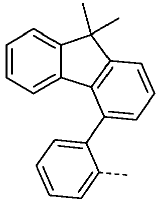 | 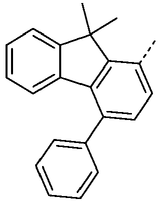 | 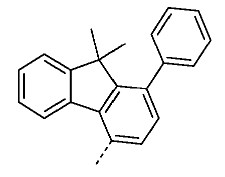 |
| Ar-152 | Ar-153 | Ar-154 |
| 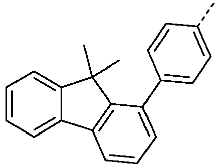 | 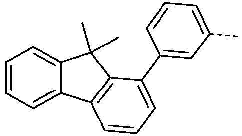 | 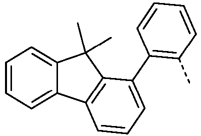 |
| Ar-155 | Ar-156 | Ar-157 |
| 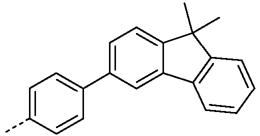 | 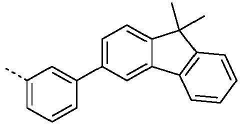 | 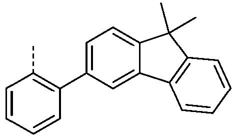 |
| Ar-158 | Ar-159 | Ar-160 |
| 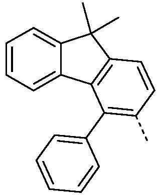 | 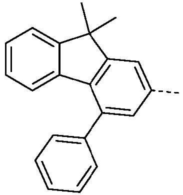 | 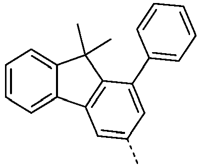 |
| Ar-161 | Ar-162 | Ar-163 |
| 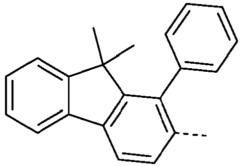 | 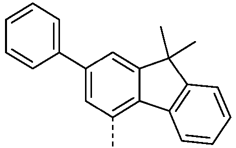 | 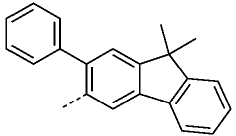 |
| Ar-164 | Ar-165 | Ar-166 |
| 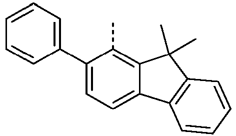 | 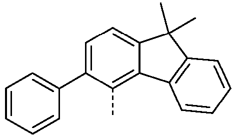 | 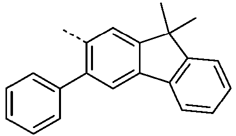 |
| Ar-167 | Ar-168 | Ar-169 |
| 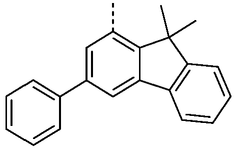 | 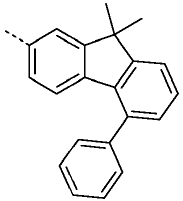 | 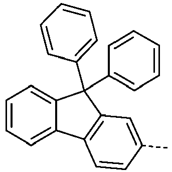 |
| Ar-170 | Ar-171 | Ar-172 |
| 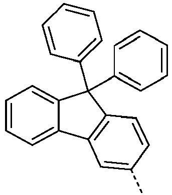 | 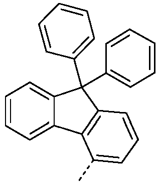 | 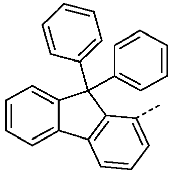 |
| Ar-173 | Ar-174 | Ar-175 |
| 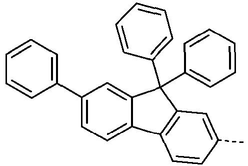 | 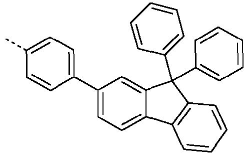 | 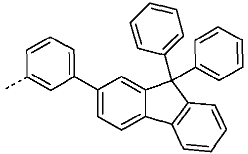 |
| Ar-176 | Ar-177 | Ar-178 |
| 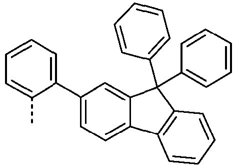 | 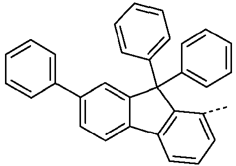 | 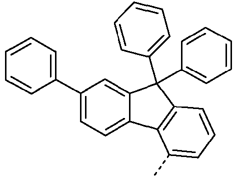 |
| Ar-179 | Ar-180 | Ar-181 |
| 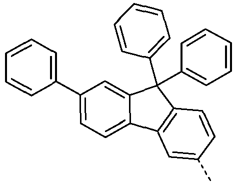 | 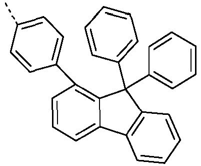 | 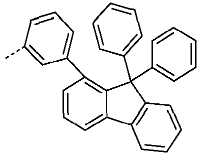 |
| Ar-182 | Ar-183 | Ar-184 |
| 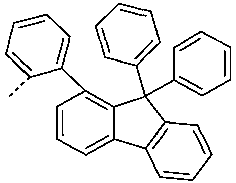 | 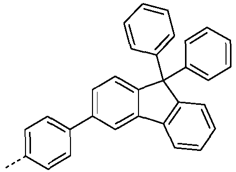 | 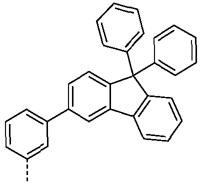 |
| Ar-185 | Ar-186 | Ar-187 |
| 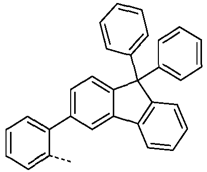 | 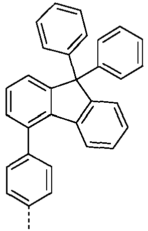 | 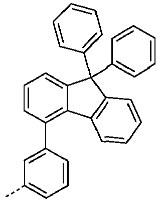 |
| Ar-188 | Ar-189 | Ar-190 |
| 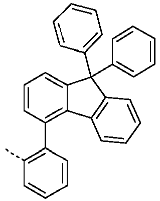 | | |
| Ar-191 | | |
| 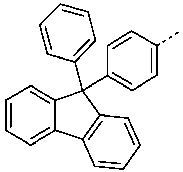 | 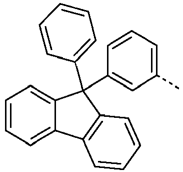 | 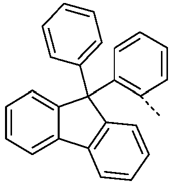 |
| Ar-192 | Ar-193 | Ar-194 |
| 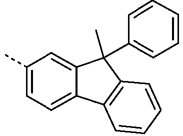 | 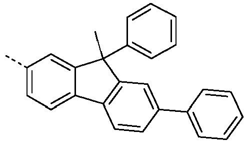 | 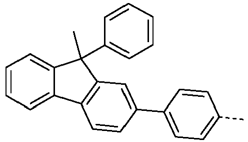 |
| Ar-195 | Ar-196 | Ar-197 |
| 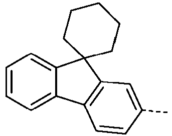 | 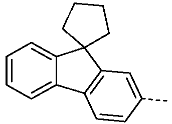 | 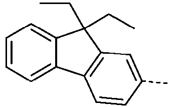 |
| Ar-198 | Ar-199 | Ar-200 |
| 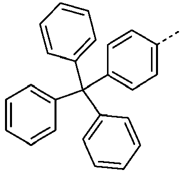 | 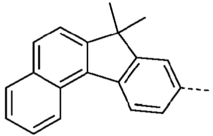 | 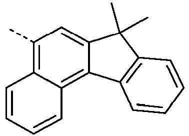 |
| Ar-201 | Ar-202 | Ar-203 |
| 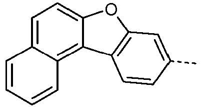 | 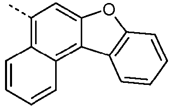 | 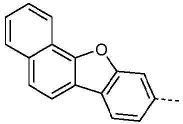 |
| Ar-204 | Ar-205 | Ar-206 |
| 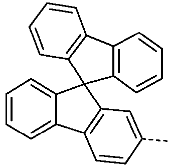 | 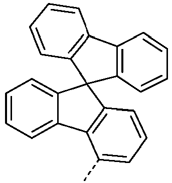 | 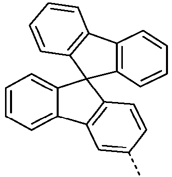 |
| Ar-207 | Ar-208 | Ar-209 |
| 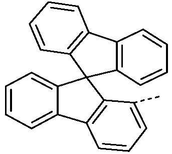 | 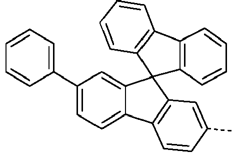 | 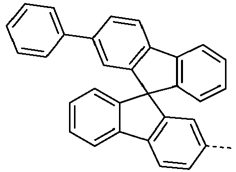 |
| Ar-210 | Ar-211 | Ar-212 |
| 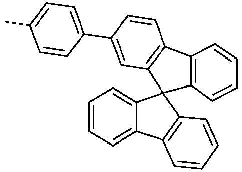 | 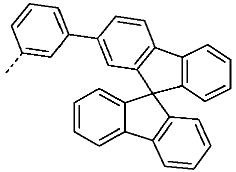 | 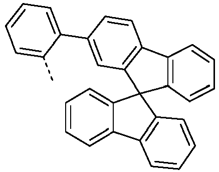 |
| Ar-213 | Ar-214 | Ar-215 |
| 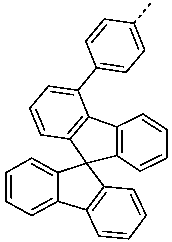 | 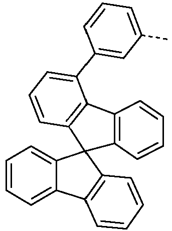 | 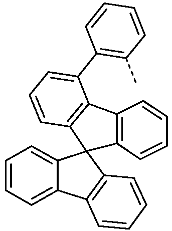 |
| Ar-216 | Ar-217 | Ar-218 |
| 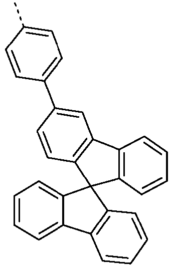 | 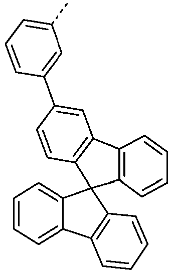 | 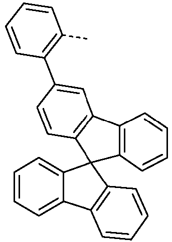 |
| Ar-219 | Ar-220 | Ar-221 |
| 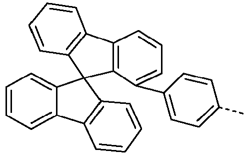 | 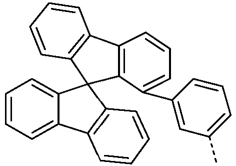 | 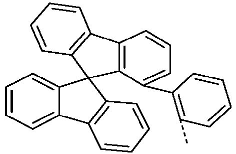 |
| Ar-222 | Ar-223 | Ar-224 |
| 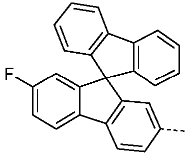 | 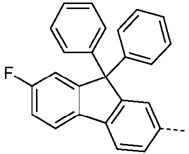 | 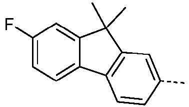 |
| Ar-225 | Ar-226 | Ar-227 |
| 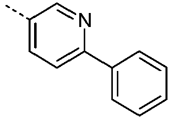 | 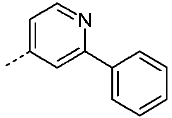 | 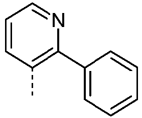 |
| Ar-228 | Ar-229 | Ar-230 |
| 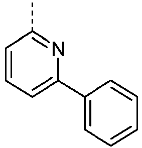 | 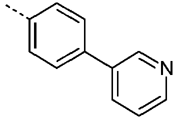 | 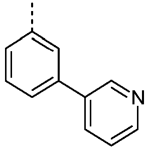 |
| Ar-231 | Ar-232 | Ar-233 |
| 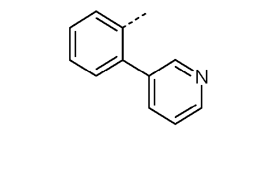 | 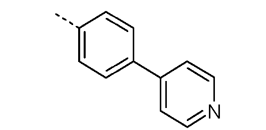 | 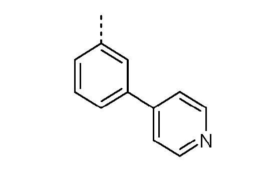 |
| Ar-234 | Ar-235 | Ar-236 |
| 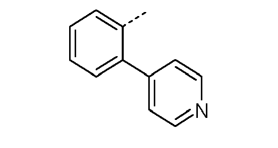 | 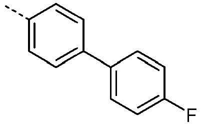 | 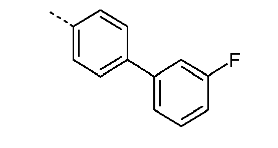 |
| Ar-237 | Ar-238 | Ar-239 |
| 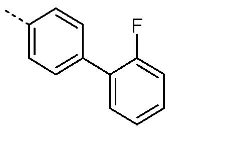 | 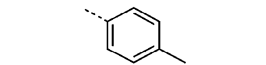 | 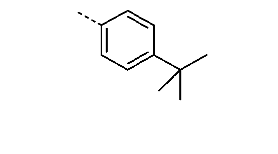 |
| Ar-240 | Ar-241 | Ar-242 |
| 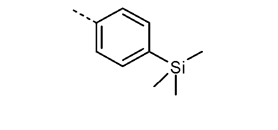 | 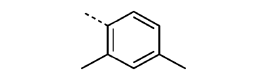 | 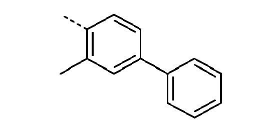 |
| Ar-243 | Ar-244 | Ar-245 |
| 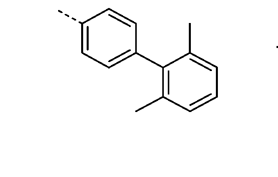 | 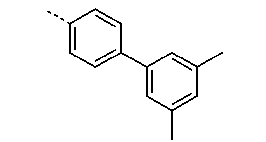 | 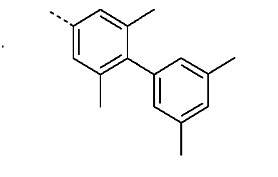 |
| Ar-246 | Ar-247 | Ar-248 |
| 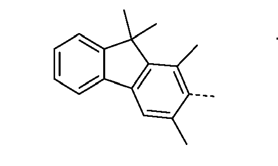 | 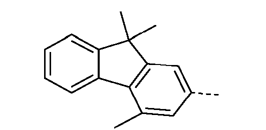 | 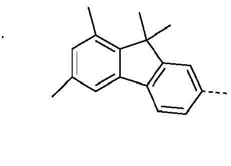 |
| Ar-250 | Ar-251 | Ar-252 |
| 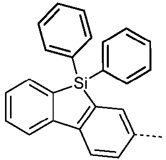 | 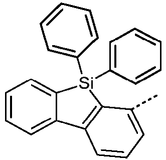 | 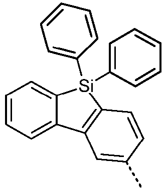 |
| Ar-253 | Ar-254 | Ar-255 |
| 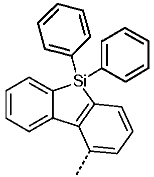 | 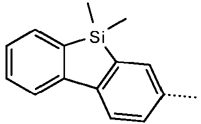 | 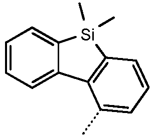 |
| Ar-256 | Ar-257 | Ar-258 |
| 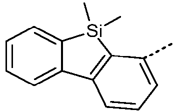 | 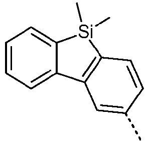 | 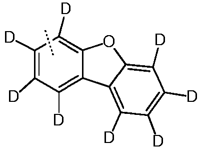 |
| Ar-259 | Ar-260 | Ar-261 |
| 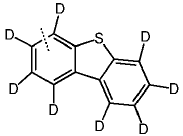 | 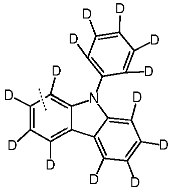 | 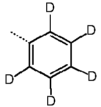 |
| Ar-262 | Ar-263 | Ar-264 |
| 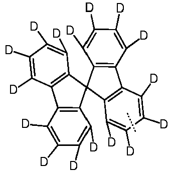 | 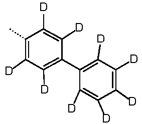 | 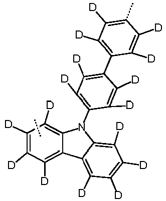 |
| Ar-265 | Ar-266 | Ar-267 |
| 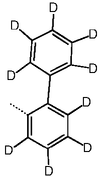 | 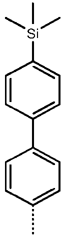 | 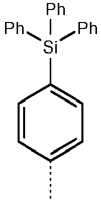 |
| Ar-268 | Ar-269 | Ar-270 |
| 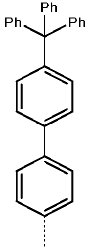 | 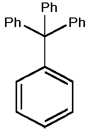 | 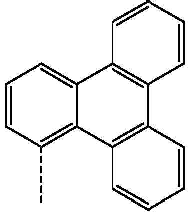 |
| Ar-271 | Ar-272 | Ar-273 |
| 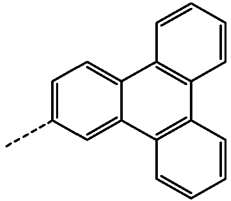 | | |
| Ar-274 | | |

wobei die Gruppen an den unsubstituiert dargestellten Positionen mit Resten R³ substituiert sind, wobei R³ in diesen Positionen bevorzugt H ist, und wobei die gestrichelte Bindung die Bindung an den Rest der Gruppe A darstellt.

Aus den oben genannten Gruppen sind die der Formeln (Ar-1), (Ar-3), (ar-16), (Ar-49), (Ar-63), (Ar-65), (Ar-66), (Ar-69), (Ar-78), (Ar-139) und (Ar-274) am meisten bevorzugt, und insbesondere Formel (Ar-1).

Gemäß einer bevorzugten Ausführungsform sind Gruppen Ar² in Formel (A) gleich gewählt. Gemäß einer anderen bevorzugten Ausführungsform sind die Gruppen Ar² verschieden gewählt.

Beispiele ganz besonders bevorzugter Gruppen ET sind die folgenden Gruppen, wobei die gestrichelten Linien die Bindungen an den Rest der Formel (I) darstellen:
Gruppen ET-23a bis ET-23s mit der allgemeinen Formel 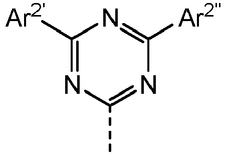 wobei gilt:

| **Gruppe ET** | **Ar^{2'}** | **Ar^{2"}** |
|---|---|---|
| Formel (ET-23a) | Formel (Ar-1) | Formel (Ar-1) |
| Formel (ET-23b) | Formel (Ar-16) | Formel (Ar-16) |
| Formel (ET-23c) | Formel (Ar-1) | Formel (Ar-16) |
| Formel (ET-23d) | Formel (Ar-63) | Formel (Ar-65) |
| Formel (ET-23e) | Formel (Ar-63) | Formel (Ar-63) |
| Formel (ET-23f) | Formel (Ar-1) | Formel (Ar-63) |
| Formel (ET-23g) | Formel (Ar-1) | Formel (Ar-65) |
| Formel (ET-23h) | Formel (Ar-65) | Formel (Ar-65) |
| Formel (ET-23i) | Formel (Ar-1) | Formel (Ar-66) |
| Formel (ET-23j) | Formel (Ar-1) | Formel (Ar-274) |
| Formel (ET-23k) | Formel (Ar-1) | Formel (Ar-69) |
| Formel (ET-23l) | Formel (Ar-69) | Formel (Ar-69) |
| Formel (ET-23m) | Formel (Ar-1) | Formel (Ar-49) |
| Formel (ET-23n) | Formel (Ar-49) | Formel (Ar-49) |
| Formel (ET-23o) | Formel (Ar-1) | Formel (Ar-3) |
| Formel (ET-23p) | Formel (Ar-3) | Formel (Ar-3) |
| Formel (ET-23q) | Formel (Ar-66) | Formel (Ar-66) |
| Formel (ET-23r) | Formel (Ar-1) | Formel (Ar-139) |
| Formel (ET-23s) | Formel (Ar-139) | Formel (Ar-139) |

Gruppen ET-24a bis ET-24s mit der allgemeinen Formel 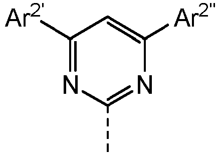 wobei gilt:

| **Gruppe ET** | **Ar^{2'}** | **Ar^{2"}** |
|---|---|---|
| Formel (ET-24a) | Formel (Ar-1) | Formel (Ar-1) |
| Formel (ET-24b) | Formel (Ar-16) | Formel (Ar-16) |
| Formel (ET-24c) | Formel (Ar-1) | Formel (Ar-16) |
| Formel (ET-24d) | Formel (Ar-63) | Formel (Ar-65) |
| Formel (ET-24e) | Formel (Ar-63) | Formel (Ar-63) |
| Formel (ET-24f) | Formel (Ar-1) | Formel (Ar-63) |
| Formel (ET-24g) | Formel (Ar-1) | Formel (Ar-65) |
| Formel (ET-24h) | Formel (Ar-65) | Formel (Ar-65) |
| Formel (ET-24i) | Formel (Ar-1) | Formel (Ar-66) |
| Formel (ET-24j) | Formel (Ar-1) | Formel (Ar-274) |
| Formel (ET-24k) | Formel (Ar-1) | Formel (Ar-69) |
| Formel (ET-24l) | Formel (Ar-69) | Formel (Ar-69) |
| Formel (ET-24m) | Formel (Ar-1) | Formel (Ar-49) |
| Formel (ET-24n) | Formel (Ar-49) | Formel (Ar-49) |
| Formel (ET-24o) | Formel (Ar-1) | Formel (Ar-3) |
| Formel (ET-24p) | Formel (Ar-3) | Formel (Ar-3) |
| Formel (ET-24q) | Formel (Ar-66) | Formel (Ar-66) |
| Formel (ET-24r) | Formel (Ar-1) | Formel (Ar-139) |
| Formel (ET-24s) | Formel (Ar-139) | Formel (Ar-139) |

Gruppen ET-25a bis ET-25s mit der allgemeinen Formel 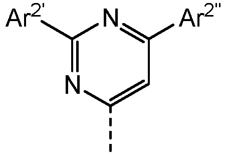 wobei gilt:

| **Gruppe ET** | **Ar^{2'}** | **Ar^{2"}** |
|---|---|---|
| Formel (ET-25a) | Formel (Ar-1) | Formel (Ar-1) |
| Formel (ET-25b) | Formel (Ar-16) | Formel (Ar-16) |
| Formel (ET-25c) | Formel (Ar-1) | Formel (Ar-16) |
| Formel (ET-25d) | Formel (Ar-63) | Formel (Ar-65) |
| Formel (ET-25e) | Formel (Ar-63) | Formel (Ar-63) |
| Formel (ET-25f) | Formel (Ar-1) | Formel (Ar-63) |
| Formel (ET-25g) | Formel (Ar-1) | Formel (Ar-65) |
| Formel (ET-25h) | Formel (Ar-65) | Formel (Ar-65) |
| Formel (ET-25i) | Formel (Ar-1) | Formel (Ar-66) |
| Formel (ET-25j) | Formel (Ar-1) | Formel (Ar-274) |
| Formel (ET-25k) | Formel (Ar-1) | Formel (Ar-69) |
| Formel (ET-25l) | Formel (Ar-69) | Formel (Ar-69) |
| Formel (ET-25m) | Formel (Ar-1) | Formel (Ar-49) |
| Formel (ET-25n) | Formel (Ar-49) | Formel (Ar-49) |
| Formel (ET-25o) | Formel (Ar-1) | Formel (Ar-3) |
| Formel (ET-25p) | Formel (Ar-3) | Formel (Ar-3) |
| Formel (ET-25q) | Formel (Ar-66) | Formel (Ar-66) |
| Formel (ET-25r) | Formel (Ar-1) | Formel (Ar-139) |
| Formel (ET-25s) | Formel (Ar-139) | Formel (Ar-139) |

Gruppen ET-26a bis ET-26k mit der allgemeinen Formel 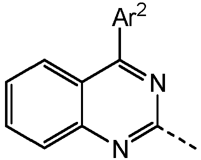 wobei gilt:

| **Gruppe ET** | **Ar²** |
|---|---|
| Formel (ET-26a) | Formel (Ar-1) |
| Formel (ET-26b) | Formel (Ar-16) |
| Formel (ET-26c) | Formel (Ar-3) |
| Formel (ET-26d) | Formel (Ar-49) |
| Formel (ET-26e) | Formel (Ar-63) |
| Formel (ET-26f) | Formel (Ar-65) |
| Formel (ET-26g) | Formel (Ar-66) |
| Formel (ET-26h) | Formel (Ar-69) |
| Formel (ET-26i) | Formel (Ar-78) |
| Formel (ET-26i) | Formel (Ar-139) |
| Formel (ET-26k) | Formel (Ar-274) |

Gruppen ET-27a bis ET-27k mit der allgemeinen Formel 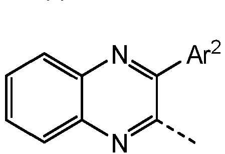 wobei gilt:

| **Gruppe ET** | **Ar²** |
|---|---|
| Formel (ET-27a) | Formel (Ar-1) |
| Formel (ET-27b) | Formel (Ar-16) |
| Formel (ET-27c) | Formel (Ar-3) |
| Formel (ET-27d) | Formel (Ar-49) |
| Formel (ET-27e) | Formel (Ar-63) |
| Formel (ET-27f) | Formel (Ar-65) |
| Formel (ET-27g) | Formel (Ar-66) |
| Formel (ET-27h) | Formel (Ar-69) |
| Formel (ET-27i) | Formel (Ar-78) |
| Formel (ET-27i) | Formel (Ar-139) |
| Formel (ET-27k) | Formel (Ar-274) |

Gruppen ET-28a bis ET-28k mit der allgemeinen Formel 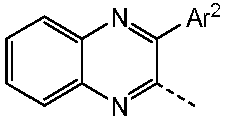 wobei gilt:

| **Gruppe ET** | **Ar²** |
|---|---|
| Formel (ET-28a) | Formel (Ar-1) |
| Formel (ET-28b) | Formel (Ar-16) |
| Formel (ET-28c) | Formel (Ar-3) |
| Formel (ET-28d) | Formel (Ar-49) |
| Formel (ET-28e) | Formel (Ar-63) |
| Formel (ET-28f) | Formel (Ar-65) |
| Formel (ET-28g) | Formel (Ar-66) |
| Formel (ET-28h) | Formel (Ar-69) |
| Formel (ET-28i) | Formel (Ar-78) |
| Formel (ET-28i) | Formel (Ar-139) |
| Formel (ET-28k) | Formel (Ar-274) |

Bevorzugt ist R³ bei jedem Auftreten gleich oder verschieden gewählt aus H, D, F, CN, Si(R⁵)₃, N(R⁵)₂, geradkettigen Alkyl- oder Alkoxygruppen mit 1 bis 20 C-Atomen, verzweigten oder cyclischen Alkyl- oder Alkoxygruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl- und Alkoxygruppen, die genannten aromatischen Ringsysteme und die genannten heteroaromatischen Ringsysteme jeweils mit Resten R⁵ substituiert sind; und wobei in den genannten Alkyl- oder Alkoxygruppen eine oder mehrere CH₂-Gruppen durch -C=C-, -R⁵C=CR⁵-, Si(R⁵)₂, C=O, C=NR⁵, -NR⁵-, -O-, -S-, -C(=O)O- oder -C(=O)NR⁵- ersetzt sein können.

Besonders bevorzugt ist R³ bei jedem Auftreten gleich oder verschieden gewählt aus H, D, Si(R⁵)₃, geradkettigen Alkylgruppen mit 1 bis 20 C-Atomen, die deuteriert sein können, verzweigten oder cyclischen Alkylgruppen mit 3 bis 20 C-Atomen, die deuteriert sein können, aromatischen Ringsystemen mit 6 bis 40 aromatischen Ringatomen, die deuteriert sein können, und heteroaromatischen Ringsystemen mit 5 bis 40 aromatischen Ringatomen, die deuteriert sein können, wobei die genannten Alkylgruppen, die genannten aromatischen Ringsysteme und die genannten heteroaromatischen Ringsysteme jeweils mit Resten R⁵ substituiert sind, die bevorzugt gleich H sind. Ganz besonders bevorzugt ist R³ gleich H.

Bevorzugt ist R⁵ bei jedem Auftreten gleich oder verschieden gewählt aus H, D, F, CN, Si(R⁶)₃, geradkettigen Alkyl- oder Alkoxygruppen mit 1 bis 20 C-Atomen, verzweigten oder cyclischen Alkyl- oder Alkoxygruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl- und Alkoxygruppen, die genannten aromatischen Ringsysteme und die genannten heteroaromatischen Ringsysteme jeweils mit Resten R⁶ substituiert sind; und wobei in den genannten Alkyl- oder Alkoxygruppen eine oder mehrere CH₂-Gruppen durch -C≡C-, -R⁶C=CR⁶-, Si(R⁶)₂, C=O, C=NR⁶, -NR⁶-, -O-, - S-, -C(=O)O- oder -C(=O)NR⁶- ersetzt sein können. Besonders bevorzugt ist R⁵ bei jedem Auftreten gleich oder verschieden gewählt aus H, D, Si(R⁶)₃, geradkettigen Alkylgruppen mit 1 bis 20 C-Atomen, die deuteriert sein können, verzweigten oder cyclischen Alkylgruppen mit 3 bis 20 C-Atomen, die deuteriert sein können, aromatischen Ringsystemen mit 6 bis 40 aromatischen Ringatomen, die deuteriert sein können, und heteroaromatischen Ringsystemen mit 5 bis 40 aromatischen Ringatomen, die deuteriert sein können, wobei die genannten Alkylgruppen, die genannten aromatischen Ringsysteme und die genannten heteroaromatischen Ringsysteme jeweils mit Resten R⁶ substituiert sind, die bevorzugt gleich H sind. Ganz besonders bevorzugt ist R⁵ gleich H.

In einer bevorzugten Ausführungsform der Erfindung sind die beiden Reste R⁰ nicht miteinander verknüpft und bilden keinen aliphatischen oder heteroaliphatischen Ring, so dass keine Spiroverbindung, insbesondere kein Spirobifluoren-Derivat, in Position 8 des Fluoren-Derivats entsteht.

Dementsprechend entspricht Formel (I) in einer bevorzugten Ausführungsform der Erfindung der folgenden Formel (I-A) 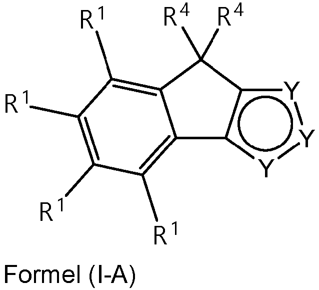
wobei die Variablen wie vorstehend für Formel (I) definiert sind und mindestens eine Gruppe A wie vorstehend definiert je Formel vorliegt; und
R⁴ bei jedem Auftreten gleich oder verschieden gewählt ist aus H, D, F, Cl, Br, **I,** C(=O)R¹, CN, Si(R¹)3, N(R¹)₂, P(=O)(R¹)₂, OR¹, S(=O)R¹, S(=O)₂R¹, geradkettigen Alkyl-, Alkoxy- oder Thioalkylgruppen mit 1 bis 20 C-Atomen, verzweigten oder cyclischen Alkyl-, Alkoxy- oder Thioalkylgruppen mit 3 bis 20 C-Atomen, Alkenyl- oder Alkinylgruppen mit 2 bis 20 C-Atomen, aromatischen Ringsystemen mit 6 bis 40 aromatischen Ringatomen, heteroaromatischen Ringsystemen mit 5 bis 40 aromatischen Ringatomen, Aryloxy- oder Heteroaryloxygruppen mit 5 bis 40 aromatischen Ringatomen, und Aralkylgruppen mit 5 bis 40 aromatischen Ringatomen; wobei ausgeschlossen ist, dass die beiden Reste R⁴ miteinander verknüpft sind und einen aliphatischen oder heteroaliphatischen Ring bilden; wobei die genannten Alkyl-, Alkoxy-, Thioalkyl-, Alkenyl-, Alkinyl-, Aryloxy-, Heteroaryloxy- und Aralkylgruppen und die genannten aromatischen Ringsysteme und heteroaromatischen Ringsysteme jeweils mit Resten R¹ substituiert sind; und wobei eine oder mehrere CH₂-Gruppen in den genannten Alkyl-, Alkoxy-, Thioalkyl-, Alkenyl-, Alkinyl-, Aryloxy-, Heteroaryloxy- und Aralkylgruppen durch -R¹C=CR¹-, -C=C-, Si(R¹)₂, C=O, C=NR¹, -C(=O)O-, -C(=O)NR¹-, NR¹, P(=O)(R¹), -O-, -S-, SO oder SO₂ ersetzt sein können.

Bevorzugte Ausführungsformen der Formel (I-A) sind die folgenden Formeln (II-A), (III-A) und (IV-A): 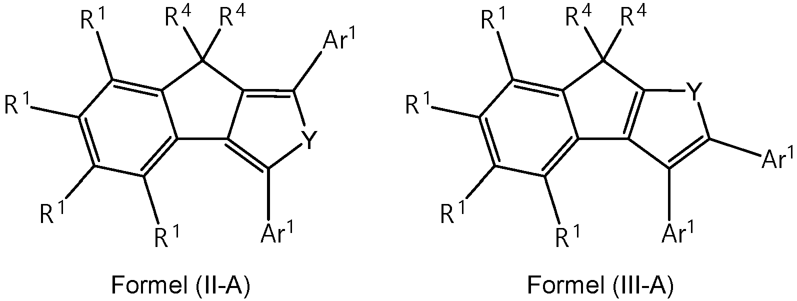 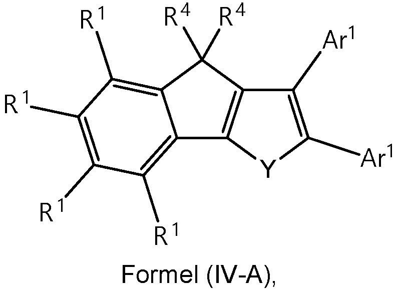 wobei die Variablen wie vorstehend definiert sind und mindestens eine Gruppe A wie vorstehend definiert je Formel vorliegt.

Bevorzugt ist R⁴ bei jedem Auftreten gleich oder verschieden gewählt ist aus F, CN, Si(R¹)₃, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkylgruppen und die genannten aromatischen Ringsysteme und heteroaromatischen Ringsysteme jeweils mit Resten R¹ substituiert sind und wobei ausgeschlossen ist, dass die beiden Reste R⁴ miteinander verknüpft sind und einen aliphatischen oder heteroaliphatischen Ring bilden.

Für die oben genannten Formeln (II-A), (III-A) und (IV-A) gelten bevorzugt die vorstehend genannten bevorzugten Ausführungsformen der Varialblen. Bevorzugt sind in den oben genannten Gruppen genau zwei oder eine Gruppe A je Formel gebunden, besonders bevorzugt genau eine (das heißt, (besonders) bevorzugt sind ein oder zwei Reste R¹, oder ein oder zwei Gruppen Ar¹, oder ein Rest R¹ und eine Gruppe Ar¹ entsprechend durch eine bzw. zwei Gruppen A ersetzt). Wenn zwei Gruppen A in den Verbindungen der Formeln (II-A), (III-A) und (IV-A) vorhanden sind, so können diese entweder beide an den heteroaromatischen Fünfring gebunden sein; oder eine Gruppe A ist an den heteroaromatischen Fünfring gebunden und die andere Gruppe A ist an den Benzolring (aromatischen Sechsring) gebunden; oder beide Gruppen A sind an Benzolring gebunden. Bevorzugt sind die eine oder beiden Gruppen A an den heteroaromatischen Fünfring gebunden. Die bevorzugten Bindungspositionen an dem Benzolring des Fluoren-Derivat-Grundgerüsts sind die Positionen 4 und 6. Für den Fall, dass keine Gruppe A an den heteroaromatischen Fünfring gebunden ist, können beide Gruppen Ar¹ gleich H sein, oder eine Gruppe Ar¹ ist H, die andere ein wie vorstehend definierter Rest, oder beide Gruppen Ar¹ können gleich oder veschieden aus den vorstehend definierten Resten gewählt sein. Für den Fall, dass eine Gruppe A an den heteroaromatischen Fünfring gebunden ist, kann die eine Gruppe Ar¹ entweder gleich H oder ein wie vorstehend definierter Rest sein. X ist in den oben genannten Formeln bevorzugt gleich S oder O, besonders bevorzugt gleich S. Bevorzugt unter den oben genannten Formeln sind die Formeln (II-A) und (IV-A), insbesondere die Formel (II-A).

Weiterhin bevorzugt sind in den oben genannten Formeln (II-A), (III-A) und (IV-A) alle an den Benzolring gebundenen Reste R¹ gleich H. Gemäß einer weiteren bevorzugten Ausführungsform sind in den oben genannten Formeln (II-A), (III-A) und (IV-A) zusätzlich zu der/den vorliegenden Gruppe(n) A genau zwei Reste R¹, die an den Benzolring gebunden sind, besonders bevorzugt genau einer, ungleich H, insbesondere dann, wenn die Gruppe(n) A an den heteroaromatischen Fünfring gebunden ist/sind. Die bevorzugten Bindungspositionen der Reste R¹, die ungleich H sind, sind die Positionen 4 und 6 an dem Benzolring des Fluoren-Derivat-Grundgerüsts, aber selbstverständlich nur dann, wenn diese Positionen nicht durch die Gruppe(n) A besetzt ist/sind. Für den Fall, dass R¹ ungleich H ist, sind besonders bevorzugte Gruppen R¹ die weiter oben definierten Gruppen (R¹-1), (R¹-8), (R¹-31), (R¹-33), (R¹-163), (R¹-189) und (R¹-190).

Gemäß einer weiter bevorzugten Ausführungsform der Erfindung ist R⁴ in bei jedem Auftreten gleich gewählt. Besonders bevorzugt ist R⁴ in den oben genannten Formeln bei jedem Auftreten gleich gewählt aus geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen, wobei die genannten Alkylgruppen und die genannten aromatischen Ringsysteme und die genannten heteroaromatischen Ringsysteme jeweils mit Resten R¹ substituiert sind, und wobei ausgeschlossen ist, dass die beiden Reste R⁴ miteinander verknüpft sind und einen aliphatischen oder heteroaliphatischen Ring bilden. Ganz besonders bevorzugt sind beide Reste R⁴ dabei gleich gewählt aus geradkettigen Alkylgruppen mit 1 bis 4 C-Atomen und Phenyl, die jeweils mit Resten R¹ substituiert sind, wobei R¹ bevorzugt in diesem Fall H ist.

Gemäß einer anderen weiter bevorzugten Ausführungsform der Erfindung ist R⁴ bei jedem Auftreten verschieden gewählt. Besonders bevorzugt ist R⁴ in den oben genannten Formeln bei jedem Auftreten verschieden gewählt aus geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen, wobei die genannten Alkylgruppen und die genannten aromatischen Ringsysteme und die genannten heteroaromatischen Ringsysteme jeweils mit Resten R¹ substituiert sind, und wobei ausgeschlossen ist, dass die beiden Reste R⁴ miteinander verknüpft sind und einen aliphatischen oder heteroaliphatischen Ring bilden. Ganz besonders bevorzugt sind beide Reste R⁴ dabei verschieden gewählt aus einer geradkettigen Alkylgruppen mit 1 bis 4 C-Atomen und Phenyl, die jeweils mit Resten R¹ substituiert sind, wobei R¹ bevorzugt in diesem Fall H ist.

Bevorzugte Ausführungsformen der Formeln (II-A), (III-A) und (IV-A) entsprechen den folgenden Formeln:

| | |
|---|---|
| 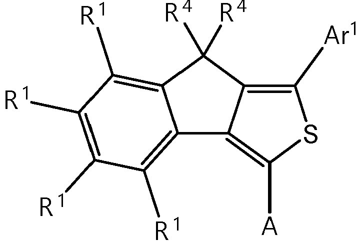 | 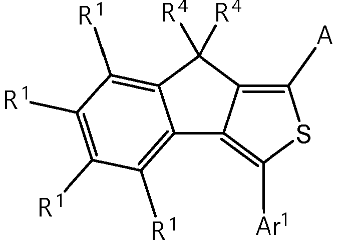 |
| Formel (II-A-S1) | Formel (II-A-S2) |
| 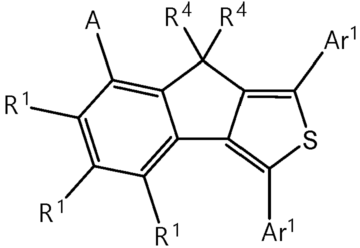 | 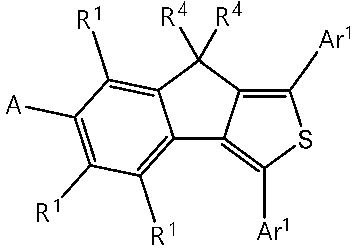 |
| Formel (II-A-S3) | Formel (II-A-S4) |
| 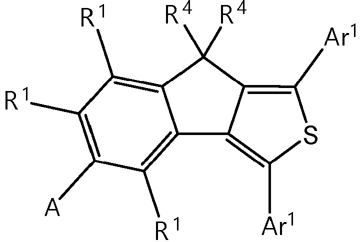 | 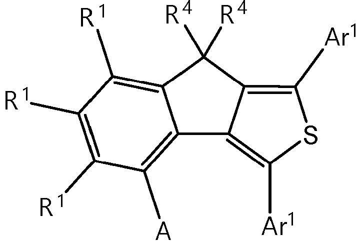 |
| Formel (II-A-S5) | Formel (II-A-S6) |
| 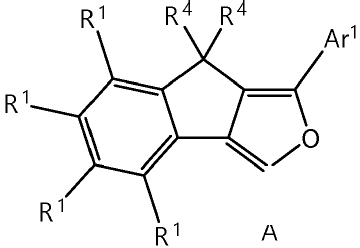 | 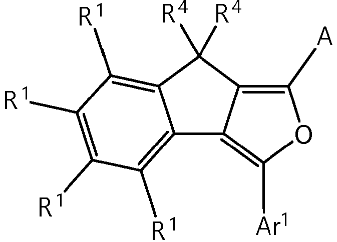 |
| Formel (II-A-O1) | Formel (II-A-O2) |
| 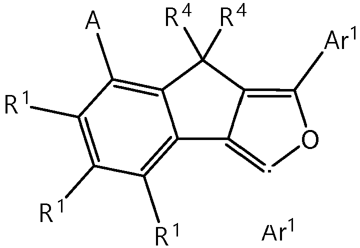 | 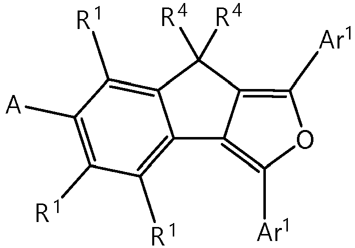 |
| Formel (II-A-O3) | Formel (II-A-O4) |
| 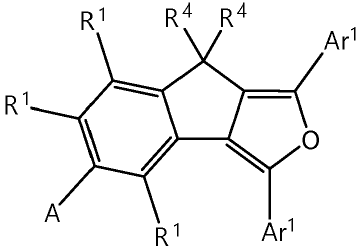 | 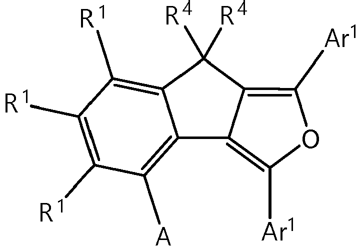 |
| Formel (II-A-O5) | Formel (II-A-O6) |
| 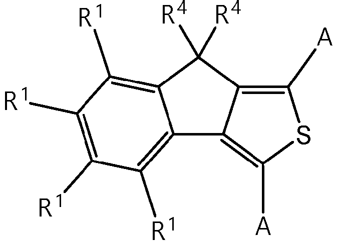 | 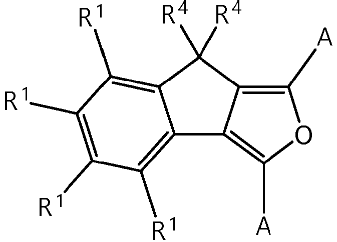 |
| Formel (II-A-S7) | Formel (II-A-O7) |
| 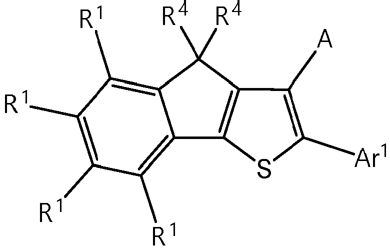 | 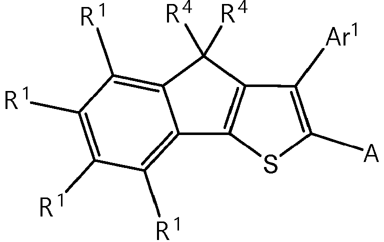 |
| Formel (IV-A-S1) | Formel (IV-A-S2) |
| 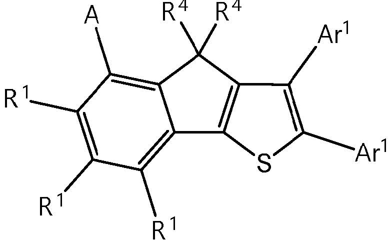 | 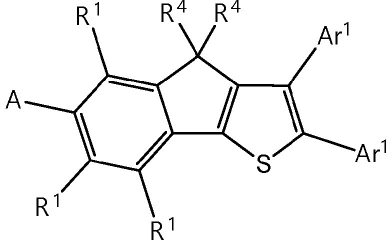 |
| Formel (IV-A-S3) | Formel (IV-A-S4) |
| 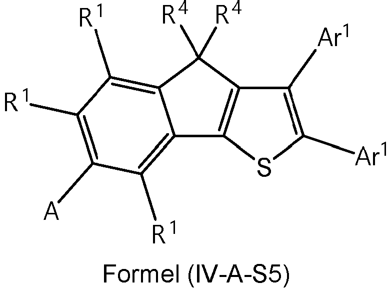 | 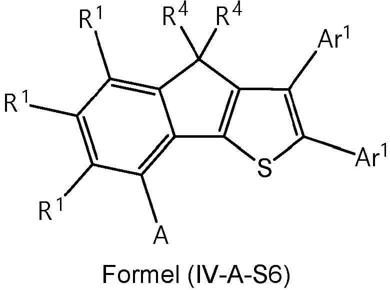 |
| | |
| 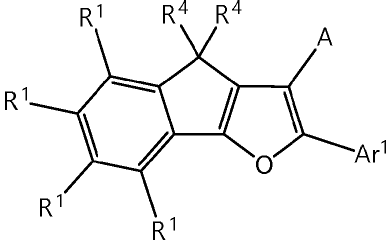 | 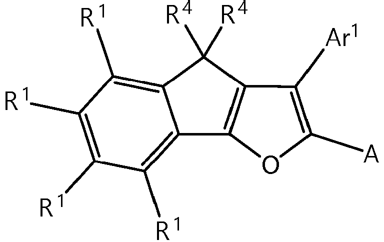 |
| Formel (IV-A-O1) | Formel (IV-A-O2) |
| 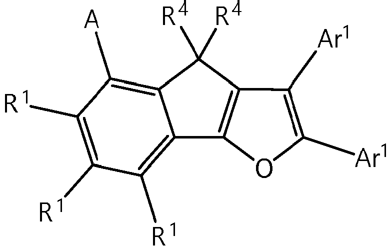 | 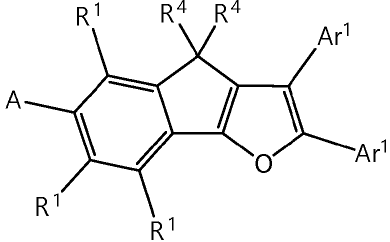 |
| Formel (IV-A-O3) | Formel (IV-A-O4) |
| 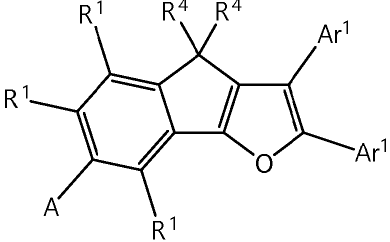 | 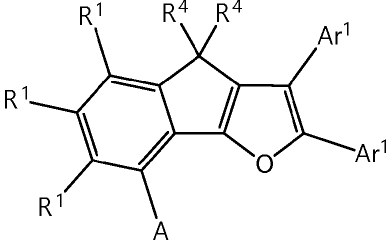 |
| Formel (IV-A-O5) | Formel (IV-A-O6) |
| 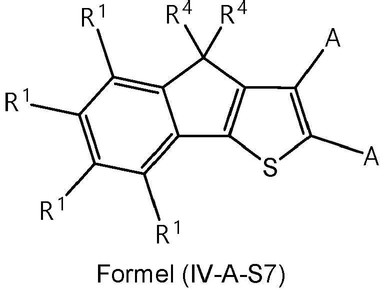 | 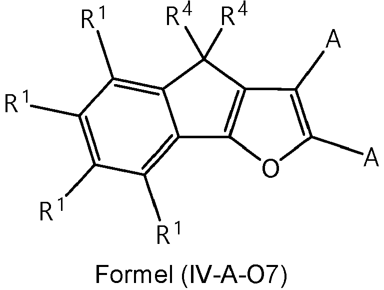 |
| | |
| 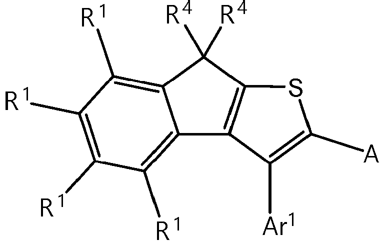 | 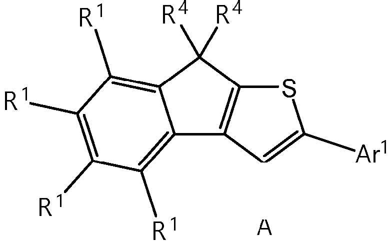 |
| Formel (III-A-S1) | Formel (III-A-S2) |
| 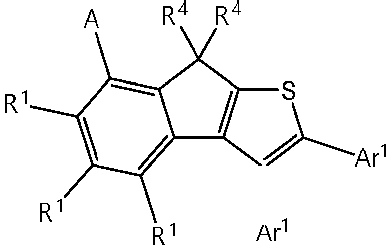 | 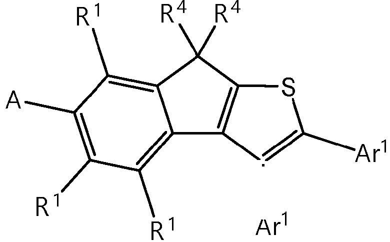 |
| Formel (III-A-S3) | Formel (III-A-S4) |
| 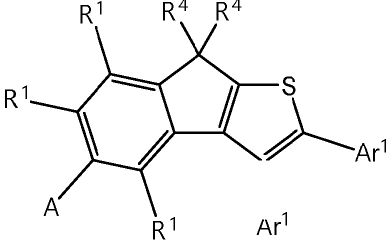 | 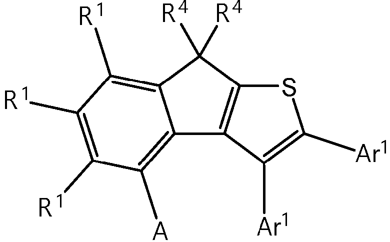 |
| Formel (III-A-S5) | Formel (III-A-S6) |
| 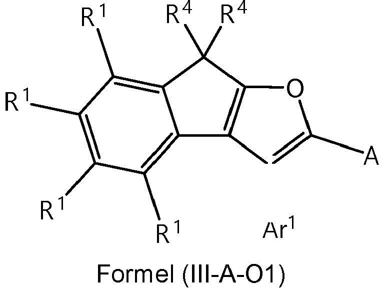 | 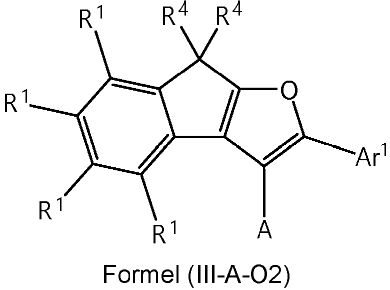 |
| | |
| 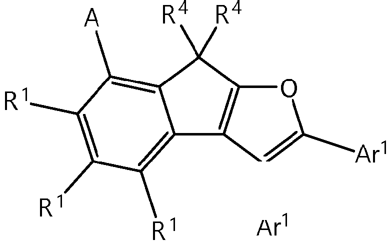 | 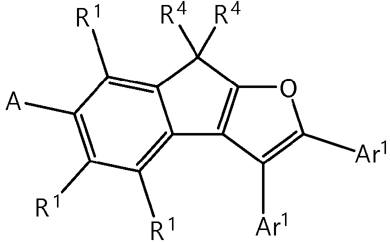 |
| Formel (III-A-O3) | Formel (III-A-O4) |
| 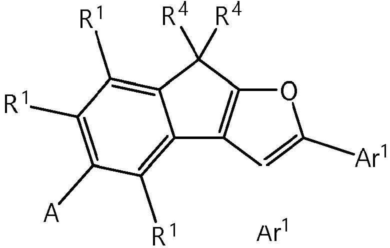 | 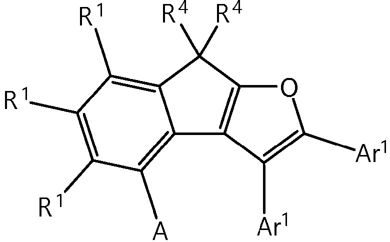 |
| Formel (III-A-O5) | Formel (III-A-O6) |
| 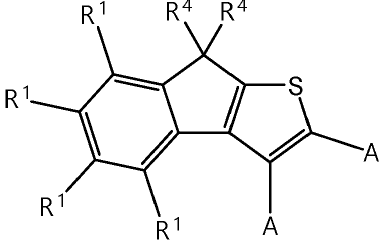 | 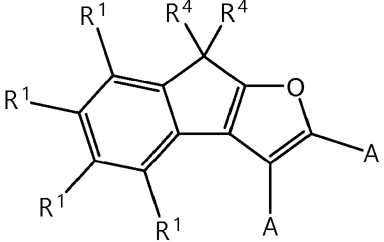 |
| Formel (III-A-S7) | Formel (III-A-O7) |

wobei die Variablen die oben genannten Bedeutungen haben und bevorzugt ihren oben genannten bevorzugten Ausführungsformen entsprechen.

In den Fällen in denen die Gruppe

Unter den oben genannten Formeln sind die Formeln (II-A-S1) bis (IV-AS1), (II-A-S2) bis (IV-A-S2), (II-A-S4) bis (IV-A-S4), (II-A-S6) bis (IV-A-S6), (II-A-O1) bis (IV-A-O1), (II-A-O2) bis (IV-A-O2), (II-A-O4) bis (IV-A-O4) und (II-A-O6) bis (IV-A-O6) besonders bevorzugt. Ganz besonders bevorzugt sind die Formeln (II-A-S1) bis (IV-A-S1), (II-A-S2) bis (IV-A-S2), (II-A-S4) bis (IV-A-S4), (II-A-O1) bis (IV-A-O1), (II-A-O2) bis (IV-A-O2) und (II-A-O4) bis (IV-A-O4).

Weiterhin ganz besonders bevorzugt sind die Formeln (II-A-S1), (IV-A-S1), (II-A-S2), (IV-A-S2), (II-A-S4), (IV-A-S4), (II-A-S6), (IV-A-S6), (II-A-O1), (IV-A-O1), (II-A-O2), (IV-A-O2), (II-A-O4), (IV-A-O4), (II-A-O6) und (IV-A-O6).

Am meisten bevorzugt unter den oben genannten Formeln sind die Formeln (II-A-S1), (IV-A-S1), (II-A-S2), (IV-A-S2), (II-A-S4), (IV-A-S4), (II-A-O1), (IV-A-O1), (II-A-O2), (IV-A-O2), (II-A-O4) und (IV-A-O4).

Besonders bevorzugt entspricht die anmeldungsgemäße Verbindung daher einer Formel (II-A-S1) bis (IV-A-S1), (II-A-S2) bis (IV-A-S2), (II-A-S4) bis (IV-A-S4), (II-A-S6) bis (IV-A-S6), (II-A-O1) bis (IV-A-O1), (II-A-O2) bis (IV-A-O2), (II-A-O4) bis (IV-A-O4) oder (II-A-O6) bis (IV-A-O6), wobei für die auftretenden Variablen gilt:
Ar¹ ist bei jedem Auftreten gleich oder verschieden gewählt aus Phenyl, Biphenyl, Terphenyl, Quaterphenyl, Naphthyl, Fluorenyl, insbesondere 9,9'-Dimethylfluorenyl und 9,9'-Diphenylfluorenyl, Benzofluorenyl, Spirobifluorenyl, Indenofluorenyl, Indenocarbazolyl, Dibenzofuranyl, Dibenzothiophenyl, Benzofuranyl, Benzothiophenyl, benzokondensiertem Dibenzofuranyl, benzokondensiertem Dibenzothiophenyl, Naphthyl-substituiertem Phenyl, Fluorenyl-substituiertem Phenyl, Spirobifluorenyl-substituiertem Phenyl, Dibenzofuranyl-substituiertem Phenyl, Dibenzothiophenyl-substituiertem Phenyl, Carbazolyl-substituiertem Phenyl, Pyridyl-substituiertem Phenyl, Pyrimidyl-substituiertem Phenyl, Triazinyl-substituiertem Phenyl, wobei die genannten Gruppen jeweils mit Resten R² substituiert sind, und H;
R¹ ist bei jedem Auftreten gleich oder verschieden gewählt aus H, D, Si(R⁵)₃, geradkettigen Alkylgruppen mit 1 bis 20 C-Atomen, die deuteriert sein können, verzweigten oder cyclischen Alkylgruppen mit 3 bis 20 C-Atomen, die deuteriert sein können, aromatischen Ringsystemen mit 6 bis 40 aromatischen Ringatomen, die deuteriert sein können, und heteroaromatischen Ringsystemen mit 5 bis 40 aromatischen Ringatomen, die deuteriert sein können, wobei die genannten Alkylgruppen, die genannten aromatischen Ringsysteme und die genannten heteroaromatischen Ringsysteme jeweils mit Resten R⁵ substituiert sind, die bevorzugt gleich H sind;
R⁴ ist bei jedem Auftreten gleich oder verschieden gewählt ist aus geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen, wobei die genannten Alkylgruppen und die genannten aromatischen Ringsysteme und die genannten heteroaromatischen Ringsysteme jeweils mit Resten R¹ substituiert sind, und wobei ausgeschlossen ist, dass die beiden Reste R⁴ miteinander verknüpft sind und einen aliphatischen oder heteroaliphatischen Ring bilden;
A ist eine Einheit der Formel (A)

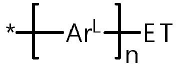

Formel (A), wobei die Variablen in Formel (A) wie folgt definiert sind:
Ar^{L} ist bei jedem Auftreten gleich oder verschieden gewählt aus divalenten Gruppen abgeleitet von Benzol, Biphenyl, Terphenyl, Naphthalin, Fluoren, Indenofluoren, Indenocarbazol, Spirobifluoren, Dibenzofuran und Dibenzothiophen, die jeweils mit Resten R² substituiert sind;
ET ist bei jedem Auftreten gleich oder verschieden gewählt aus Gruppen abgeleitet von Pyridin, Pyrazin, Pyrimidin, Pyridazin, 1,2,4-Triazin, 1,3,5-Triazin, Chinolin, Isochinolin, Chinoxalin, Chinazolin, Pyrazol, Imidazol, Benzimidazol, Thiazol, Benzothiazol, Oxazol, Oxadiazol und Benzooxazol, die jeweils mit Gruppen Ar² substituiert sind;
Ar² ist bei jedem Auftreten gleich oder verschieden gewählt aus Phenyl, Biphenyl, Terphenyl, Quaterphenyl, Naphthyl, Fluorenyl, insbesondere 9,9'-Dimethylfluorenyl und 9,9'-Diphenylfluorenyl, Benzofluorenyl, Spirobifluorenyl, Indenofluorenyl, Indenocarbazolyl, Dibenzofuranyl, Dibenzothiophenyl, Carbazolyl, Benzofuranyl, Benzothiophenyl, benzokondensiertem Dibenzofuranyl, benzokondensiertem Dibenzothiophenyl, Naphthyl-substituiertem Phenyl, Fluorenyl-substituiertem Phenyl, Spirobifluorenyl-substituiertem Phenyl, Dibenzofuranyl-substituiertem Phenyl, Dibenzothiophenyl-substituiertem Phenyl, Carbazolyl-substituiertem Phenyl, Pyridyl-substituiertem Phenyl, Pyrimidyl-substituiertem Phenyl, Triazinyl-substituiertem Phenyl, wobei die genannten Gruppen jeweils mit Resten R³ substituiert sind, und H; $n = 1 oder 0 ;$
und die weiteren Variablen sind definiert gemäß ihrer oben angegebenen breitesten Ausführungsform, bevorzugt den oben angegebenen bevorzugten Ausführungsformen.

Bevorzugte Verbindungen der Formel (I) gemäß dieser Ausführungsform sind in untenstehender Tabelle 1 gezeigt.

In einer anderen bevorzugten Ausführungsform der Erfindung sind die beiden Reste R⁰ miteinander verknüpft und bilden einen aliphatischen oder heteroaliphatischen Ring, so dass eine Spiroverbindung, insbesondere ein Spirobifluoren-Derivat, in Position 8 des Fluoren-Derivats entsteht.

Dementsprechend entspricht Formel (I) in einer anderen bevorzugten Ausführungsform der Erfindung der folgenden Formel (I-B): 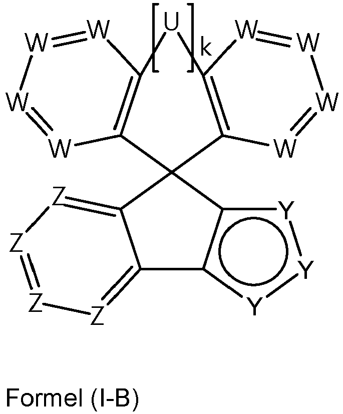 wobei
W bei jedem Auftreten gleich oder verschieden gewählt ist aus N und CR¹;
U gewählt ist aus O und S;
k gleich 0 oder 1 ist, wobei im Fall k=0 das betreffende Heteroatom U wegfällt und U stattdessen eine Einfachbindung ist, die die entsprechenden beiden aromatischen Sechsringe verbindent; und
die Variablen Z, Y und R¹ wie vorstehend definiert sind, und wobei mindestens eine Gruppe A wie vorstehend definiert in der Formel (I-B) vorliegt.

Bevorzugt sind in Formel (I-B) höchstens drei Gruppen W, besonders bevorzugt höchstens zwei Gruppen W, ganz besonders bevorzugt höchstens eine Gruppe W, am stärksten bevorzugt ist keine Gruppe W in einem aromatischen Sechring gleich N. Die verbleibenden Gruppen sind entsprechend gleich CR¹. Weiterhin ist es bevorzugt, dass benachbarte Gruppen W in einem aromatischen Sechsring nicht gleich N sind. Nochmals weiterhin ist es bevorzugt, dass höchstens 3 Gruppen W in einer Formel (I-B) gleich N sind, besonders bevorzugt höchstens 2 Gruppen W in einer Formel (I-B) gleich N sind, ganz besonders bevorzugt höchstens eine Gruppe W in einer Formel (I-B) gleich N ist, am stärksten keine Gruppe W gleich N ist.

Bevorzugte Ausführungsformen der Formel (I-B) entsprechen den folgenden Formeln (II-B) bis (IV-B) und (II-C) bis (IV-C):

| | |
|---|---|
| 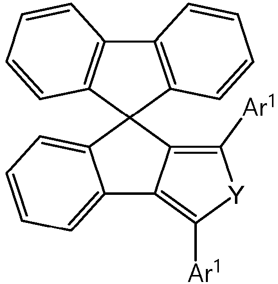 | 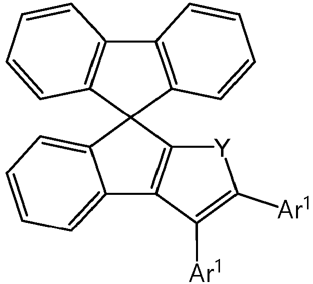 |
| Formel (II-B) | Formel (III-B) |
| 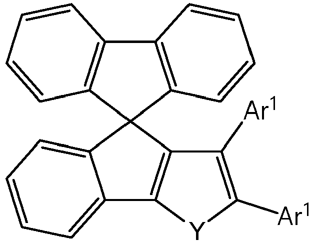 | 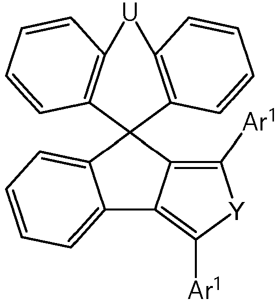 |
| Formel (IV-B) | Formel (II-C) |
| 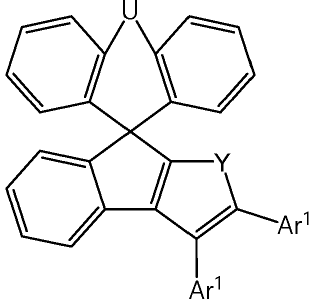 | 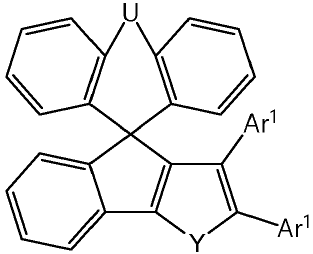 |
| Formel (III-C) | Formel (IV-C) |

wobei die Variablen wie vorstehend definiert sind und die freien Positionen an den Benzolringen jeweils mit Resten R¹ substituiert sind, und wobei mindestens eine Gruppe A wie vorstehend definiert je Formel vorliegt, wobei in den oben genannten Formeln bevorzugt genau eine Gruppe A je Formel gebunden ist, die entweder an einen der Benzolringe bindet (das heißt, ein Rest R¹ ist entsprechend durch eine Gruppe A ersetzt), oder an den heteroaromatischen Fünfring (das heißt, eine Gruppe Ar¹ ist entsprechend durch eine Gruppe A ersetzt), wobei es besonders bevorzugt ist, wenn die Gruppe A an den heteroaromatischen Fünfring bindet. Die bevorzugten Bindungspositionen an den Benzolringen des Spirobifluoren-Derivat-Grundgerüsts sind die Positionen 4, 6, 9, 11 12 und 14. Für den Fall, dass keine Gruppe A an den heteroaromatischen Fünfring gebunden ist, können beide Gruppen Ar¹ gleich H sein, oder eine Gruppe Ar¹ ist H, die andere ein wie vorstehend definierter Rest, oder beide Gruppen Ar¹ können gleich oder veschieden aus den vorstehend definierten Resten gewählt sein. Für den Fall, dass eine Gruppe A an den heteroaromatischen Fünfring gebunden ist, kann die eine Gruppe Ar¹ entweder gleich H oder ein wie vorstehend definierter Rest sein. X ist in den oben genannten Formeln bevorzugt gleich S oder O, besonders bevorzugt gleich S.

Weiterhin bevorzugt sind in den oben genannten Formeln (II-B) bis (IV-B) und (II-C) bis (IV-C) alle an die Benzolringe gebundenen Reste R¹ gleich H.

Gemäß einer weiteren bevorzugten Ausführungsform sind in den oben genannten Formeln (II-B) bis (IV-B) und (II-C) bis (IV-C) zusätzlich zu der vorliegenden mindestens einen Gruppe A genau zwei Reste R¹, die an die Benzolringe gebunden sind, besonders bevorzugt genau ein Rest R¹, ungleich H, insbesondere dann, wenn die mindestens eine Gruppe A an den heteroaromatischen Fünfring gebunden ist. Die bevorzugten Bindungspositionen für Reste R¹, die ungleich H sind, sind die Positionen 4, 6, 9, 11 12 und 14 an den Benzolringen des Spirobifluoren-Derivat-Grundgerüsts, und selbstverständlich nur dann, wenn diese Positionen nicht durch die mindestens eine Gruppe A besetzt sind. Für den Fall, dass R¹ ungleich H ist, sind besonders bevorzugte Gruppen R¹ die weiter oben definierten Gruppen (R¹-1), (R¹-8), (R¹-31), (R¹-33), (R¹-163), (R¹-189) und (R¹-190).

Bevorzugt ist k gleich 0. Bevorzugt unter den oben genannten Formeln sind demensprechend die Formeln (II-B) bis (IV-B), insbesondere die Formel (II-B).

Bevorzugte Ausführungsformen der Formeln (II-B) bis (IV-B) entsprechen den folgenden Formeln:

| | |
|---|---|
| 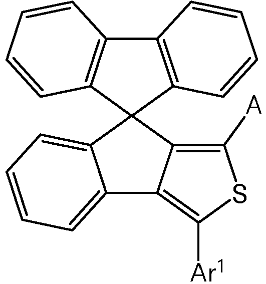 | 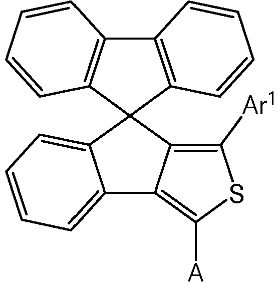 |
| Formel (II-B-S1) | Formel (II-B-S2) |
| 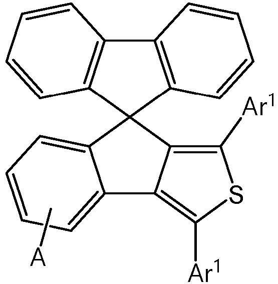 | 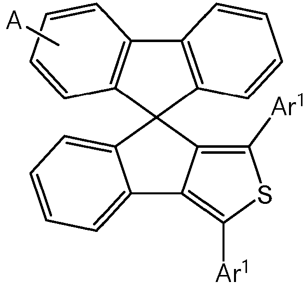 |
| Formel (II-B-S3) | Formel (II-B-S4) |
| 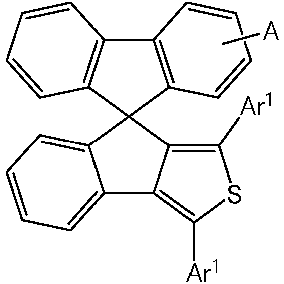 | 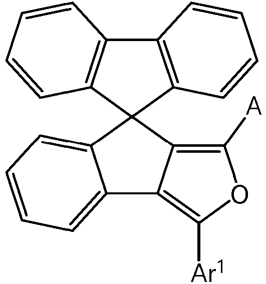 |
| Formel (II-B-S5) | Formel (II-B-O1) |
| 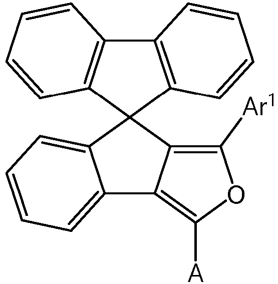 | 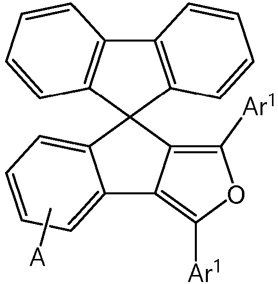 |
| Formel (II-B-O2) | Formel (II-B-O3) |
| 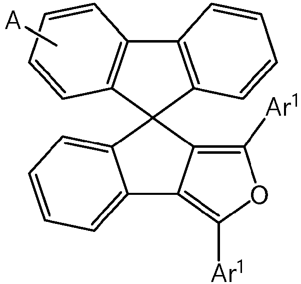 | 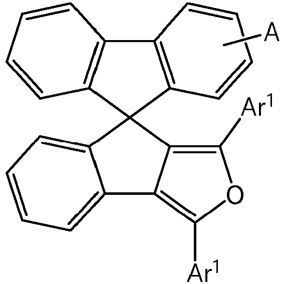 |
| Formel (II-B-O4) | Formel (II-B-O5) |
| 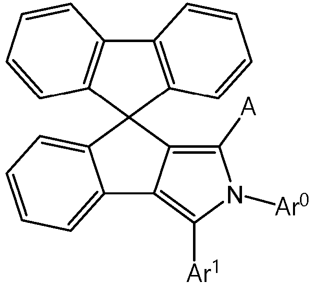 | 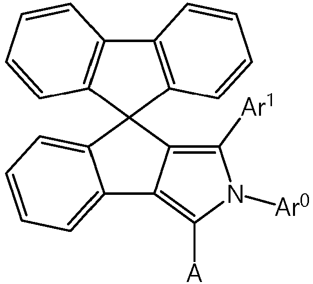 |
| Formel (II-B-N1) | Formel (II-B-N2) |
| 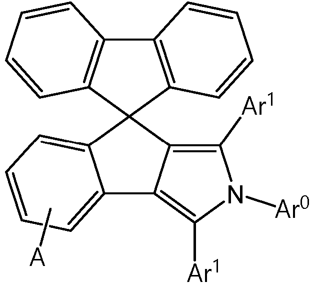 | 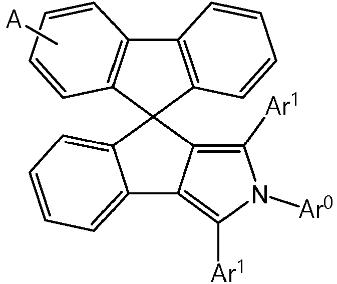 |
| Formel (II-B-N3) | Formel (II-B-N4) |
| 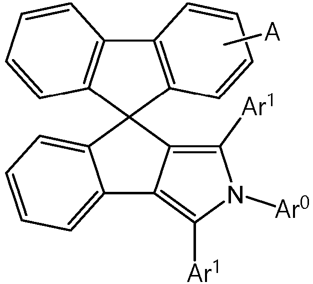 | 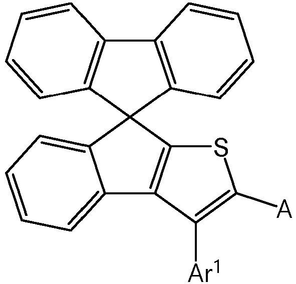 |
| Formel (II-B-N5) | Formel (III-B-S1) |
| 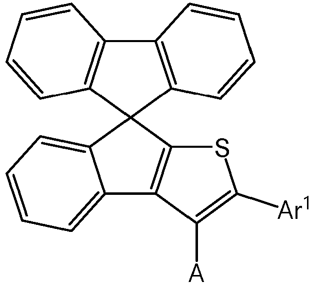 | 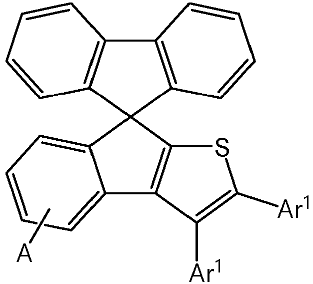 |
| Formel (III-B-S2) | Formel (III-B-S3) |
| 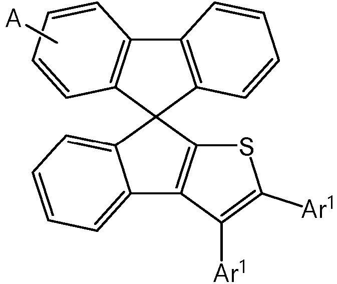 | 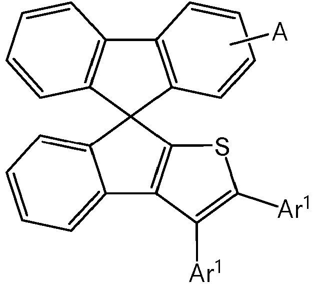 |
| Formel (III-B-S4) | Formel (III-B-S5) |
| 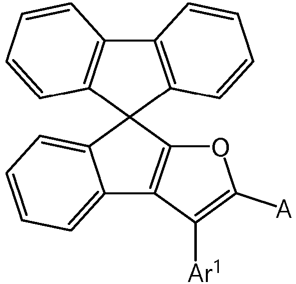 | 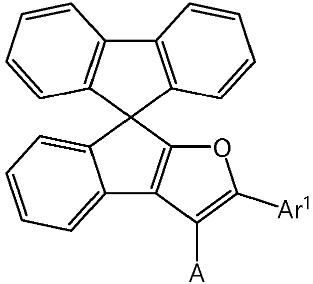 |
| Formel (III-B-O1) | Formel (III-B-O2) |
| 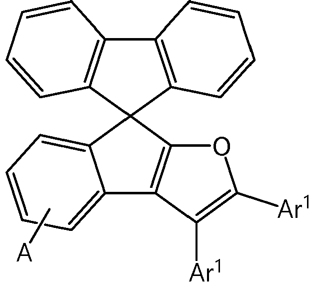 | 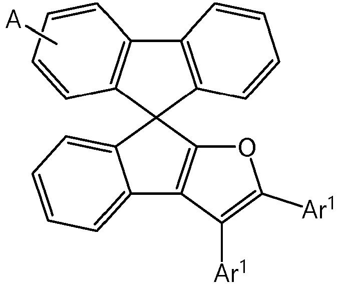 |
| Formel (III-B-O3) | Formel (III-B-O4) |
| 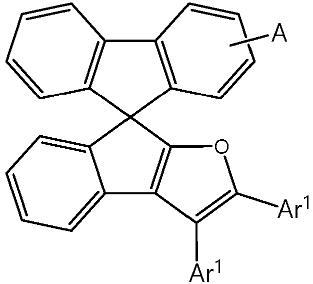 | 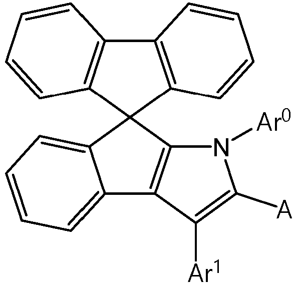 |
| Formel (III-B-O5) | Formel (III-B-N1) |
| 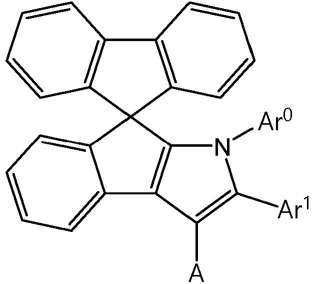 | 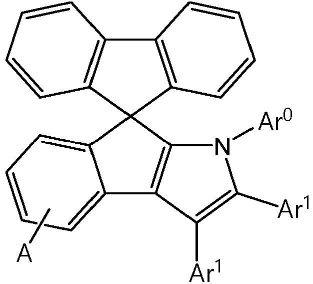 |
| Formel (III-B-N2) | Formel (III-B-N3) |
| 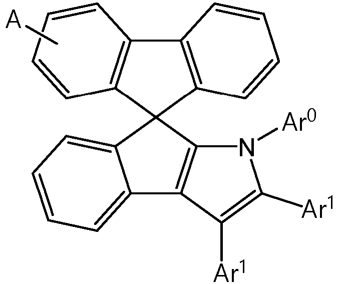 | 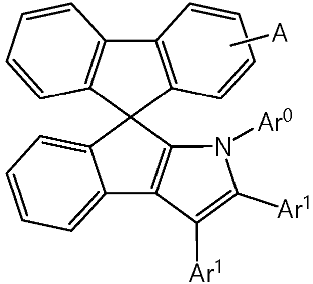 |
| Formel (III-B-N4) | Formel (III-B-N5) |
| 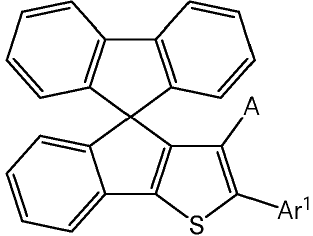 | 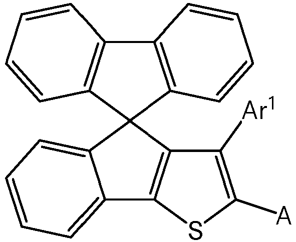 |
| Formel (IV-B-S1) | Formel (IV-B-S2) |
| 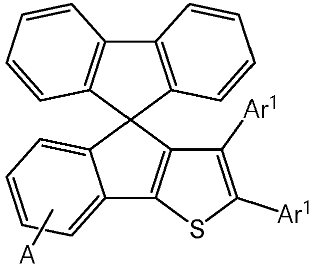 | 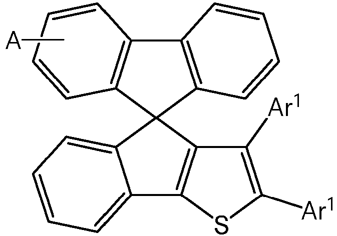 |
| Formel (IV-B-S3) | Formel (IV-B-S4) |
| 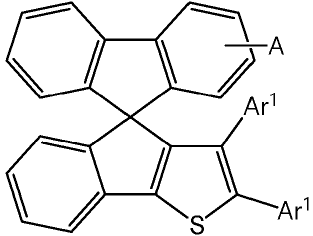 | 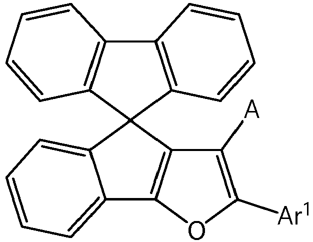 |
| Formel (IV-B-S5) | Formel (IV-B-O1) |
| 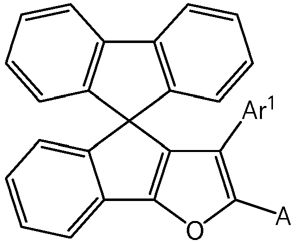 | 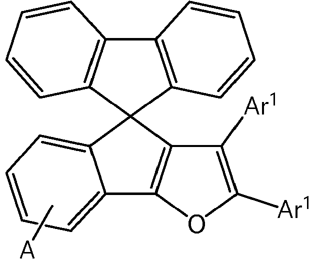 |
| Formel (IV-B-O2) | Formel (IV-B-O3) |
| 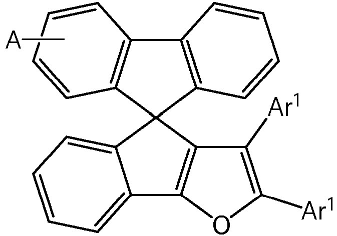 | 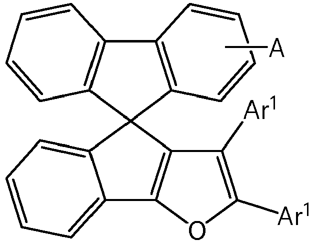 |
| Formel (IV-B-O4) | Formel (IV-B-O5) |
| 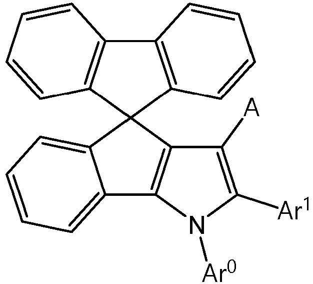 | 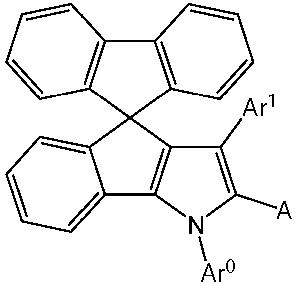 |
| Formel (IV-B-N1) | Formel (IV-B-N2) |
| 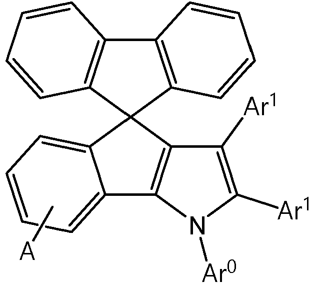 | 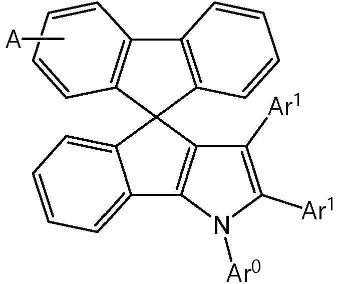 |
| Formel (IV-B-N3) | Formel (IV-B-N4) |
| 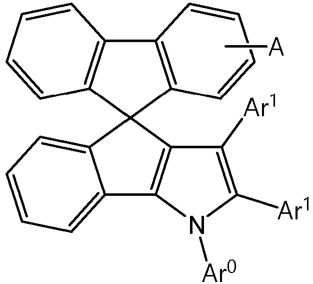 | |
| Formel (IV-B-N5), | |

wobei die Variablen die oben genannten Bedeutungen haben und bevorzugt ihren oben genannten bevorzugten Ausführungsformen entsprechen, und wobei die freien Positionen an den Benzolringen jeweils mit Resten R¹ substituiert sind, welche bevorzugt gleich H sind.

Ar⁰ ist in den oben genannten Formeln bevorzugt Phenyl, das mit Resten R² substituiert ist, wobei R² in diesen Fällen bevorzugt H ist.

Unter den oben genannten Formeln sind die Formeln (II-B-S1) bis (II-B-S4), (II-B-N1) bis (II-B-N4) und (II-B-O1) bis (II-B-O4), (III-B-S1) bis (III-B-S4), (III-B-N1) bis (III-B-N4) und (III-B-O1) bis (III-B-O4), und (IV-B-S1) bis (IV-B-S4), (IV-B-N1) bis (IV-B-N4) und (IV-B-O1) bis (IV-B-O4) bevorzugt. Besonders bevorzugt sind die Formeln (II-B-S1) bis (II-B-S4) und (II-B-O1) bis (II-B-O4), (III-B-S1) bis (III-B-S4) und (III-B-O1) bis (III-B-O4), und (IV-B-S1) bis (IV-B-S4) und (IV-B-O1) bis (IV-B-O4). Ganz besonders bevorzugt sind die Formeln (II-B-S1) bis (II-B-S4) und (II-B-O1) bis (II-B-O4).

Weiterhin besonders bevorzugt sind die Formeln (II-B-S1) bis (II-B-N1) und (II-B-S2) bis (II-B-N2).

Am meisten bevorzugt sind die Formeln (II-B-S1), (II-B-O1), (II-B-S2) und (II-B-O2).

Weiterhin besonders bevorzugt sind die Formeln (II-B-S1), (II-B-S2), (II-B-S3), (II-B-S4), (II-B-O1), (II-B-O2), (II-B-O3), (II-B-O4), (III-B-S1), (III-B-S2), (III-B-S3), (III-B-S4), (III-B-O1), (III-B-O2), (III-B-O3), (III-B-O4), (IV-B-S1), (IV-B-S2), (IV-B-S3), (IV-B-S4), (IV-B-O1), (IV-B-O2), (IV-B-O3), (IV-B-O4), (II-B-N1) und (II-B-N2).

Bevorzugte Ausführungsformen der Formeln (II-B-S3), (II-B-S4), (III-B-S3), (III-B-S4), (IV-B-S3) und (IV-B-S4) sind die folgenden Formeln:

| | |
|---|---|
| 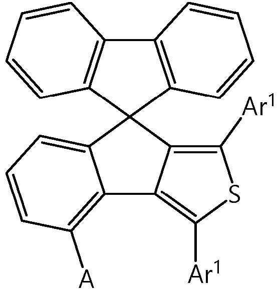 | 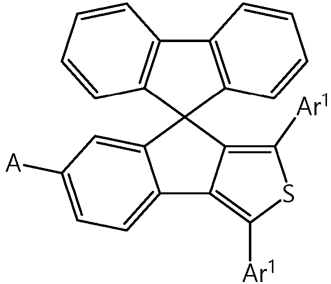 |
| Formel (II-B-S3-1) | Formel (II-B-S3-2) |
| 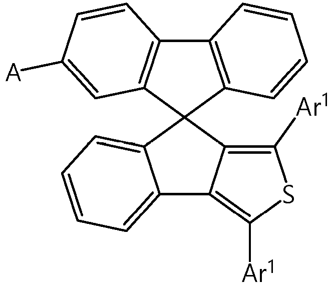 | 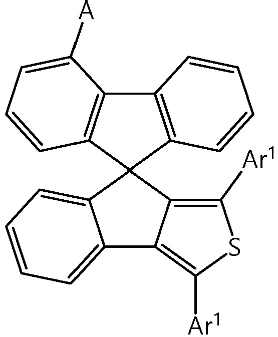 |
| Formel (II-B-S4-1) | Formel (II-B-S4-2) |
| 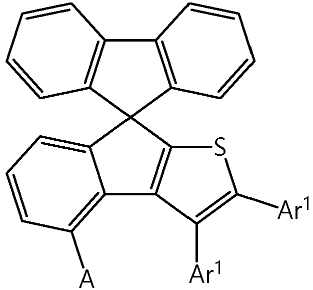 | 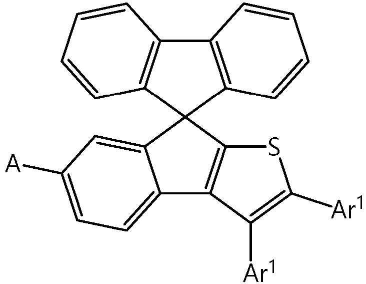 |
| Formel (III-B-S3-1) | Formel (III-B-S3-2) |
| 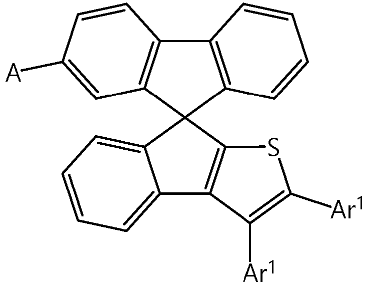 | 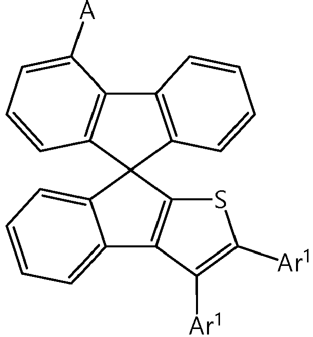 |
| Formel (III-B-S4-1) | Formel (III-B-S4-2) |
| 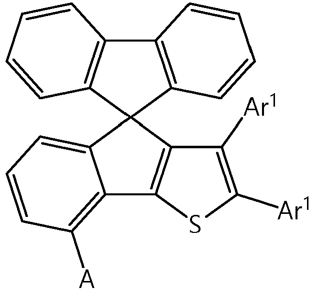 | 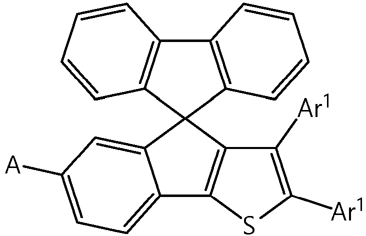 |
| Formel (IV-B-S3-1) | Formel (IV-B-S3-2) |
| 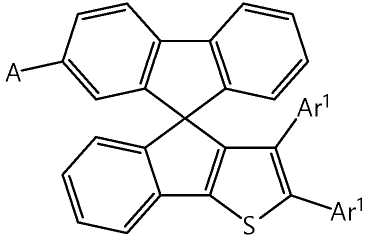 | 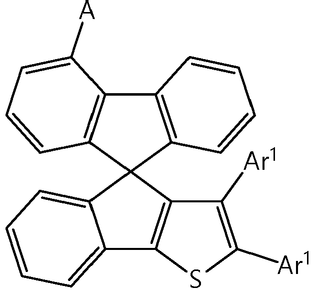 |
| Formel (IV-B-S4-1) | Formel (IV-B-S4-2), |

wobei die auftretenden Variablen definiert sind wie oben und bevorzugt ihren bevorzugten Ausführungsformen entsprechen, und wobei die freien Positionen an den Benzolringen jeweils mit Resten R¹ substituiert sind, welche bevorzugt gleich H sind.

Bevorzugte Ausführungsformen der Formeln (II-B-O3), (II-B-O4), (III-B-O3), (III-B-O4), (IV-B-O3) und (IV-B-O4) sind die folgenden Formeln:

| | |
|---|---|
| 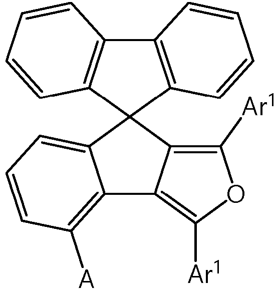 | 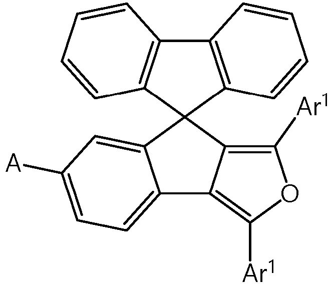 |
| Formel (II-B-O3-1) | Formel (II-B-O3-2) |
| 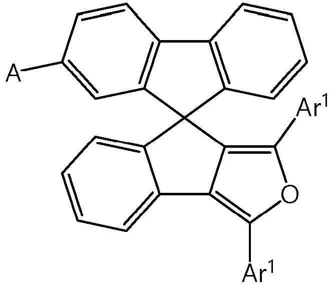 | 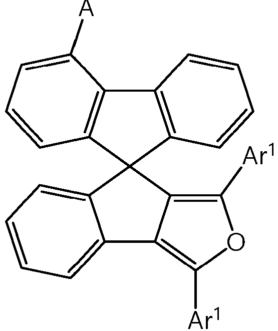 |
| Formel (II-B-O4-1) | Formel (II-B-O4-2) |
| 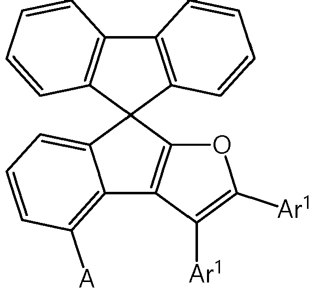 | 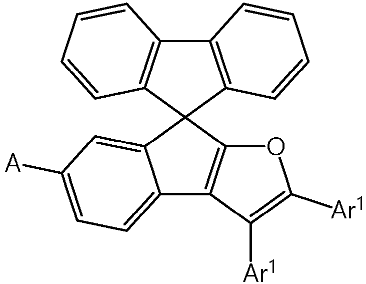 |
| Formel (III-B-O3-1) | Formel (III-B-O3-2) |
| 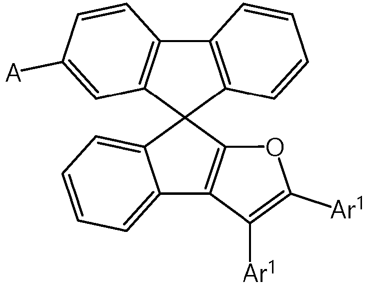 | 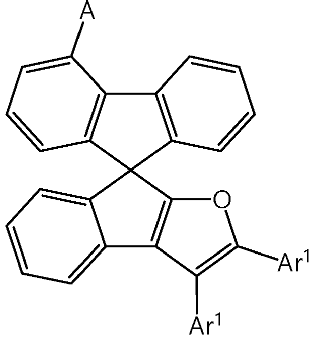 |
| Formel (III-B-O4-1) | Formel (III-B-O4-2) |
| 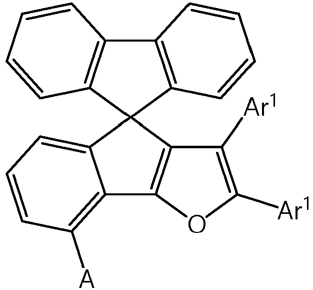 | 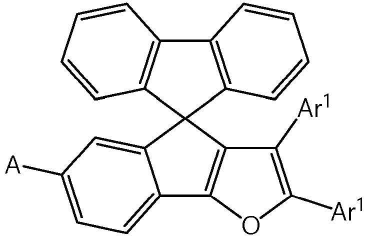 |
| Formel (IV-B-O3-1) | Formel (IV-B-O3-2) |
| 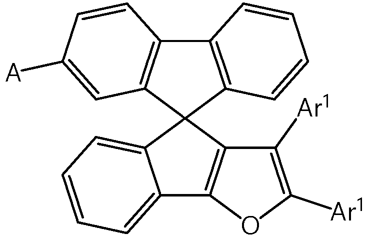 | 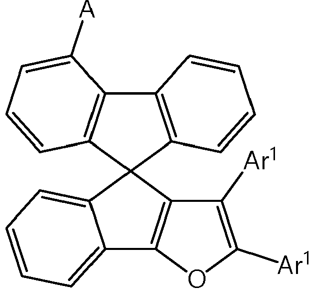 |
| Formel (IV-B-O4-1) | Formel (IV-B-O4-2), |

wobei die auftretenden Variablen definiert sind wie oben und bevorzugt ihren bevorzugten Ausführungsformen entsprechen, und wobei die freien Positionen an den Benzolringen jeweils mit Resten R¹ substituiert sind, welche bevorzugt gleich H sind.

Unter den oben genannten Formeln sind die Formeln (II-B-S3-1), (II-B-S3-2), (II-B-S4-1) (II-B-S4-2), (II-B-O3-1), (II-B-O3-2), (II-B-O4-1) (II-B-O4-2), besonders bevorzugt.

Am stärksten bevorzugt entsprechen Verbindungen der Formel (I) in dieser Ausführungsform einer der Formeln (II-B-S1), (II-B-O1), (II-B-S2), (II-B-O2), (II-B-S3-1), (II-B-S3-2), (II-B-S4-1) (II-B-S4-2), (II-B-O3-1), (II-B-O3-2), (II-B-04-1) und (II-B-O4-2), wobei die Variablen in diesen Fällen bevorzugt ihren oben angegebenen bevorzugten Ausführungsformen entsprechen.

Besonders bevorzugt entspricht die anmeldungsgemäße Verbindung daher ebenso einer Formel (II-B-S1), (II-B-S2), (II-B-S3), (II-B-S4), (II-B-O1), (II-B-O2), (II-B-O3), (II-B-O4), (III-B-S1), (III-B-S2), (III-B-S3), (III-B-S4), (III-B-O1), (III-B-O2), (III-B-O3), (III-B-O4), (IV-B-S1), (IV-B-S2), (IV-B-S3), (IV-B-S4), (IV-B-O1), (IV-B-O2), (IV-B-O3), (IV-B-O4), (II-B-N1) und (II-B-N2), wobei für die auftretenden Variablen gilt:
Ar¹ ist bei jedem Auftreten gleich oder verschieden gewählt aus Phenyl, Biphenyl, Terphenyl, Quaterphenyl, Naphthyl, Fluorenyl, insbesondere 9,9'-Dimethylfluorenyl und 9,9'-Diphenylfluorenyl, Benzofluorenyl, Spirobifluorenyl, Indenofluorenyl, Indenocarbazolyl, Dibenzofuranyl, Dibenzothiophenyl, Benzofuranyl, Benzothiophenyl, benzokondensiertem Dibenzofuranyl, benzokondensiertem Dibenzothiophenyl, Naphthyl-substituiertem Phenyl, Fluorenyl-substituiertem Phenyl, Spirobifluorenyl-substituiertem Phenyl, Dibenzofuranyl-substituiertem Phenyl, Dibenzothiophenyl-substituiertem Phenyl, Carbazolyl-substituiertem Phenyl, Pyridyl-substituiertem Phenyl, Pyrimidyl-substituiertem Phenyl, Triazinyl-substituiertem Phenyl, wobei die genannten Gruppen jeweils mit Resten R² substituiert sind, und H;
R¹ ist bei jedem Auftreten gleich oder verschieden gewählt aus H, D, Si(R⁵)₃, geradkettigen Alkylgruppen mit 1 bis 20 C-Atomen, die deuteriert sein können, verzweigten oder cyclischen Alkylgruppen mit 3 bis 20 C-Atomen, die deuteriert sein können, aromatischen Ringsystemen mit 6 bis 40 aromatischen Ringatomen, die deuteriert sein können, und heteroaromatischen Ringsystemen mit 5 bis 40 aromatischen Ringatomen, die deuteriert sein können, wobei die genannten Alkylgruppen, die genannten aromatischen Ringsysteme und die genannten heteroaromatischen Ringsysteme jeweils mit Resten R⁵ substituiert sind, die bevorzugt gleich H sind;
R⁴ ist bei jedem Auftreten gleich oder verschieden gewählt ist aus geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen, wobei die genannten Alkylgruppen und die genannten aromatischen Ringsysteme und die genannten heteroaromatischen Ringsysteme jeweils mit Resten R¹ substituiert sind, und wobei ausgeschlossen ist, dass die beiden Reste R⁴ miteinander verknüpft sind und einen aliphatischen oder heteroaliphatischen Ring bilden;
A ist eine Einheit der Formel (A) 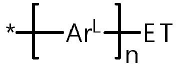
Formel (A), wobei die Variablen in Formel (A) wie folgt definiert sind:
   Ar^{L} ist bei jedem Auftreten gleich oder verschieden gewählt aus divalenten Gruppen abgeleitet von Benzol, Biphenyl, Terphenyl, Naphthalin, Fluoren, Indenofluoren, Indenocarbazol, Spirobifluoren, Dibenzofuran und Dibenzothiophen, die jeweils mit Resten R² substituiert sind;
   ET ist bei jedem Auftreten gleich oder verschieden gewählt aus Gruppen abgeleitet von Pyridin, Pyrazin, Pyrimidin, Pyridazin, 1,2,4-Triazin, 1,3,5-Triazin, Chinolin, Isochinolin, Chinoxalin, Chinazolin, Pyrazol, Imidazol, Benzimidazol, Thiazol, Benzothiazol, Oxazol, Oxadiazol und Benzooxazol, die jeweils mit Gruppen Ar² substituiert sind;
   Ar² ist bei jedem Auftreten gleich oder verschieden gewählt aus Phenyl, Biphenyl, Terphenyl, Quaterphenyl, Naphthyl, Fluorenyl, insbesondere 9,9'-Dimethylfluorenyl und 9,9'-Diphenylfluorenyl, Benzofluorenyl, Spirobifluorenyl, Indenofluorenyl, Indenocarbazolyl, Dibenzofuranyl, Dibenzothiophenyl, Carbazolyl, Benzofuranyl, Benzothiophenyl, benzokondensiertem Dibenzofuranyl, benzokondensiertem Dibenzothiophenyl, Naphthyl-substituiertem Phenyl, Fluorenyl-substituiertem Phenyl, Spirobifluorenyl-substituiertem Phenyl, Dibenzofuranyl-substituiertem Phenyl, Dibenzothiophenyl-substituiertem Phenyl, Carbazolyl-substituiertem Phenyl, Pyridyl-substituiertem Phenyl, Pyrimidyl-substituiertem Phenyl, Triazinyl-substituiertem Phenyl, wobei die genannten Gruppen jeweils mit Resten R³ substituiert sind, und H;
   n = 1 oder 0;
   und die weiteren Variablen sind definiert gemäß ihrer oben angegebenen breitesten Ausführungsform, bevorzugt den oben angegebenen bevorzugten Ausführungsformen.

Bevorzugte Verbindungen der Formel (I) gemäß dieser Ausführungsform sind in folgender Tabelle 1 gezeigt:

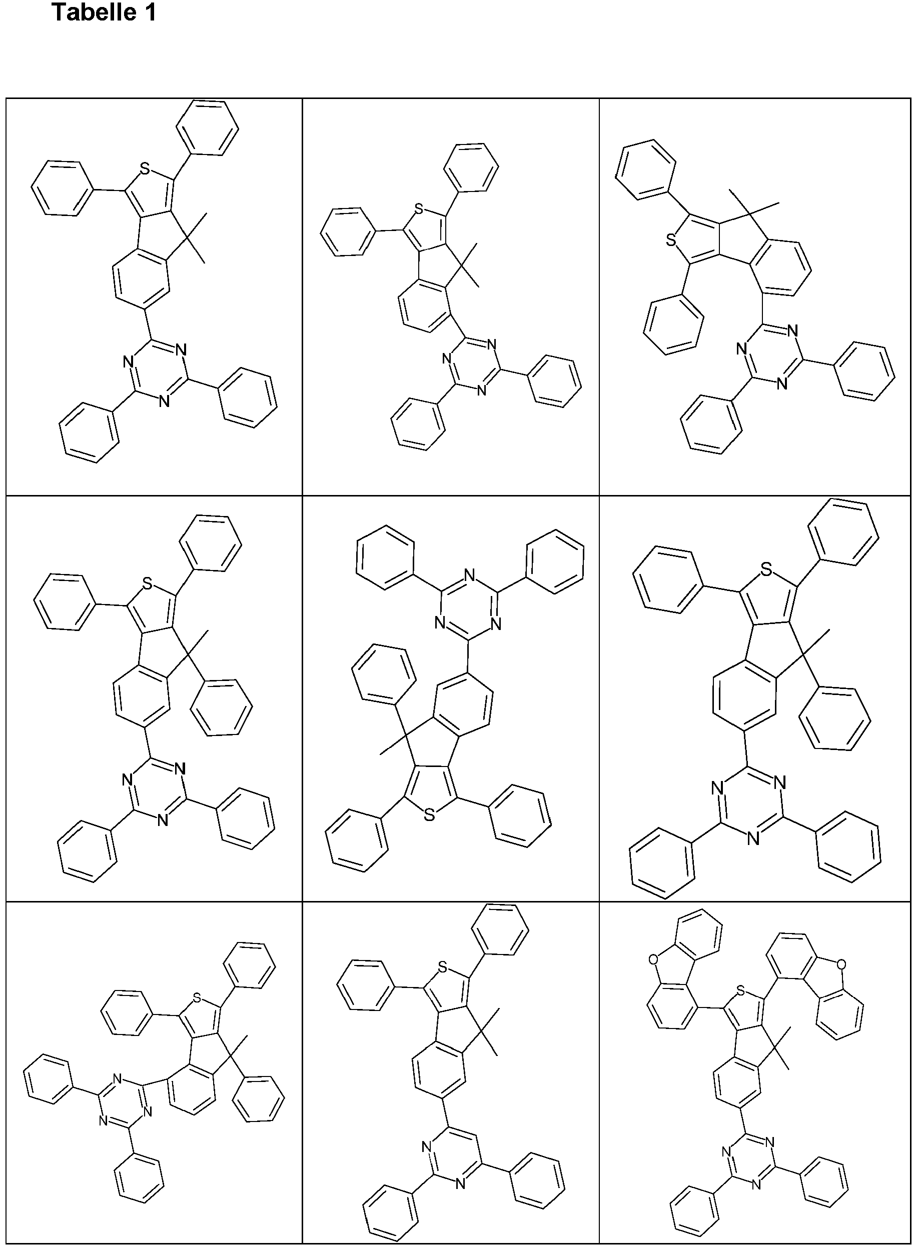

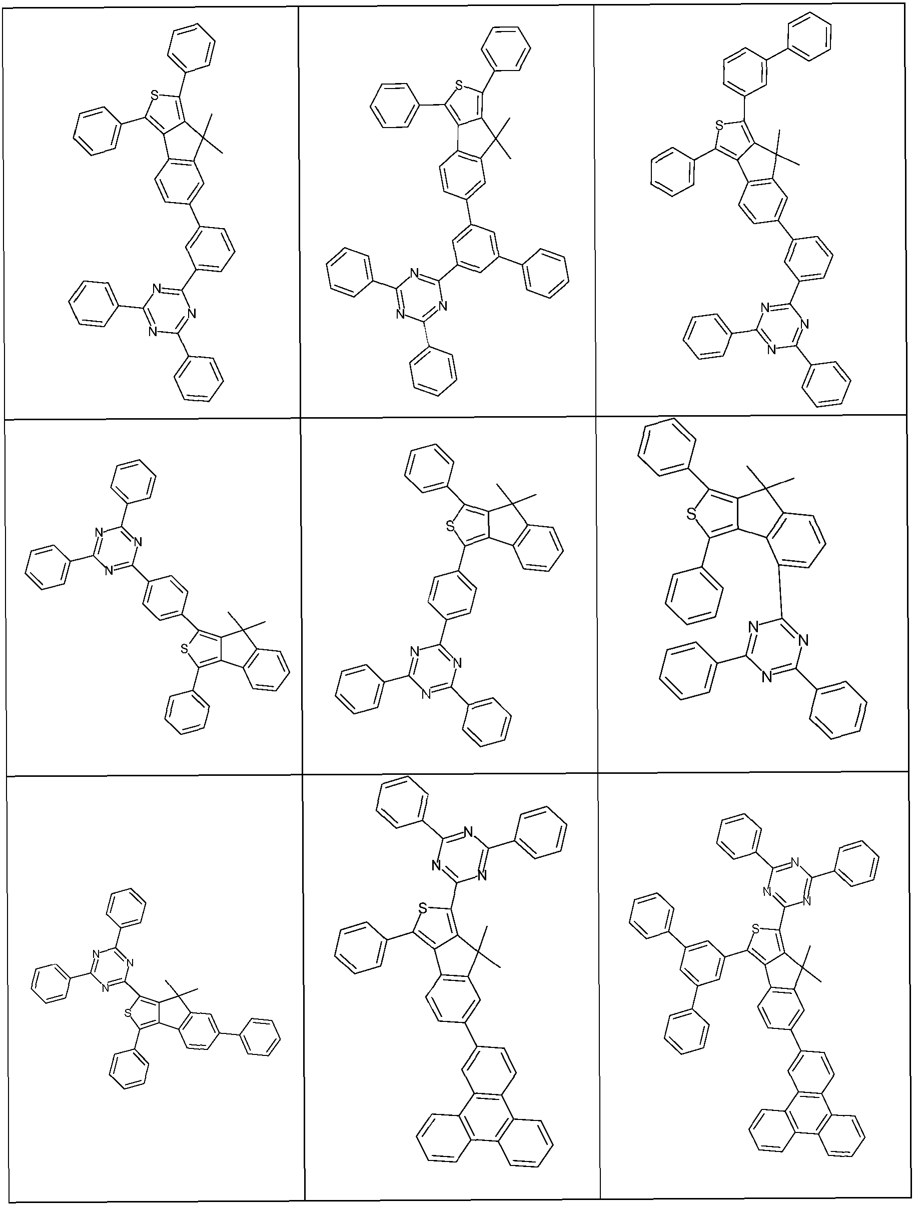

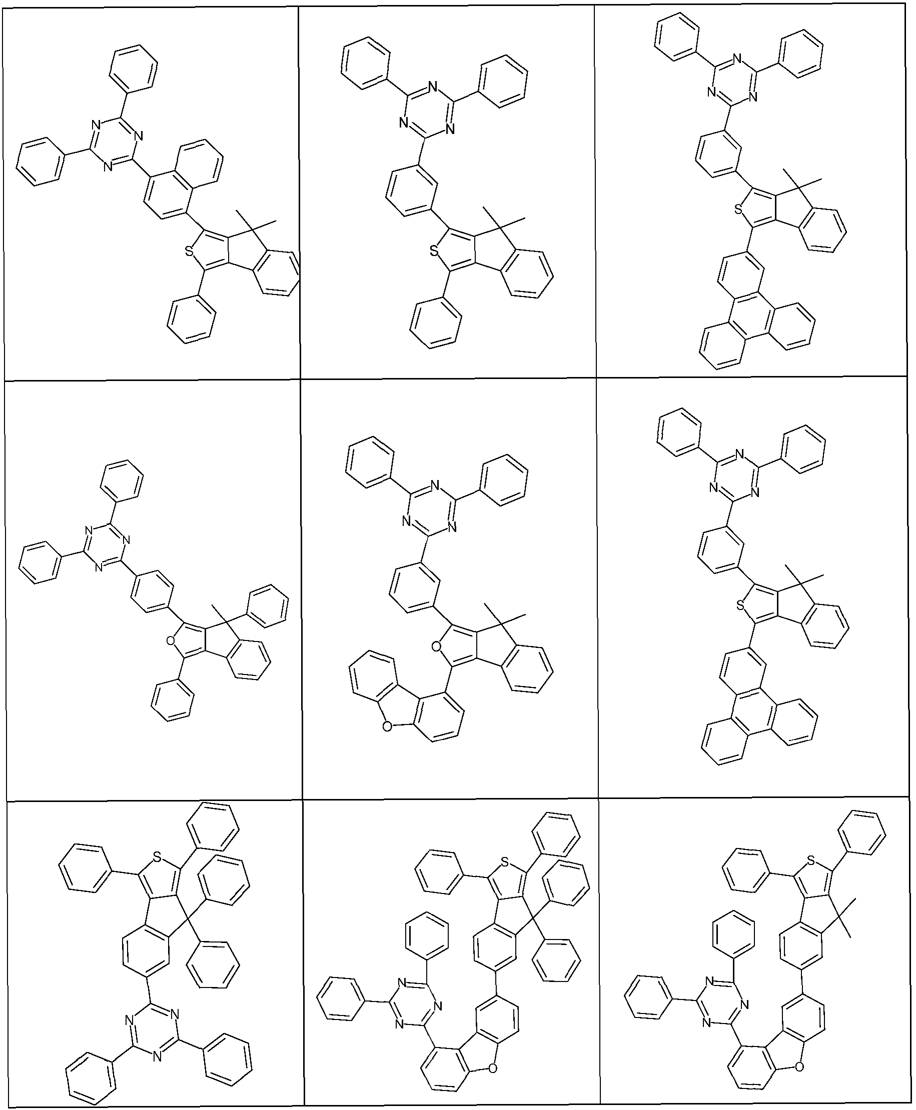

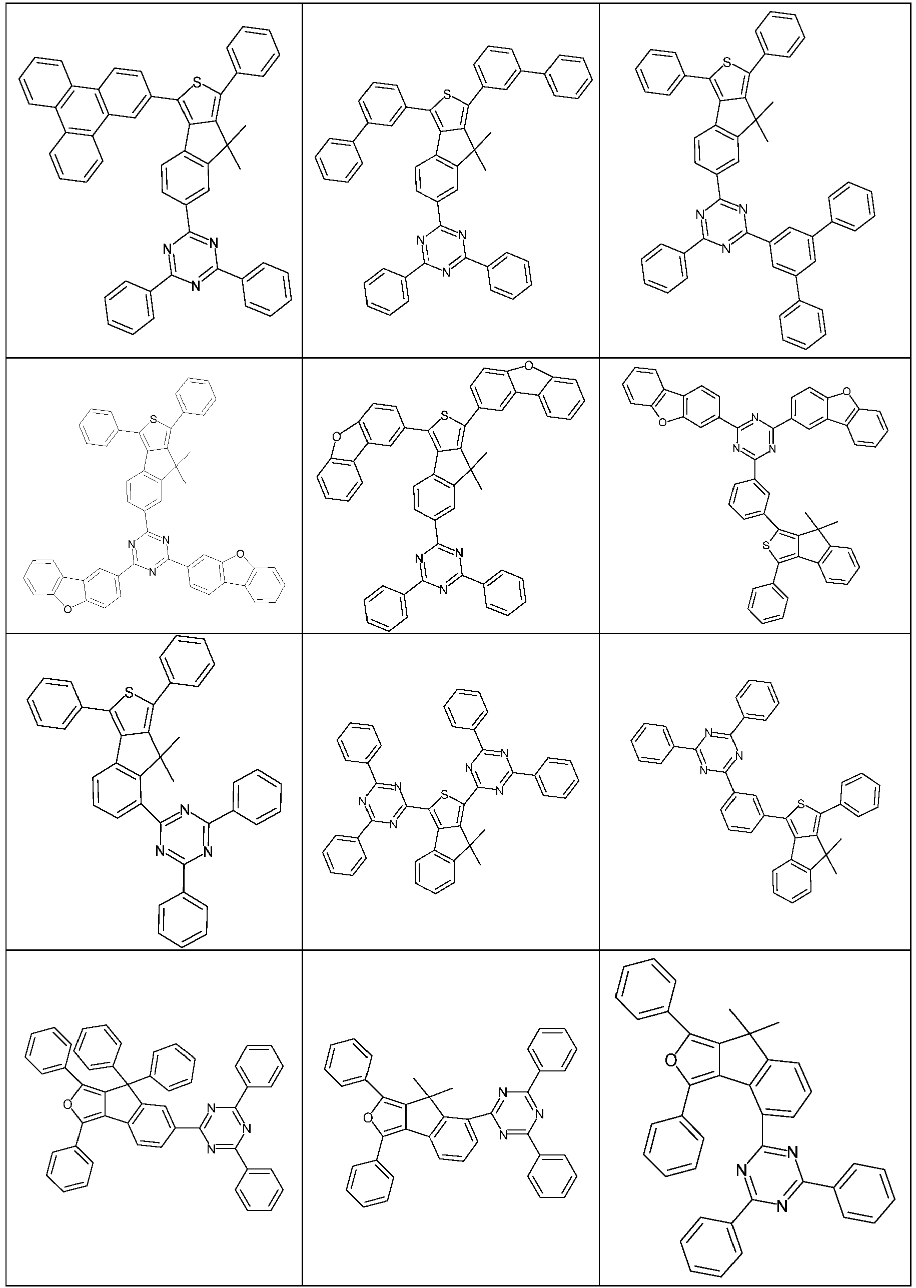

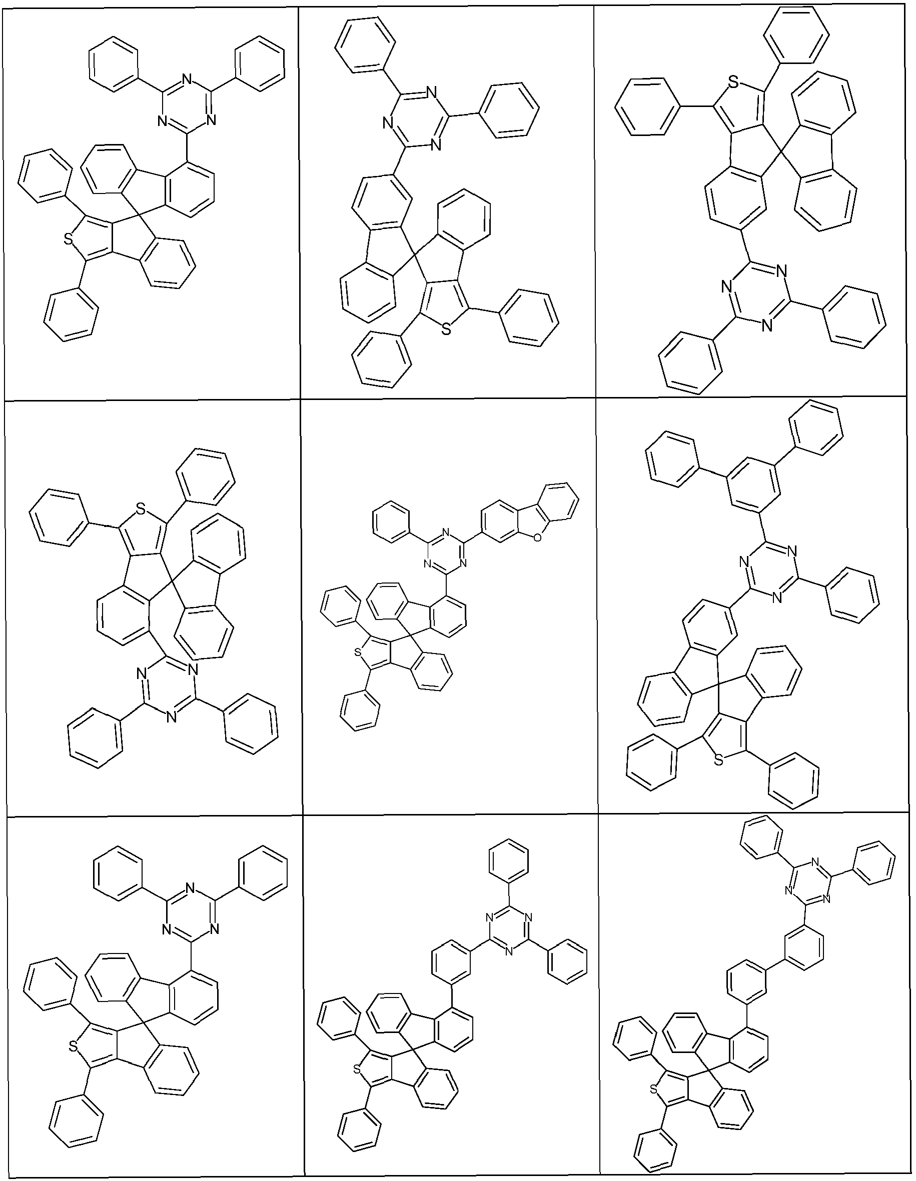

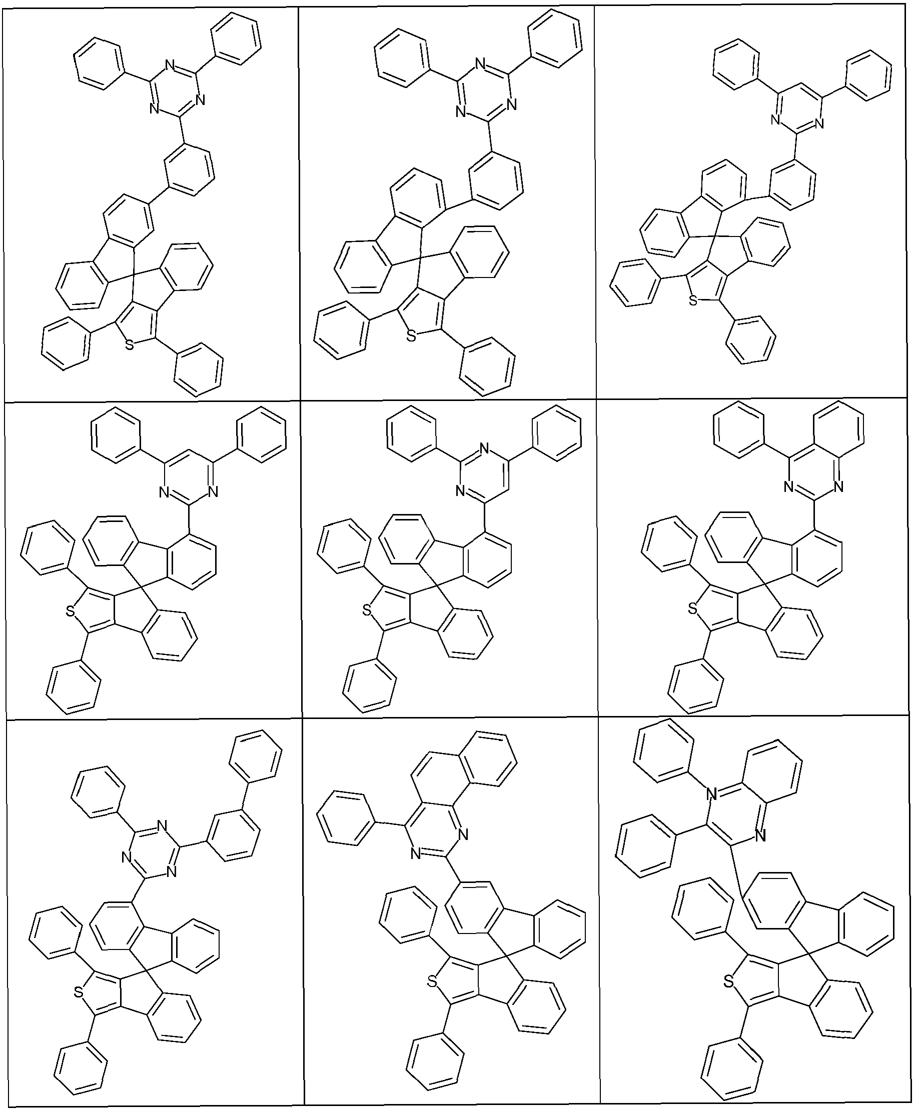

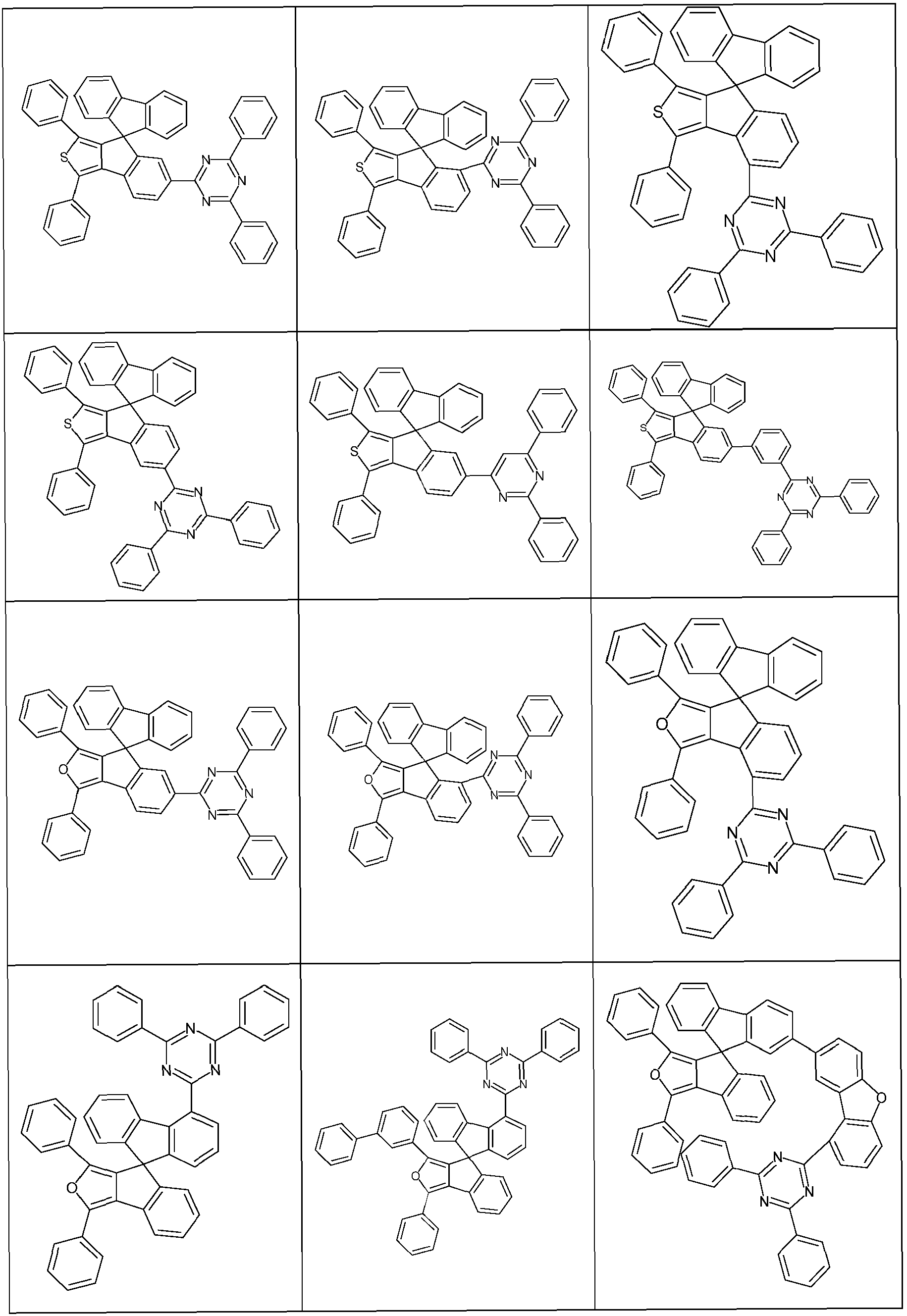

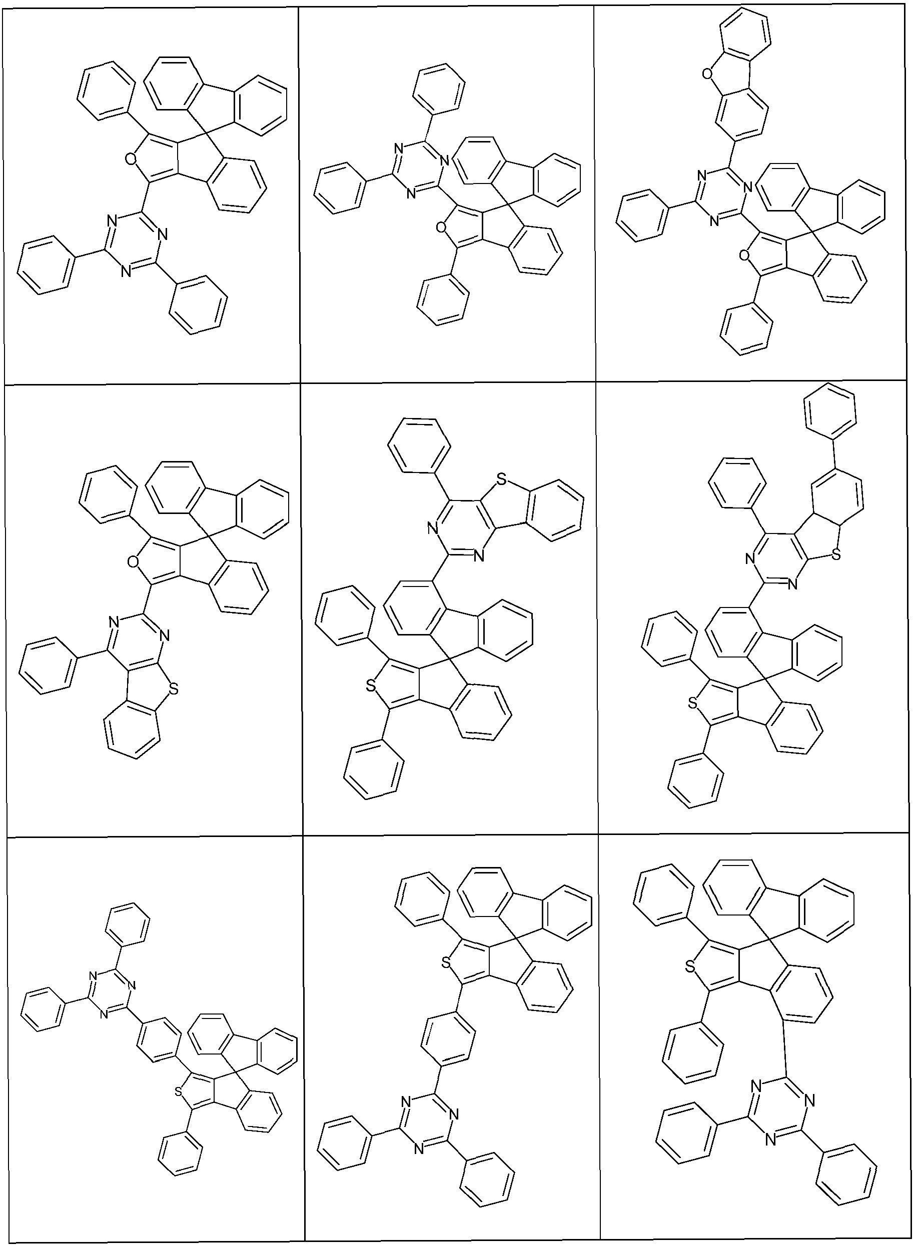

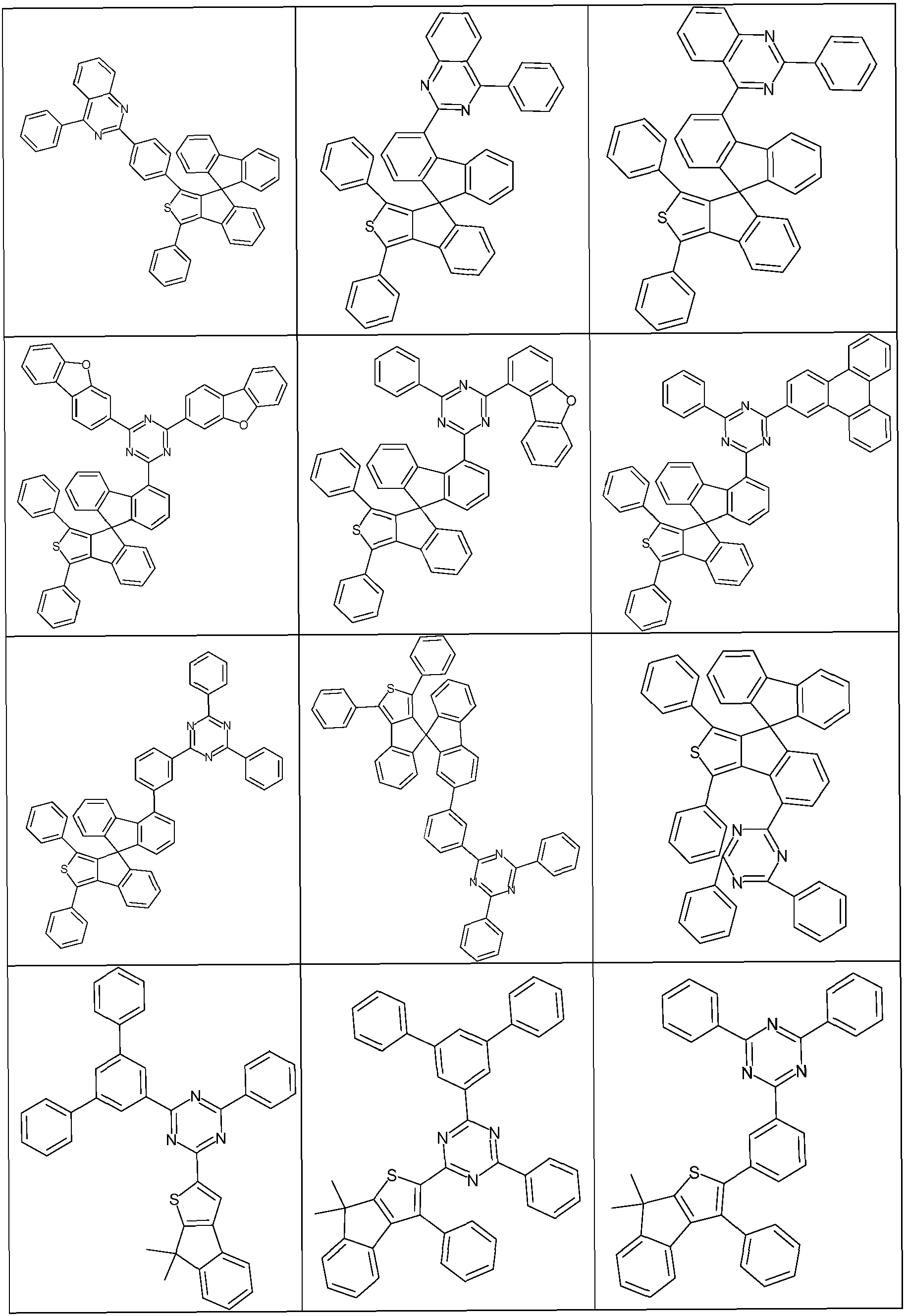

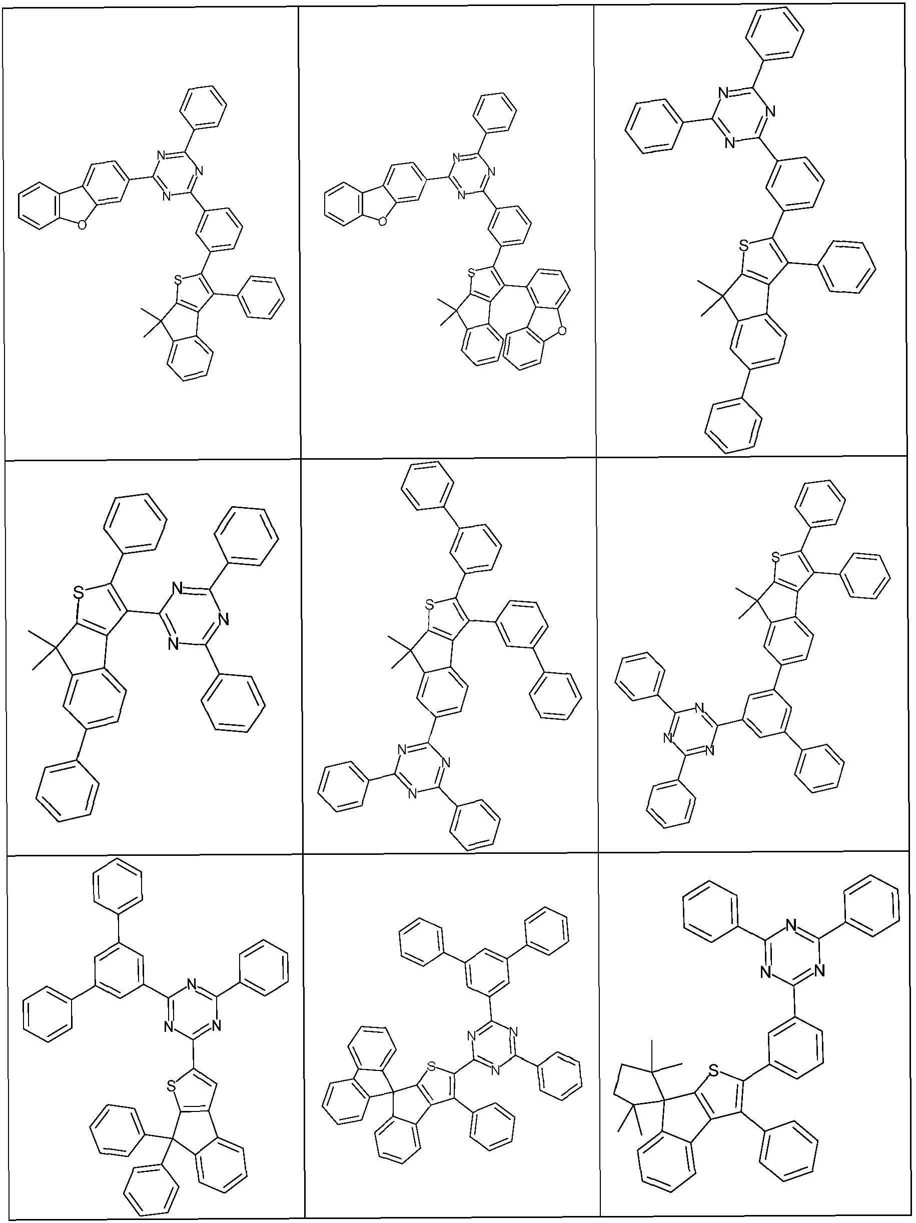

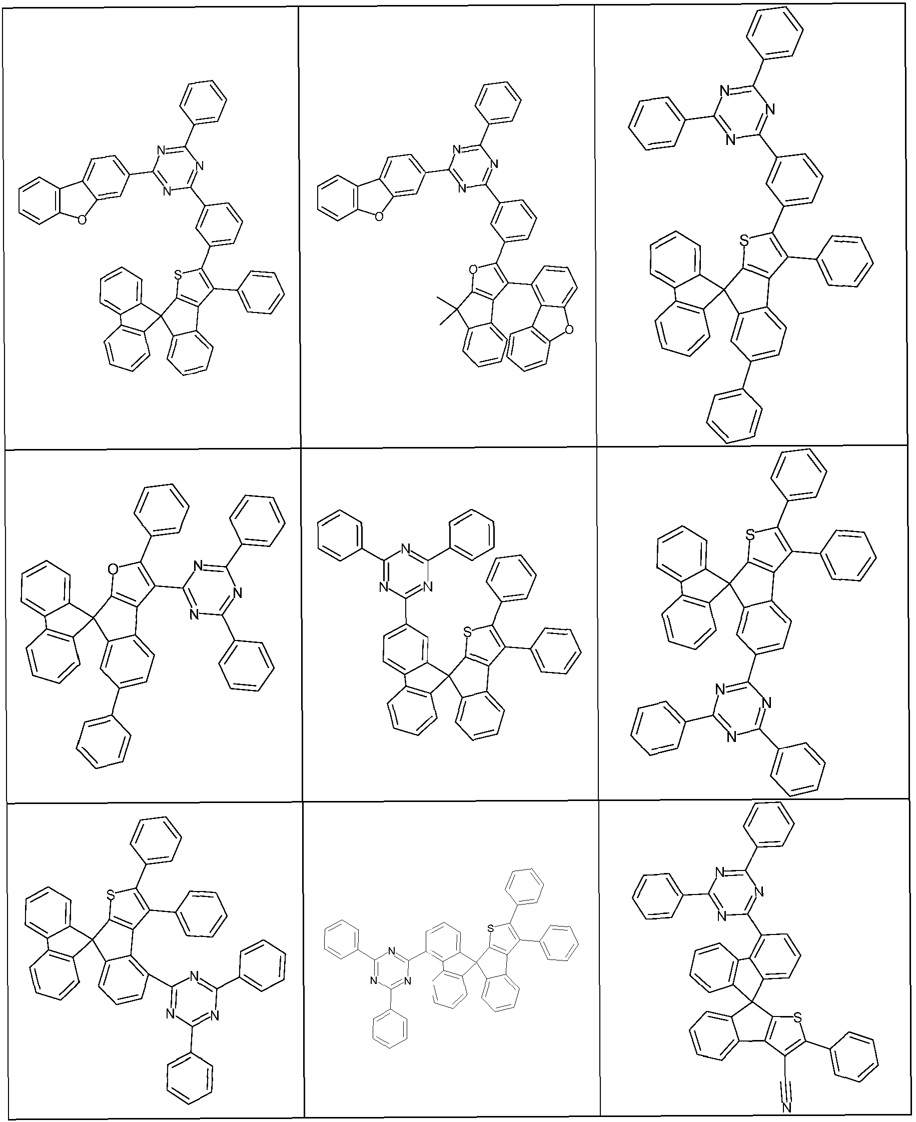

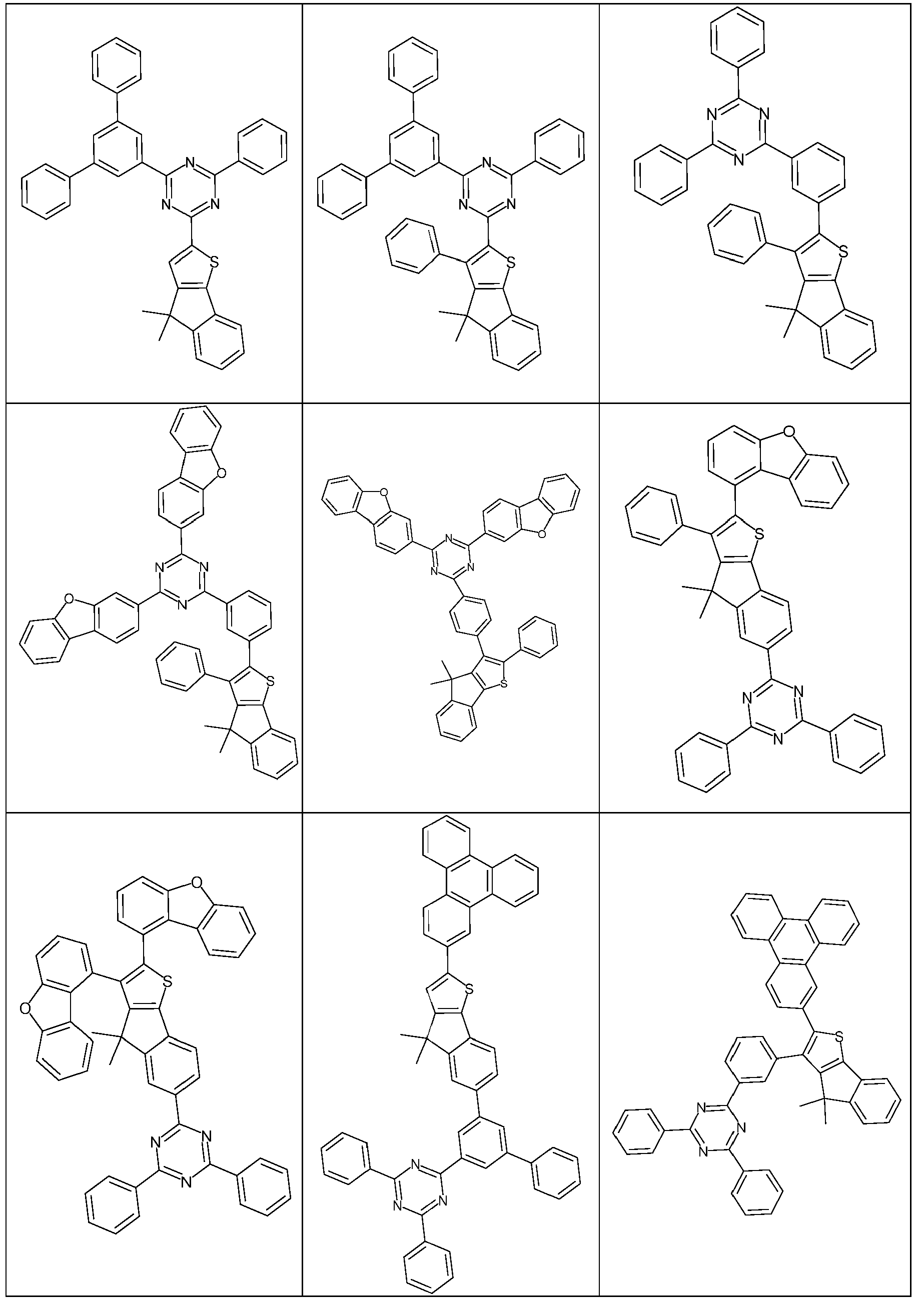

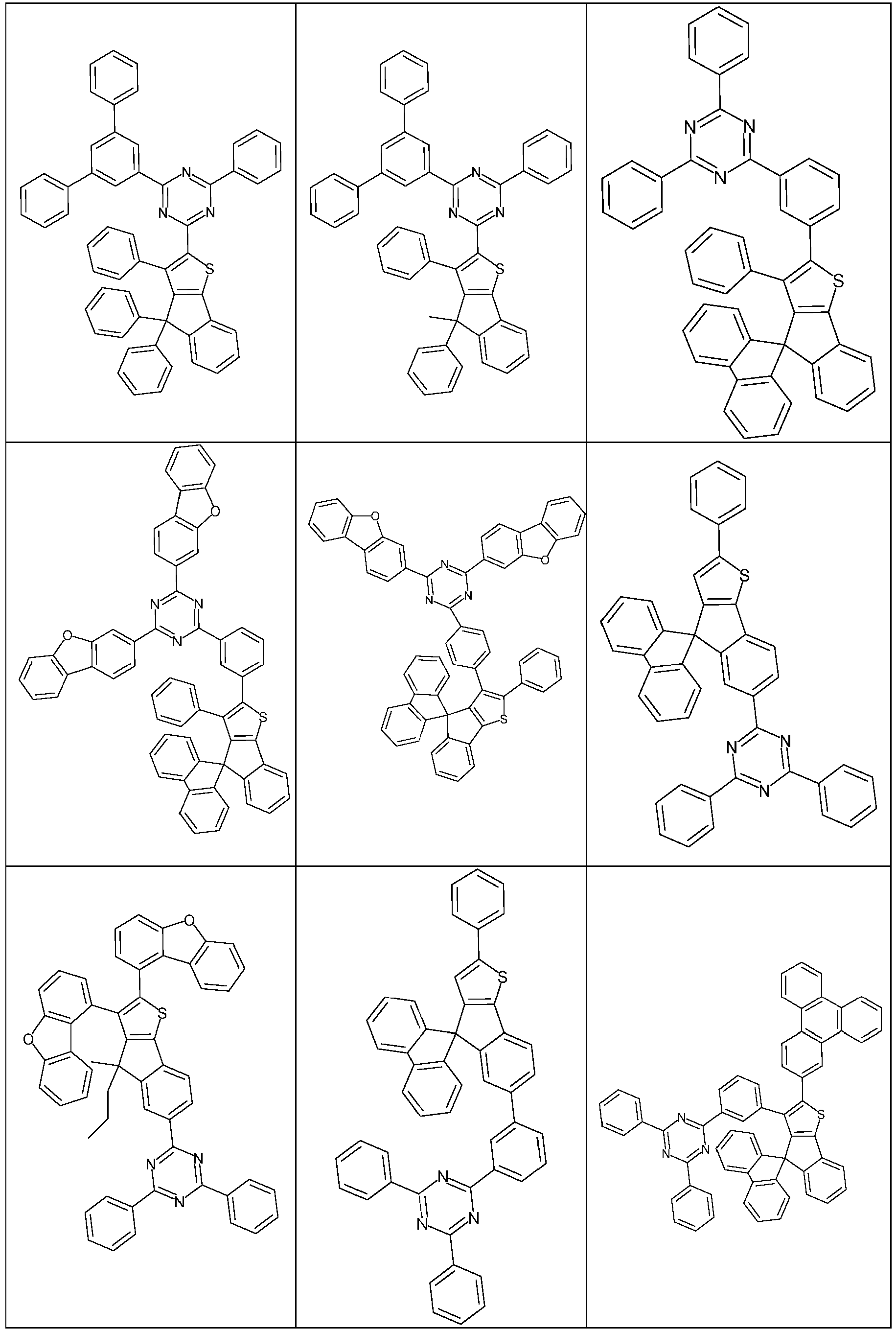

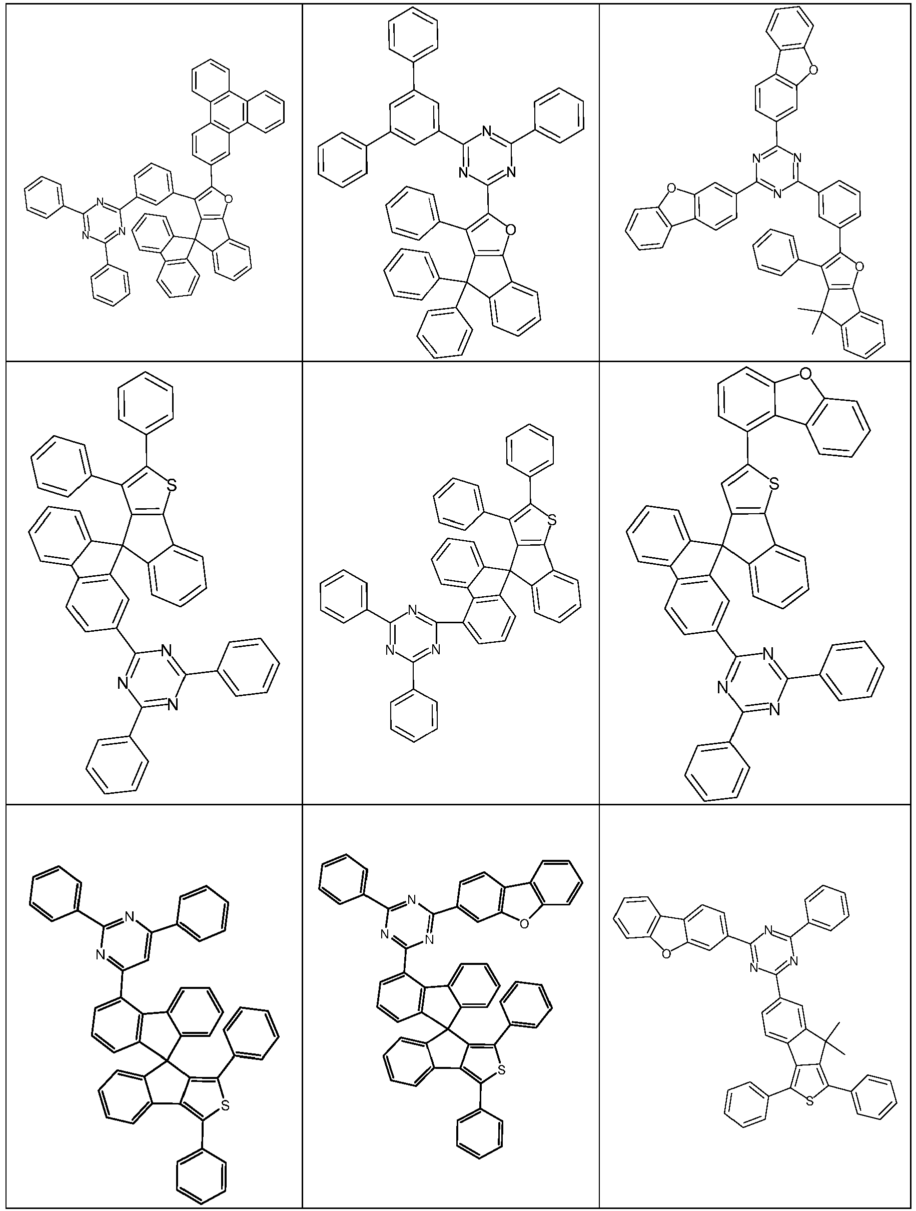

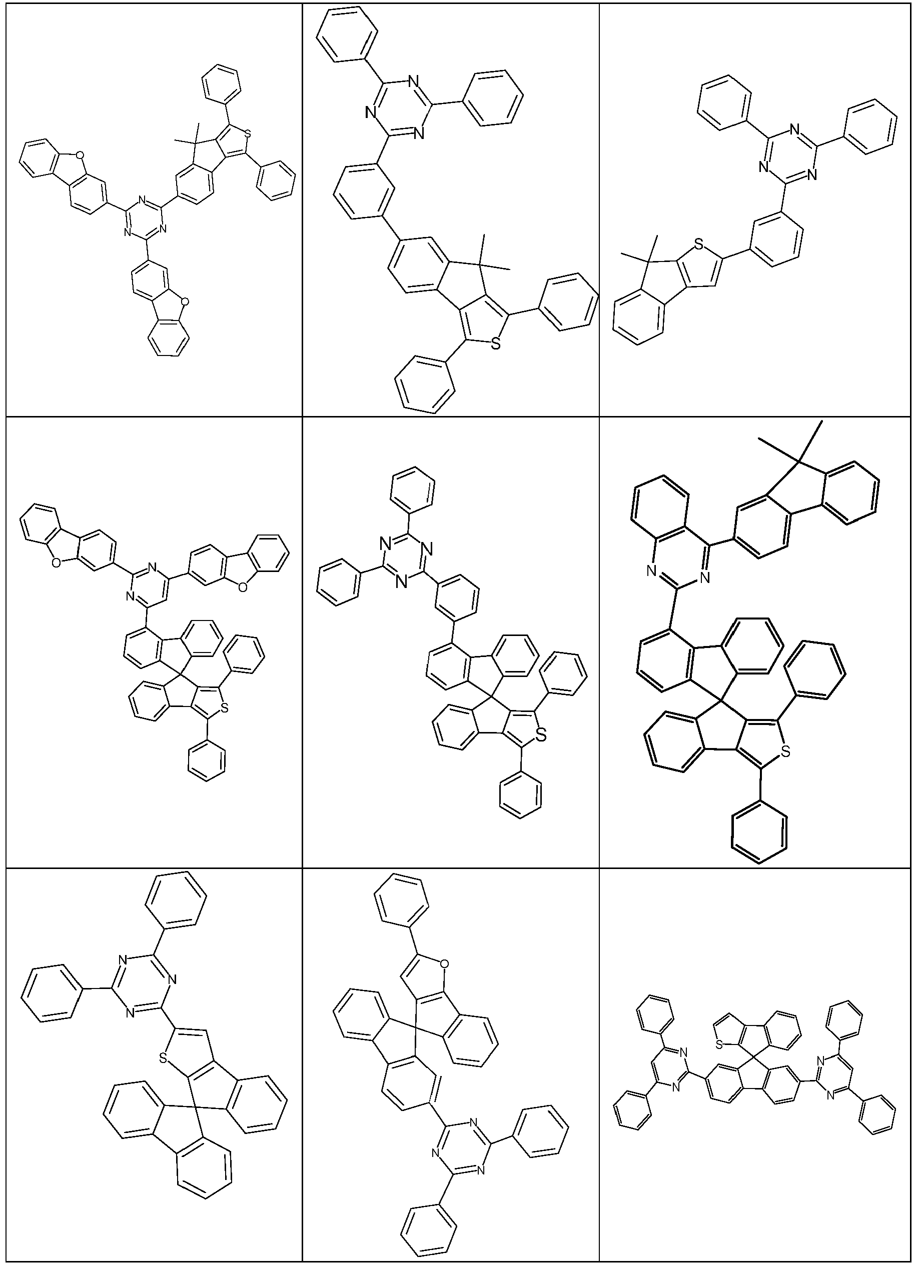

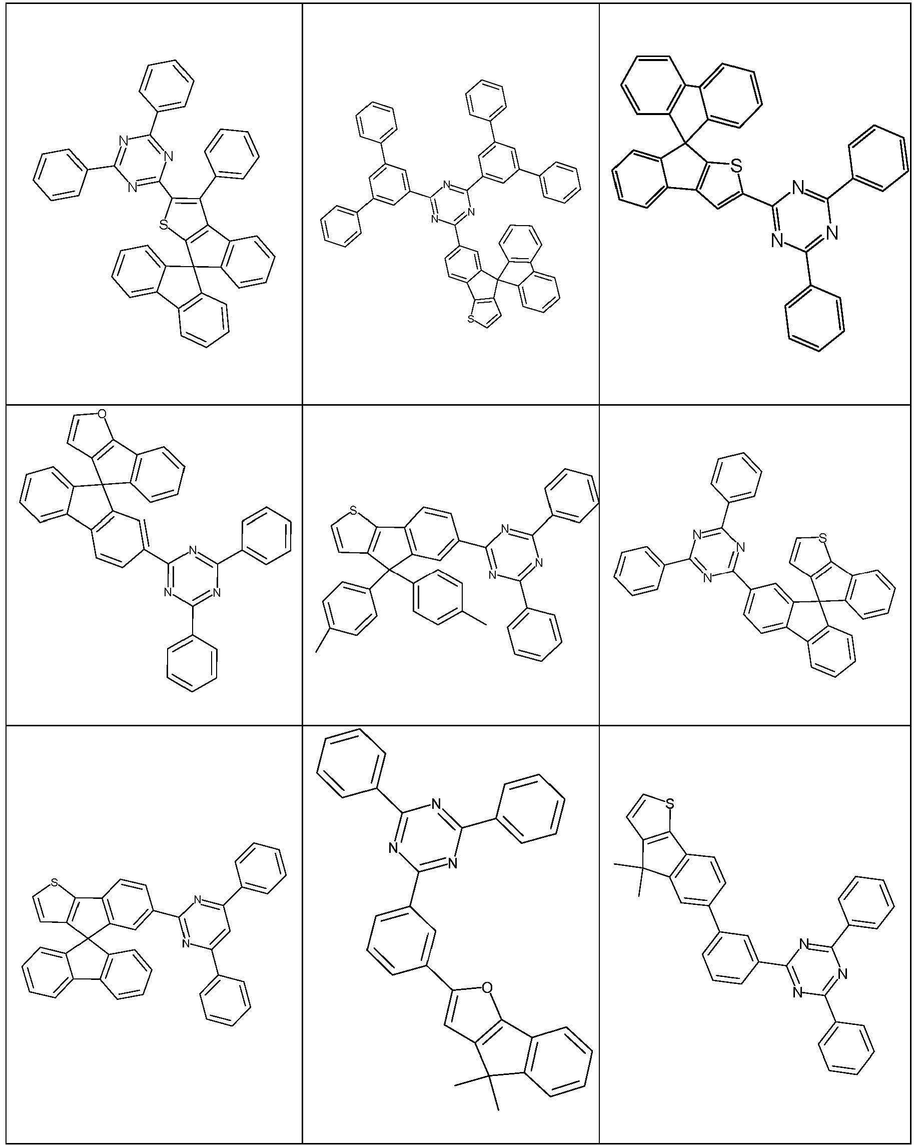

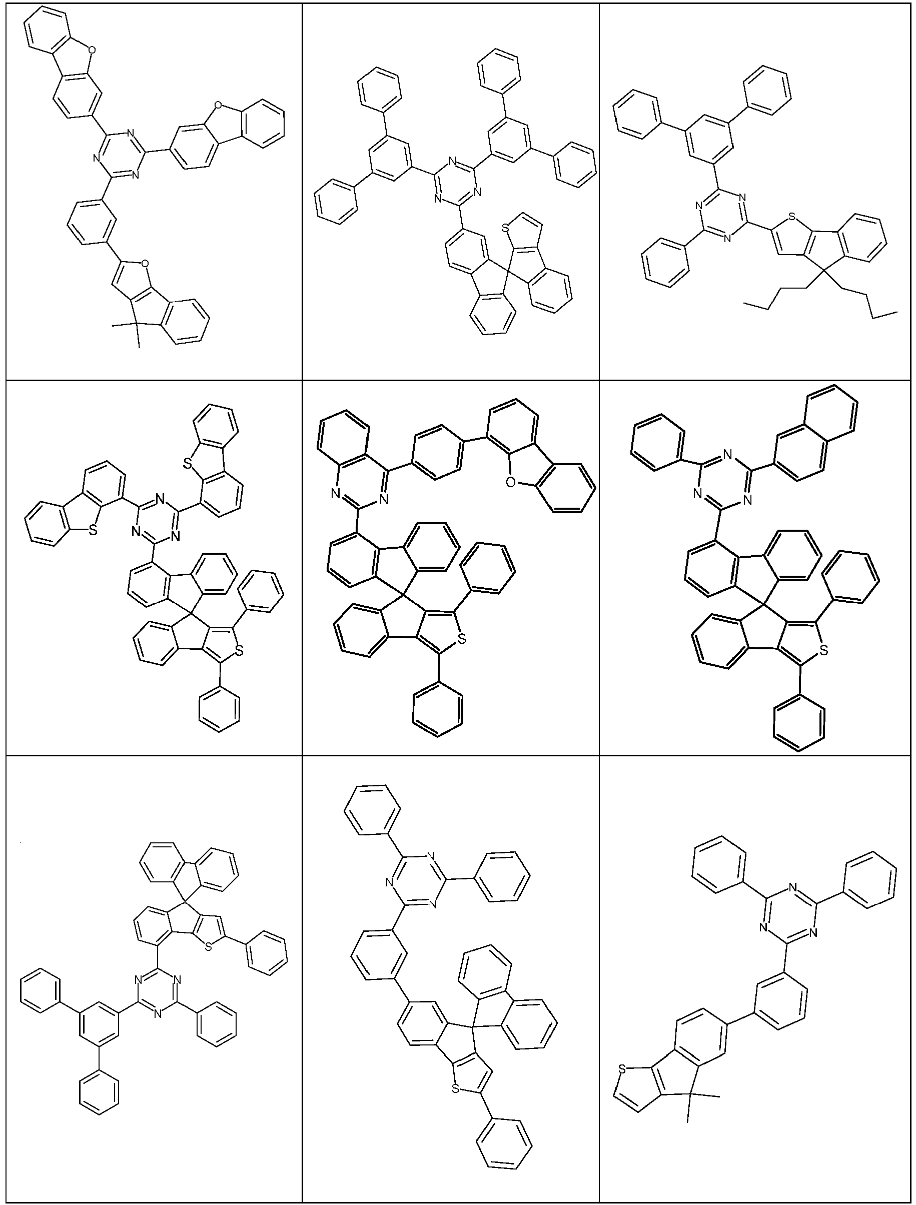

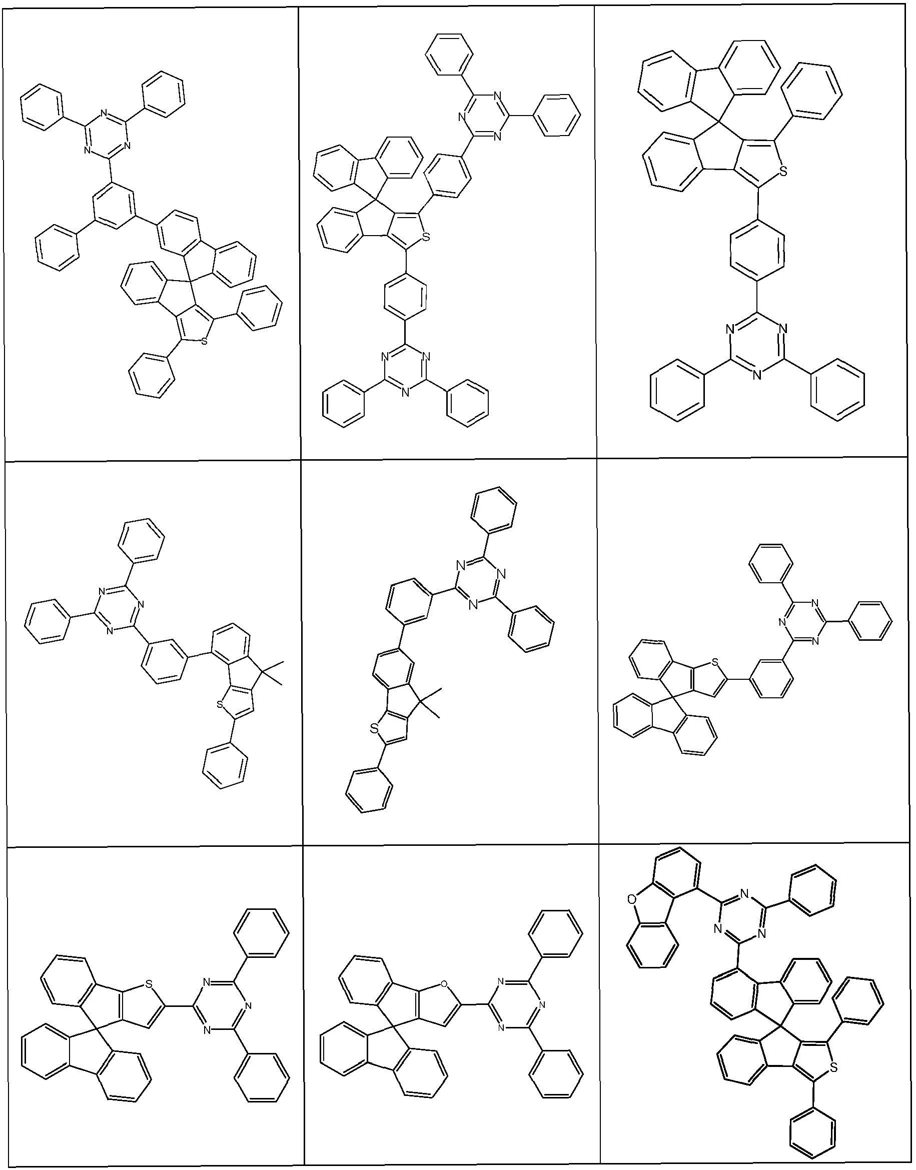

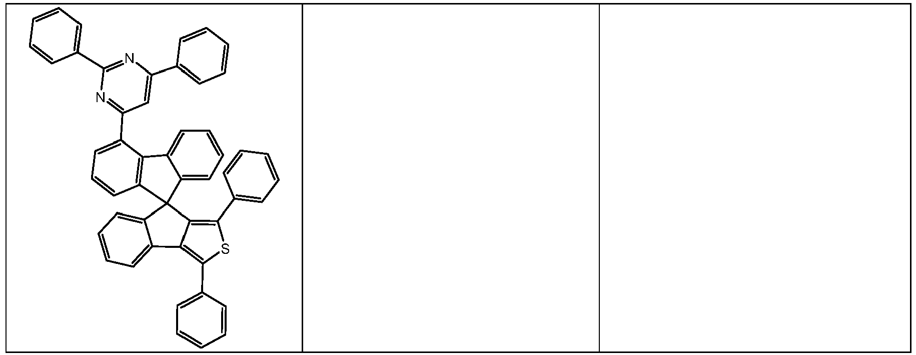

Die anmeldungsgemäßen Verbindungen können mittels dem Fachmann bekannten Syntheseschritten der organischen Chemie hergestellt werden, beispielsweise mittels Metallierung, Addition von Nukleophilen an Carbonylgruppen, Suzuki-Reaktion und Hartwig-Buchwald-Reaktion.

Bevorzugte Verfahren zur Herstellung von anmeldungsgemäßen Verbindungen sind im Folgenden gezeigt. Die Verfahren sind beispielhaft und nicht beschränkend zu verstehen. Der Fachmann kann von den gezeigten beispielhaften Verfahren abweichen und Änderungen im Rahmen seines allgemeinen Fachwissens durchführen, wenn dies technisch vorteilhaft ist, um zu den anmeldungsgemäßen Verbindungen zu gelangen.

Gemäß einem bevorzugten Verfahren wird in einem ersten Schritt in einer Suzuki-Reaktion ein heteroaromatischer Fünfring (besonders bevorzugt Pyrrol, Furan oder Thiophen), der eine Carbonsäuregruppe oder eine Carbonsäureestergruppe trägt, an einen Benzolring gekuppelt (Schema 1). Alternativ dazu kann die Carbonsäure- oder Carbonsäureestergruppe auch an dem Benzolring gebunden sein.

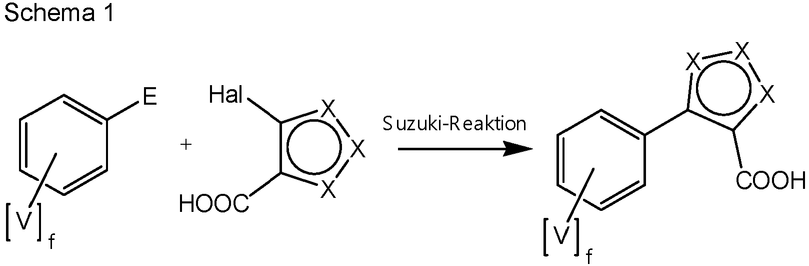

Die Variablen sind dabei wie folgt definiert:
V ist bei jedem Auftreten gleich oder verschieden gewählt aus reaktiven Gruppen, bevorzugt Cl, Br oder I;
X ist bei jedem Auftreten gleich oder verschieden gewählt aus S, O, NAr⁰, C-H und C-V, wobei Ar⁰ wie in Formel (I) definiert ist und bevorzugt genau zwei X, besonders bevorzugt genau ein X gewählt ist aus S, O und NAr⁰;
Hal ist Cl, Br oder I;
E ist eine reaktive Gruppe, bevorzugt eine Boronsäuregruppe oder eine Boronestergruppe; und
f ist 0, 1 oder 2;
wobei die Verbindungen an den freien Positionen am Benzolring jeweils mit einem Rest R¹, wie oben für Formel (I) definiert, substituiert sind, und wobei mindestens eine Gruppe V vorhanden ist.

Aus den gemäß Schema 1 erhaltenen Verbindungen werden durch Ringschlussreaktion unter sauren Bedingungen Intermediate gemäß einer Formel (Int-1) hergestellt (Schema 2).

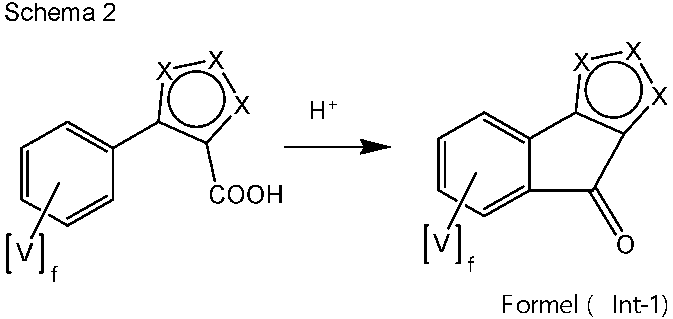

Die Variablen sind dabei definiert wie für Schema 1
Zur Herstellung der anmeldungsgemäße Fluoren-Derivate gemäß Formel (I) bzw. Formel (I-A), in denen jeweils ein Benzolring gegen einen Heteroaryl-Fünfring (besonders bevorzugt Pyrrol-, Furan- oder Thiophenring) ausgetauscht ist, werden die Verbindungen der Formel (Int-1) zu Verbindungen der Formel (Int-2) reduziert, beispielsweise mittels Zugabe einer Lösung aus Hydarzinhydrat in Anwesenheit von Raney-Nickel als Katalysator. Die Verbindungen der Formel (Int-2) werden in einem folgenden Schritt durch Zugabe einer Organohalogenverbindungen oder eines Organometallreagenzes, bevorzugt Grignard-Reagenz, zu Intermediat-Verbindungen der Formel (Int-3) überführt (Schema 3).

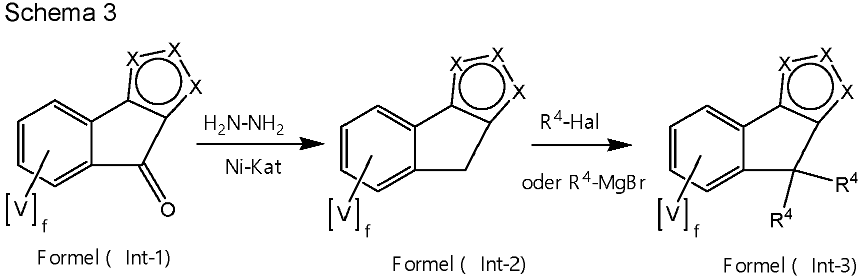

Die Variablen sind dabei definiert wie für Schema 1, wobei R⁴ wie oben für Formel (I-A) definiert ist.

Zur Einführung der mindestens einen Gruppe A an den Heteroaryl-Fünfring und/oder den Bezolring, wird die Verbindung der Formel (Int-3) in einer Suzuki-Reaktion, nach Überführung der entsprechenden Gruppe(n) V in eine oder mehrere reaktive Gruppen E, die bevorzugt eine Boronsäuregruppe oder eine Boronestergruppe sind, an mindestens eine Gruppe A (Hal-A) gekoppelt. Zuvor können gegbenfalls auch ein oder zwei Gruppen Ar¹, ebenfalls bevorzugt durch Suzuki-Reaktion, an den Heteroaryl-Fünfring (wenn X gleich C-V entspricht) gebunden werden. Dadurch werden Verbindungen gemäß Formel (I-A) erhalten, wie in Schema 4 gezeigt ist:

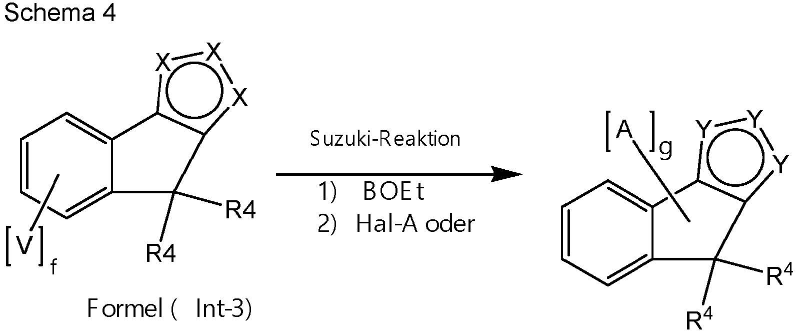

Die Variablen sind wie oben für Formel (I-A) definiert, wobei Index g 1 oder 2, bevorzugt 1 ist und wobei die Formeln an den freien Positionen an dem Benzolring jeweils mit einem Rest R¹ substituiert sind.

Zu Herstellung der anmedlungsgemäßen Spirobifluoren-Derivate gemäß Formel (I) bzw. Formel (I-B), in denen jeweils ein Benzolring gegen einen Heteroaryl-Fünfring (besonders bevorzugt Pyrrol-, Furan- oder Thiophenring) ausgetauscht ist, werden die Verbindungen der Formel (Int-1) in einem folgenden Schritt umgesetzt z.B. durch Addition von orthohalogeniertem oder ortho-metalliertem Bisaryl an die Carbonylfunktion der Verbindungen der Formel (Int-1) und anschließendem säurekatalysierten Ringschluss, wodurch Verbindungen der Formeln (Int-4) erhalten werden (Schema 5).

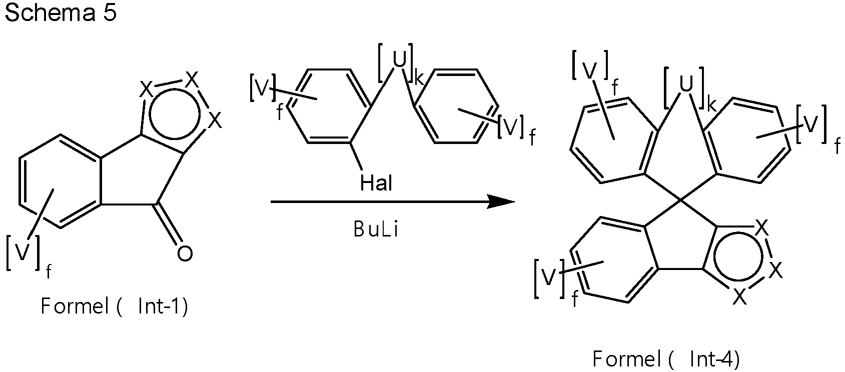

Die Variablen sind bei den Verbindungen der Formel (Int-4) definiert wie oben, wobei Index f bevorzugt gleich 0 oder 1 ist und Index k gleich 0 oder 1 ist, bevorzugt gleich 0, und wobei mindestens eine Gruppe V vorhanden ist, und wobei die Formeln an den freien Positionen an den Benzolringen jeweils mit einem Rest R¹ substituiert sind.

Zur Einführung der mindestens einen Gruppe A an den Heteroaryl-Fünfring und/oder die Bezolringe wird die Verbindung der Formel (Int-4) in einer Suzuki-Reaktion, nach Überführung der entsprechenden Gruppe(n) V in eine oder mehrere reaktive Gruppen E, die bevorzugt eine Boronsäuregruppe oder eine Boronestergruppe sind, an mindestens eine Gruppe A (Hal-A) gekoppelt. Zuvor können gegbenfalls auch ein oder zwei Gruppen Ar¹, ebenfalls bevorzugt durch Suzuki-Reaktion, an den Heteroaryl-Fünfring (wenn X gleich C-V entspricht) gebunden werden. Dadurch werden Verbindungen gemäß Formel (I-B) erhalten, wie in Schema 6 gezeigt ist:

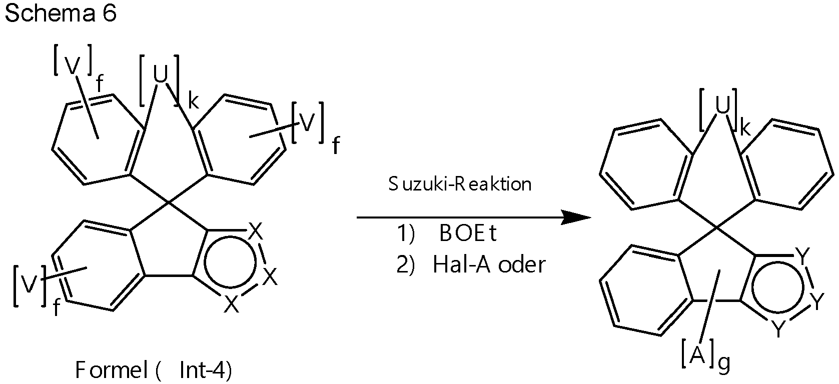

Die Variablen sind wie oben für Formel (I-B) definiert, wobei Index g 1 oder 2, bevorzugt 1 ist und wobei die Formeln an den freien Positionen an den Benzolringen jeweils mit einem Rest R¹ substituiert sind.

Wie in Schema 4 bzw. 6 oben gezeigt ist, können die Intermediate der Formeln (Int-3) bzw. (Int-4) über eine Suzuki-Kupplung mit einer Heteroarylverbindung entsprechend der Gruppe A (Hal-A) umgesetzt werden. Dadurch werden Verbindungen gemäß Formel (I) erhalten.

Gegenstand der vorliegenden Anmeldung ist damit ein Verfahren zur Herstellung eines Fluoren-Derivats der Formel (I), insbesondere der Formeln (I-A), oder eines Spirobifluoren-Derivats der Formel (I), insbesondere der Formel (I-B), dadurch gekennzeichnet, dass in einem ersten Schritt eine Suzuki-Kupplung durchgeführt wird, in der ein heteroaromatischer Fünfring an einen Benzolring gekuppelt wird, wobei der heteroaromatische Fünfring oder der Benzolring eine Carbonsäuregruppe trägt; dass in einem zweiten Schritt durch Ringschlussreaktion unter sauren Bedingungen die Carbonsäuregruppe cyclisiert zu einer verbrückenden Carbonylgruppe zwischen dem heteroaromatischen Fünfring und dem Benzolring; dass in einem dritten Schritt die Carbonylgruppe reduziert wird und unter Zugabe einer Organohalogenverbindungen oder eines Organometallreagenzes eine substituierte Methylenbrücke zwischen dem heteroaromatischen Fünfring und dem Benzolring erhalten wird, und dass in einem vierten Schritt eine Suzuki-Kupplung mit einer Heteroarylverbindung durchgeführt wird, wobei eine Verbindung der Formel (I), insbesondere der Formel (I-A) erhalten wird; oder dass in einem dritten Schritt ein ortho-halogeniertes oder ortho-metalliertes Bisaryl addiert wird und eine weitere Ringschlussreaktion durchgeführt wird; und dass in einem vierten Schritt eine Suzuki-Kupplung mit einer Heteroarylverbindung durchgeführt wird, wobei eine Verbindung der Formel (I), insbesondere der Formel (I-B) erhalten wird.

Die oben beschriebenen erfindungsgemäßen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, Polymere oder Dendrimere enthaltend eine oder mehrere Fluoren-Derivate bzw. Spirobifluoren-Derivate gemäß Formel (I), bzw. Formel (I-A) oder (I-B), wobei die Bindung(en) zum Polymer, Oligomer oder Dendrimer an beliebigen, in den Formel (I) bzw. (I-A) oder (I-B) mit R⁰, R¹, R², R³ oder R⁴ substituierten Positionen lokalisiert sein können. Je nach Verknüpfung der Verbindung gemäß Formel (I), bzw. Formel (I-A) oder (I-B), ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nichtkonjugiert sein. Die erfindungsgemäßen Oligomere oder Polymere können linear, verzweigt oder dendritisch sein. In den linear verknüpften Strukturen können die Einheiten gemäß Formel (I) bzw. (I-A) oder (I-B) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formel (I) bzw. (I-A) oder (I-B) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (I) bzw. (I-A) oder (I-B) in Oligomeren, Dendrimeren und Polymeren gelten dieselben Bevorzugungen wie oben für Verbindungen gemäß Formel (I) bzw. (I-A) oder (I-B) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen, Spirobifluorenen, Paraphenylenen, Carbazolen, Thiophenen, Dihydrophenanthrenen, cis- und trans-Indenofluorenen, Ketonen, Phenanthrenen oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine oder phosphoreszierende Metallkomplexe, und/oder Ladungstransporteinheiten, insbesondere solche basierend auf Triarylaminen.

Die erfindungsgemäßen Polymere, Oligomere und Dendrimere weisen vorteilhafte Eigenschaften, insbesondere hohe Lebensdauern, hohe Effizienzen und gute Farbkoordinaten auf.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (I) bzw. (I-A) oder (I-B) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
(A) SUZUKI-Polymerisation;
(B) YAMAMOTO-Polymerisation;
(C) STILLE-Polymerisation; und
(D) **HARTWIG-BUCHWALD-Polymerisation.**

Wie die Polymerisation nach diesen Methoden durchgeführt werden kann und wie die Polymere dann vom Reaktionsmedium abgetrennt und aufgereinigt werden können, ist dem Fachmann bekannt und in der Literatur im Detail beschrieben.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, alpha-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethyl-ether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Gegenstand der Erfindung ist daher weiterhin eine Formulierung, insbesondere eine Lösung, Dispersion oder Emulsion, enthaltend mindestens ein Fluoren-Derivat oder Spirobifluoren-Derivat gemäß Formel (I) bzw. (I-A) oder (I-B), oder mindestens ein Polymer, Oligomer oder Dendrimer enthaltend mindestens eine Einheit gemäß Formel (I) bzw. (I-A) oder (I-B) sowie mindestens ein Lösungsmittel, bevorzugt ein organisches Lösungsmittel. Wie solche Lösungen hergestellt werden können, ist dem Fachmann bekannt.

Die erfindungsgemäßen Verbindungen oder die erfindungsgemäßen Polymere, Oligomere und Dendrimere können des Weiteren in einer Zusammensetzung mit mindestens einer weitere Verbindung, insbesondere mindestens einer weiteren organischen oder anorganischen Verbindung, die ebenfalls in der elektronischen Vorrichtung eingesetzt wird, vorliegen, beispielsweise ein Lochinjektionsmaterial, Lochtransportmaterial, Lochblockiermaterial, *wide-band-gap-*Material, fluoreszierender Emitter, *delayed fluorescent* Material, phosphoreszierender Emitter, Hostmaterial, Matrixmaterial, Elektronenblockiermaterial, Elektronentransportmaterial und Elektroneninjektionsmaterial, n-Dotand und p-Dotand.

Gegenstand der Erfindung ist daher weiterhin eine Zusammensetzung enthaltend mindestens eine erfindungsgemäße Verbindung oder mindestens ein erfindungsgemäßes Polymer, Oligomer oder Dendrimer eine weitere Verbindung ausgewählt aus Lochinjektionsmaterialien, Lochtransportmaterialien, Lochblockiermaterialien, *wide-band-gap-*Materialien, fluoreszierenden Emittern, *delayed fluorescent* Material, phosphoreszierenden Emittern, Hostmaterialien, Matrixmaterialien, Elektronenblockiermaterialien, Elektronentransportmaterialien und Elektroneninjektionsmaterialien, n-Dotanden und p-Dotanden. Geeignete Beispiele dieser weiteren Verbindungen sind untenstehend aufgeführt.

Die Verbindung gemäß Formel (I) eignet sich für den Einsatz in einer elektronischen Vorrichtung, insbesondere einer organischen Elektrolumineszenzvorrichtung (OLED). Abhängig von der Substitution kann die Verbindung der Formel (I) in unterschiedlichen Funktionen und Schichten eingesetzt werden. Bevorzugt ist die Verwendung als elektronentransportierendes Material in einer elektronentransportierenden Schicht und/oder als Matrixmaterial in einer emittierenden Schicht, besonders bevorzugt in Kombination mit einem phosphoreszierenden Emitter.

Weiterer Gegenstand der Erfindung ist daher die Verwendung einer Verbindung gemäß Formel (I) in einer elektronischen Vorrichtung. Dabei ist die elektronische Vorrichtung bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt organischen Elektrolumineszenzvorrichtungen (OLEDs).

Gegenstand der Erfindung ist weiterhin eine elektronische Vorrichtung, enthaltend mindestens eine Verbindung gemäß Formel (I). Dabei ist die elektronische Vorrichtung bevorzugt ausgewählt aus den oben genannten Vorrichtungen.

Besonders bevorzugt ist eine organische Elektrolumineszenzvorrichtung, enthaltend Anode, Kathode und mindestens eine emittierende Schicht, dadurch gekennzeichnet, dass mindestens eine organische Schicht in der Vorrichtung enthalten ist, welche mindestens eine Verbindung gemäß Formel (I) enthält. Bevorzugt ist eine organische Elektrolumineszenzvorrichtung, enthaltend Anode, Kathode und mindestens eine emittierende Schicht, dadurch gekennzeichnet, dass mindestens eine organische Schicht in der Vorrichtung, gewählt aus elektronentransportierenden und emittierenden Schichten, mindestens eine Verbindung gemäß Formel (I) enthält.

Unter einer elektronentransportierenden Schicht werden dabei alle Schichten verstanden, die zwischen Kathode und emittierender Schicht angeordnet sind, bevorzugt Elektroneninjektionsschicht (EIL), Elektronentransportschicht (ETL), und Lochblockierschicht (HBL). Unter eine Elektroneninjektionsschicht wird dabei eine Schicht verstanden, die direkt an die Kathode angrenzt. Unter einer Elektronentransportschicht wird dabei eine Schicht verstanden, die zwischen Kathode und emittierender Schicht vorliegt, aber nicht direkt an die Kathode angrenzt, bevorzugt auch nicht direkt an die emittierende Schicht angrenzt. Unter einer Lochblockierschicht wird dabei eine Schicht verstanden, die zwischen Kathode und emittierender Schicht vorliegt und direkt an die emittierende Schicht angrenzt.

Eine Lochblockierschicht weist bevorzugt ein energetisch ein organisches Material auf, das ein tiefer liegendes HOMO aufweist als das erste organische Matrixmaterial und/oder das zweite organische Matrixmaterial. So werden die Löcher (deren Injektion und Transport in der Regel das kleinere Problem darstellt) durch die Elektronentransportschicht daran gehindert, direkt zur Kathode zu wandern (aus diesem Grund wird die Elektronentransportschicht häufig auch als Lochblockierschicht bezeichnet.

Außer Kathode, Anode und emittierender Schicht kann die elektronische Vorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) und/oder organischen oder anorganischen p/n-Übergängen. Es sei aber darauf hingewiesen, dass nicht notwendigerweise jede dieser Schichten vorhanden sein muss und die Wahl der Schichten immer von den verwendeten Verbindungen abhängt und insbesondere auch von der Tatsache, ob es sich um eine fluoreszierende oder phosphoreszierende Elektrolumineszenzvorrichtung handelt.

Die Abfolge der Schichten der elektronischen Vorrichtung ist bevorzugt wie folgt:
- Anode-
- Lochinjektionsschicht-
- Lochtransportschicht-
- optional weitere Lochtransportschichten-
- optional Elektronenblockierschicht-
- emittierende Schicht-
- optional Lochblockierschicht-
- Elektronentransportschicht-
- Elektroneninjektionsschicht-
- Kathode-.

Dabei soll erneut darauf hingewiesen werden, dass nicht alle der genannten Schichten vorhanden sein müssen, und/oder dass zusätzlich weitere Schichten vorhanden sein können.

Die erfindungsgemäße organische Elektrolumineszenzvorrichtung kann mehrere emittierende Schichten enthalten. Besonders bevorzugt weisen diese Emissionsschichten insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues, grünes, gelbes, orangefarbenes oder rotes Licht emittieren. Insbesondere bevorzugt sind Dreischichtsysteme, also Systeme mit drei emittierenden Schichten, wobei jeweils eine der drei Schichten blaue, jeweils eine der drei Schichten grüne und jeweils eine der drei Schichten orangefarbene oder rote Emission zeigt. Die erfindungsgemäßen Verbindungen sind dabei bevorzugt in einer lochtransportierenden Schicht oder in der emittierenden Schicht vorhanden. Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert.

Es ist bevorzugt, dass die Verbindung der Formel (I) als Elektronentransportmaterial (ETM) verwendet wird. Dabei kann die emittierende Schicht eine fluoreszierende emittierende Schicht sein, oder sie kann eine phosphoreszierende emittierende Schicht sein. Bevorzugt ist die emittierende Schicht eine blau fluoreszierende Schicht oder eine grün phosphoreszierende Schicht.

Wenn die Vorrichtung enthaltend die Verbindung der Formel (I) eine phosphoreszierende emittierende Schicht enthält, ist es bevorzugt, dass diese Schicht zwei oder mehr, bevorzugt genau zwei, verschiedene Matrixmaterialien enthält (mixed-Matrix-System). Bevorzugte Ausführungsformen von mixed-Matrix-Systemen sind weiter unten näher beschrieben.

Wird die Verbindung gemäß Formel (I) als Elektronentransportmaterial in einer Elektronentransportschicht, einer Elektroneninjektionsschicht oder einer Lochblockierschicht eingesetzt, so kann die Verbindung als Reinmaterial, d.h. in einem Anteil von 100 %, in der Elektronentransport-, Elektroneninjektions- oder Lochblockierschicht eingesetzt werden, oder sie kann in Kombination mit einer oder mehreren weiteren Verbindungen, vorzugsweise organischen Verbindungen, eingesetzt werden.

Gemäß einer bevorzugten Ausführungsform enthält eine Elektronentransportschicht enthaltend die Verbindung der Formel (I) zusätzlich eine oder mehrere weitere elektronentransportierende Verbindungen. Diese weiteren elektronentransportierenden Verbindungen sind bevorzugt gewählt aus den weiter unten angegebenen bevorzugten Ausführungsformen von Elektronentransportmaterialien. Ganz besonders bevorzugt enthält eine elektronentransportierende Schicht die Verbindung der Formel (I) zusammen mit LiQ (lithium 8-quinolinolate) oder einem LiQ-Derivat als weitere elektronentransportierende Verbindung. In der beschriebenen bevorzugten Ausführungsform sind die Verbindung der Formel (I) und die eine oder mehrere weiteren elektronentransportierenden Verbindungen bevorzugt jeweils in einem Anteil von mindestens 10% vorhanden, besonders bevorzugt jeweils in einem Anteil von mindestens 20% vorhanden.

Gemäß einer anderen bevorzugten Ausführungsform enthält eine Elektronentransportschicht die Verbindung der Formel (I) als Reinmaterial, , und eine Elektroneninjektionsschicht enthält eine oder mehrere weitere elektronentransportierende Verbindungen, besonders bevorzugt LiQ oder ein LiQ-Derivat.

Gemäß einer bevorzugten Ausführungsform enthält eine lochtransportierende Schicht enthaltend die Verbindung der Formel (I) zusätzlich einen oder mehrere p-Dotanden. Als p-Dotanden werden gemäß der vorliegenden Erfindung bevorzugt solche organischen Elektronenakzeptorverbindungen eingesetzt, die eine oder mehrere der anderen Verbindungen der Mischung oxidieren können.

Besonders bevorzugt als p-Dotanden sind Chinodimethanverbindungen, Azaindenofluorendione, Azaphenalene, Azatriphenylene, I₂, Metallhalogenide, bevorzugt Übergangsmetallhalogenide, Metalloxide, bevorzugt Metalloxide enthaltend mindestens ein Übergangsmetall oder ein Metall der 3. Hauptgruppe, und Übergangsmetallkomplexe, bevorzugt Komplexe von Cu, Co, Ni, Pd und Pt mit Liganden enthaltend mindestens ein Sauerstoffatom als Bindungsstelle. Bevorzugt sind weiterhin Übergangsmetalloxide als Dotanden, bevorzugt Oxide von Rhenium, Molybdän und Wolfram, besonders bevorzugt Re₂O₇, MoO₃, WO₃ und ReOs. Nochmals weiterhin bevorzugt sind Komplexe von Bismut in der Oxidationsstufe (III), insbesondere Bismut(III)-Komplexe mit elektronenarmen Liganden, insbesondere Carboxylat-Liganden.

Die p-Dotanden liegen bevorzugt weitgehend gleichmäßig verteilt in den p-dotierten Schichten vor. Dies kann beispielsweise durch Co-Verdampfung des p-Dotanden und der Lochtransportmaterial-Matrix erreicht werden. Der p-Dotand liegt bevorzugt in einem Anteil von 1 bis 10 % in der p-dotierten Schicht vor.

Bevorzugt sind als p-Dotanden insbesondere die folgenden Verbindungen:

| | | |
|---|---|---|
| 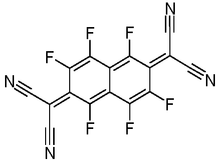 | 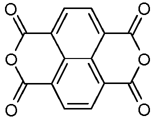 | 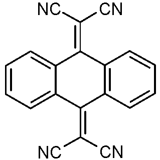 |
| (D-1) | (D-2) | (D-3) |
| 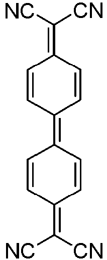 | 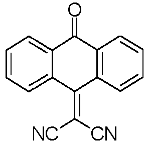 | 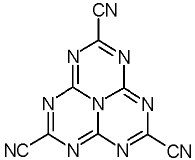 |
| (D-4) | (D-5) | (D-6) |
| 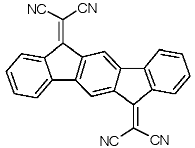 | 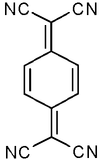 | 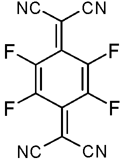 |
| (D-7) | (D-8) | (D-9) |
| 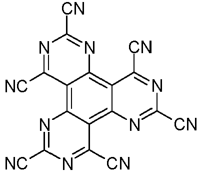 | 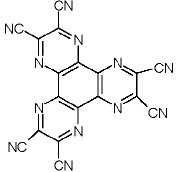 | 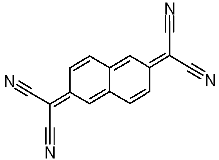 |
| (D-10) | (D-11) | (D-12) |
| 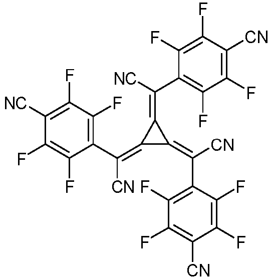 | 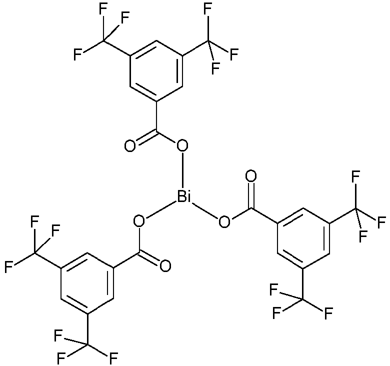 | |
| (D-13) | (D-14) | |

Gemäß einer bevorzugten Ausführungsform ist in der Vorrichtung eine Lochinjektionsschicht vorhanden, die einer der folgenden Ausführungsformen entspricht: a) sie enthält ein Triarylamin und einen p-Dotanden; oder b) sie enthält ein einzelnes elektronenarmes Material (Elektronenakzeptor). Gemäß einer bevorzugten Ausführungsform der Ausführungsform a) ist das Triarylamin ein Mono-Triarylamin, insbesondere eines der weiter unten genannten bevorzugten Triarylamin-Derivate.

Gemäß einer bevorzugten Ausführungsform der Ausführungsform b) ist das elektronenarme Material ein Hexaazatriphenylenderivat, wie in US 2007/0092755 beschrieben.

Die Verbindung der Formel (I) kann in einer Elektroneninjektionsschicht, in einer Elektronentransportschicht, und/oder in einer Lochblockierschicht der Vorrichtung enthalten sein. Wenn die Verbindung in einer Elektroneninjektionsschicht oder in einer Elektronentransportschicht vorliegt, ist sie bevorzugt p-dotiert, das heißt sie liegt gemischt mit einem p-Dotanden, wie oben beschrieben, in der Schicht vor.

Bevorzugt ist die Verbindung der Formel (I) in einer Elektronentransportschicht und/oder in einer Lochblockierschicht enthalten. Ganz besonders bevorzugt ist die Verbindung der Formel (I) in einer Elektronentransportschicht enthalten. Bevorzugt ist sie in diesem Fall nicht p-dotiert. Weiterhin bevorzugt liegt sie in diesem Fall nicht als Einzelverbindung in der Elektronentransportschicht vor, sondern in Kombination mit einer oder mehreren weiteren Verbindungen, bevorzugt mit weiteren Elektronentransport- und/oder Elektroneninjektions materialien, und ganz besonders bevorzugt mit LiQ (lithium 8-quinolinolate) oderLiQ-Derivaten.

Gemäß einer alternativen bevorzugten Ausführungsform liegt die Verbindung der Formel (I) in einer emittierenden Schicht als Matrixmaterial in Kombination mit einer oder mehreren emittierenden Verbindungen, vorzugsweise phosphoreszierenden emittierenden Verbindungen, eingesetzt. Die phosphoreszierenden emittierenden Verbindungen sind dabei bevorzugt gewählt aus rot phosphoreszierenden und grün phosphoreszierenden Verbindungen.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt zwischen 85.0 und 97.0 Vol.-%.

Entsprechend beträgt der Anteil der emittierenden Verbindung zwischen 0.1 und 50.0 Vol.-%, bevorzugt zwischen 0.5 und 20.0 Vol.-% und besonders bevorzugt zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere emittierende Verbindungen enthalten. Auch in diesem Fall sind die emittierenden Verbindungen im Allgemeinen diejenigen Verbindungen, deren Anteil im System der kleinere ist und die Matrixmaterialien sind diejenigen Verbindungen, deren Anteil im System der größere ist. In Einzelfällen kann jedoch der Anteil eines einzelnen Matrixmaterials im System kleiner sein als der Anteil einer einzelnen emittierenden Verbindung.

Es ist bevorzugt, dass die Verbindungen gemäß Formel (I) als eine Komponente von Mixed-Matrix-Systemen, bevorzugt für phosphoreszierende Emitter, verwendet werden. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit elektronentransportierenden Eigenschaften und das andere Material ein Material mit lochtransportierenden Eigenschaften dar. Bevorzugt ist weiterhin, wenn eines der Materialien gewählt ist aus Verbindungen mit großer Energiedifferenz zwischen HOMO und LUMO (Wide-Bandgap-Materialien). Die Verbindung der Formel (I) stellt in einem mixed-Matrix-System bevorzugt das Matrixmaterial mit elektronentransportierenden Eigenschaften dar. Entsprechend ist, wenn die Verbindung der Formel (I) als Matrixmaterial für einen phosphoreszierenden Emitter in der emittierenden Schicht einer OLED eingesetzt wird, eine zweite Matrixverbindung in der emittierenden Schicht vorhanden, die lochtransportierende Eigenschaften aufweist. Insbesondere bevorzugt ist hierbei, wenn die Verbindung der Formel (I) als elektronentransportierendes Material mit Carbazol-Derivaten, insbesondere Biscarbazolen, als lochtransportierendes Material als Mischung in der EML vorliegt. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen.

Bevorzugte Biscarbazole als Matrixmaterial sind solche der folgenden Formel

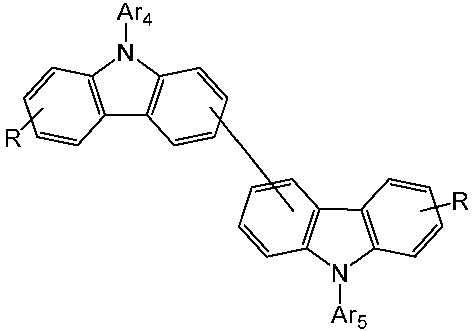

Ganz bevorzugte Biscarbazole sind solche der folgenden Formel s 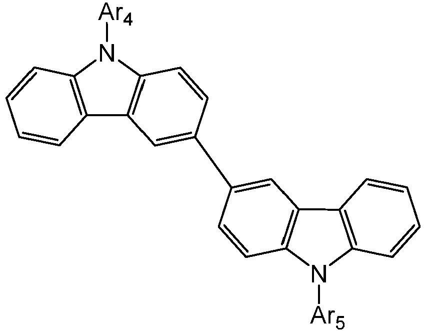 wobei die Gruppe R die gleiche Bedeutung hat wie die oben und/oder in Anspruch 1 definierte Gruppe R⁰; Ar₄ und Ar₅ haben die gleiche Bedeutung wie die oben und/oder in Anspruch 1 definierte Gruppe Ar⁰.

Besonders bevorzugte Biscarbazole sind solche der folgenden Formeln

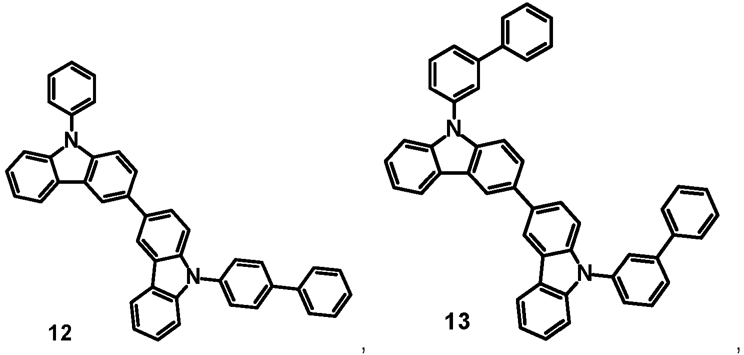

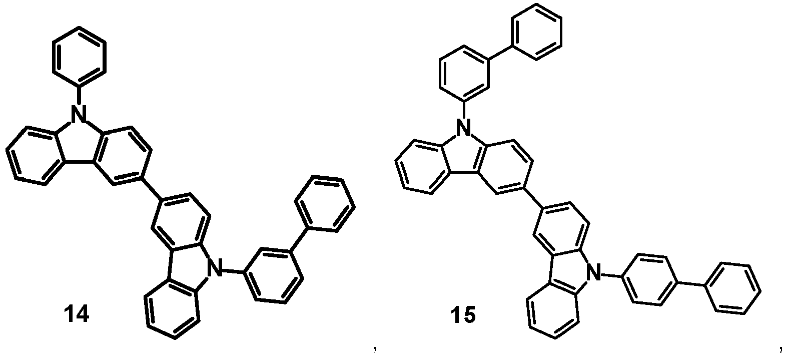

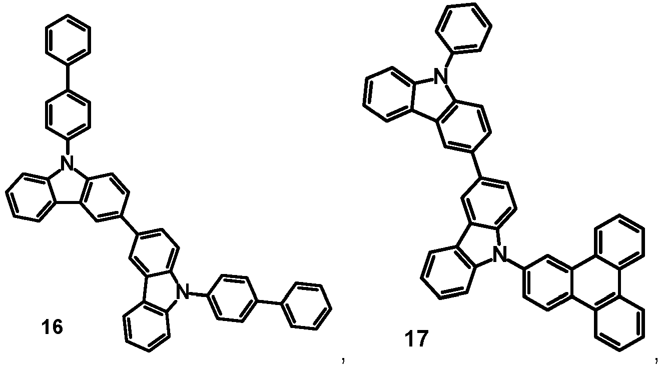

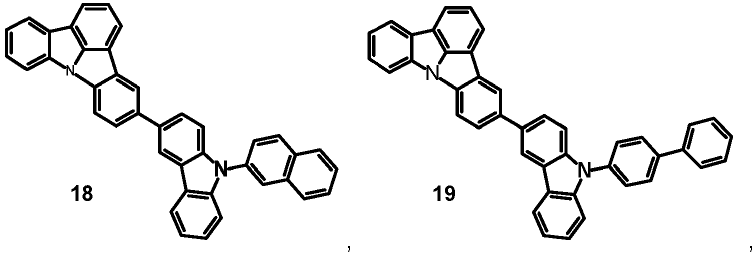

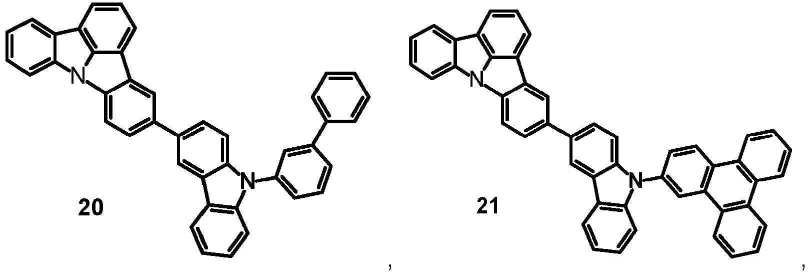

Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen.

In den oben genannten Schichten der Vorrichtung werden bevorzugt die folgenden Materialklassen eingesetzt:
Phosphoreszierende Emitter:
Vom Begriff phosphoreszierende Emitter sind typischerweise Verbindungen umfasst, bei denen die Lichtemission durch einen spin-verbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Emitter eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Emitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Erfindung alle lumineszierenden Iridium-, Platin- oder Kupferkomplexe als phosphoreszierende Verbindungen angesehen.

Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind, zur Verwendung in den erfindungsgemäßen Vorrichtungen. Weitere Beispiele für geeignete phosphoreszierende Emitter sind in der folgenden Tabelle gezeigt:

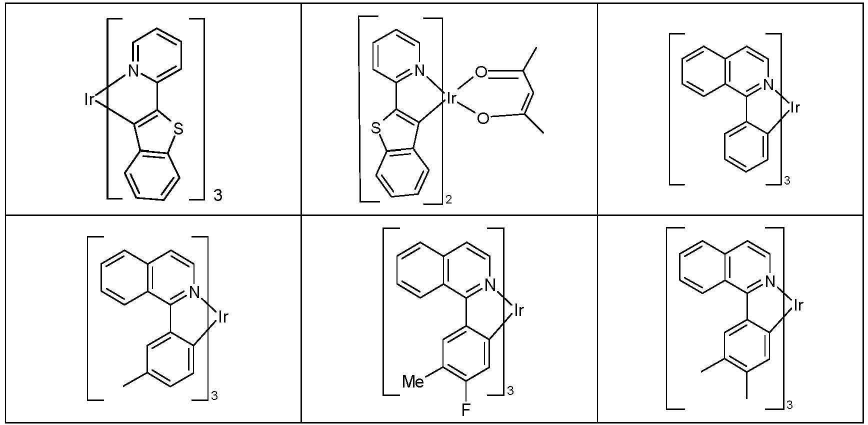

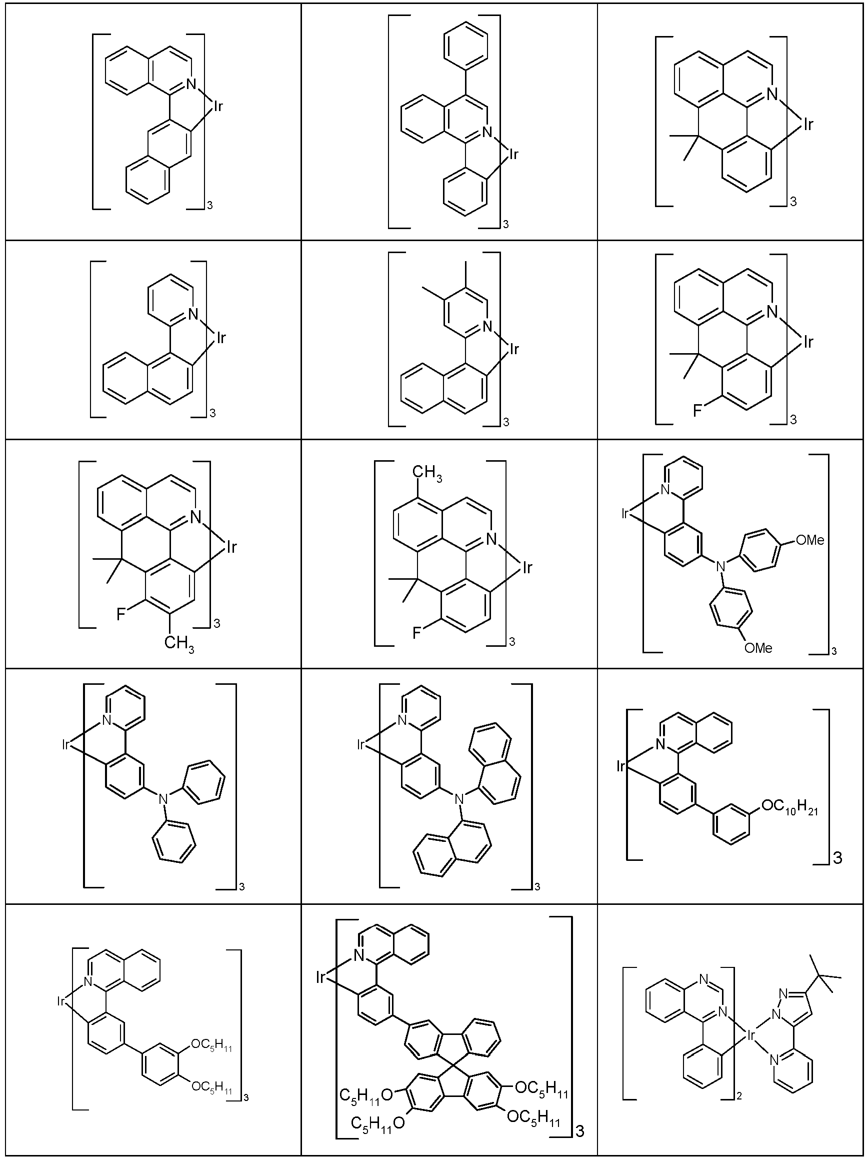

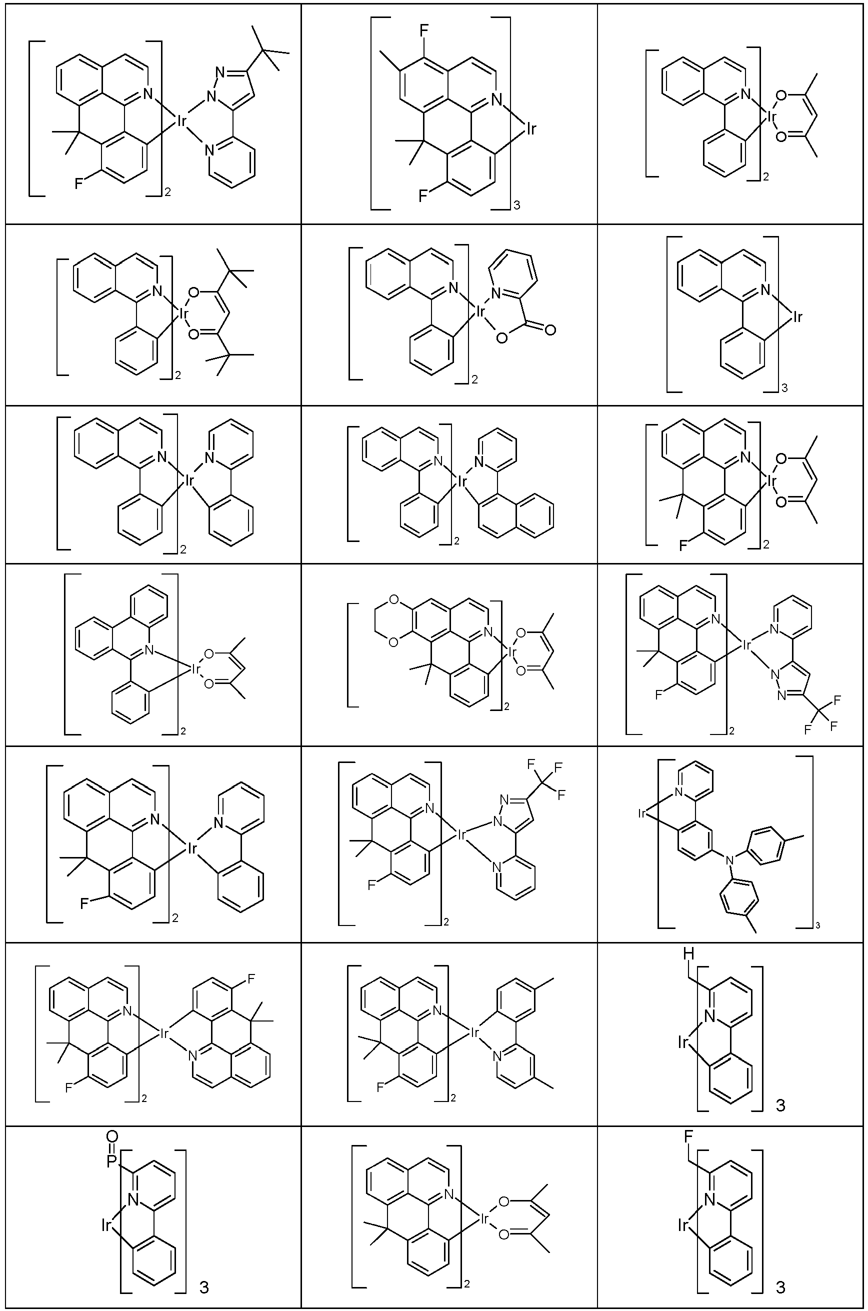

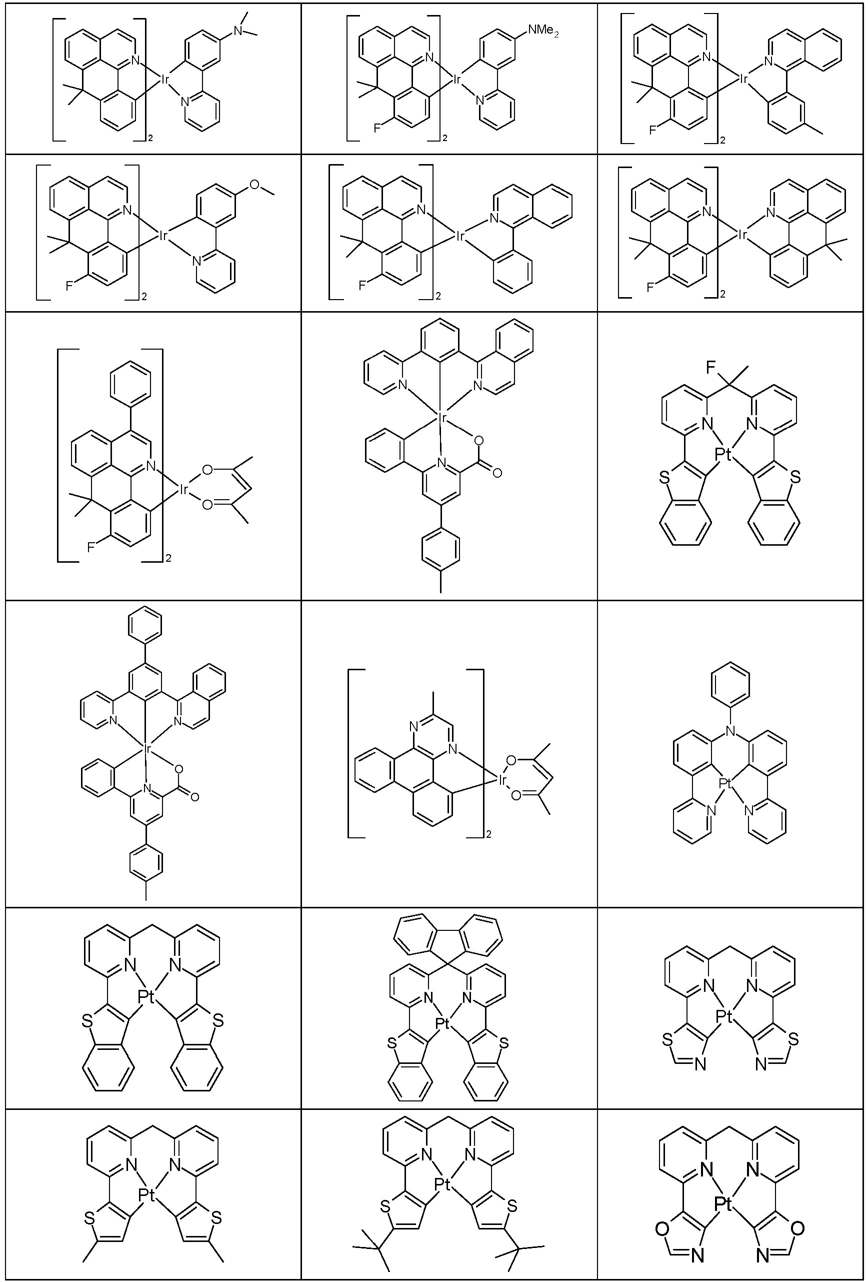

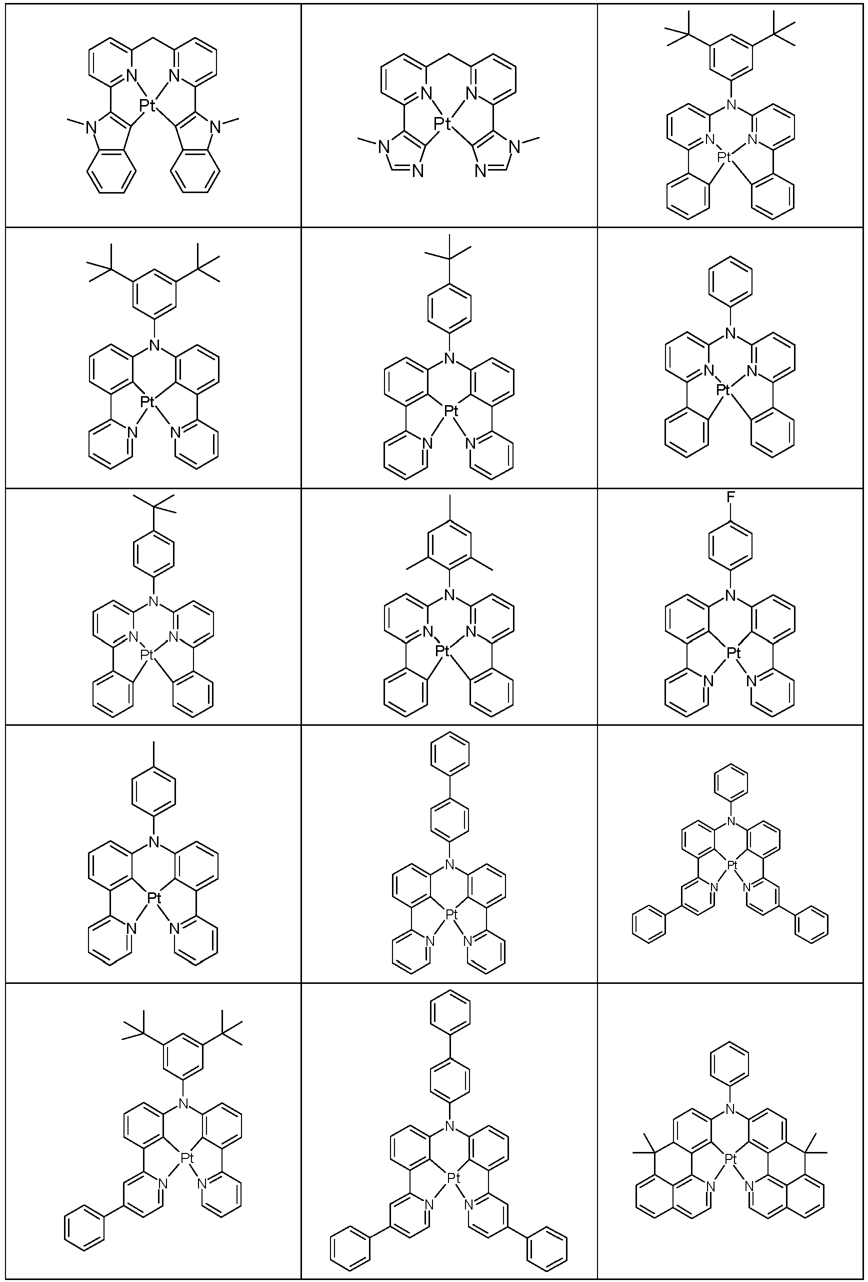

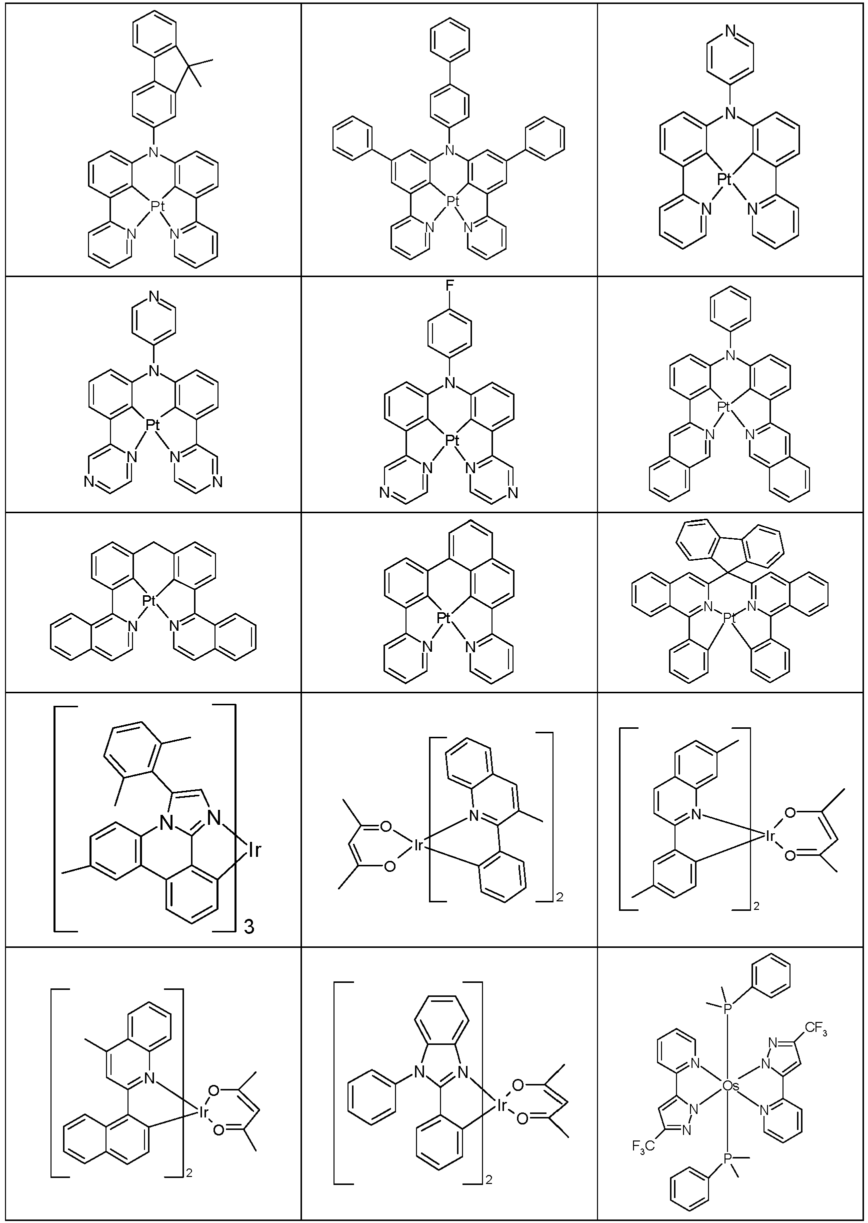

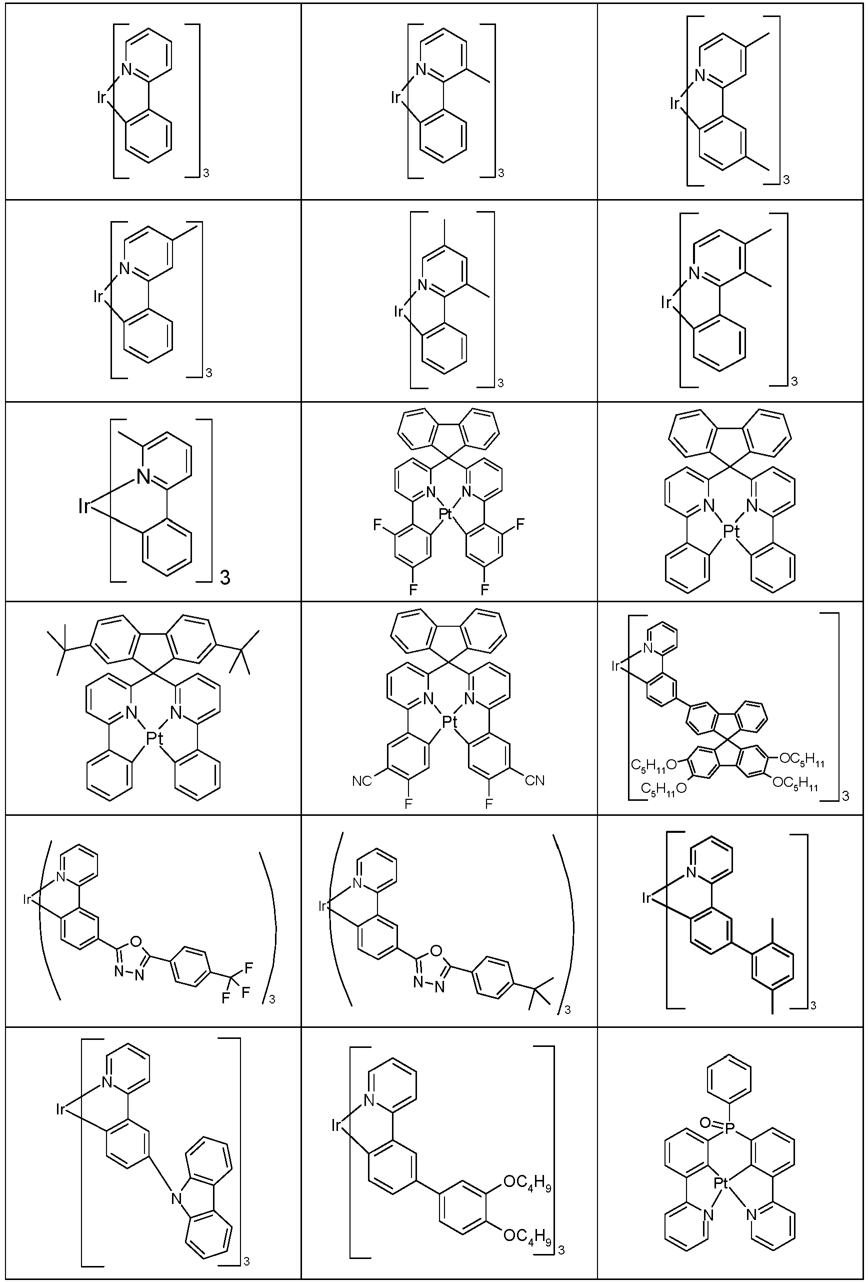

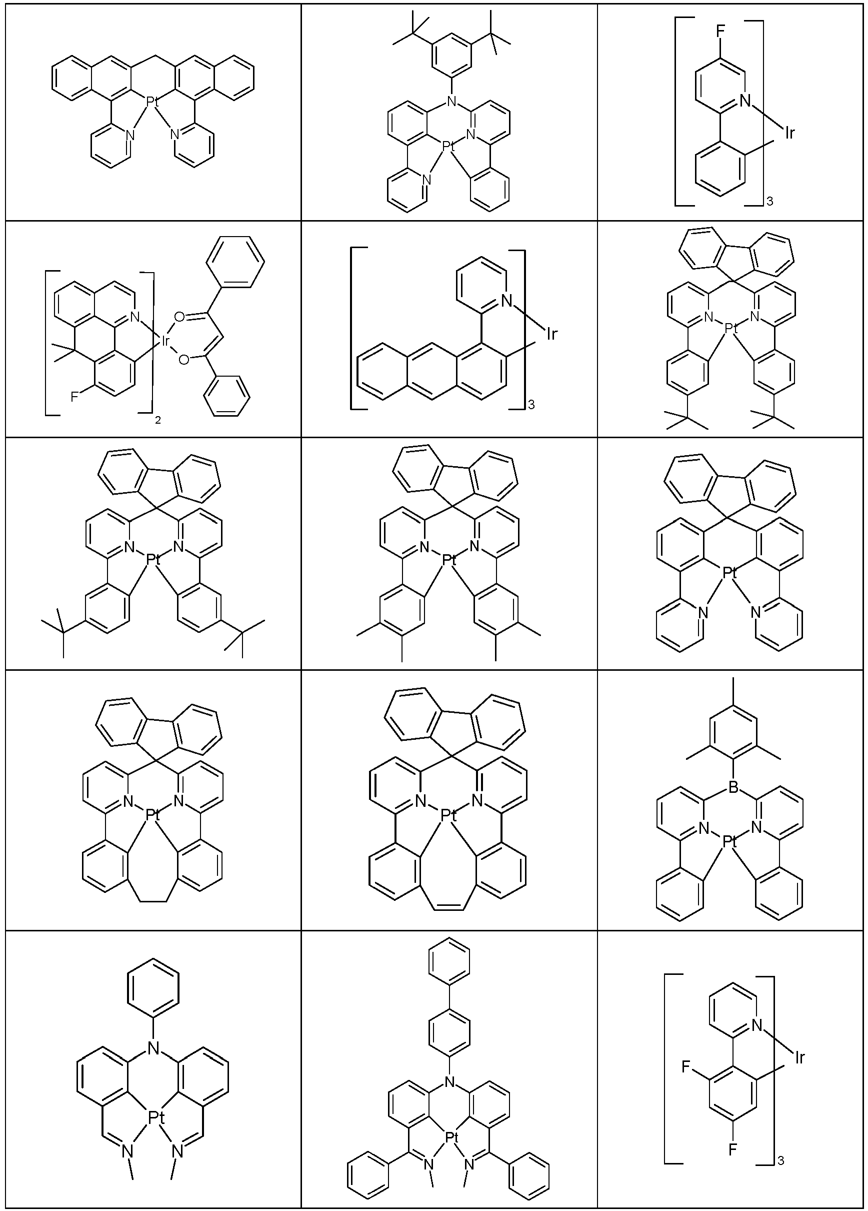

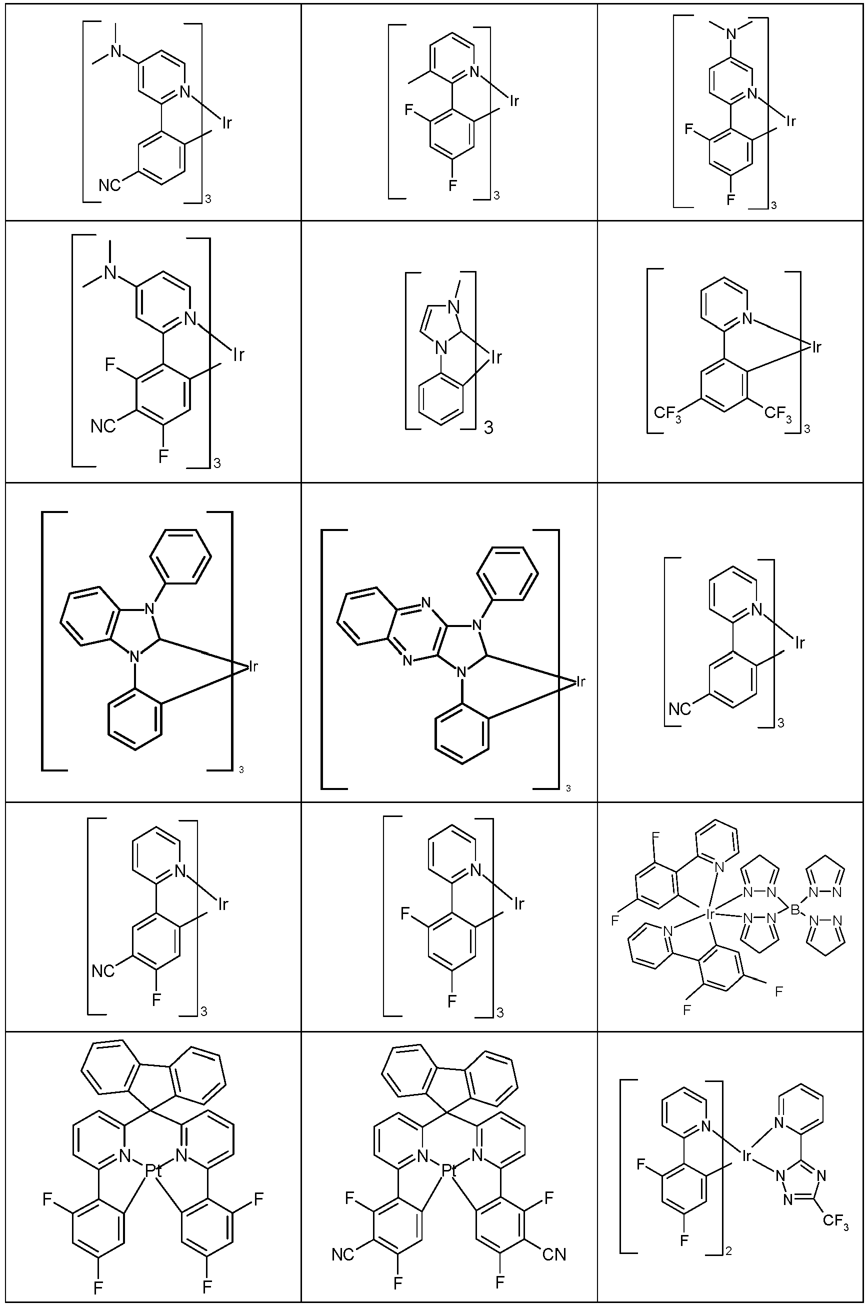

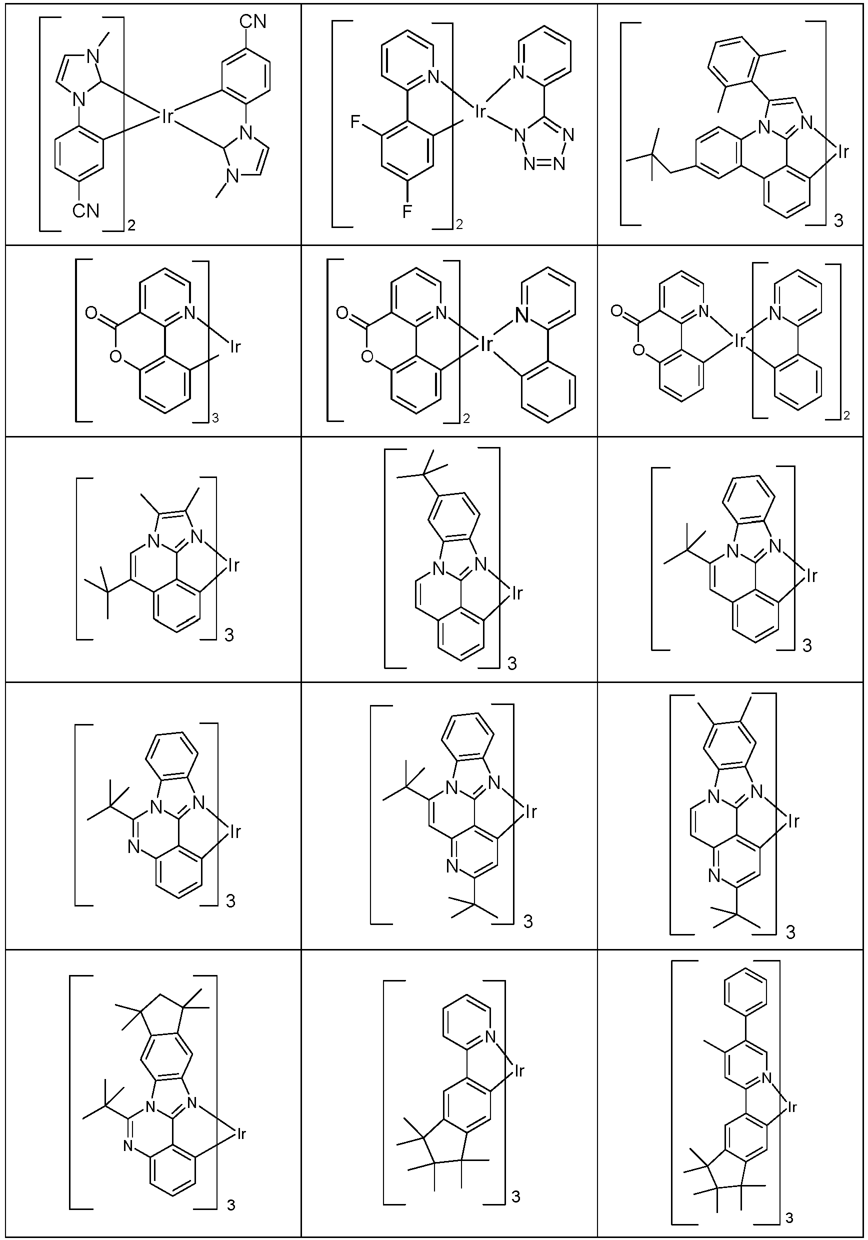

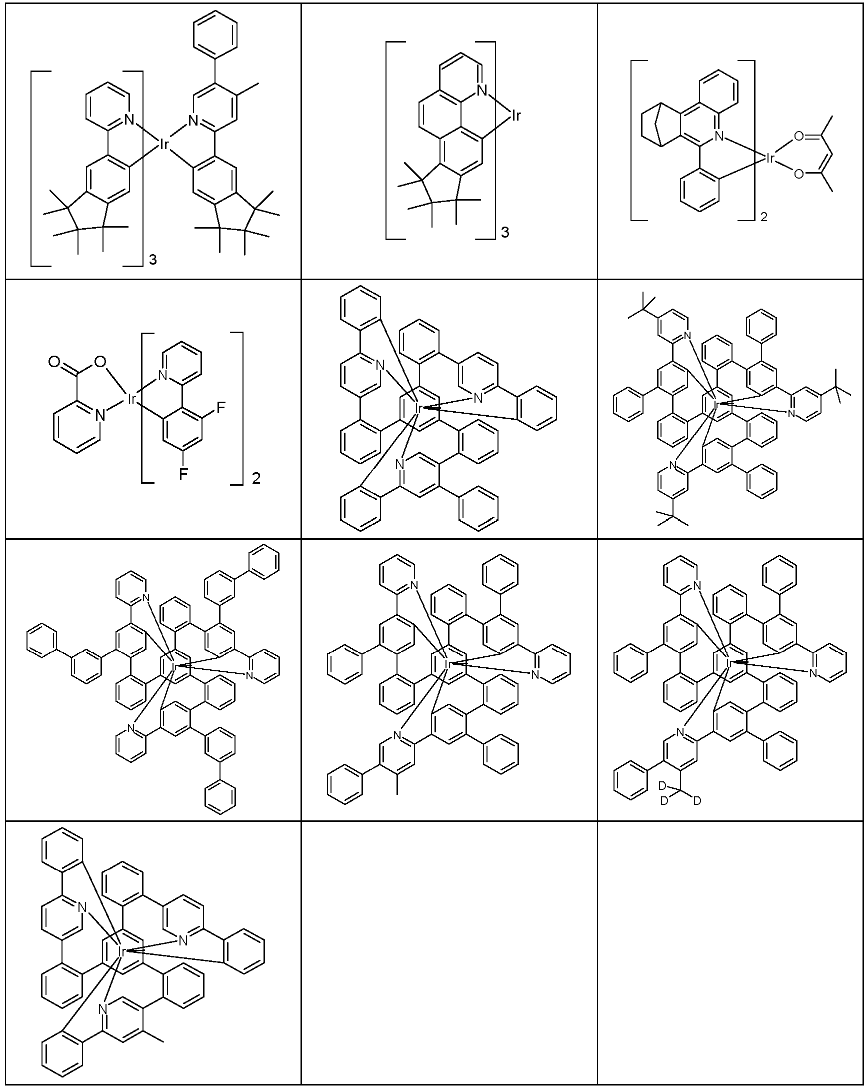

### Fluoreszierende Emitter:

Bevorzugte fluoreszierende emittierende Verbindungen sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte emittierende Verbindungen sind Indenofluorenamine bzw. - diamine, Benzoindenofluorenamine bzw. -diamine, und Dibenzoindenofluorenamine bzw. -diamine, sowie Indenofluorenderivate mit kondensierten Arylgruppen. Ebenfalls bevorzugt sind Pyren-Arylamine. Ebenfalls bevorzugt sind Benzoindenofluoren-Amine, Benzofluoren-Amine, erweiterte Benzoindenofluorene, Phenoxazine, und Fluoren-Derivate, die mit Furan-Einheiten oder mit Thiophen-Einheiten verbunden sind.

### Delayed fluorescent-Materialien:

Geeignete delayed fluorescent-Materialien, wie delayed fluorescent Emitter oder delayed fluorescent Hosts, sind im Stand der Technik gut bekannt, z.B. in Ye Tao et al., Adv. Mater. 2014, 26, 7931-7958, M. Y. Wong et al., Adv. Mater. 2017, 29, 1605444, WO 2011/070963, WO 2012/133188, WO 2015/022974 und WO 2015/098975. Typischerweise sind delayed fluorescent-Materialien dadurch charakterisiert, dass einen ziemlich kleine Energielücke zwischen dem Singulett-Energieniveau (S₁) und dem Triplett-Energieniveau (T₁) aufweisen. Bevorzugt ist ΔE_{ST} kleiner als 0,5 eV, besonders bevorzugt kleiner als 0,3 eV und ganz besonders bevorzugt klaeiner als 0,2 eV, wobei ΔE_{ST} die Energiedifferenz zischen dem Singulett-Energieniveau (S₁) und dem Triplett-Energieniveau (T₁) darstellt.

### Matrixmaterialien für fluoreszierende Emitter:

Bevorzugte Matrixmaterialien für fluoreszierende Emitter sind ausgewählt aus den Klassen der Oligoarylene (z. B. 2,2',7,7'-Tetraphenylspirobifluoren), insbesondere der Oligoarylene enthaltend kondensierte aromatische Gruppen, der Oligoarylenvinylene, der polypodalen Metallkomplexe, der lochleitenden Verbindungen, der elektronenleitenden Verbindungen, insbesondere Ketone, Phosphinoxide, und Sulfoxide; der Atropisomere, der Boronsäurederivate oder der Benzanthracene. Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

### Matrixmaterialien für phosphoreszierende Emitter:

Bevorzugte Matrixmaterialien für phosphoreszierende Emitter sind neben den Verbindungen der Formel (I) aromatische Ketone, aromatische Phosphinoxide oder aromatische Sulfoxide oder Sulfone, Triarylamine, Carbazolderivate, z. B. CBP (N,N-Biscarbazolylbiphenyl) oder Carbazol-derivate, Indolocarbazolderivate, Indenocarbazolderivate, Azacarbazolderivate, bipolare Matrixmaterialien, Silane, Azaborole oder Boronester, Triazinderivate, Zinkkomplexe, Diazasilol- bzw. Tetraazasilol-Derivate, Diazaphosphol-Derivate, überbrückte Carbazol-Derivate, Triphenylenderivate, oder Lactame.

### Elektronentransportierende Materialien:

Weitere Verbindungen, die neben den Verbindungen der Formel (I) bevorzugt in elektronetransportierenden Schichten der erfindungsgemäßen OLEDs eingesetzt werden, sind beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Weitere Verbindungen, die neben den Verbindungen der Formel (I) in der Elektronentransportschicht eingesetzt werden, sind alle Materialien, die gemäß dem Stand der Technik als Elektronentransportmaterialien in der Elektronentransportschicht verwendet werden können. Insbesondere eignen sich Aluminiumkomplexe, beispielsweise Alq₃, Zirkoniumkomplexe, beispielsweise Zrq₄, Lithiumkomplexe, beispielsweise Liq, Benzimidazolderivate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate. Bevorzugte elektronentransportierende Verbindungen sind in der folgenden Tabelle gezeigt:

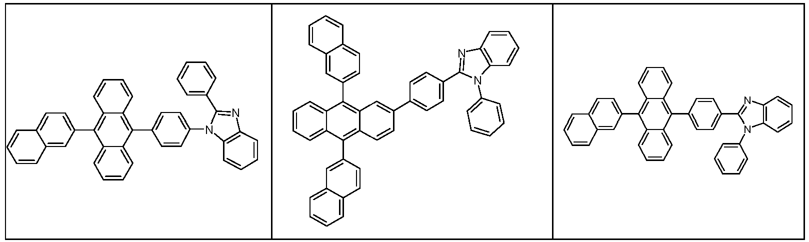

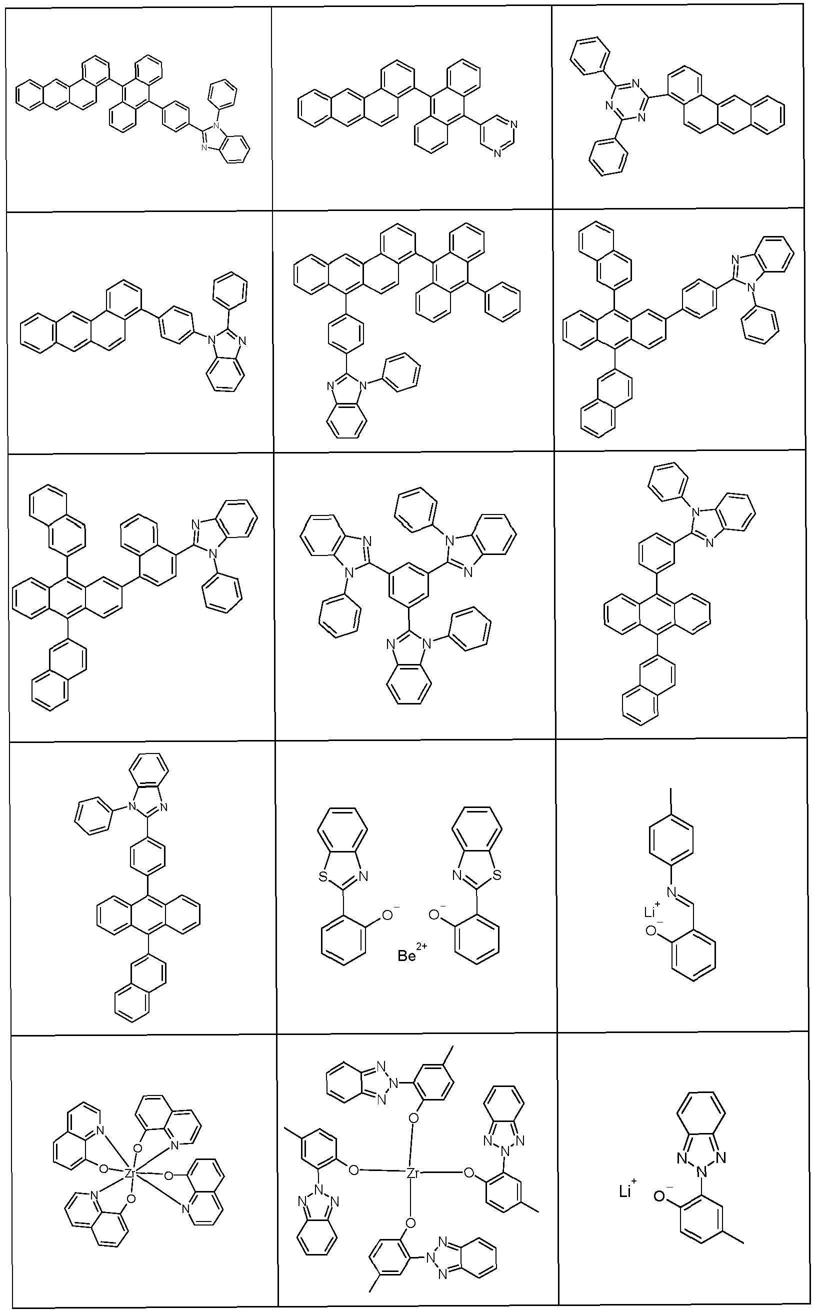

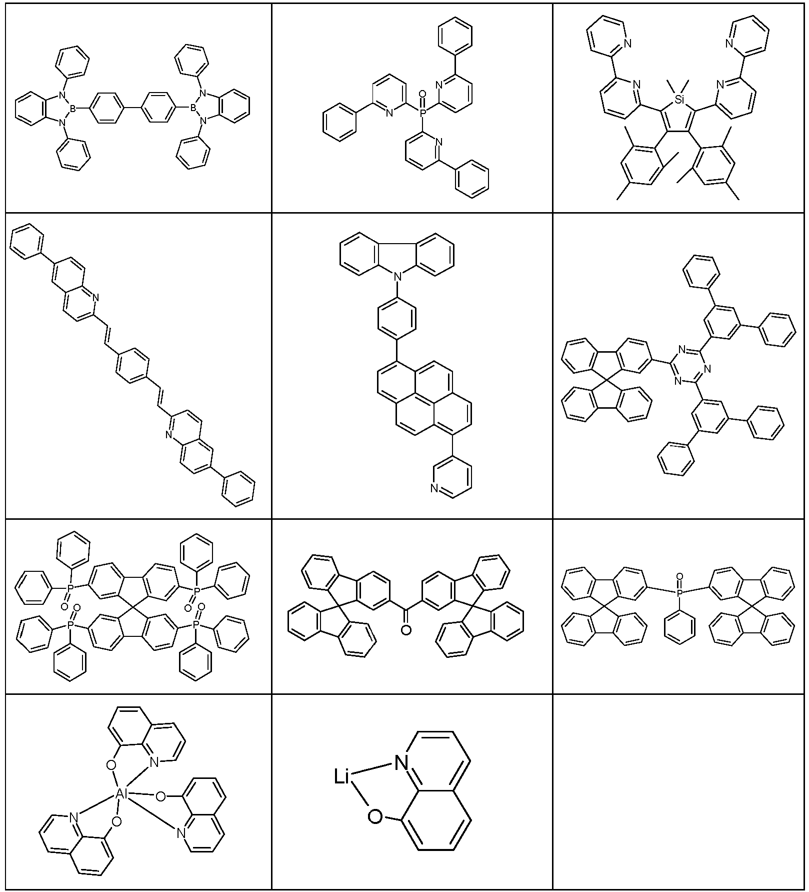

### Lochtransportierende Materialien:

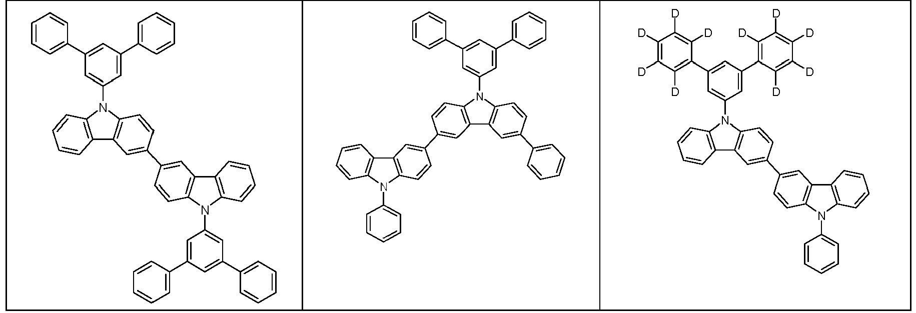

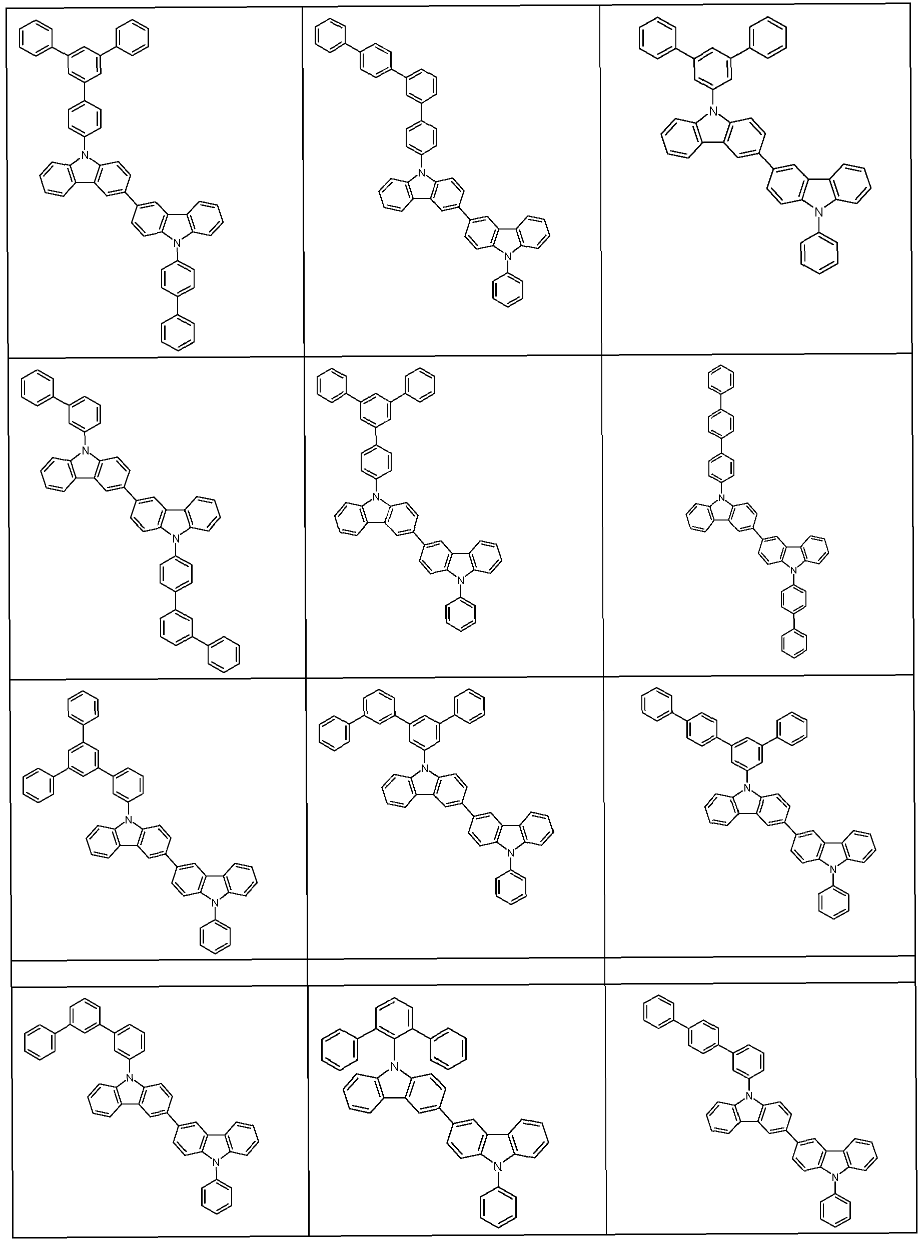

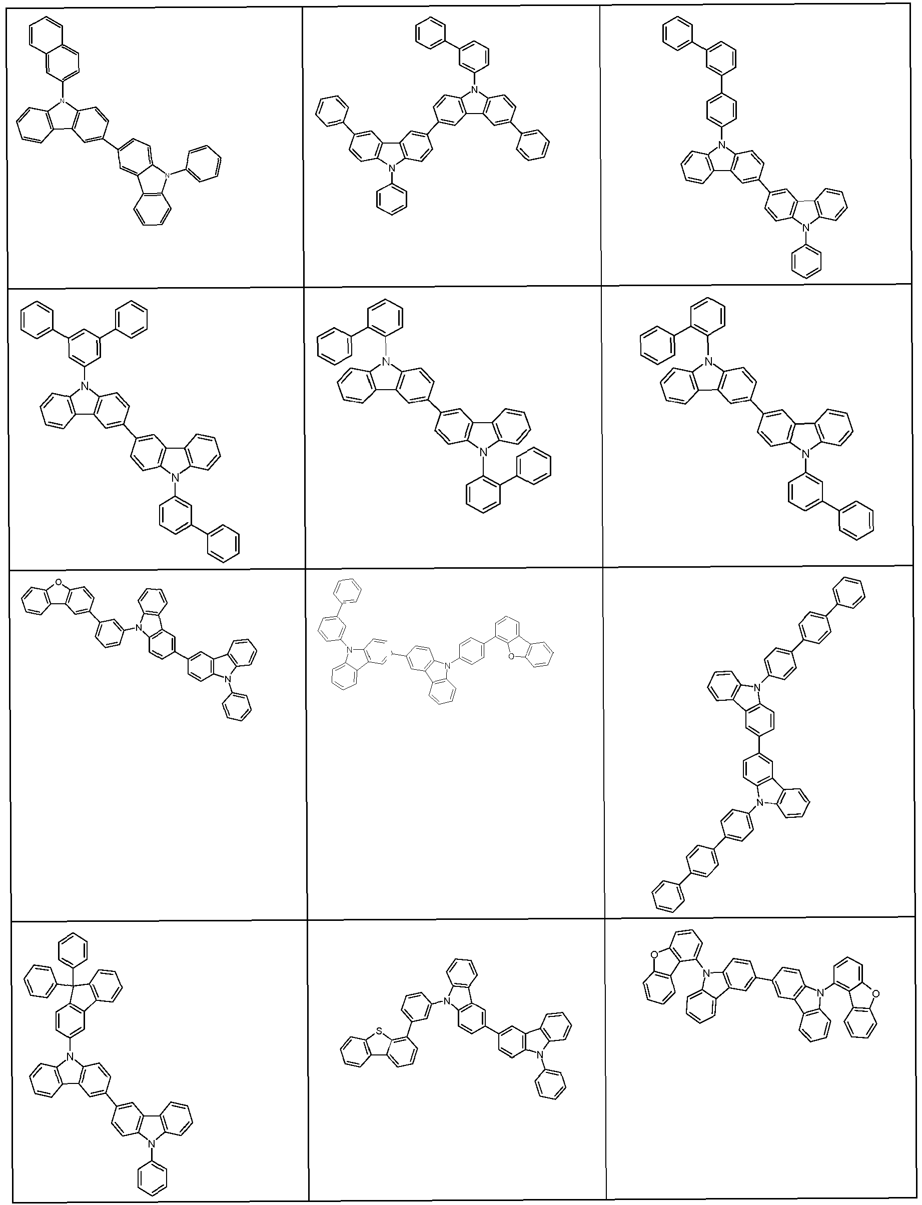

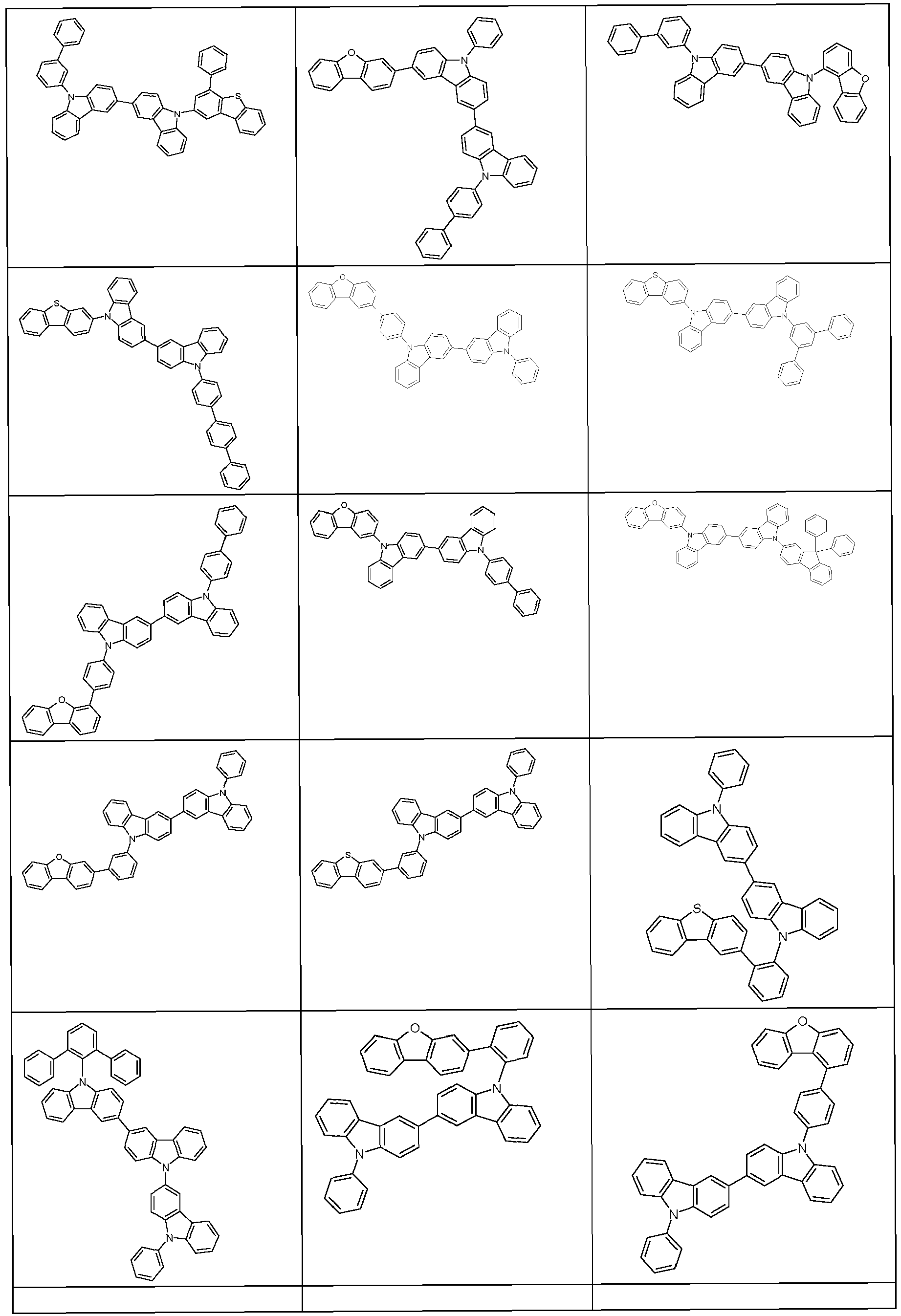

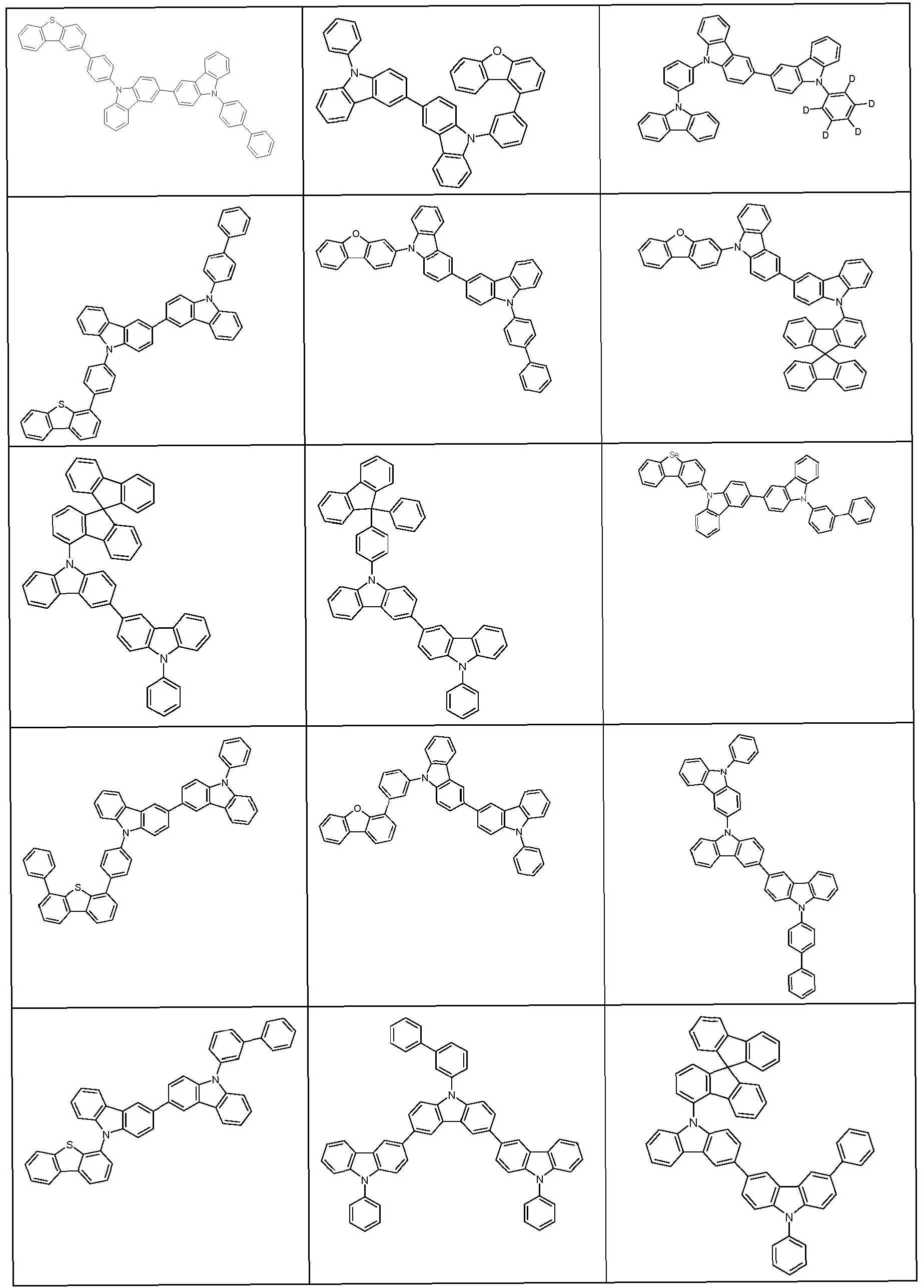

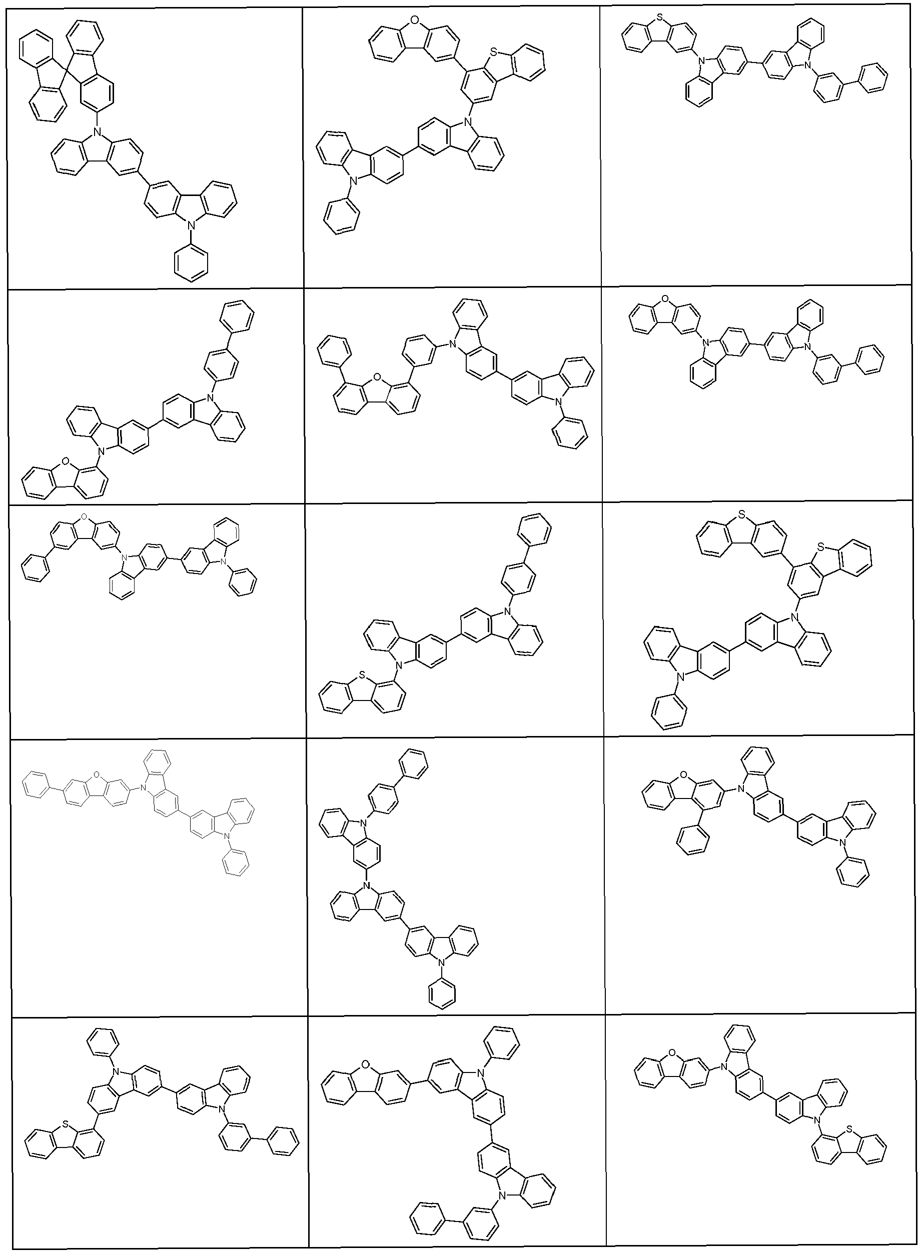

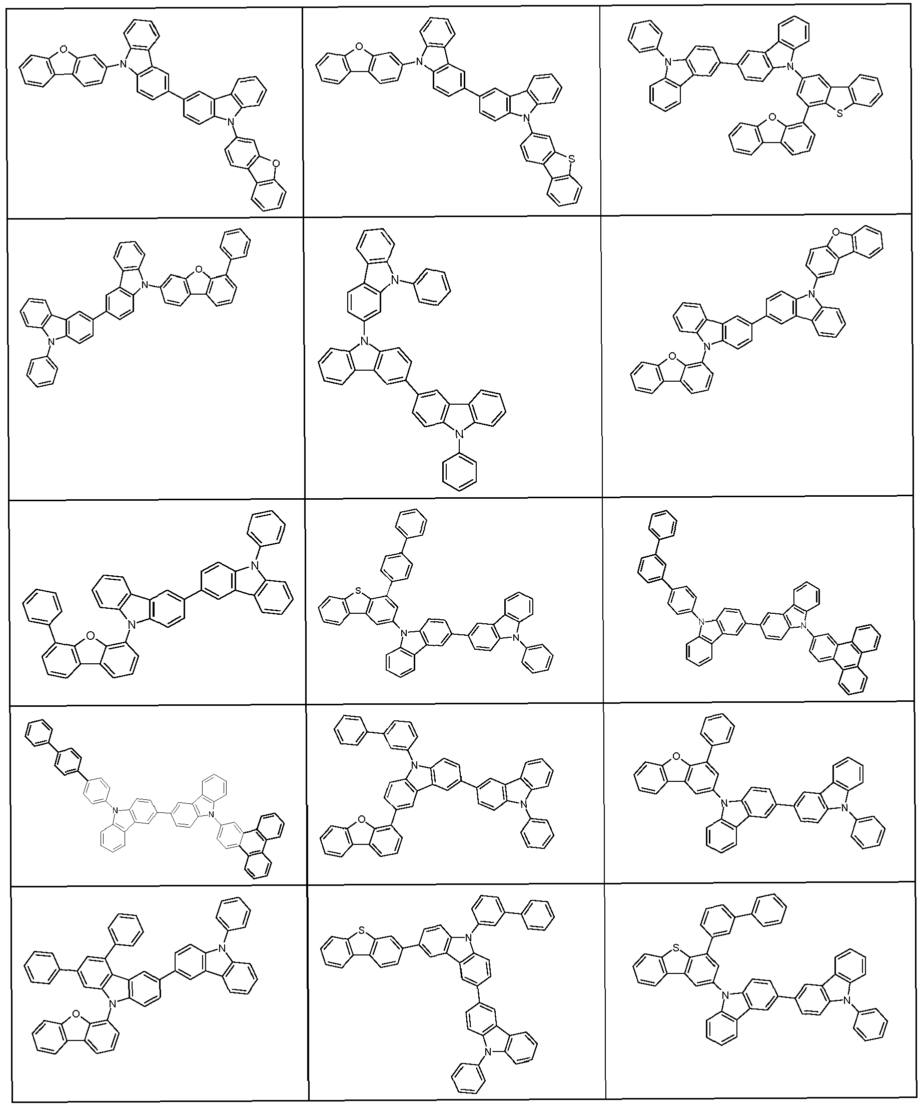

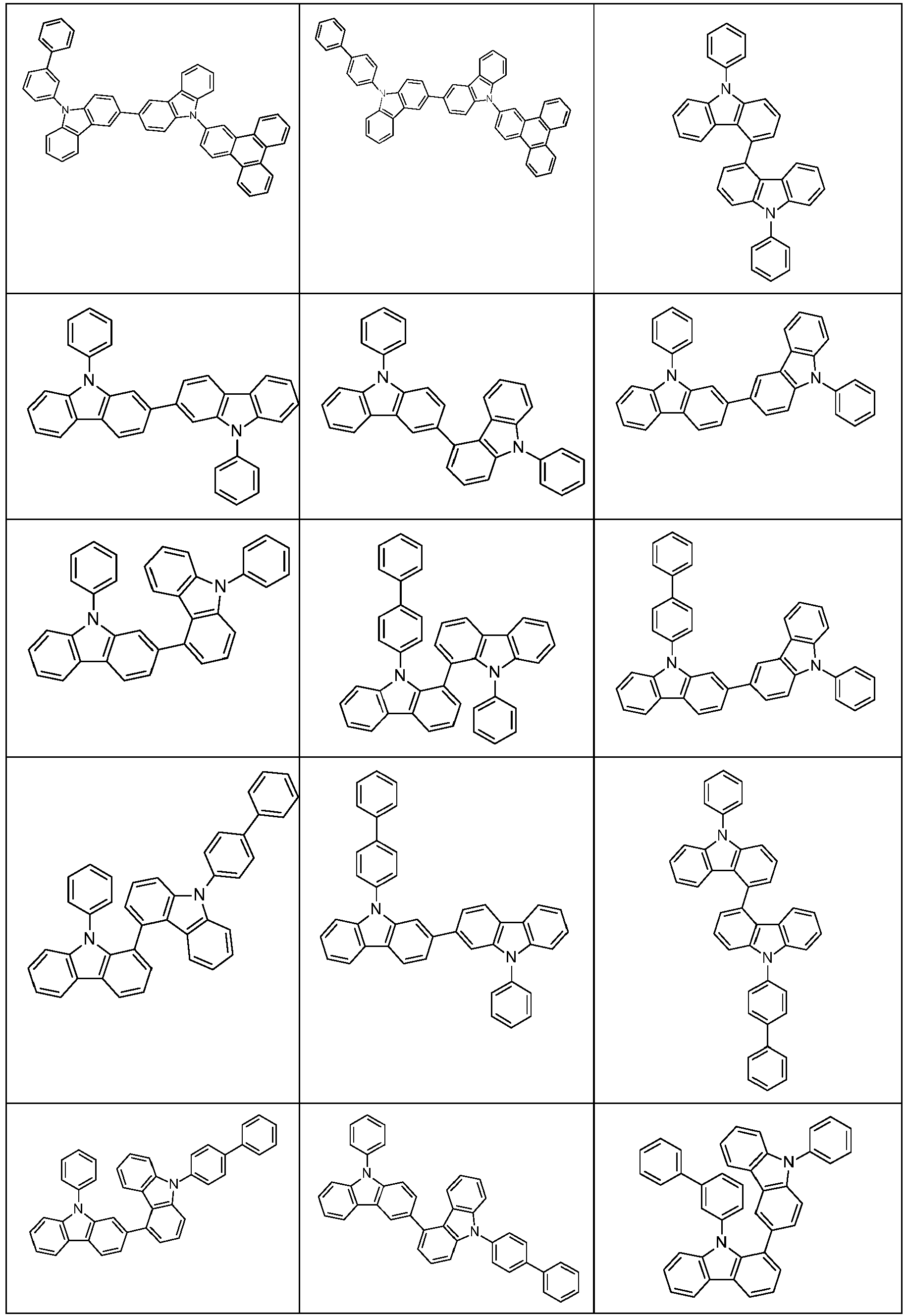

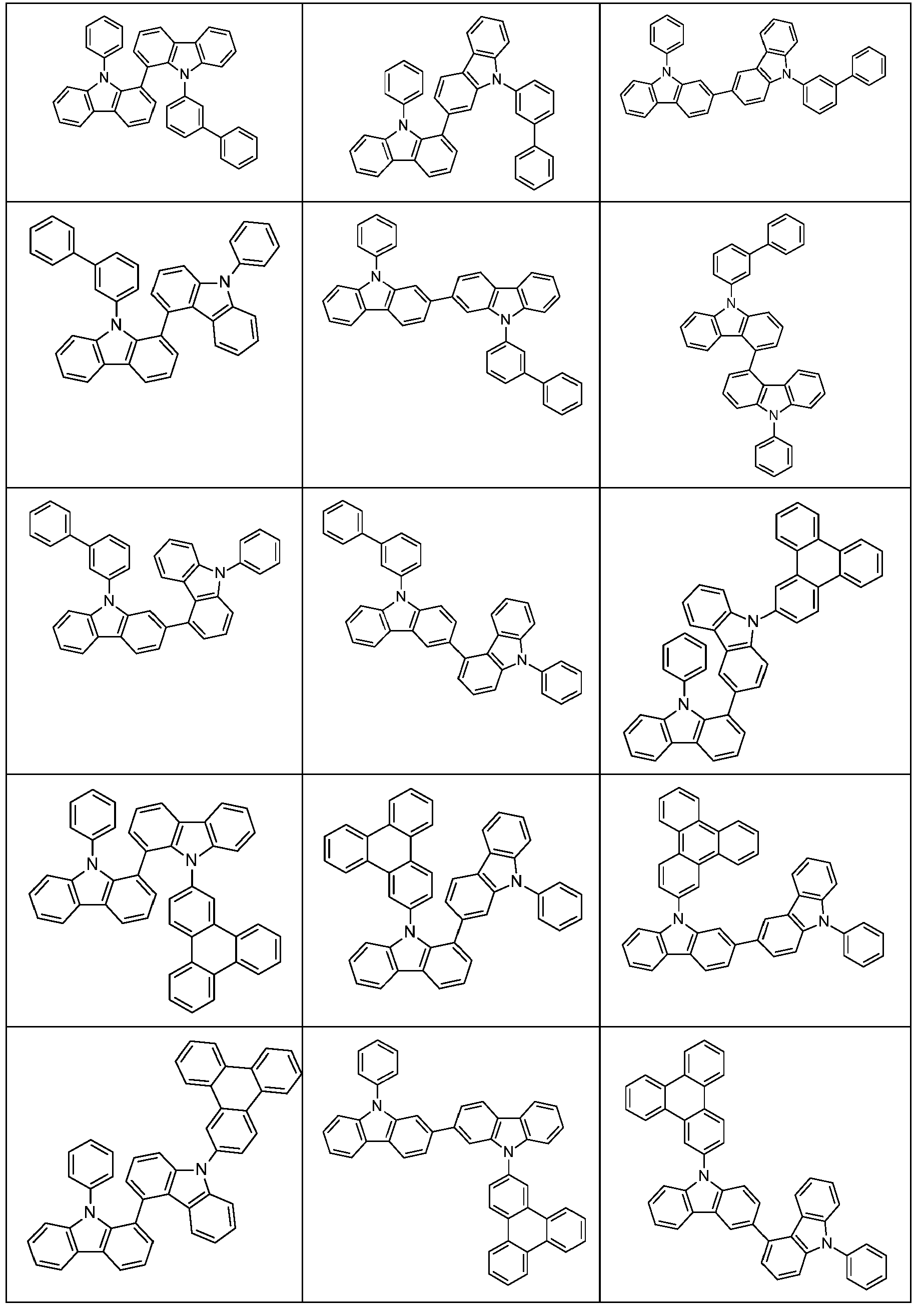

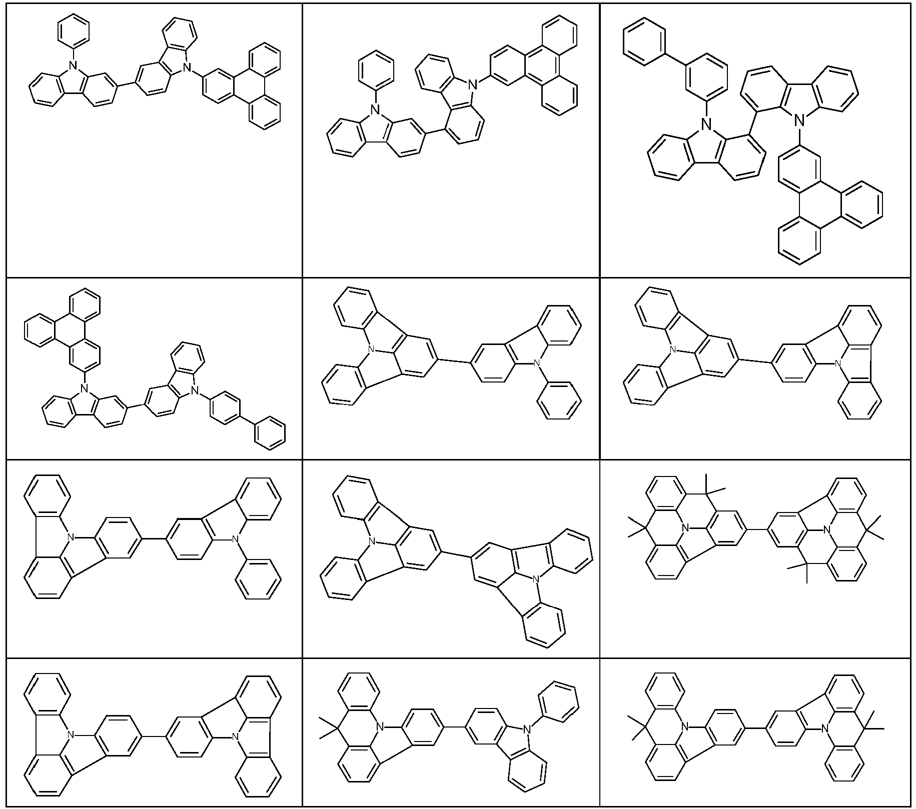

Als Materialien für lochtransportierenden Schichten können alle Materialien eingesetzt werden, die gemäß dem Stand der Technik in diesen Schichten eingesetzt werden. Insbesondere geeignet sind Indenofluorenamin-Derivate, Aminderivate, Hexaazatriphenylenderivate, Aminderivate mit kondensierten Aromaten, Monobenzoindenofluorenamine, Dibenzoindenofluorenamine, Spirobifluoren-Amine, Fluoren-Amine, Spiro-Dibenzopyran-Amine, Dihydroacridin-Derivate, Spirodibenzofurane und Spirodibenzothiophene, Phenanthren-Diarylamine, Spiro-Tribenzotropolone, Spirobifluorene mit meta-Phenyldiamingruppen, Spiro-Bisacridine, Xanthen-Diarylamine, und 9,10-Dihydroanthracen-Spiroverbindungen mit Diarylaminogruppen. Bevorzugte lochtransportierende Verbindungen sind in der folgenden Tabelle gezeigt:

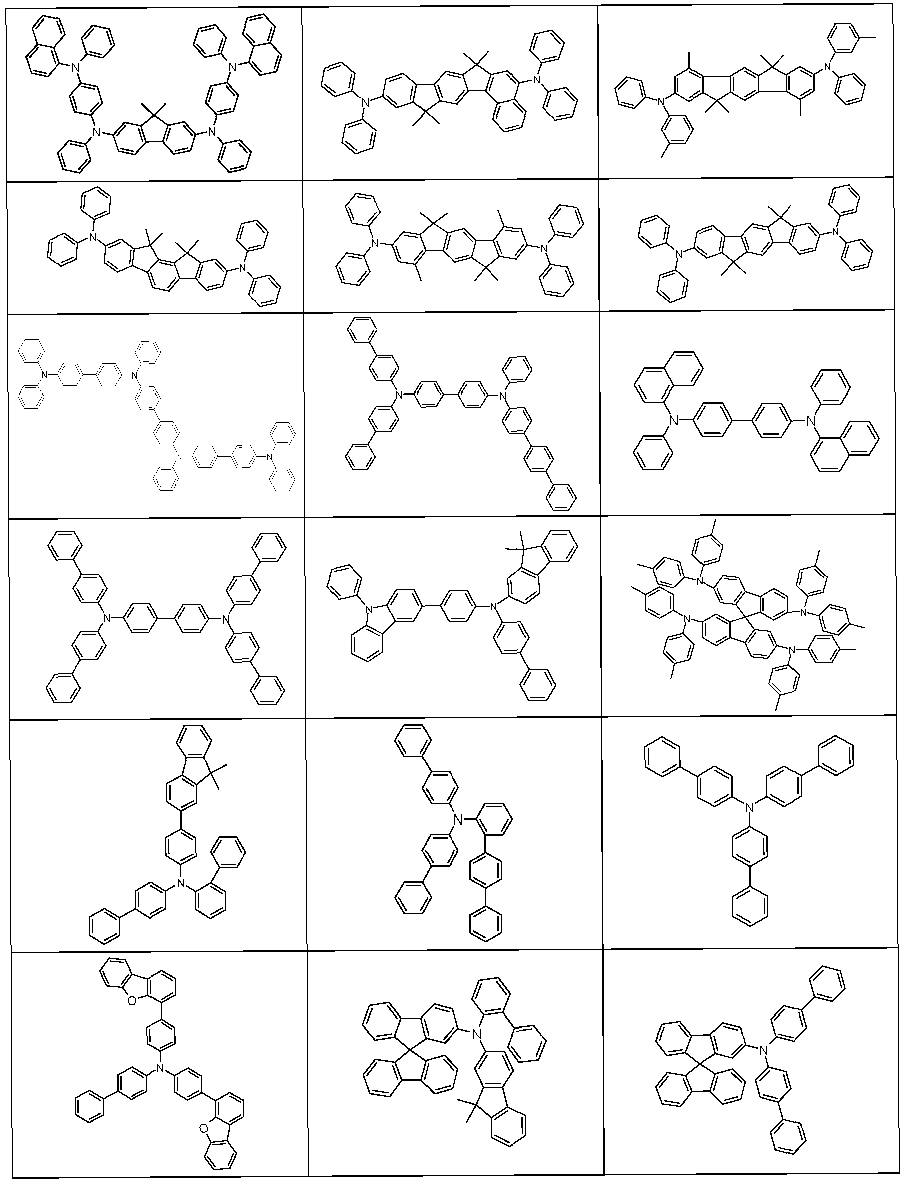

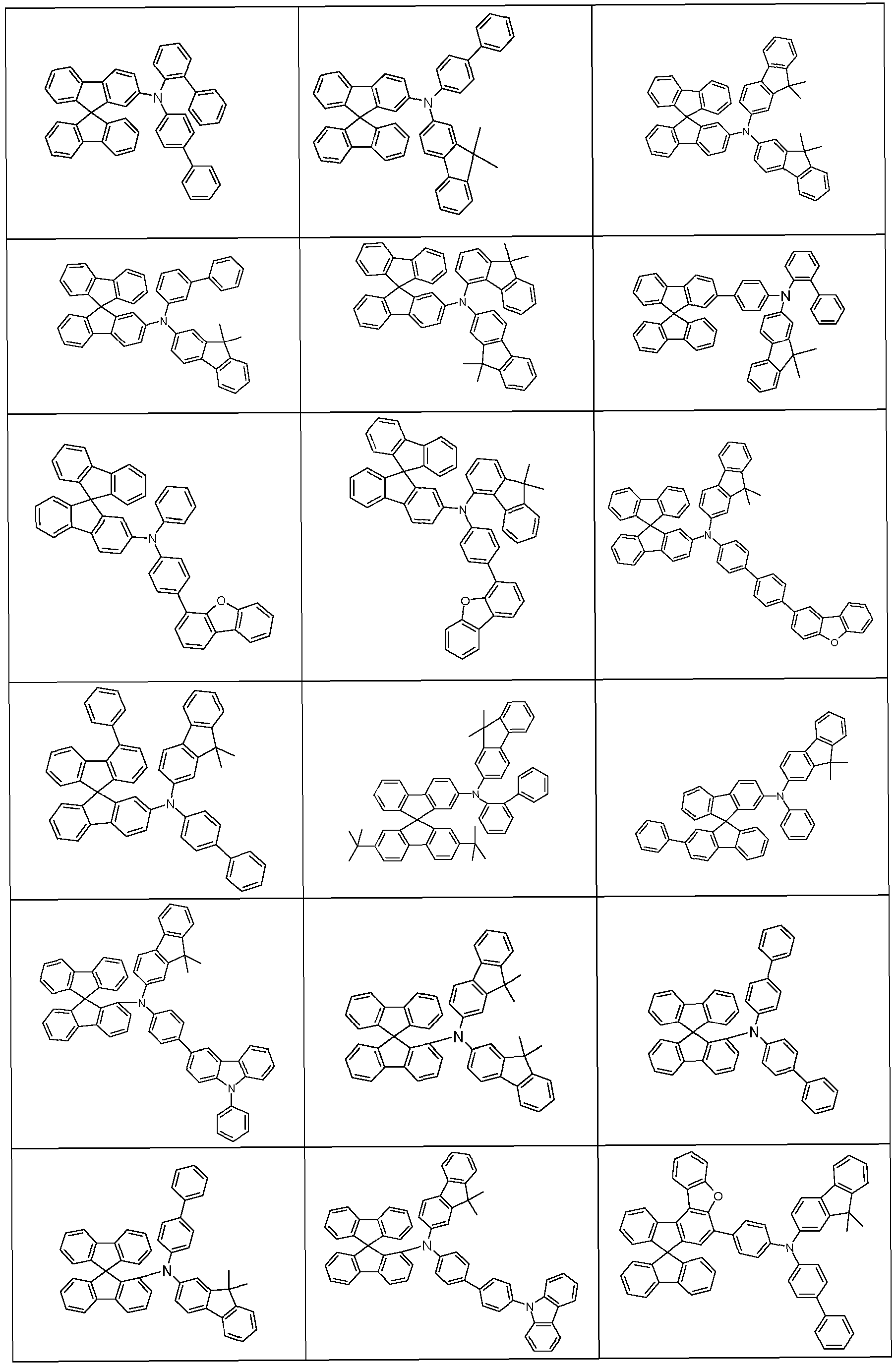

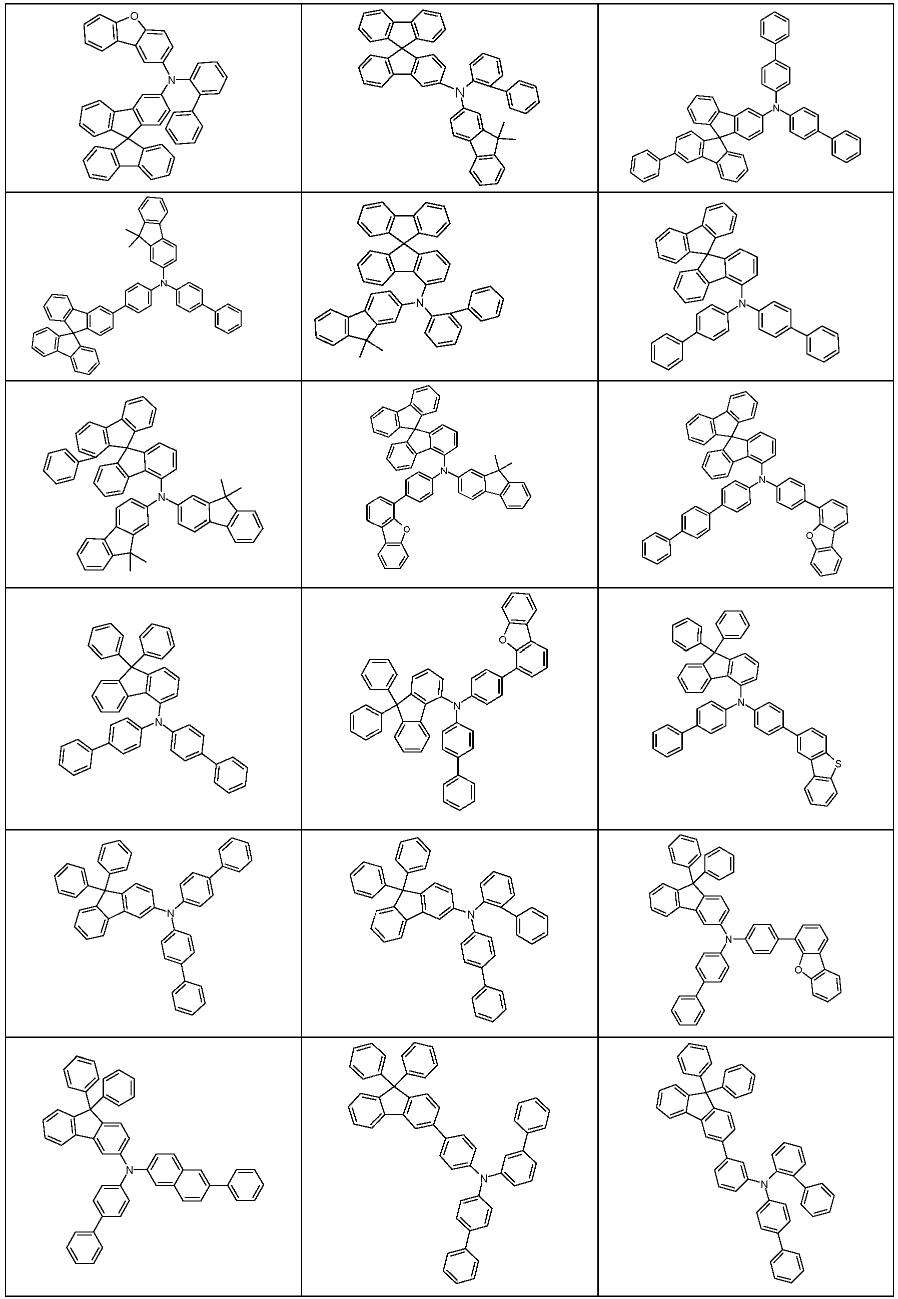

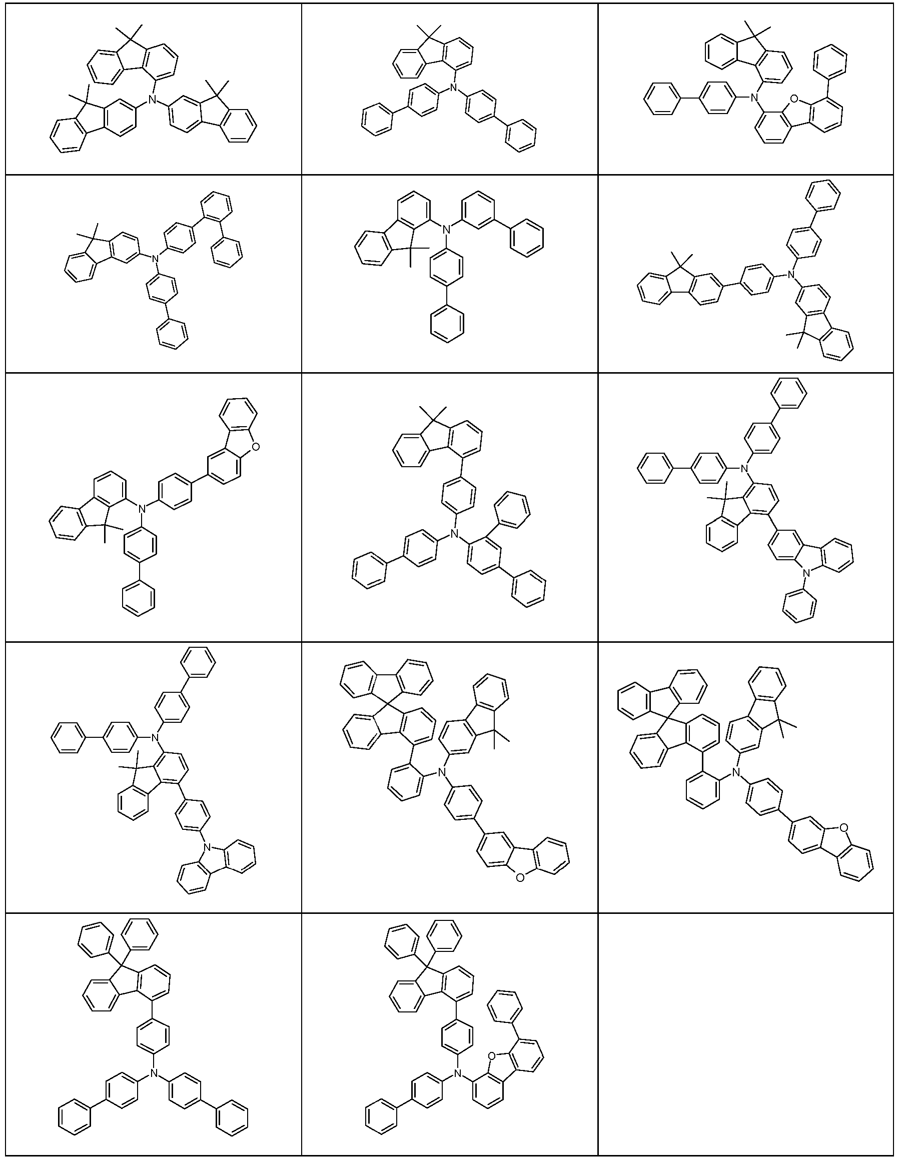

### Wide-band-gap-Materialien:

Als wide-band-gap-Materialien sollen im Rahmen der vorliegenden Anmeldung solche Materialien verstanden werden, wie sie in der US 7,294,849 offenbart sind, die dadurch charakterisiert sind, dass sie ein Bandlücke von mindestens 3,5 eV aufweisen, wobei der Begriff "Bandlücke" die Energielücke zwischen dem höchsten besetzten Molekülorbital (HOMO) und dem niedrigsten unbesetzten Molekülorbital (LUMO) bezeichnet. Derartige Systems sind im Stand der Technik gut bekannt und weisen besonders vorteilhafte Leistungseigenschaften in Elektrolumineszenzvorrichtungen auf.

### n-Dotanden:

Im Fall von n-Typ-ETLs (n-ETL) sollte das Ionisationspotential des n-Dotierstoffs über dem Niveau des LUMO von Elektronentransportmatrizen liegen. Das LUMO-Niveau der n-ETLs beträgt normalerweise ungefähr 3,0 eV, und eine höhere Energie des HOMO (<3,0 eV) für einen n-Dotierstoff (n-Dotierstoff) ist erforderlich. Mögliche Kandidaten für n-Dotierstoffe sind Alkalimetalle wie Li und Cs oder hoch liegende HOMO-Moleküle wie Ru (terpy) 2.

Das Einbringen einer LiF-Schicht zwischen der Elektronentransportschicht und dem Kontaktmetall wurde gezeigt, um die Betriebsspannung von OLEDs zu senken. Das wird erklärt durch eine Art n-Dotierungseffekt durch freigesetztes Li in Alq3.

### p-Dotanden:

Bevorzugt ist weiterhin, wenn auf die Anode ein p-dotiertes Lochtransportmaterial als Lochinjektionsschicht aufgebracht wird, wobei sich als p-Dotanden Metalloxide, beispielsweise MoO₃ oder WO₃, oder (per)fluorierte elektronenarme Aromaten eignen. Weitere geeignete p-Dotanden sind HAT-CN (Hexacyano-hexaazatriphenylen) oder die Verbindung NPD9 von Novaled. Eine solche Schicht vereinfacht die Lochinjektion in Materialien mit einem tiefen HOMO, also einem betragsmäßig großen HOMO.

Als Kathode der elektronischen Vorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

In einer bevorzugten Ausführungsform ist die elektronische Vorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine elektronische Vorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine elektronische Vorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I) nötig. Hohe Löslichkeit lässt sich durch geeignete Substitution der Verbindungen erreichen.

Weiterhin bevorzugt ist es, dass zur Herstellung einer erfindungsgemäßen elektronischen Vorrichtung eine oder mehrere Schichten aus Lösung und eine oder mehrere Schichten durch ein Sublimationsverfahren aufgetragen werden.

Die Vorrichtung wird nach Aufbringen der Schichten, je nach Anwendung, strukturiert, kontaktiert und schließlich versiegelt, um schädigende Effekte von Wasser und Luft auszuschließen.

Erfindungsgemäß können die elektronischen Vorrichtungen enthaltend eine oder mehrere Verbindungen gemäß Formel (I) in Displays, als Lichtquellen in Beleuchtungsanwendungen sowie als Lichtquellen in medizinischen und/oder kosmetischen Anwendungen eingesetzt werden.

### Beispiele

### A) Synthesebeispiele

Die nachfolgenden Synthesen werden, sofern nicht anders angegeben, unter einer Schutzgasatmosphäre in getrockneten Lösungsmitteln durchgeführt. Die Edukte können von ALDRICH (Kaliumfluorid (sprühgetrocknet), Tri-*tert*-butylphosphin, Palladium(II)acetat) bezogen werden. 3-Chlor-5,6-diphenyl-1,2,4-triazin kann analog zu EP 577559 dargestellt werden. 2',7'-Di-*tert*-butyl-spiro-9,9'-bifluoren-2,7-bisboronsäureglycolester kann nach WO 02/077060 und 2-Chlor-4,6-diphenyl-1,3,5-triazin nach US 5,438138 dargestellt werden. Spiro-9,9'-bifluoren-2,7-bis(boronsäureglycolester) kann analog zu WO 02/077060 dargestellt werden.

### a) 6-chloroindeno[1,2-c]thiophen-4-on 1a

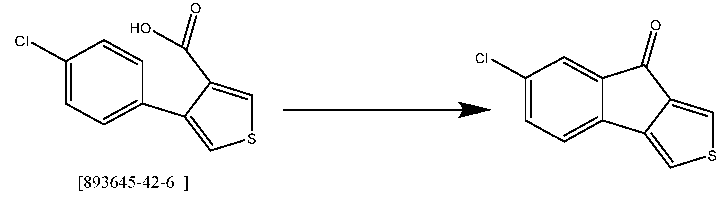

10 g (41,9 mmol) 4-(4-chlorphenyl)thiophen-3-carbonsäure wird in 73 ml Trifluormethansulfonsäure suspendiert und 2 Stunden bei 90 °C erhitzt. Nach Erkalten wird die der Rückstand auf Eis gegossene und mit Ethylacetat extrahiert. Nach der Phasentrennung wird das Lösungsmittel im Vakuum entfernt du der Rückstand chromatographisch getrennt (Heptan/ Ethylacetat, 1.1).

Die Ausbeute beträgt 2,9 g (13,4 mmol), entsprechend 32 % der Theorie.

### b) 6-chloro-4H-indeno[1,2-c]thiophen 1b

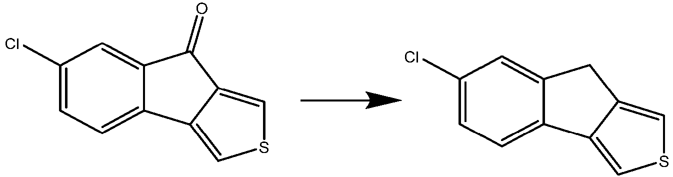

Eine gut gerührte, unter Rückfluss kochende Suspension von 55 g (250 mmol) 6-chloroindeno[1,2-c]thiophen-4-on in einem Gemisch von 1000 ml Toluol und 2000 ml Ethanol wird mit 49 ml (1000 mmol) Hydrazinhydrat und dann mit 3 g frisch bereitetem Raney-Nickel versetzt. Nach 2 h unter Rückfluss lässt man die Mischung erkalten, entfernt das Lösemittel im Vakuum, nimmt den Rückstand in 1000 ml warmem Chloroform auf, filtriert die Lösung über Kieselgel, engt die klare Lösung auf 100 ml ein und gibt 300 ml Ethanol zu. Nach 12 h Stehenlassen werden die farblosen Kristalle abgesaugt und anschließend zweimal aus Chloroform/Ethanol umkristallisiert.

Ausbeute: 49 g (238 mmol), 96 % d. Th.; Reinheit: 94 % nach ¹H-NMR.

Analog können folgende Verbindungen erhalten werden:

| | Edukt 1 | Produkt | Ausbeute |
|---|---|---|---|
| 2b | 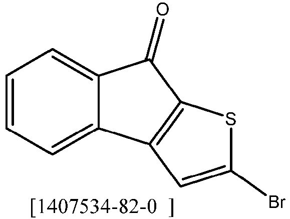 | 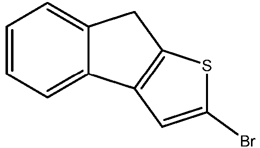 | 89% |
| 3b | 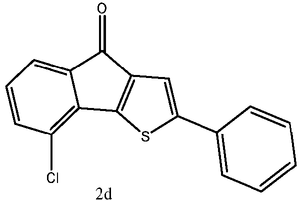 | 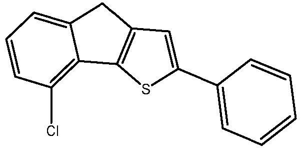 | 875 |
| 4b | 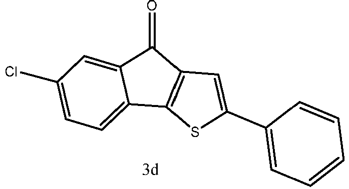 | 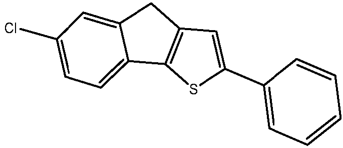 | 90% |
| 5b | 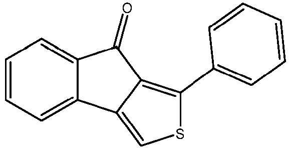 [6263-90-7 ] | 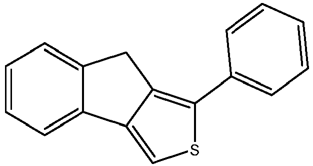 | 89% |

### c) 6-chloro-4,4-dimethyl-indeno[1,2-c]thiophene 1c

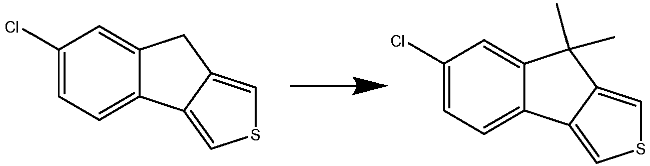

31 g (152 mmol) 6-chloro-4H-indeno[1,2-c]thiophen werden in einem ausgeheizten Kolben in 600 mL getrocknetem DMSO gelöst. Bei Raumtemperatur werden 43.9 g (457 mmol) NaO*^{t}*Bu zugegeben. Die nunmehr blaue Suspension wird auf eine Innentemperatur von 80 °C gebracht. Bei dieser Temperatur werden zur jetzt violetten Lösung 64.8 g (457 mmol) lodmethan so zugetropft, dass die Innentemperatur 90 °C nicht übersteigt (Dauer: ca. 30 min). Der Ansatz wird für weitere 30 min bei 80-90 °C Innentemperatur

Ausbeute: 30,5 g (132 mmol), 88 % d. Th.; Reinheit: 96 % nach ¹H-NMR

Analog können folgende Verbindungen erhalten werden:

| | Edukt 1 | Produkt | Ausbeute |
|---|---|---|---|
| 2c | 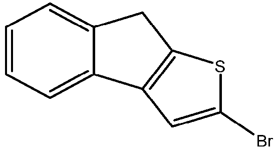 | 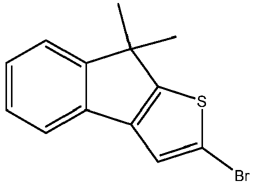 | 66% |
| 3c | 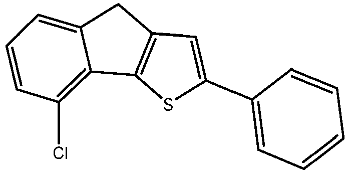 | 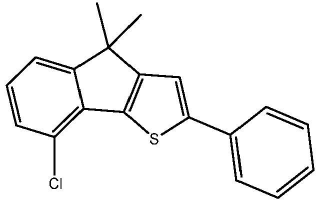 | 67% |
| 4c | 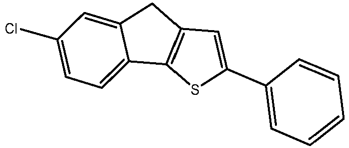 | 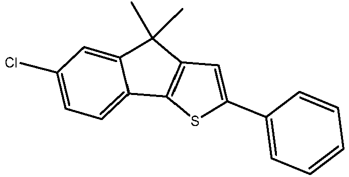 | 58% |
| 5c | 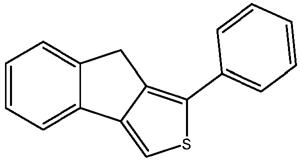 | 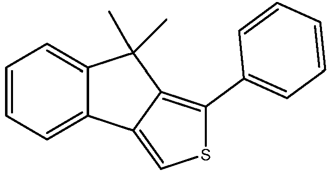 | 60% |

### d) 6-chloro-1,3-diphenyl-indeno[1,2-c]thiophen-4-on 1d

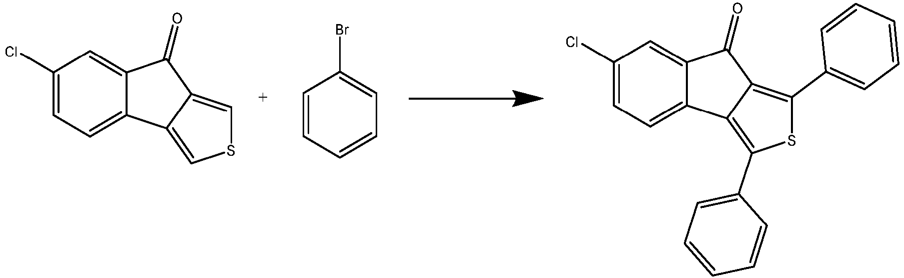

Molsieb 4A wird vorgelegt und ausgeheizt dann unter Schutzgas mit 30 g (136 mmol) 6-chloroindeno[1,2-c]thiophen-4-on, 72 g (220 mmol) Casiumcarbonat, 46 g (294 mmol) Brombenzol, 2,2 g (7,3 mmol) {[1,1'-biphenyl]-2-yl}di-tert-butyl)phosphan und 0,83 g (3,6 mmol) Palladium(II)-acetat in 325 ml trockenem DMF vorgelegt und Über Nacht bei 150 °C gerührt. Danach wird die Mischung heiß über Cellite filtriert mit Ethylacetat nachgewachsen und eingeengt und in Toluol/Heptan umkristallisiert.

Die Ausbeute beträgt 30,4 g (82 mmol), entsprechend 60 % der Theorie Analog können folgende Verbindungen erhalten werden:

| | Edukt 1 | Edukt 2 | Produkt | Ausbeute |
|---|---|---|---|---|
| 2d | 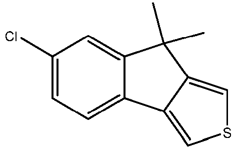 | 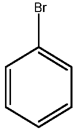 | 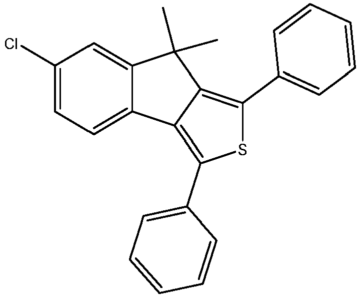 | 67% |
| 3d | 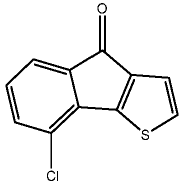 [1367747-08-7 ] | 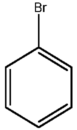 | 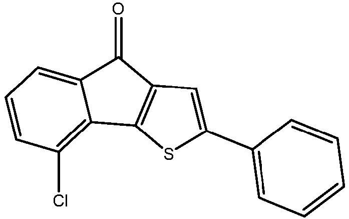 | 57% |
| 4d | 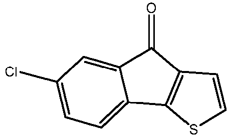 [1511966-22-5 ] | 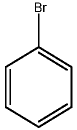 | 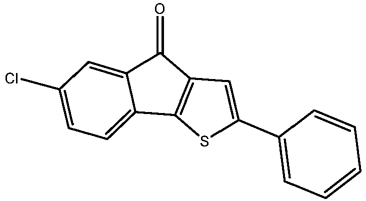 | 61% |
| 5d | 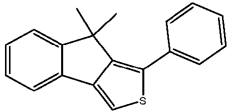 | 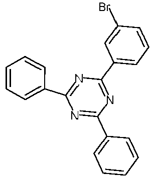 [864377-31-1] | 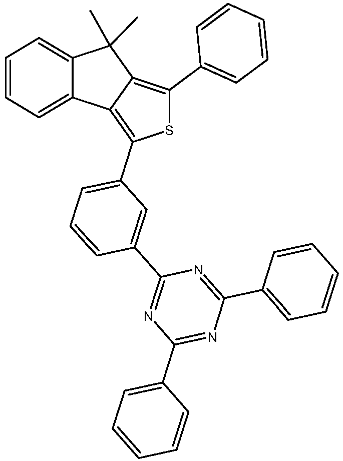 | 60 % |

### e) 4-bromo-1',3'-diphenyl-spiro[fluorene-9,4'-indeno[1,2-c]thiophene] 1e

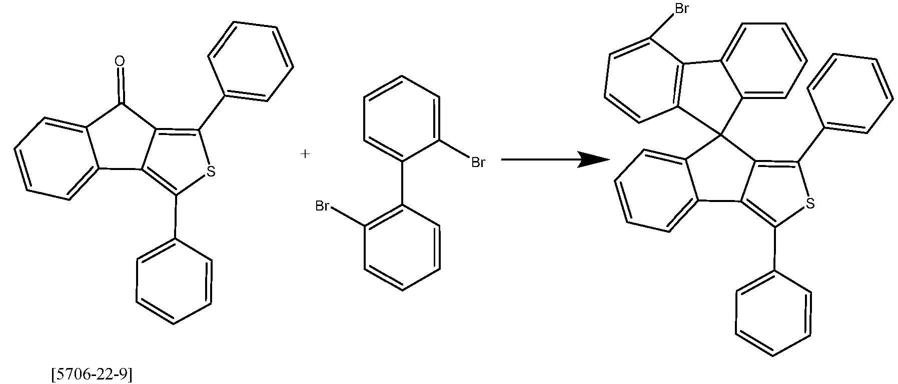

In einem 1-L-Vierhalskolben werden 40 g (112,2 mmol) 2,2'-Dibrombiphenyl in 350 mL THF vorgelegt und auf -78 °C gekühlt. Über einen Tropftrichter werden 49 mL (123,5 mmol) *n*-Butyllithium (2.5 M in *n-*Hexan) bei dieser Temperatur hinzu getropft und 1 h gerührt. Anschließend werden 38 g (112 mmol) 1,3-diphenyl-8*H*-Indeno[1,2-*c*]thiophen-8-one, gelöst in 300 mL THF, über einen Tropftrichter hinzu gegeben und über Nacht auf Raumtemperatur komm lassen. Daraufhin wird THF abrotiert, dann mit 500 mL Wasser /200 ml Ethylacetat hydrolysiert ausgeschüttelt, danach getrocknet. Der Feststoff wird in 440 ml Toluol gelöst und mit 2,1, (11,198 mmol) Toluol-4-sulfonsäure-Monohydrat über Nacht unter Rückfluss gekocht. Danach mit Wasser und Ethylacetat versetzt, Phasen getrennt und die organischen Lösungsmittel am Rotationsverdampfer entfernt. Der Feststoff wird über Aluminiumoxid mit *n*-Heptan/Toluol (1:1) heißextrahiert und in Heptan/Toluol umkristallisiert.

Die Ausbeute beträgt 58,8 g (106 mmol), entsprechend 90 % der Theorie.

Analog können folgende Verbindungen erhalten werden:

| | Edukt 1 | Edukt 2 | Produkt | Ausbeute |
|---|---|---|---|---|
| 2e | 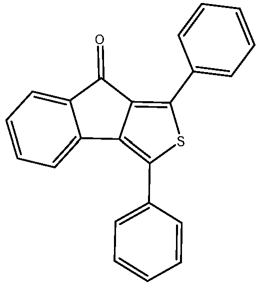 [5706-22-9] | 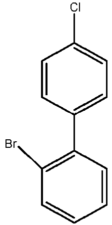 | 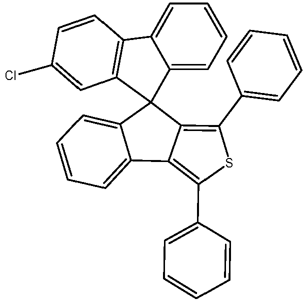 | 93% |
| 3e | 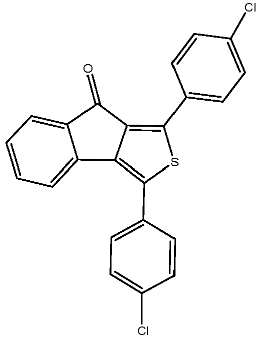 [6072-53-3 ] | 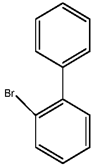 | 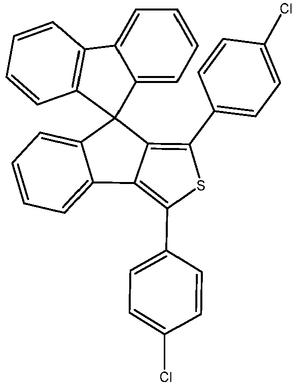 | 89% |
| 4e | 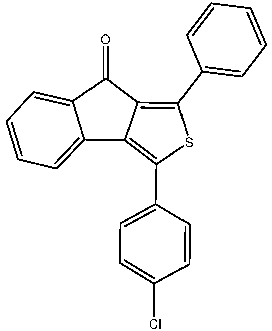 [1924664-17-4] | 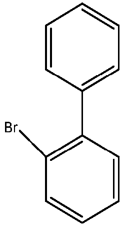 | 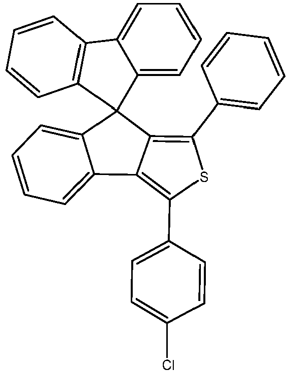 | 90% |
| 5e | 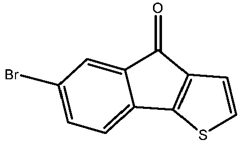 [1346266-91-8 ] | 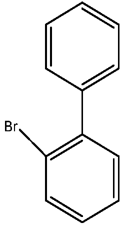 | 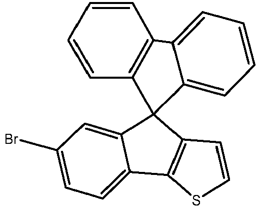 | 87% |
| 6e | 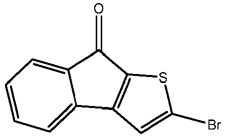 [1407534-82-0 ] | 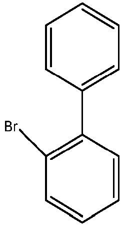 | 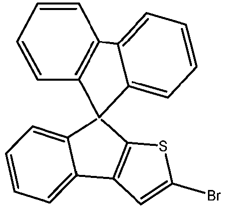 | 84% |
| 7e | 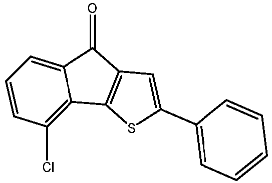 | 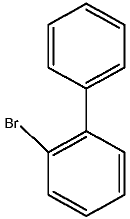 | 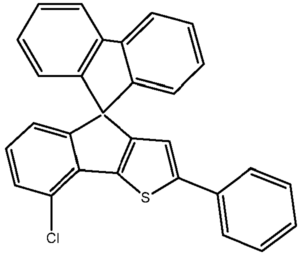 | 91% |
| 8e | 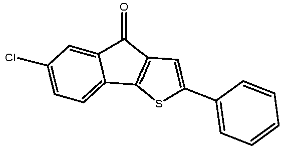 | 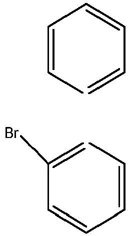 | 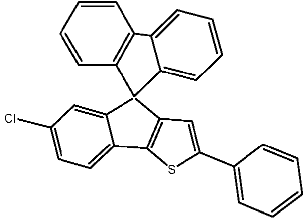 | 92% |

### f) 2-bromospiro[4a,8a-dihydroindeno[1,2-b]furan-4,9'-fluorene] 1f

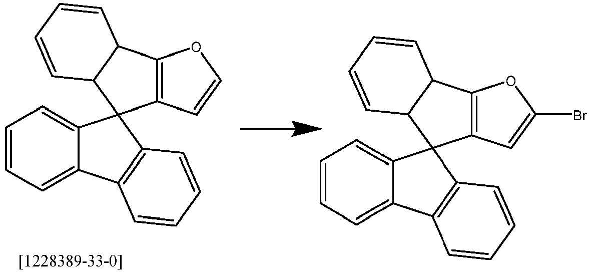

Eine Lösung von 46,2 g, (150 mmol) Spiro[4a,8a-dihydroindeno[1,2-b]furan-4,9'-fluorene]
in Chloroform (900 mL) wird bei 0 °C unter Lichtausschluss portionsweise mit N-Bromsuccinimid (26,6 g, 150 mmol) versetzt und 2 h bei dieser Temperatur gerührt. Die Reaktion wird durch Zugabe von Natriumsulfit-Lösung beendet und weitere 30 min bei Raumtemperatur gerührt. Nach Phasentrennung wird die organische Phase mit Wasser gewaschen und die wässrige Phase mit Dichlormethan extrahiert. Die vereinigten organischen Phasen werden über Natriumsulfat getrocknet und im Vakuum eingeengt. Der Rückstand wird in Toluol gelöst und über Kieselgel filtriert. Anschließend wird das Rohprodukt aus Toluol/Heptan umkristallisiert.

Ausbeute: 44 g (115 mmol), 77 % d. Th., farbloser Feststoff.

### g) (1',3'-diphenylspiro[fluorene-9,4'-indeno[1,2-c]thiophene]-4-yl)boronsäure 1g

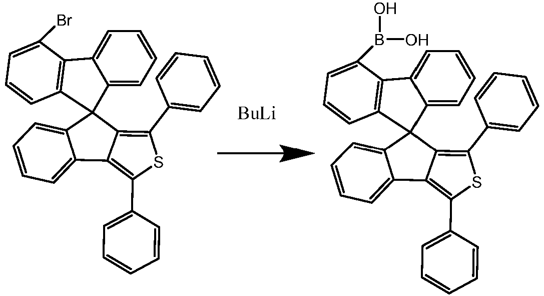

Eine auf -78 °C gekühlte Lösung von 144 g (270 mmol) 4-bromo-1',3'-diphenyl-spiro[fluorene-9,4'-indeno[1,2-c]thiophene] in 1500 ml Diethylether wird tropfenweise mit 110 ml (276 mmol) n-Buthyllithium (2.5 M in Hexan) versetzt. Die Reaktionsmischung wird 30 min. bei -78 °C gerührt. Man lässt auf Raumtemperatur kommen, kühlt erneut auf -78 °C und versetzt dann schnell mit einer Mischung von 40 ml (351 mmol) Trimethylborat in 50 ml Diethylether. Nach Erwärmen auf -10 °C wird mit 135 ml 2 N Salzsäure hydrolysiert. Die organische Phase wird abgetrennt, mit Wasser gewaschen, über Natriumsulfat getrocknet und zur Trocken eingeengt. Der Rückstand wird in 300 ml n-Heptan aufgenommen, der farblose Feststoff wird abgesaugt, mit n-Heptan gewaschen und im Vakuum getrocknet.

Ausbeute: 135 g (262 mmol), 97 % d. Th.; Reinheit: 96 % nach HPLC.

Analog können folgende Verbindungen erhalten werden:

| | Edukt 1 | Produkt | Ausbeute |
|---|---|---|---|
| 2g | 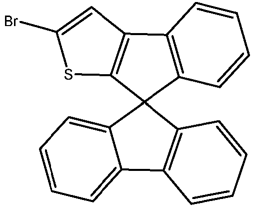 [91155-62-7] | 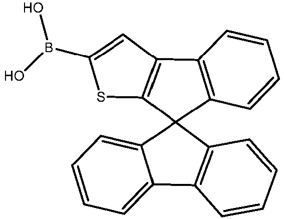 | 87% |
| 3g | 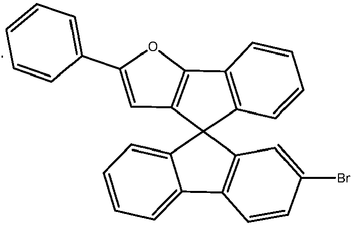 [2291155-93-4] | 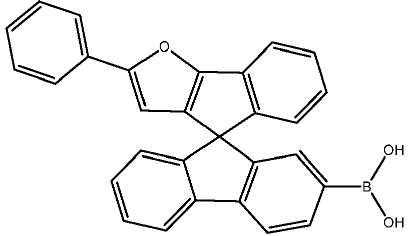 | 84% |
| 4g | 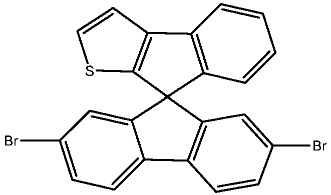 [899793-65-8] | 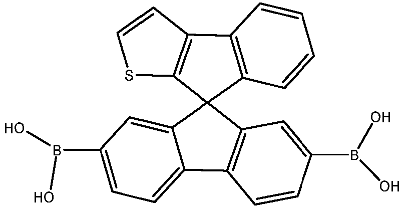 | 71% |
| 5g | 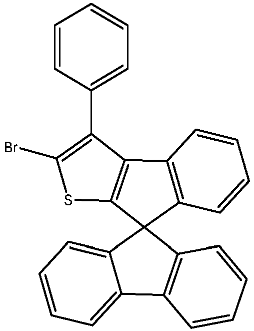 [885484-69-5 | 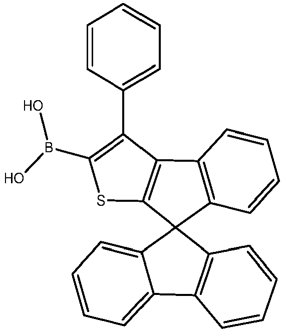 | 87% |
| 6g | 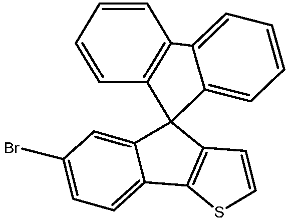 | 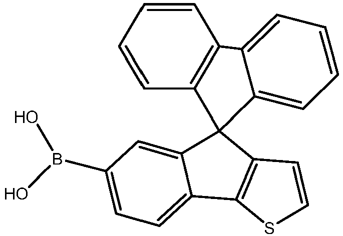 | 76% |
| 7g | 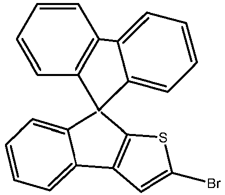 | 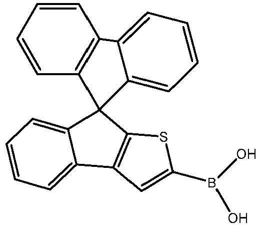 | 91% |
| 8g | 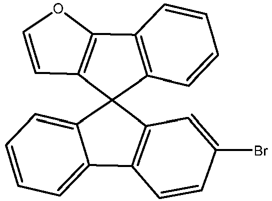 [2291155-80-9 | 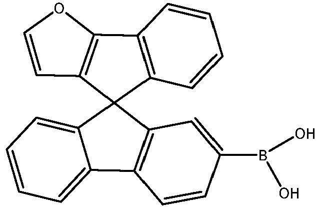 | 77% |
| 9g | 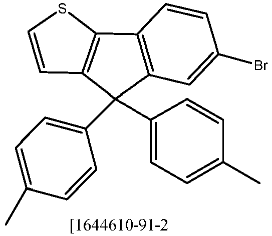 | 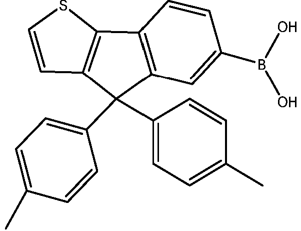 | 67% |
| 10g | 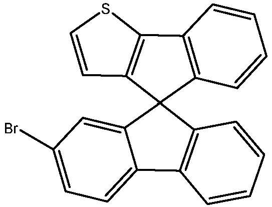 [2291155-62-7] | 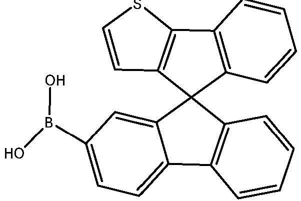 | 65% |
| 11g | 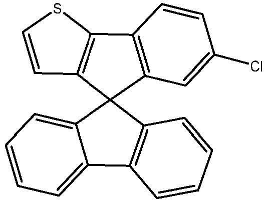 [2165343-88-2 | 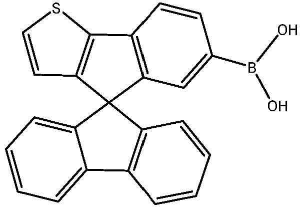 | 52% |
| 12g | 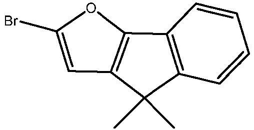 [2104697-12-10] | 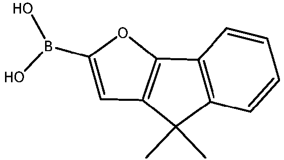 | 86% |
| 13g | 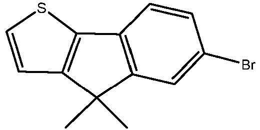 [1346266-93-0] | 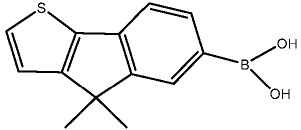 | 66% |
| 14g | 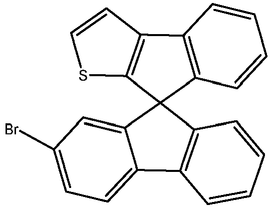 [911649-11-1] | 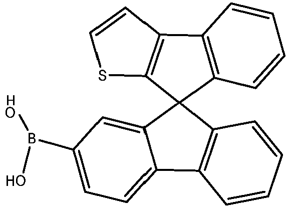 | 62% |
| 15g | 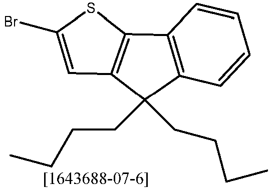 | 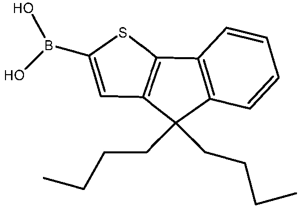 | 87% |
| 16g | 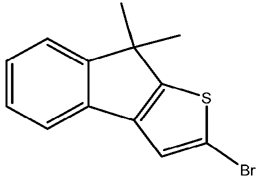 | 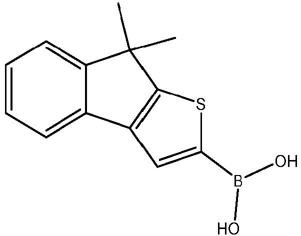 | 67% |
| 17g | 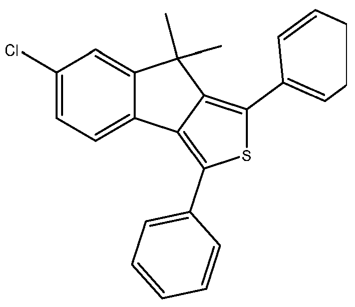 | 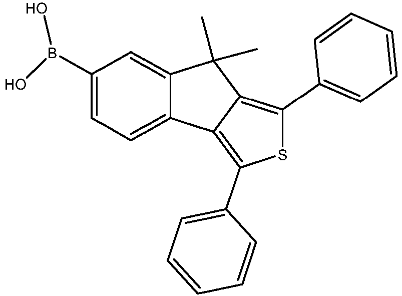 | 59% |

### h) 2-(1',3'-diphenylspiro[fluorene-9,4'-indeno[1,2-c]thiophene]-4-yl)-4,6-diphenyl-1,3,5-triazine 1h

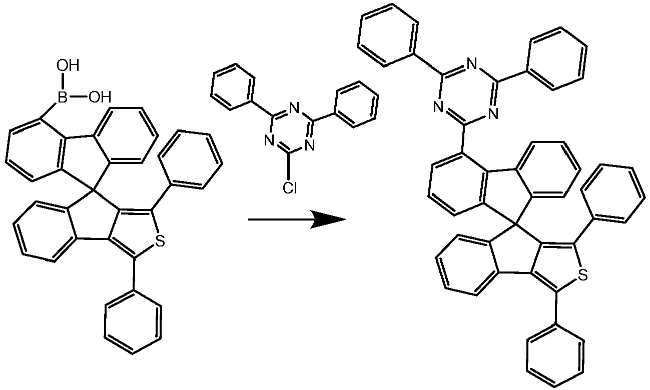

57 g (110 mmol) (1',3'-diphenylspiro[fluorene-9,4'-indeno[1,2-c]thiophene]-4-yl)boronsäure, 30 g (110.0 mmol) 2-Chlor-4,6-diphenyl-1,3,5-triazin und 45 g (210.0 mmol) Trikaliumphosphat werden in 500 mL Toulol, 500 mL Dioxan und 500 mL Wasser suspendiert. Zu dieser Suspension werden 913 mg (3.0 mmol) Tri-o-tolylphosphin und dann 112 mg (0.5 mmol) Palladium (II)acetat gegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trocken eingeengt. Der Rückstand wird aus Toluol und aus Dichlormethan / *iso*-Propanol umkristallisiert und abschließend im Hochvakuum (p = 5 x 10⁻⁵ mbar, T = 377 °C) sublimiert. Die Ausbeute beträgt 63,6 g (90 mmol), entsprechend 82 % der Theorie.

Analog können folgende Verbindungen erhalten werden:

| | Edukt 1 | Edukt 2 | Produkt | Ausbeute |
|---|---|---|---|---|
| 2h | 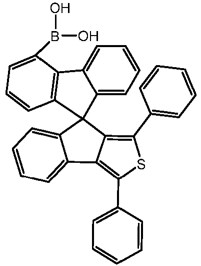 | 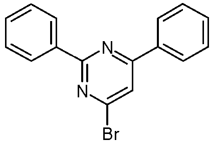 40734-4-5 | 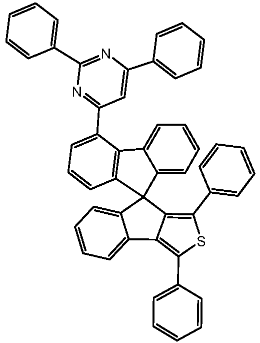 | 84% |
| 3h | 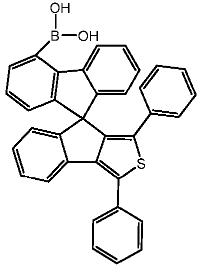 | 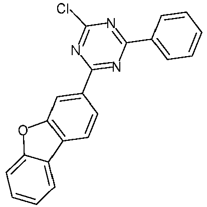 [2142681-84-1] | 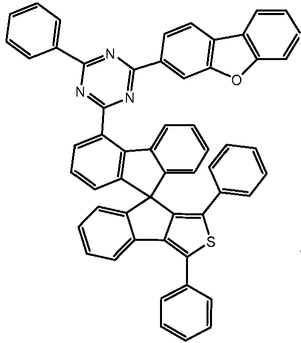 | 88% |
| 4h | 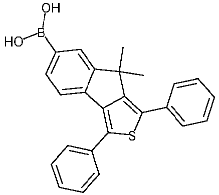 | 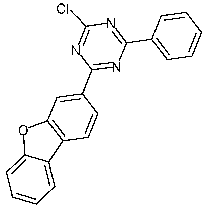 [2142681-84-1] | 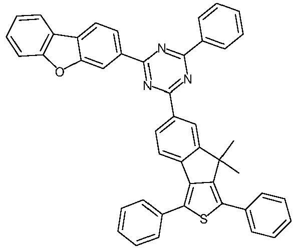 | 67% |
| 5h | 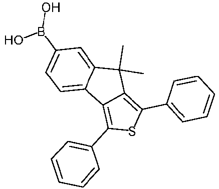 | 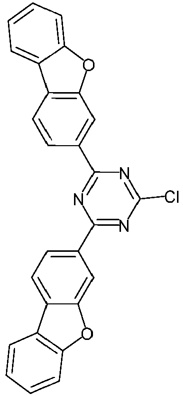 [2251105-15-2 ] | 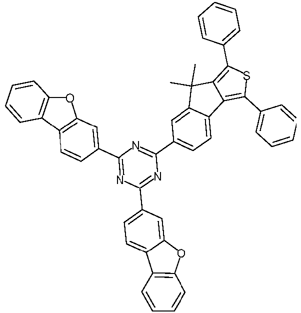 | 71% |
| 6h | 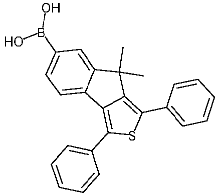 | 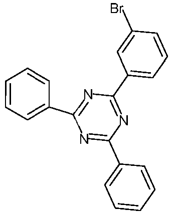 [864377-31-1] | 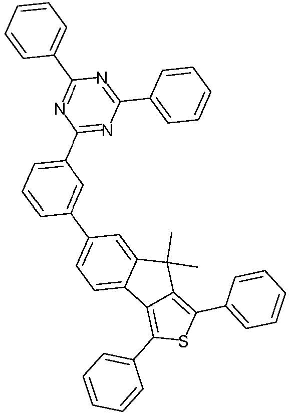 | 75% |
| 7h | 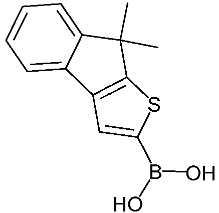 | 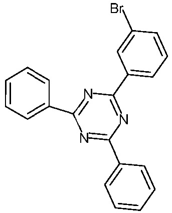 [864377-31-1] | 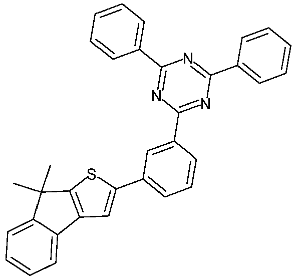 | 79% |
| 8h | 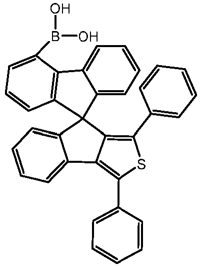 | 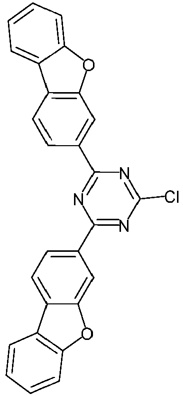 [2251105-15-2 ] | 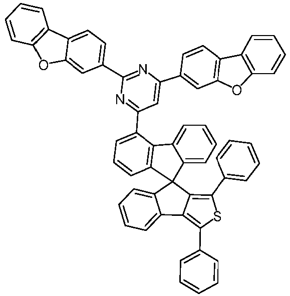 | 85% |
| 9h | 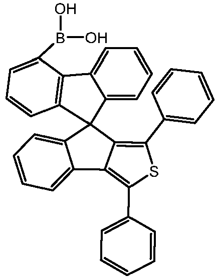 | 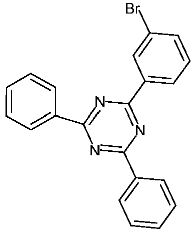 [864377-31-1] | 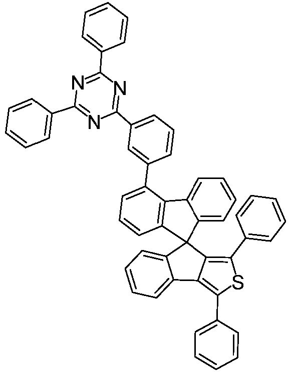 | 78% |
| 10h | 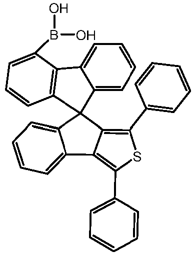 | 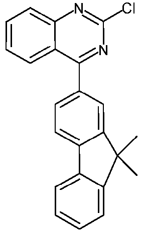 [1373265-66-7] | 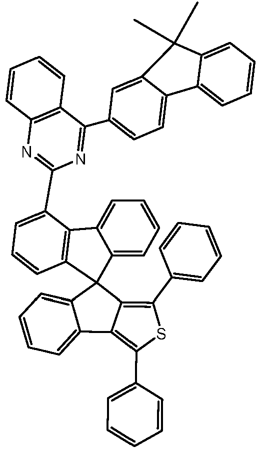 | 80% |
| 11h | 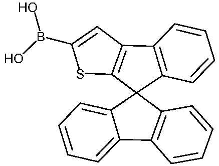 | 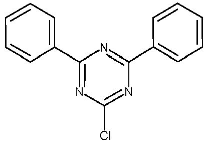 [3842-55-5] | 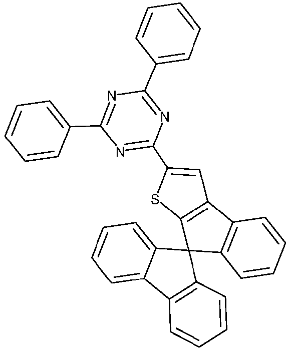 | 81% |
| 12h | 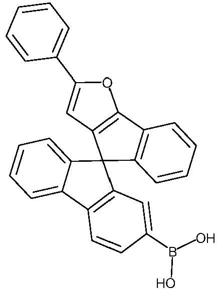 | 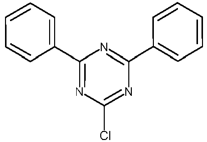 [3842-55-5] | 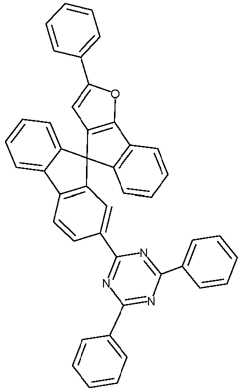 | 82 % |
| 13h | 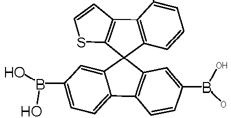 | 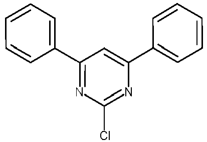 [2915-16-4] | 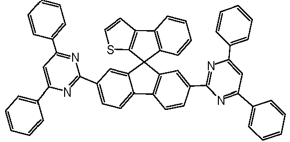 | 79% |
| 14h | 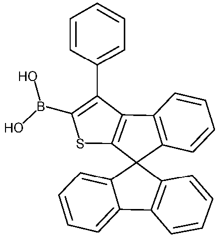 | 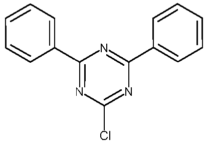 [3842-55-5] | 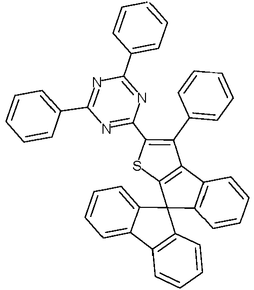 | 78% |
| 15h | 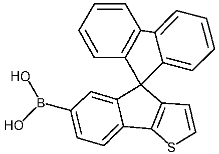 | 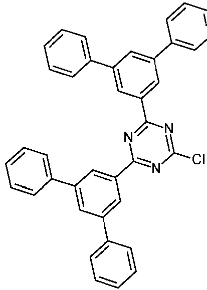 [1205748-51-1] | 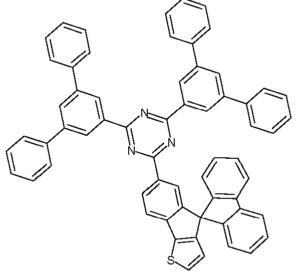 | 87% |
| 16h | 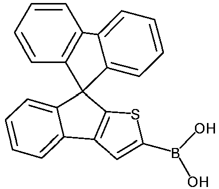 | 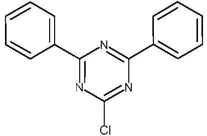 [3842-55-5] | 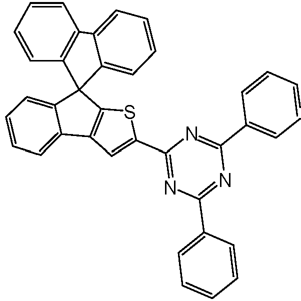 | 72% |
| 17h | 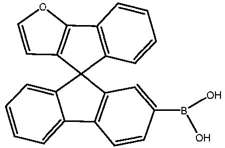 | 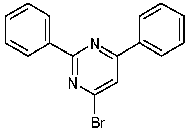 [40734-4-5] | 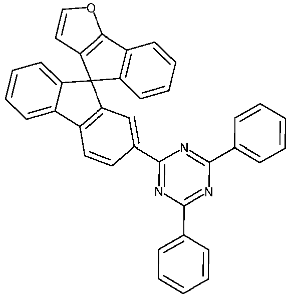 | 61% |
| 18h | 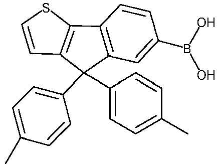 | 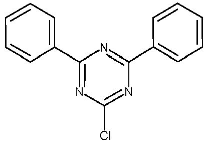 [3842-55-5] | 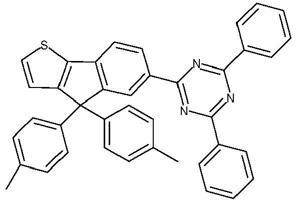 | 79% |
| 19h | 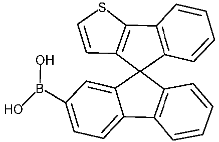 | 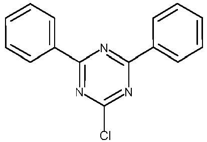 [3842-55-5] | 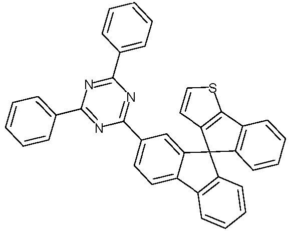 | 75% |
| 20h | 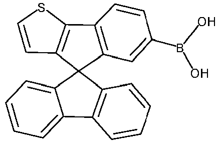 | 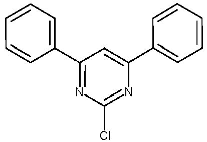 [2915-16-4] | 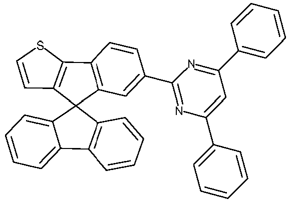 | 67% |
| 21h | 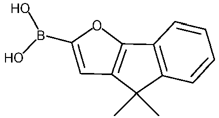 | 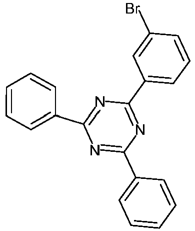 [864377-31-1] | 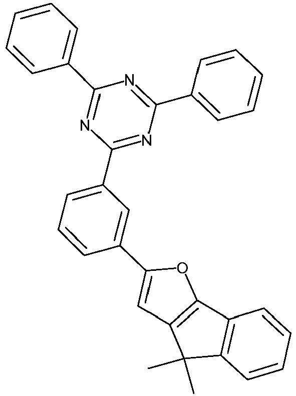 | 82% |
| 22h | 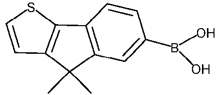 | 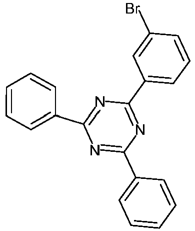 [864377-31-1] | 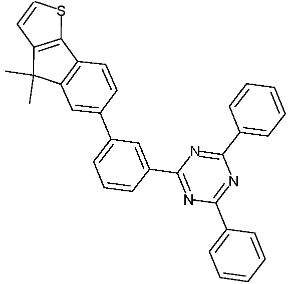 | 76% |
| 23h | 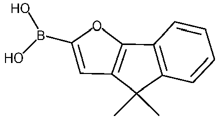 | 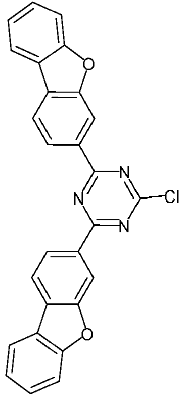 [2251105-15-2 ] | 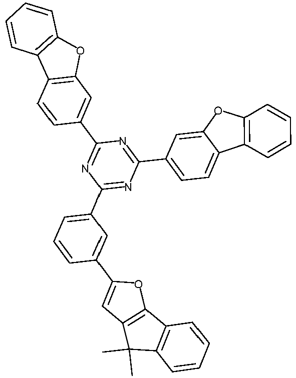 | 71% |
| 24h | 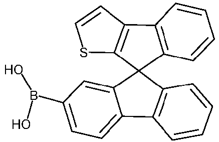 | 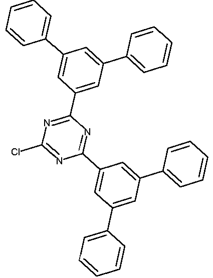 [1205748-51-1] | 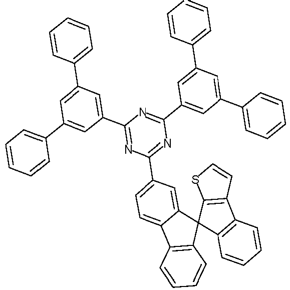 | 73% |
| 25h | 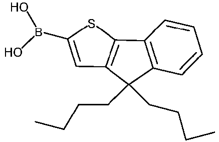 | 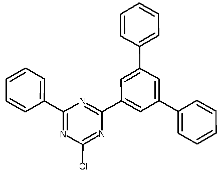 [1616231-59-41 | 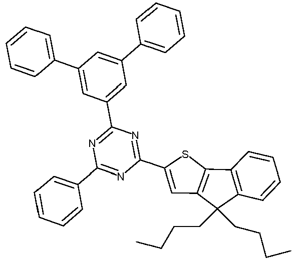 | 70% |
| 26h | 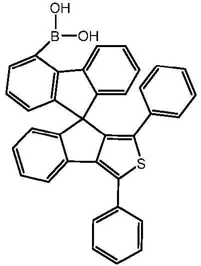 | 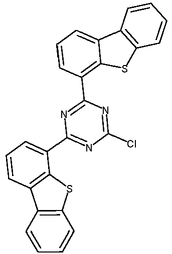 [1387596-01-1] | 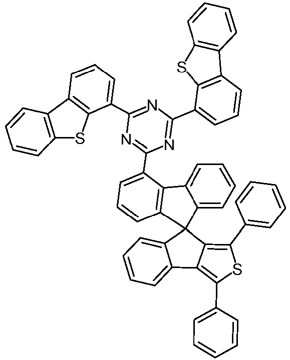 | 67% |
| 27h | 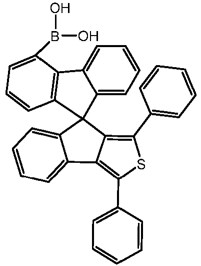 | 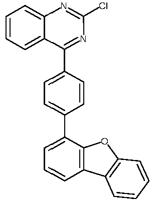 [1403252-58-31 | 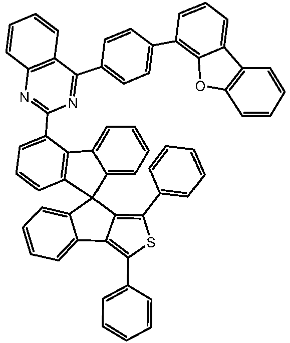 | 81% |
| 28h | 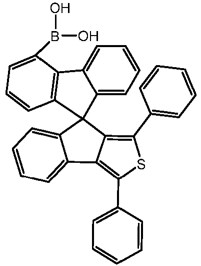 | 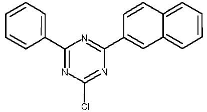 [1342819-12-8] | 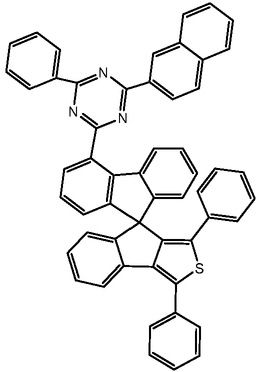 | 76% |
| 29h | 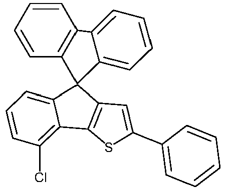 | 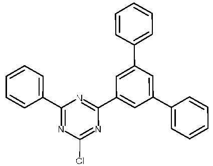 [1616231-59-4] | 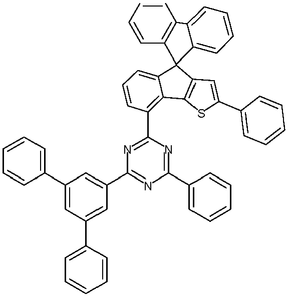 | 74% |
| 30h | 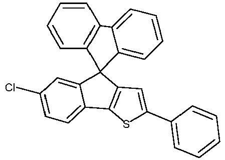 | 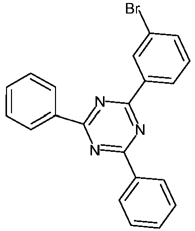 [864377-31-1] | 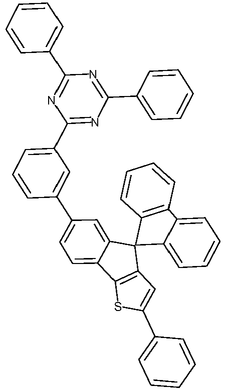 | 75% |
| 31h | 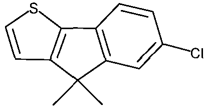 [2165343-79-1] | 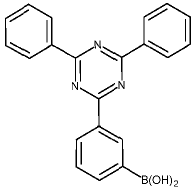 [1612243-82-9] | 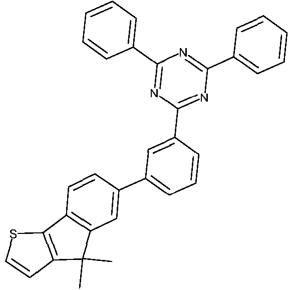 | 72% |
| 32h | 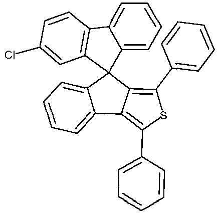 | 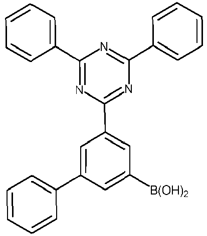 [1518823-39-6] | 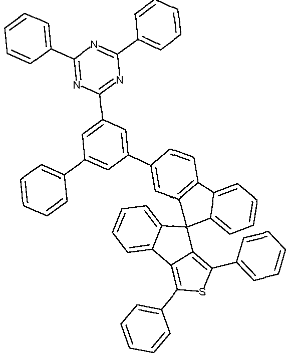 | 70% |
| 33h | 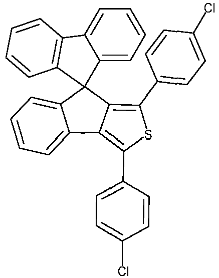 | 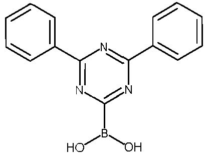 [1252825-65-6] | 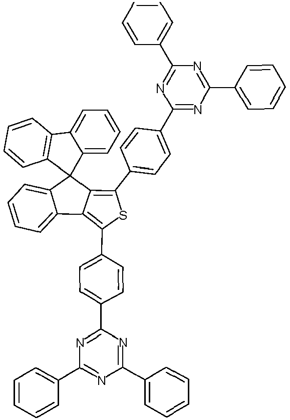 | 69% |
| 34h | 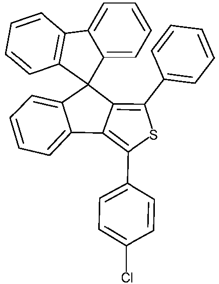 | 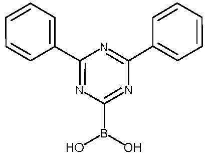 [1252825-65-6] | 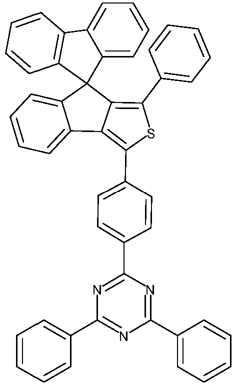 | 81% |
| 35h | 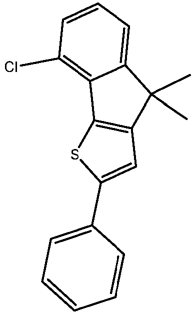 | 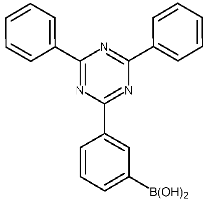 [1612243-82-9] | 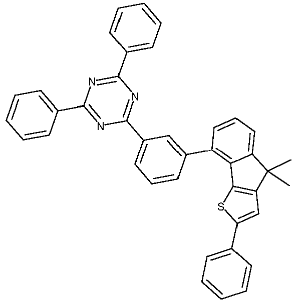 | 76% |
| 36h | 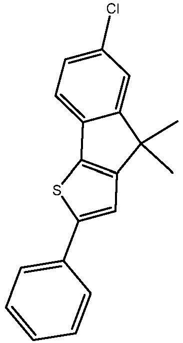 | 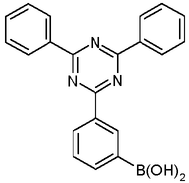 [1612243-82-9] | 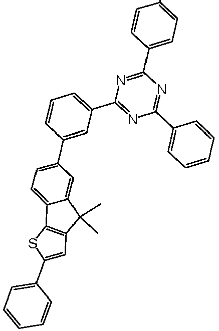 | 66% |
| 37h | 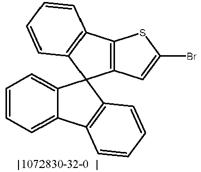 | 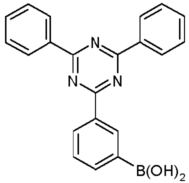 [1612243-82-9] | 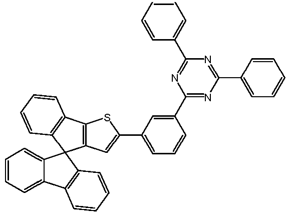 | 64% |
| 36h | 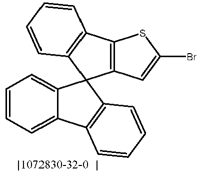 | 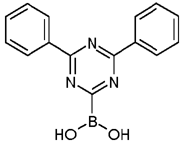 [1252825-65-6] | 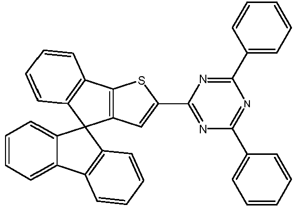 | 67% |
| 37h | 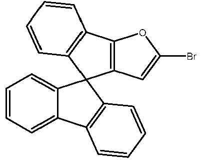 | 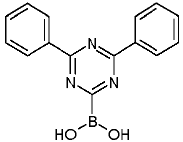 [1252825-65-6] | 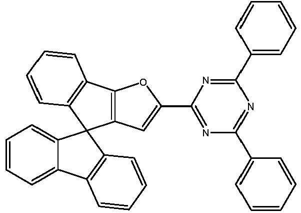 | 65% |
| 38h | 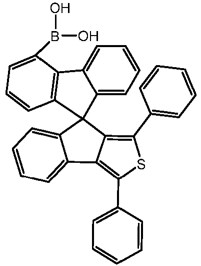 | 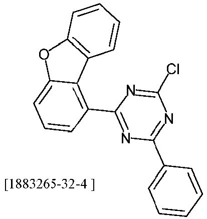 | 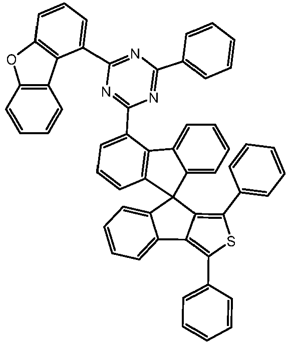 | 83% |

### B) Devicebeispiele

### 1) Allgemeines Herstellungsverfahren für die OLEDs und Charakterisierung der OLEDs

Glasplättchen, die mit strukturiertem ITO (Indium Zinn Oxid) der Dicke 50 nm beschichtet sind, bilden die Substrate, auf welche die OLEDs aufgebracht werden.

Die OLEDs haben prinzipiell folgenden Schichtaufbau: Substrat / optionale interlayer (IL) /Lochinjektionsschicht (HIL) / Lochtransportschicht (HTL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist den folgenden Tabellen zu entnehmen. Der Aufbau der hergestellten OLEDs und die zur Herstellung der OLEDs verwendeten Materialien sind in untenstehenden Tabellen 2 und 3 gezeigt.

Alle Materialien werden in einer Vakuumkammer thermisch aufgedampft. Dabei besteht die Emissionsschicht aus mindestens einem Matrixmaterial (Hostmaterial) und einem emittierenden Dotierstoff, der dem Matrixmaterial bzw. den Matrixmaterialien durch Co-Verdampfung in einem bestimmten Volumenanteil beigemischt wird. Eine Angabe wie EG1:TER5 (97%:3%) bedeutet hierbei, dass das Material EG1 in einem Volumenanteil von 97% und TER5 in einem Anteil von 3% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (SE, gemessen in cd/A) und die externe Quanteneffizienz (EQE, gemessen in %) in Abhängigkeit der Leuchtdichte, berechnet aus Strom-Spannungs-Leuchtdichte-Kennlinien unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 4 bezeichnet die Spannung, die für eine Leuchtdichte von 1000 cd/m² benötigt wird. SE1000 und EQE1000 bezeichnen die Stromeffizienz bzw. die externe Quanteneffizienz, die bei 1000cd/m² erreicht werden.

Als Lebensdauer LD wird die Zeit definiert, nach der die Leuchtdichte bei Betrieb mit konstanter Stromdichte j₀ von der Startleuchtdichte auf einen gewissen Anteil L1 absinkt. Eine Angabe L1=95% inTabelle 4 bedeutet, dass die in Spalte LD angegebene Lebensdauer der Zeit entspricht, nach der die Leuchtdichte auf 95% ihres Anfangswertes absinkt.

### 2) Verwendung und Vorteil der erfindungsgemäßen Verbindungen der Formel (I) in OLEDs

Üblicherweise kommt in der Emissionsschicht von OLEDs eine Mischung aus zwei Hostmaterialien zum Einsatz, um eine optimale Ladungsbalance und damit sehr gute Leistungsdaten der OLED zu erreichen. Hinsichtlich einer vereinfachten Herstellung von OLEDs ist eine Reduzierung der zu verwendenen Materialien erstrebenswert. In der Emissionsschicht ist also der Einsatz von lediglich einem Hostmaterial vorteilhaft.

### 2a) Verwendung von erfindungsgemäßen Verbindungen in der Emissionsschicht phosphoreszierender roter OLEDs

Mit dem Einsatz der erfindungsgemäßen Verbindungen EG1 bis EG2 in den Beispielen E1 und E2 sowie E5 und E6 als Matrixmaterial in der Emissionsschicht phosphoreszierender roter OLEDs kann gezeigt werden, dass die Verwendung als Einzelmaterial (E1 und E5) und besonders in Mischung mit einem zweiten Hostmaterial IC2 (E2 und E6) verbesserte Leistungsdaten der OLEDs gegenüber dem Stand der Technik, vor allem bezüglich Lebensdauer und Effizienz, liefert.

### 2b) Verwendung von erfindungsgemäßen Verbindungen in der Emissionsschicht phosphoreszierender grüner OLEDs

Mit dem Einsatz der erfindungsgemäßen Verbindungen EG3 bis EG6 mit höhere Triplett-Energie in den Beispielen E9 bis E12 als Matrixmaterial in der Emissionsschicht phosphoreszierender grüne OLEDs kann gezeigt werden, dass die Verwendung in Mischung mit einem zweiten Hostmaterial IC2 eine gute Lebensdauer liefert.

In Tabelle 4 sind die Ergebnisse der Leistungsdaten der OLEDs aus den Beispielen E1 bis E12 zusammengefasst.

### 2c) Verwendung von erfindungsgemäßen Verbindungen als Elektronentransportmaterial in der Elektronentransportschicht von OLEDs

Bei Verwendung der erfindungsgemäßen Verbindungen EG3 bis EG6 als Elektronentransportmaterial erhält man wesentlich geringere Spannung und bessere Effizienz und Lebensdauer (Beispiele E15 bis E18) als mit der Substanz SdT1 und SdT2 gemäß dem Stand der Technik (Beispiele E13 bis E14)

In Tabelle 5 sind die Ergebnisse der Leistungsdaten der OLEDs aus den Beispielen E13 bis E18 zusammengefasst.

**Tabelle 2: Aufbau der OLEDs**

| **Bsp** | **IL** | **HIL Dicke** | **HTL Dicke** | **EBL Dicke** | **EML Dicke** | **HBL Dicke** | **ETL Dicke** | **EIL Dicke** |
|---|---|---|---|---|---|---|---|---|
| E1 | | HATCN 5nm | SpMA1 125nm | SpMA3 10nm | EG1:TER5 (97%:3%) 35nm | ST2 10nm | ST2:LiQ (50%:50%) 30nm | LiQ 1nm |
| E2 | | HATCN 5nm | SpMA1 125nm | SpMA3 10nm | EG1:IC2:TER5 (44%:44%:12%) 30nm | ST2 10nm | ST2:LiQ (50%:50%) 30nm | LiQ 1nm |
| E3 | | HATCN 5nm | SpMA1 125nm | SpMA3 10nm | SdT1:TER5 (97%:3%) 35nm | ST2 10nm | ST2:LiQ (50%:50%) 30nm | LiQ 1nm |
| E4 | | HATCN 5nm | SpMA1 125nm | SpMA3 10nm | SdT1:IC2:TER5 (44%:44%:12%) 30nm | ST2 10nm | ST2:LiQ (50%:50%) 30nm | LiQ 1nm |
| E5 | | HATCN 5nm | SpMA1 125nm | SpMA3 10nm | EG2:TER5 (97%:3%) 35nm | ST2 10nm | ST2:LiQ (50%:50%) 30nm | LiQ 1nm |
| E6 | | HATCN 5nm | SpMA1 125nm | SpMA3 10nm | EG2:IC2:TER5 (44%:44%:12%) 30nm | ST2 10nm | ST2:LiQ (50%:50%) 30nm | LiQ 1nm |
| E7 | | HATCN 5nm | SpMA1 125nm | SpMA3 10nm | SdT2:TER5 (97%:3%) 35nm | ST2 10nm | ST2:LiQ (50%:50%) 30nm | LiQ 1nm |
| E8 | | HATCN 5nm | SpMA1 125nm | SpMA3 10nm | SdT2:IC2:TER5 (44%:44%:12%) 30nm | ST2 10nm | ST2:LiQ (50%:50%) 30nm | LiQ 1nm |
| E9 | | HATCN 5nm | SpMA1 125nm | SpMA3 10nm | EG3:IC2:TEG1 (44%:44%:12%) 30nm | ST2 10nm | ST2:LiQ (50%:50%) 30nm | LiQ 1nm |
| E10 | | HATCN 5nm | SpMA1 125nm | SpMA3 10nm | EG4:IC2: TEG1 (44%:44%:12%) 30nm | ST2 10nm | ST2:LiQ (50%:50%) 30nm | LiQ 1nm |
| E11 | | HATCN 5nm | SpMA1 125nm | SpMA3 10nm | EG5:IC2: TEG1 (44%:44%:12%) 30nm | ST2 10nm | ST2:LiQ (50%:50%) 30nm | LiQ 1nm |
| E12 | | HATCN 5nm | SpMA1 125nm | SpMA3 10nm | EG6:IC2:TEG1 (44%:44%:12%) 30nm | ST2 10nm | ST2:LiQ (50%:50%) 30nm | LiQ 1nm |
| E13 | SpA1 140nm | | HATCN 5nm | SpMA1 20nm | M2:SEB (95%:5%) 20nm | - | SdT1:LiQ (50%:50%) 30nm | - |
| E14 | SpA1 70nm | | HATCN 5nm | SpMA1 90nm | IC1:TEG1 (90%:10%) 30nm | IC1 10nm | SdT2:LiQ (50%:50%) 30nm | - |
| E15 | SpA1 140nm | | HATCN 5nm | SpMA1 20nm | M2:SEB (95%:5%) 20nm | - | EG4:LiQ (50%:50%) 30nm | - |
| E16 | SpA1 70nm | | HATCN 5nm | SpMA1 90nm | IC1:TEG1 (90%:10%) 30nm | IC1 10nm | EG4:LiQ (50%:50%) 30nm | - |
| E17 | SpA1 140nm | | HATCN 5nm | SpMA1 20nm | M2:SEB (95%:5%) 20nm | - | EG5:LiQ (50%:50%) 30nm | - |
| E18 | SpA1 70nm | | HATCN 5nm | SpMA1 90nm | IC1:TEG1 (90%:10%) 30nm | - | EG6:LiQ (50%:50%) 40nm | - |

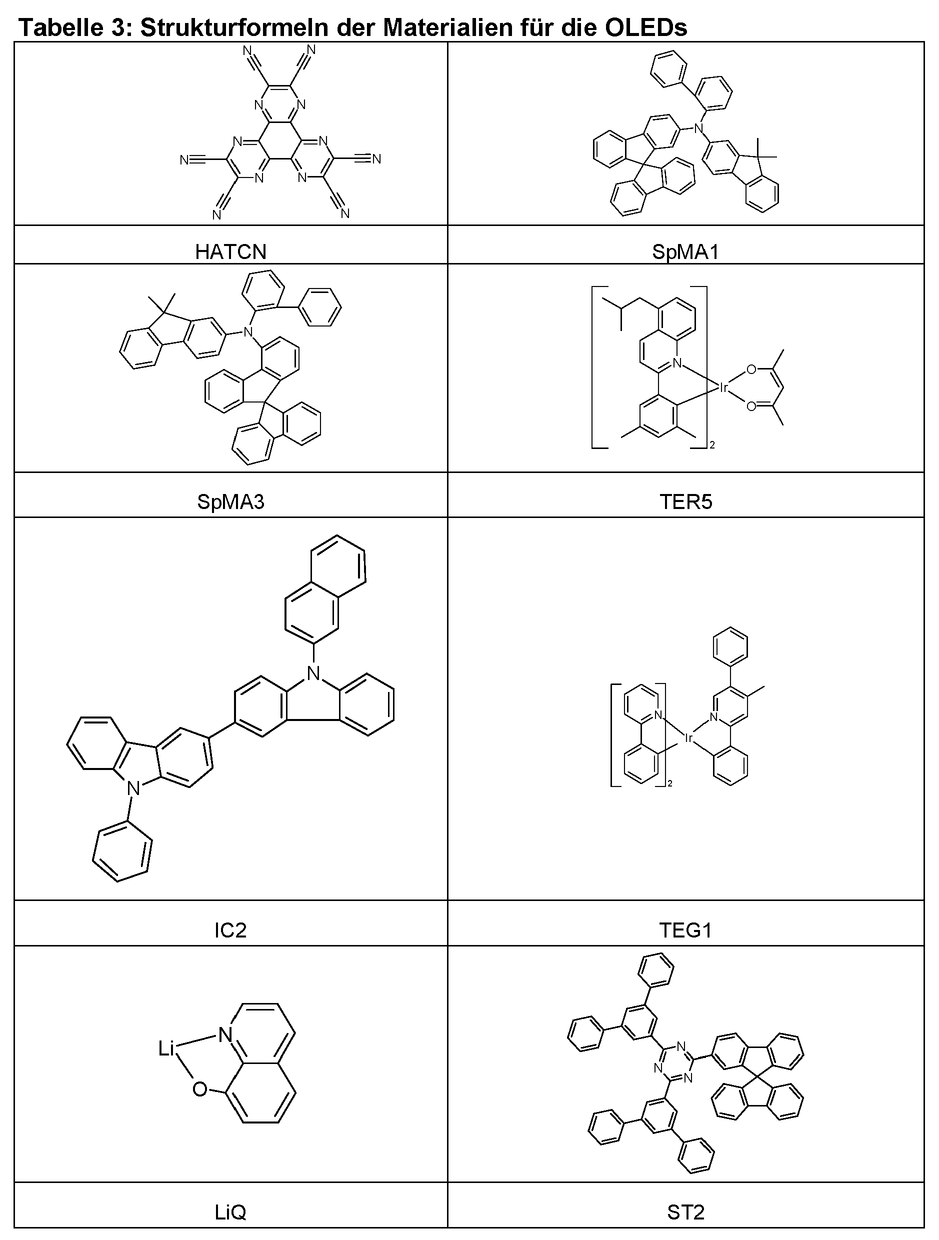

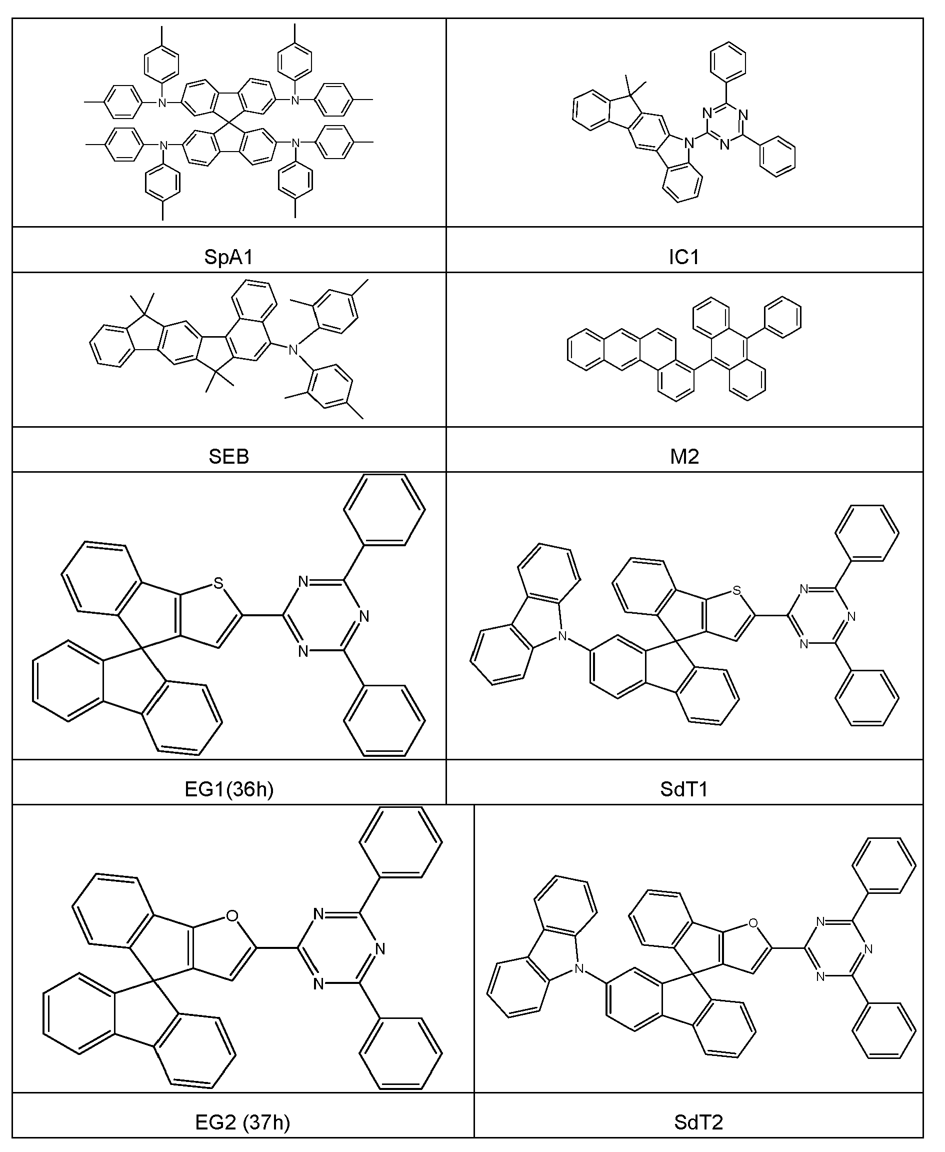

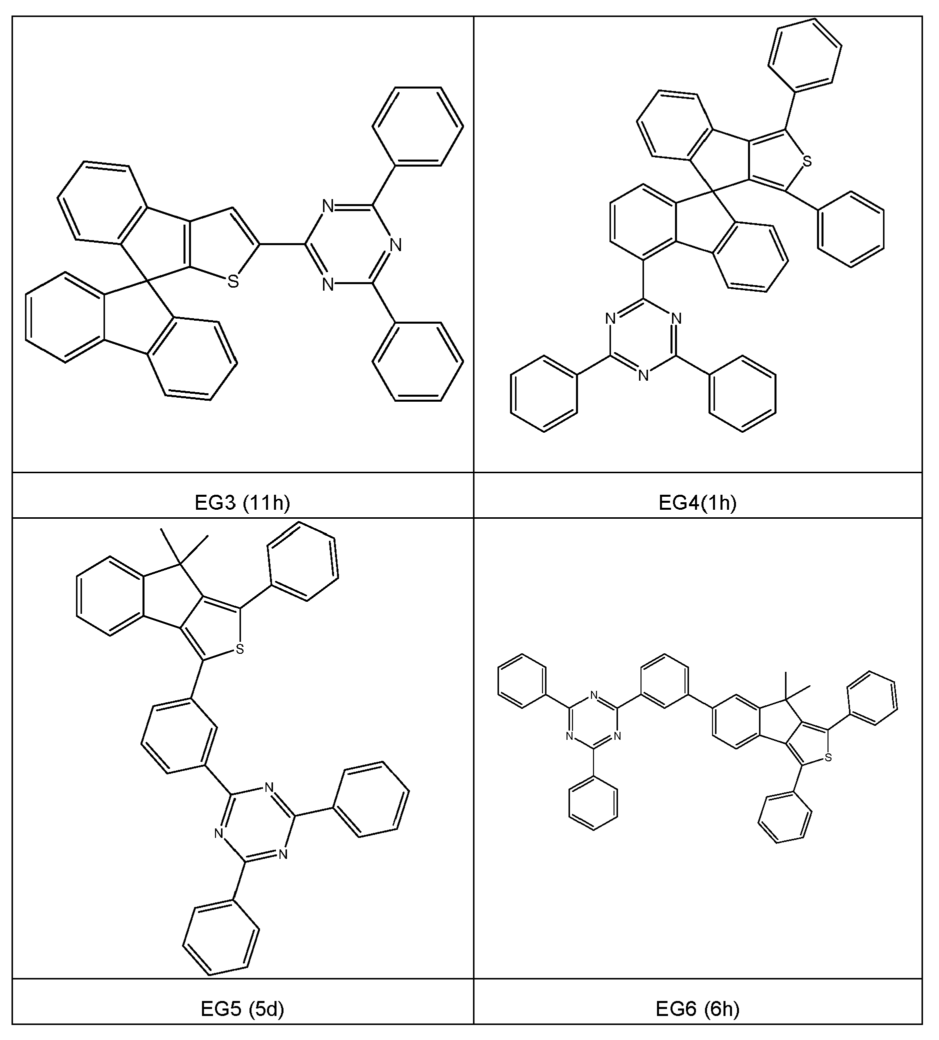

**Tabelle 4: Daten der OLEDs**

| **Bsp.** | **U1000 (V)** | **SE10 00 (cd/A)** | **EQE 1000 (%)** | **CIE x/y bei 1000 cd/m²** | **j₀ (mA/cm² )** | **L1 (%)** | **LD (h)** |
|---|---|---|---|---|---|---|---|
| E1 | 4,3 | 23 | 15 | 0.67/0.33 | 20 | 95 | 500 |
| E2 | 3.3 | 23 | 20 | 0.66/0.34 | 20 | 95 | 1110 |
| E3 | 3.9 | 23 | 14 | 0.66/0.33 | 20 | 95 | 410 |
| E4 | 3.4 | 24 | 15 | 0.67/0.34 | 20 | 95 | 840 |
| E5 | 3.9 | 23 | 20 | 0.66/0.33 | 20 | 95 | 830 |
| E6 | 3.4 | 23 | 21 | 0.66/0.34 | 20 | 95 | 1000 |
| E7 | 3.8 | 23 | 15 | 0.67/0.33 | 20 | 95 | 540 |
| E8 | 3.8 | 24 | 18 | 0.67/0.33 | 20 | 95 | 760 |
| E9 | 3.5 | 70 | 17,5 | 0.32/0.64 | 20 | 80 | 700 |
| E10 | 3.2 | 67 | 19.1 | 0.33/0.63 | 20 | 80 | 820 |
| E11 | 3.1 | 69 | 18.8 | 0.32/0.64 | 20 | 80 | 790 |
| E12 | 3.2 | 74 | 18.5 | 0.32/0.63 | 20 | 80 | 766 |

**Tabelle 5: Daten der OLEDs**

| **Bsp** | **U1000 (V)** | **CE1000 (cd/A)** | **LE1000 (Im/W)** | **EQE 1000** | **CIE x/y at 1000 cd/m²** | **L₀; j₀** | **L₁ %** | **LT (h)** |
|---|---|---|---|---|---|---|---|---|
| E13 | 6 | | 5 | 7.0% | 0.13/0.14 | 6000 cd/m² | 80 | 30 |
| E14 | 5.0 | 62 | 53 | 13% | 0.31/0.64 | 20 mA/cm² | 80 | 50 |
| E15 | 4,1 | 8 | 5 | 7.0% | 0.13/0.14 | 6000 cd/m² | 80 | 45 |
| E16 | 3.6 | 62 | 53 | 17.4% | 0.31/0.64 | 20 mA/cm² | 80 | 142 |
| E17 | 3.5 | 8 | 6 | 7.3% | 0.14/0.13 | 6000 cd/m² | 80 | 47 |
| E18 | 3.2 | 62 | 51 | 16.1% | 0.34/0.62 | 20 mA/cm² | 80 | 124 |

## Patentansprüche

1. Verbindung gemäß Formel (I) 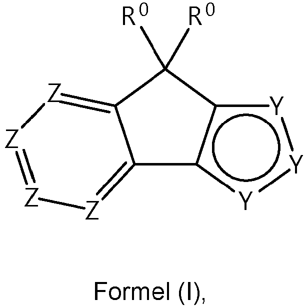 wobei gilt:
Y ist bei jedem Auftreten gleich oder verschieden gewählt aus O, S, NAr⁰ und CAr¹, wobei mindestens ein Y gewählt ist aus O, S und NAr⁰;
Z ist bei jedem Auftreten gleich oder verschieden gewählt aus N und CR¹;
Ar⁰ ist bei jedem Auftreten gleich oder verschieden gewählt aus aromatischen Ringsystemen mit 6 bis 40 aromatischen Ringatomen, die mit Resten R² substituiert sind, und heteroaromatischen Ringsystemen mit 5 bis 40 aromatischen Ringatomen, die mit Resten R² substituiert sind;
Ar¹ ist bei jedem Auftreten gleich oder verschieden gewählt aus H, D, F, Cl, Br, I, C(=O)R², CN, Si(R²)₃, P(=O)(R²)₂, OR², S(=O)R², S(=O)₂R², geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl-, Alkoxy-, Alkenyl- und Alkinylgruppen und die genannten aromatischen Ringsysteme und heteroaromatischen Ringsysteme jeweils mit Resten R² substituiert sind; und wobei eine oder mehrere CH₂-Gruppen in den genannten Alkyl-, Alkoxy-, Alkenyl- und Alkinylgruppen durch - R²C=CR²-, -C≡C-, Si(R²)₂, C=O, C=NR², -C(=O)O-, -C(=O)NR²-, NR², P(=O)(R²), -O-, -S-, SO oder SO₂ ersetzt sein können; wobei ausgeschlossen ist, dass zwei Gruppen Ar¹ miteinander verknüpft sind, um ein mono- oder polycyclisches, aliphatisches oder aromatisches Ringsystem zu bilden;
R⁰ ist bei jedem Auftreten gleich oder verschieden gewählt aus H, D, F, Cl, Br, I, C(=O)R¹, CN, Si(R¹)₃, N(R¹)₂, P(=O)(R¹)₂, OR¹, S(=O)R¹, S(=O)₂R¹, geradkettigen Alkyl-, Alkoxy- oder Thioalkylgruppen mit 1 bis 20 C-Atomen, verzweigten oder cyclischen Alkyl-, Alkoxy- oder Thioalkylgruppen mit 3 bis 20 C-Atomen, Alkenyl- oder Alkinylgruppen mit 2 bis 20 C-Atomen, aromatischen Ringsystemen mit 6 bis 40 aromatischen Ringatomen, heteroaromatischen Ringsystemen mit 5 bis 40 aromatischen Ringatomen, Aryloxy- oder Heteroaryloxygruppen mit 5 bis 40 aromatischen Ringatomen, und Aralkylgruppen mit 5 bis 40 aromatischen Ringatomen; wobei die beiden Reste R⁰ miteinander verknüpft sein können und einen aliphatischen oder heteroaliphatischen Ring bilden können, so dass eine Spiroverbindung in Position 8 des Fluoren-Derivats entsteht, insbesondere ein Spirobifluoren-Derivat; wobei die genannten Alkyl-, Alkoxy-, Thioalkyl-, Alkenyl-, Alkinyl-, Aryloxy-, Heteroaryloxy- und Aralkylgruppen und die genannten aromatischen Ringsysteme und heteroaromatischen Ringsysteme jeweils mit Resten R¹ substituiert sind; und wobei eine oder mehrere CH₂-Gruppen in den genannten Alkyl-, Alkoxy-, Thioalkyl-, Alkenyl-, Alkinyl-, Aryloxy-, Heteroaryloxy- und Aralkylgruppen durch - R¹C=CR¹-, -C≡C-, Si(R¹)₂, C=O, C=NR¹, -C(=O)O-, -C(=O)NR¹-, NR¹, P(=O)(R¹), -O-, -S-, SO oder SO₂ ersetzt sein können;
R¹ ist bei jedem Auftreten gleich oder verschieden gewählt aus H, D, F, Cl, Br, I, C(=O)R⁵, CN, Si(R⁵)₃, P(=O)(R⁵)₂, OR⁵, S(=O)R⁵, S(=O)₂R⁵, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹ miteinander verknüpft sein können und einen aliphatischen oder heteroaliphatischen Ring bilden können; wobei die genannten Alkyl-, Alkoxy-, Alkenyl- und Alkinylgruppen und die genannten aromatischen Ringsysteme und heteroaromatischen Ringsysteme jeweils mit Resten R⁵ substituiert sind; und wobei eine oder mehrere CH₂-Gruppen in den genannten Alkyl-, Alkoxy-, Alkenyl- und Alkinylgruppen durch -R⁵C=CR⁵-, -C≡C-, Si(R⁵)₂, C=O, C=NR⁵, -C(=O)O-, -C(=O)NR⁵-, NR⁵, P(=O)(R⁵), -O-, -S-, SO oder SO₂ ersetzt sein können;
R² ist bei jedem Auftreten gleich oder verschieden gewählt aus H, D, F, Cl, Br, I, C(=O)R⁵, CN, Si(R⁵)₃, P(=O)(R⁵)₂, OR⁵, S(=O)R⁵, S(=O)₂R⁵, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R² miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit Resten R⁵ substituiert sind; und wobei eine oder mehrere CH₂-Gruppen in den genannten Alkyl-, Alkoxy-, Alkenyl- und Alkinylgruppen durch -R⁵C=CR⁵-, -C=C-, Si(R⁵)₂, C=O, C=NR⁵, -C(=O)O-, -C(=O)NR⁵-, NR⁵, P(=O)(R⁵), -O-, -S-, SO oder SO₂ ersetzt sein können;
R⁵ ist bei jedem Auftreten gleich oder verschieden gewählt aus H, D, F, Cl, Br, I, C(=O)R⁶, CN, Si(R⁶)₃, P(=O)(R⁶)₂, OR⁶, S(=O)R⁶, S(=O)₂R⁶, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R⁵ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit Resten R⁶ substituiert sind; und wobei eine oder mehrere CH₂-Gruppen in den genannten Alkyl-, Alkoxy-, Alkenyl- und Alkinylgruppen durch -R⁶C=CR⁶-, -C=C-, Si(R⁶)₂, C=O, C=NR⁶, -C(=O)O-, -C(=O)NR⁶-, NR⁶, P(=O)(R⁶), -O-, -S-, SO oder SO₂ ersetzt sein können;
R⁶ ist bei jedem Auftreten gleich oder verschieden gewählt aus H, D, F, Cl, Br, I, CN, Alkyl- oder Alkoxygruppen mit 1 bis 20 C-Atomen, Alkenyl- oder Alkinylgruppen mit 2 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R⁶ miteinander verknüpft sein können und einen Ring bilden können; und wobei die genannten Alkyl-, Alkoxy-, Alkenyl- und Alkinylgruppen, aromatischen Ringsysteme und heteroaromatischen Ringsysteme mit einem oder mehreren Resten gewählt aus F und CN substituiert sein können; und
in der Formel (I) ist mindestens ein Rest R¹, eine Gruppe Ar⁰ oder eine Gruppe Ar¹ durch eine Gruppe A substituiert, die einer Formel (A) entspricht: 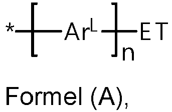
wobei gilt:
* bezeichnet die Bindung an die Struktur der Formel (I);
Ar^{L} ist bei jedem Auftreten gleich oder verschieden gewählt aus aromatischen Ringsystemen mit 6 bis 40 aromatischen Ringatomen, die mit Resten R² substituiert sind, und heteroaromatischen Ringsystemen mit 5 bis 40 aromatischen Ringatomen, die mit Resten R² substituiert sind;
ET ist bei jedem Auftreten gleich oder verschieden eine elektronentransportierende Gruppe, die gewählt ist aus elektronenarmen heteroaromatischen Gruppen, an die eine oder mehrere Gruppen Ar² gebunden sind;
Ar² ist bei jedem Auftreten gleich oder verschieden gewählt aus ist bei jedem Auftreten gleich oder verschieden gewählt aus H, D, F, Cl, Br, I, C(=O)R³, CN, Si(R³)₃, P(=O)(R³)₂, OR³, S(=O)R³, S(=O)₂R³, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl-, Alkoxy-, Alkenyl- und Alkinylgruppen und die genannten aromatischen Ringsysteme und heteroaromatischen Ringsysteme jeweils mit Resten R³ substituiert sind; wobei eine oder mehrere CH₂-Gruppen in den genannten Alkyl-, Alkoxy-, Alkenyl- und Alkinylgruppen durch -R³C=CR³-, -C=C-, Si(R³)₂, C=O, C=NR³, -C(=O)O-, -C(=O)NR³-, NR³, P(=O)(R³), -O-, -S-, SO oder SO₂ ersetzt sein können; und wobei zwei oder mehr benachbarte Gruppen Ar² miteinander ein mono- oder polycyclisches, aliphatisches oder aromatisches Ringsystem bilden können;
R³ ist bei jedem Auftreten gleich oder verschieden gewählt ist aus H, D, F, Cl, Br, I, C(=O)R⁵, CN, Si(R⁵)₃, N(R⁵)₂, P(=O)(R⁵)₂, OR⁵, S(=O)R⁵, S(=O)₂R⁵, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R³ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit Resten R⁵ substituiert sind; und wobei eine oder mehrere CH₂-Gruppen in den genannten Alkyl-, Alkoxy-, Alkenyl- und Alkinylgruppen durch -R⁵C=CR⁵-, -C=C-, Si(R⁵)₂, C=O, C=NR⁵, -C(=O)O-, -C(=O)NR⁵-, NR⁵, P(=O)(R⁵), -O-, -S-, SO oder SO₂ ersetzt sein können; und
n ist gleich 0 oder 1.

2. Verbindung nach Anspruch 1, **dadurch gekennzeichnet, dass** genau ein Y gewählt ist aus O, S und NAr⁰ und zwei Y gleich CAr¹ sind.

3. Verbindung nach Anspruch 1 oder 2, **dadurch gekennzeichnet, dass** die Gruppen Z gleich CR¹ sind.

4. Verbindung nach einem oder mehreren der Ansprüche 1 bis 3, **dadurch gekennzeichnet, dass** die Gruppe ET gleich oder verschieden bei jedem Auftreten gewählt ist aus Heteroarylgruppen mit 5 bis 40 aromatischen Ringatomen, die mit Gruppen Ar² substituiert sind.

5. Verbindung nach einem oder mehreren der Ansprüche 1 bis 4, **dadurch gekennzeichnet, dass** die Gruppe ET gleich oder verschieden bei jedem Auftreten gewählt ist aus den Gruppen der Formeln (ET-1) bis (ET-11c): 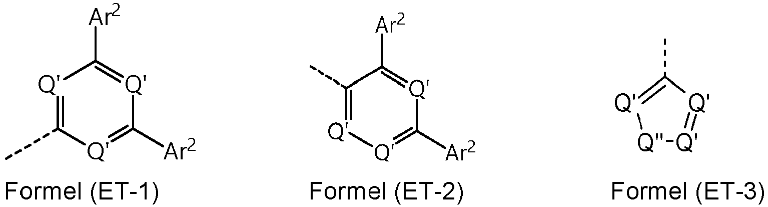 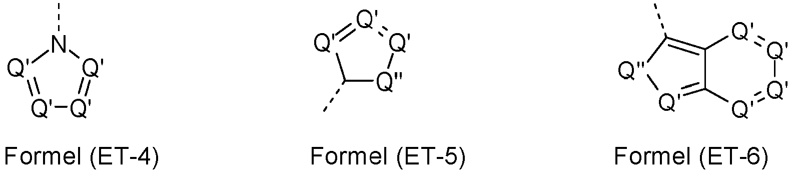 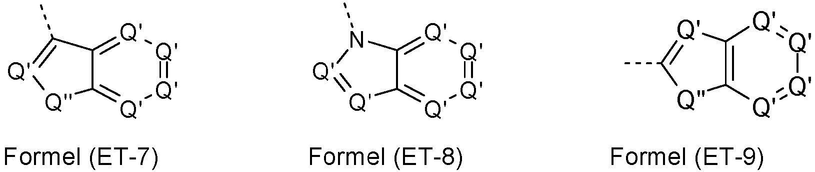 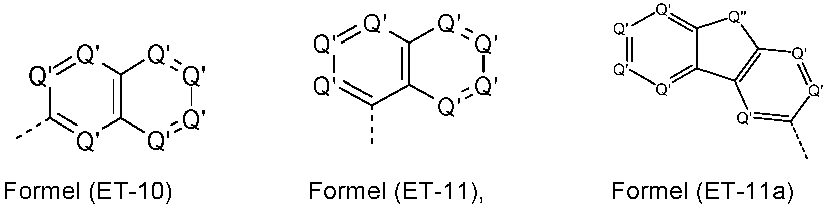 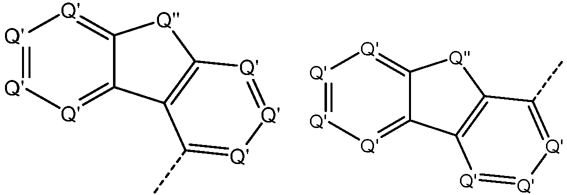 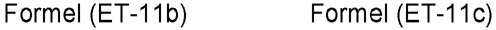 wobei
Q' bei jedem Auftreten gleich oder verschieden gewählt ist aus CAr² oder N;
Q" bei jedem Auftreten gleich oder verschieden gewählt ist aus NR², O oder S;
die gestrichelte Line jeweils die Anbindungsposition an den Rest der Formel (I) markiert und R² und Ar² wie in Anspruch 1 definiert sind; und
wobei wenigstens ein Q' gleich N ist.

6. Verbindung nach einem oder mehreren der Ansprüche 1 bis 5, **dadurch gekennzeichnet, dass** die Gruppe ET gleich oder verschieden bei jedem Auftreten gewählt ist aus den Gruppen der Formeln (ET-12) bis (ET-35) 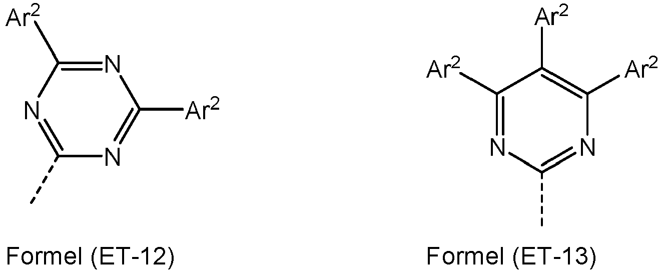 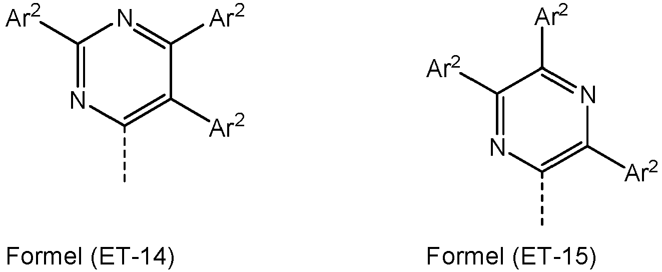 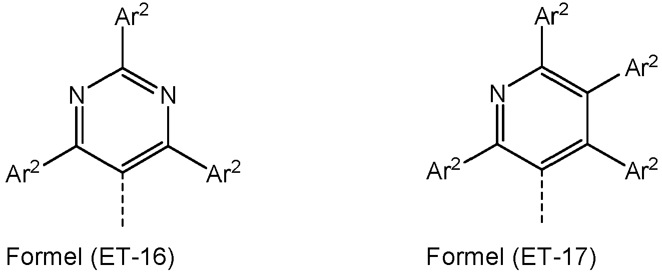 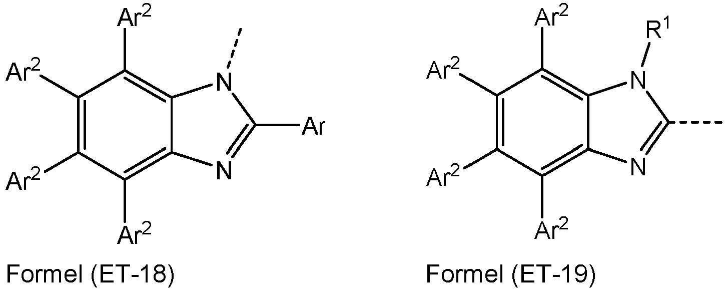 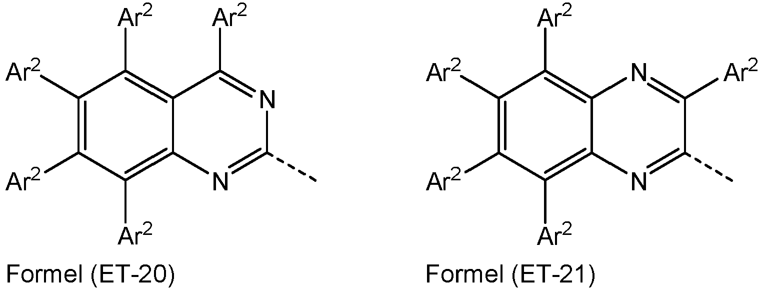 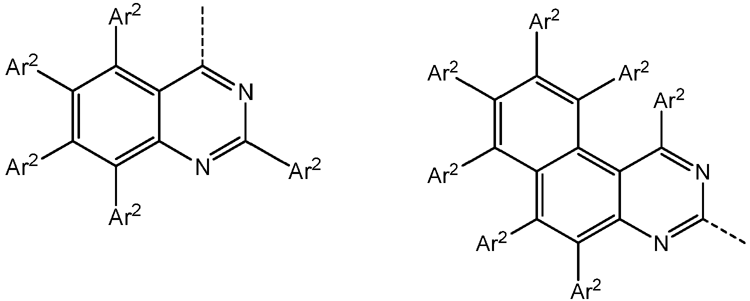 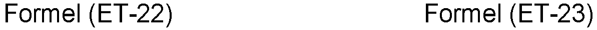 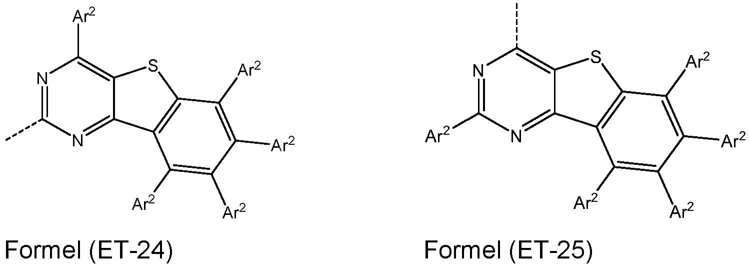 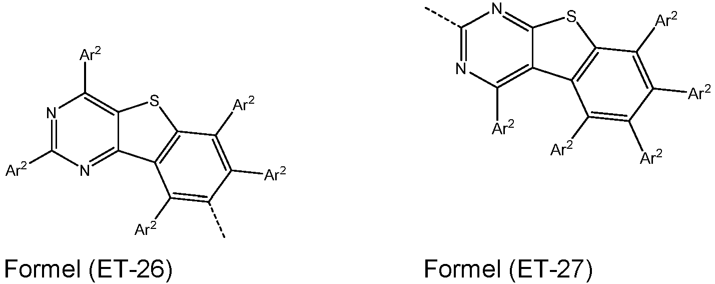 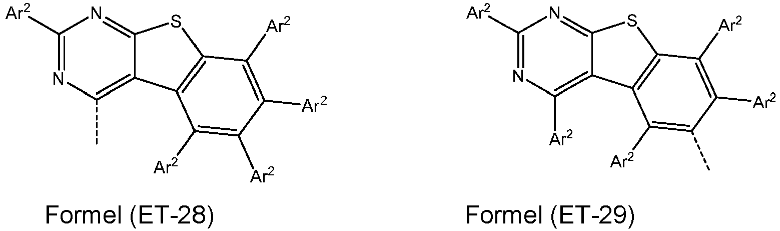 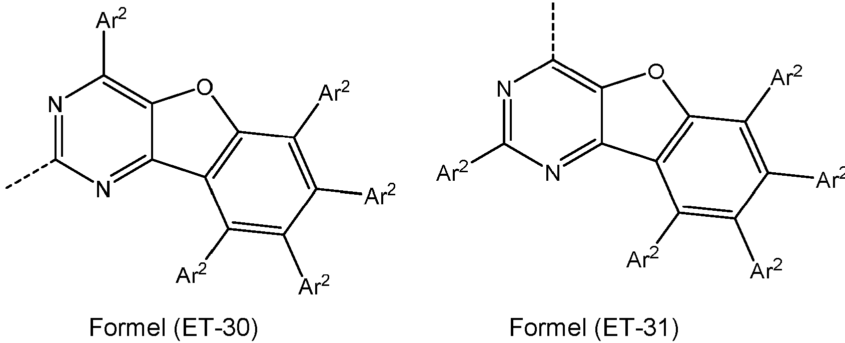 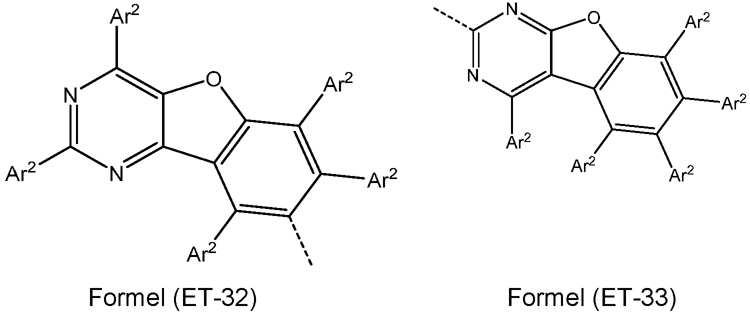 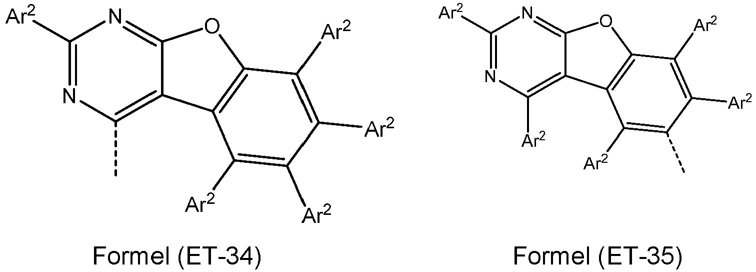 wobei die gestrichelten Linien die Bindungen an den Rest der Formel (I) darstellen und Ar² wie in Anspruch 1 definiert ist.

7. Verbindung nach einem oder mehreren der Ansprüche 1 bis 6, **dadurch gekennzeichnet, dass** sie der folgenden Formel (I-A) entspricht 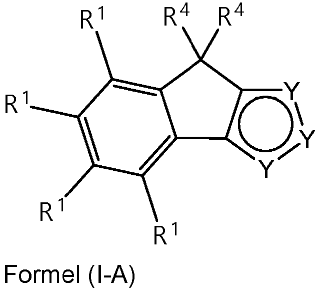
wobei die Variablen definiert sind wie in einem oder mehreren der Ansprüche 1 bis 11 und mindestens eine Gruppe A je Formel vorliegt; und
R⁴ bei jedem Auftreten gleich oder verschieden gewählt ist aus H, D, F, Cl, Br, I, C(=O)R¹, **CN,** Si(R¹)₃, N(R¹)₂, P(=O)(R¹)₂, OR¹, S(=O)R¹, S(=O)₂R¹, geradkettigen Alkyl-, Alkoxy- oder Thioalkylgruppen mit 1 bis 20 C-Atomen, verzweigten oder cyclischen Alkyl-, Alkoxy- oder Thioalkylgruppen mit 3 bis 20 C-Atomen, Alkenyl- oder Alkinylgruppen mit 2 bis 20 C-Atomen, aromatischen Ringsystemen mit 6 bis 40 aromatischen Ringatomen, heteroaromatischen Ringsystemen mit 5 bis 40 aromatischen Ringatomen, Aryloxy- oder Heteroaryloxygruppen mit 5 bis 40 aromatischen Ringatomen, und Aralkylgruppen mit 5 bis 40 aromatischen Ringatomen; wobei ausgeschlossen ist, dass die beiden Reste R⁴ miteinander verknüpft sind und einen aliphatischen oder heteroaliphatischen Ring bilden; wobei die genannten Alkyl-, Alkoxy-, Thioalkyl-, Alkenyl-, Alkinyl-, Aryloxy-, Heteroaryloxy- und Aralkylgruppen und die genannten aromatischen Ringsysteme und heteroaromatischen Ringsysteme jeweils mit Resten R¹ substituiert sind; und wobei eine oder mehrere CH₂-Gruppen in den genannten Alkyl-, Alkoxy-, Thioalkyl-, Alkenyl-, Alkinyl-, Aryloxy-, Heteroaryloxy- und Aralkylgruppen durch -R¹C=CR¹-, -C=C-, Si(R¹)₂, C=O, C=NR¹, -C(=O)O-, -C(=O)NR¹-, NR¹, P(=O)(R¹), -O-, -S-, SO oder SO₂ ersetzt sein können.

8. Verbindung nach einem oder mehreren der Ansprüche 1 bis 7, **dadurch gekennzeichnet, dass** sie einer der folgenden Formeln (II-A), (III-A) und (IV-A) entspricht 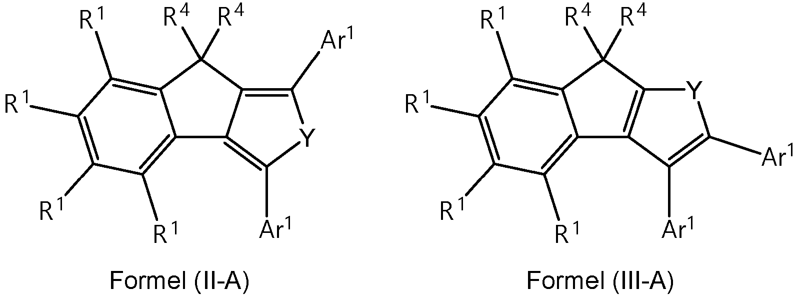 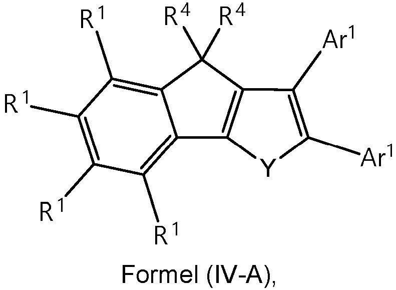
wobei die Variablen definiert sind wie in einem oder mehreren der Ansprüche 1 bis 7 und mindestens eine Gruppe A je Formel vorliegt; und
R⁴ bei jedem Auftreten gleich oder verschieden gewählt ist aus F, CN, Si(R¹)₃, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkylgruppen und die genannten aromatischen Ringsysteme und heteroaromatischen Ringsysteme jeweils mit Resten R¹ substituiert sind und wobei ausgeschlossen ist, dass die beiden Reste R⁴ miteinander verknüpft sind und einen aliphatischen oder heteroaliphatischen Ring bilden.

9. Verbindung nach einem oder mehreren der Ansprüche 1 bis 6, **dadurch gekennzeichnet, dass** sie der folgenden Formel (I-B) entsprechen: 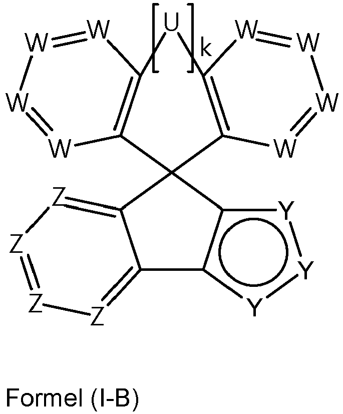 wobei
W bei jedem Auftreten gleich oder verschieden gewählt ist aus N und CR¹;
U gewählt ist aus O und S;
k gleich 0 oder 1 ist, wobei im Fall k=0 das betreffende Heteroatom U wegfällt und U stattdessen eine Einfachbindung ist, die die entsprechenden beiden aromatischen Sechsringe verbindent; und
mindestens eine Gruppe A in der Formel (I-B) vorliegt, wobei die Variablen Z, Y und R¹ sowie die mindestens eine Gruppe A wie in einem der Ansprüche 1 bis 6 definiert sind.

10. Verbindung nach Anspruch 9, **dadurch gekennzeichnet, dass** sie einer der folgenden Formeln (II-B) bis (IV-B) und (II-C) bis (IV-C) entspricht:
| | |
|---|---|
| 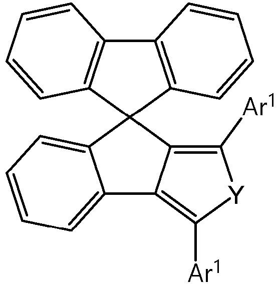 | 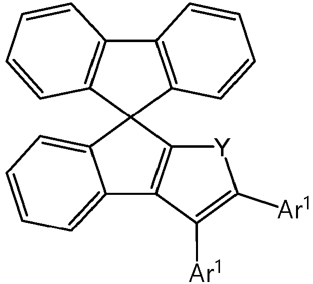 |
| Formel (II-B) | Formel (III-B) |
| 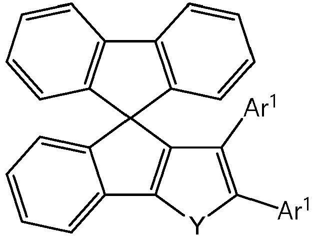 | 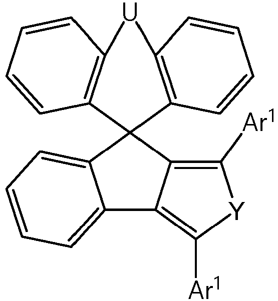 |
| Formel (IV-B) | Formel (II-C) |
| 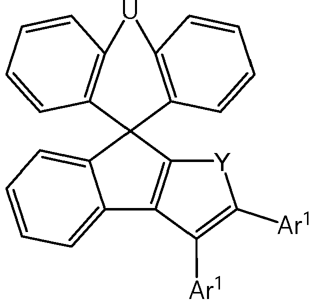 | 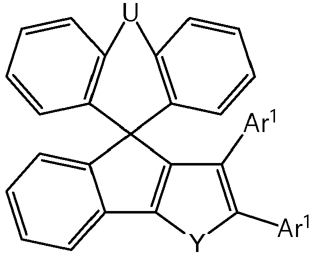 |
| Formel (III-C) | Formel (IV-C) |
wobei die die Variablen wie in einem oder mehreren der Ansprüche 9 und 1 bis 6 definiert sind und die freien Positionen an den Benzolringen jeweils mit Resten R¹ substituiert sind, und wobei mindestens eine Gruppe A je Formel vorliegt.

11. Oligomer, Polymer oder Dendrimer, enthaltend eine oder mehrere Verbindungen nach einem oder mehreren der Ansprüche 1 bis 10, wobei die Bindung(en) zum Polymer, Oligomer oder Dendrimer an beliebigen, in Formel (I) mit R⁰, R¹, R² oder R³ substituierten Positionen lokalisiert sein können.

12. Formulierung, enthaltend mindestens eine Verbindung nach einem oder mehreren der Ansprüche 1 bis 10 oder mindestens ein Polymer, Oligomer oder Dendrimer nach Anspruch 11, sowie mindestens ein Lösungsmittel.

13. Zusammensetzung, enthaltend mindestens eine Verbindung nach einem oder mehreren der Ansprüche 1 bis 10 oder mindestens ein Polymer, Oligomer oder Dendrimer nach Anspruch 11, sowie mindestens eine weitere Verbindung ausgewählt aus Lochinjektionsmaterialien, Lochtransportmaterialien, Lochblockiermaterialien, *wide-band-gap-*Materialien, fluoreszierenden Emittern, phosphoreszierenden Emittern, delayed fluorescent Materialien, Hostmaterialien, Matrixmaterialien, Elektronenblockiermaterialien, Elektronentransportmaterialien und Elektroneninjektionsmaterialien, n-Dotanden und p-Dotanden.

14. Elektronische Vorrichtung, enthaltend mindestens eine Verbindung nach einem oder mehreren der Ansprüche 1 bis 10, mindestens ein Polymer, Oligomer oder Dendrimer nach Anspruch 11, oder eine Zusammensetzung nach Anspruch 13.

15. Elektronische Vorrichtung nach Anspruch 14, **dadurch gekennzeichnet, dass** sie eine organische Elektrolumineszenzvorrichtung ist und Anode, Kathode und mindestens eine emittierende Schicht enthält, und dass die Verbindung in einer elektronentransportierenden Schicht oder in einer emittierenden Schicht der Vorrichtung enthalten ist.

16. Elektronische Vorrichtung nach Anspruch 15, **dadurch gekennzeichnet, dass** die Verbindung als elektronentransportierendes Material in der Elektronentransportschicht ist und eine oder mehrere weitere elektronentransportierenden Verbindungen zusätzlich in der Elektronenantransportschicht oder in der Elektroneninjektionsschicht enthalten sind, wobei es sich es sich bei den weiteren elektronentransportierenden Verbindungen bevorzugt um ein Hydroxychinolinat, ganz bevorzugt um ein Liq-Derivat und ganz besonders bevorzugt um Liq handelt.

17. Elektronische Vorrichtung nach Anspruch 15, **dadurch gekennzeichnet, dass** die Verbindung als elektronentransportierendes Material in der emittierenden Schicht zusammen mit einem lochtransportierendes Material enthalten ist.

18. Verwendung einer Verbindung nach einem oder mehreren der Ansprüche 1 bis 10 in einer elektronischen Vorrichtung.

## Claims

1. Compound according to formula (I) 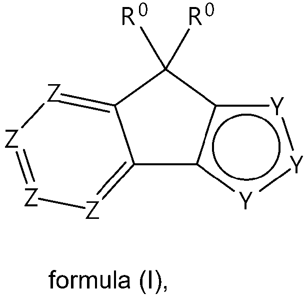 wherein
Y is on each occurrence, identically or differently, selected from O, S, NAr⁰ and CAr¹, wherein at least one Y is selected from O, S and NAr⁰;
Z is on each occurrence, identically or differently, selected from N and CR¹;
Ar⁰ is on each occurrence, identically or differently, selected from aromatic ring systems having 6 to 40 aromatic ring atoms which are substituted by radicals R² and heteroaromatic ring systems having 5 to 40 aromatic ring atoms which are substituted by radicals R²;
Ar¹ is on each occurrence, identically or differently, selected from H, D, F, Cl, Br, I, C(=O)R², CN, Si(R²)₃, P(=O)(R²)₂, OR², S(=O)R², S(=O)₂R², straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; wherein said alkyl, alkoxy, alkenyl and alkynyl groups and said aromatic ring systems and heteroaromatic ring systems are each substituted by radicals R²; and wherein one or more CH₂ groups in said alkyl, alkoxy, alkenyl and alkynyl groups may be substituted by -R²C=CR²-, -C≡C-, Si(R²)₂, C=O, C=NR², -C(=O)O-, -C(=O)NR²-, NR², P(=O)(R²), -O-, -S-, SO or SO₂; wherein it is ruled out that two groups Ar¹ are linked together to form a mono- or polycyclic, aliphatic or aromatic ring system;
R⁰ is on each occurrence, identically or differently, selected from **H, D,** F, Cl, Br, I, C(=O)R¹, CN, Si(R¹)₃, N(R¹)₂, P(=O)(R¹)₂, OR¹, S(=O)R¹, S(=O)₂R¹, straight-chain alkyl, alkoxy or thioalkyl groups having 1 to 20 C atoms, branched or cyclic alkyl, alkoxy or thioalkyl groups having 3 to 20 C atoms, alkenyl or alkynyl groups having 2 to 20 C atoms, aromatic ring systems having 6 to 40 aromatic ring atoms, heteroaromatic ring systems having 5 to 40 aromatic ring atoms, aryloxy or heteroaryloxy groups having 5 to 40 aromatic ring atoms, and aralkyl groups having 5 to 40 aromatic ring atoms; wherein the two radicals R⁰ may be linked together and may form an aliphatic or heteroaliphatic ring, so that a spiro compound is formed in position 8 of the fluorene derivative, in particular a spirobifluorene derivative; wherein said alkyl, alkoxy, thioalkyl, alkenyl, alkynyl, aryloxy, heteroaryloxy and aralkyl groups and said aromatic ring systems and heteroaromatic ring systems are each substituted with radicals R¹; and wherein one or more CH₂ groups in said alkyl, alkoxy, thioalkyl, alkenyl, alkynyl, aryloxy, heteroaryloxy and aralkyl groups may be substituted by -R¹C=CR¹-, -C≡C-, Si(R¹)₂, C=O, C=NR¹, -C(=O)O-, -C(=O)NR¹-, NR¹, P(=O)(R¹), -O-, -S-, SO or SO₂;
R¹ is on each occurrence, identically or differently, selected from **H, D,** F, Cl, Br, I, C(=O)R⁵, CN, Si(R⁵)₃, P(=O)(R⁵)₂, OR⁵, S(=O)R⁵, S(=O)₂R⁵, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; wherein two or more radicals R¹ may be linked together and may form an aliphatic or heteroaliphatic ring; wherein said alkyl, alkoxy, alkenyl and alkynyl groups and said aromatic ring systems and heteroaromatic ring systems are each substituted by radicals R⁵; and wherein one or more CH₂ groups in said alkyl, alkoxy, alkenyl and alkynyl groups may be substituted by -R⁵C=CR⁵-, -C≡C-, Si(R⁵)₂, C=O, C=NR⁵, -C(=O)O-, - C(=O)NR⁵-, NR⁵, P(=O)(R⁵), -O-, -S-, SO or SO₂;
R² is on each occurrence, identically or differently, selected from H, D, F, Cl, Br, I, C(=O)R⁵, CN, Si(R⁵)₃, P(=O)(R⁵)₂, OR⁵, S(=O)R⁵, S(=O)₂R⁵, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; wherein two or more radicals R² may be linked together and may form a ring; wherein said alkyl, alkoxy, alkenyl and alkynyl groups and said aromatic ring systems and heteroaromatic ring systems each may be substituted by radicals R⁵; and wherein one or more CH₂ groups in said alkyl, alkoxy, alkenyl and alkynyl groups may be substituted by -R⁵C=CR⁵-, -C≡C-, Si(R⁵)₂, C=O, C=NR⁵, -C(=O)O-, -C(=O)NR⁵-, NR⁵, P(=O)(R⁵), -O-, -S-, SO or SO₂;
R⁵ is on each occurrence, identically or differently, selected from H, D, F, Cl, Br, I, C(=O)R⁶, CN, Si(R⁶)₃, P(=O)(R⁶)₂, OR⁶, S(=O)R⁶, S(=O)₂R⁶, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; wherein two or more radicals R⁵ may be linked together and may form a ring; wherein said alkyl, alkoxy, alkenyl and alkynyl groups and said aromatic ring systems and heteroaromatic ring systems each may be substituted by radicals R⁶; and wherein one or more CH₂ groups in said alkyl, alkoxy, alkenyl and alkynyl groups may be substituted by -R⁶C=CR⁶-, -C=C-, Si(R⁶)₂, C=O, C=NR⁶, -C(=O)O-, -C(=O)NR⁶-, NR⁶, P(=O)(R⁶), -O-, -S-, SO or SO₂;
R⁶ is on each occurrence, identically or differently, selected from H, D, F, CI, Br, I, CN, alkyl or alkoxy groups having 1 to 20 C atoms, alkenyl or alkynyl groups having 2 to 20 C atoms, aromatic ring systems having 6 to 40 aromatic ring atoms and heteroaromatic ring systems having 5 to 40 aromatic ring atoms; wherein two or more radicals R⁶ may be linked together and form a ring; and wherein said alkyl, alkoxy, alkenyl and alkynyl groups, aromatic ring systems and heteroaromatic ring systems may be substituted by one or more radicals selected from F and CN; and
in formula (I), at least one radical R¹, a group Ar⁰ or a group Ar¹ is substituted by a group A which corresponds to a formula (A): 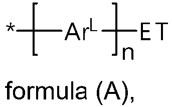
wherein
* denotes the bond to the structure of formula (I);
Ar^{L} is on each occurrence, identically or differently, selected from aromatic ring systems having 6 to 40 aromatic ring atoms which are substituted by radicals R² and heteroaromatic ring systems having 5 to 40 aromatic ring atoms which are substituted by radicals R²;
ET is on each occurence, identically or differently, an electron-transporting group selected from electron-poor heteroaromatic groups to which one or more Ar² groups are bonded;
Ar² is on each occurrence, identically or differently, selected from H, D, F, Cl, Br, I, C(=O)R³, CN, Si(R³)₃, P(=O)(R³)₂, OR³, S(=O)R³, S(=O)₂R³, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; wherein said alkyl, alkoxy, alkenyl and alkynyl groups and said aromatic ring systems and heteroaromatic ring systems are each substituted by radicals R³; wherein one or more CH₂ groups in said alkyl, alkoxy, alkenyl and alkynyl groups may be substituted by -R³C=CR³-, -C≡C-, Si(R³)₂, C=O, C=NR³, -C(=O)O-, -C(=O)NR³-, NR³, P(=O)(R³), -O-, -S-, SO or SO₂; and wherein two or more adjacent groups Ar² can form a mono- or polycyclic, aliphatic or aromatic ring system;
R³ is on each occurrence, identically or differently, selected from H, D, F, Cl, Br, I, C(=O)R⁵, CN, Si(R⁵)₃, N(R⁵)₂, P(=O)(R⁵)₂, OR⁵, S(=O)R⁵, S(=O)₂R⁵, straight-chain alkyl or alkoxy groups having 1 to 20 C atoms, branched or cyclic alkyl or alkoxy groups having3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; wherein two or more radicals R³ may be linked together and may form a ring; wherein said alkyl, alkoxy, alkenyl and alkynyl groups and said aromatic ring systems and heteroaromatic ring systems are each substituted by radicals R⁵; and wherein one or more CH₂ groups in said alkyl, alkoxy, alkenyl and alkynyl groups may be substituted by -R⁵C=CR⁵-, -C≡C-, Si(R⁵)₂, C=O, C=NR⁵, -C(=O)O-, -C(=O)NR⁵-, NR⁵, P(=O)(R⁵), -O-, -S-, SO or SO₂; and
n is equal to 0 or 1.

2. Compound according to claim 1, **characterised in that** exactly one Y is selected from O, S and NAr⁰ and two Y are equal to CAr¹.

3. Compound according to claim 1 or 2, **characterised in that** the groups Z are equal to CR¹.

4. Compound according to one or more of claims 1 to 3, **characterised in that** the group ET is on each occurrence, identically or differently, selected from heteroaryl groups having 5 to 40 aromatic ring atoms substituted by groups Ar².

5. Compound according to one or more of claims 1 to 4, **characterised in that** the group ET is on each occurrence, identically or differently, selected from the groups of formulae (ET-1) to (ET-11c): 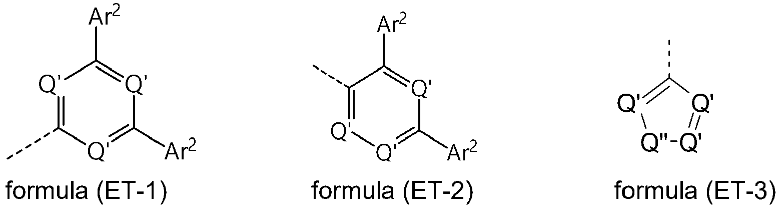 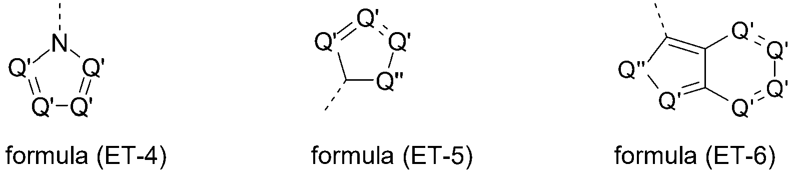 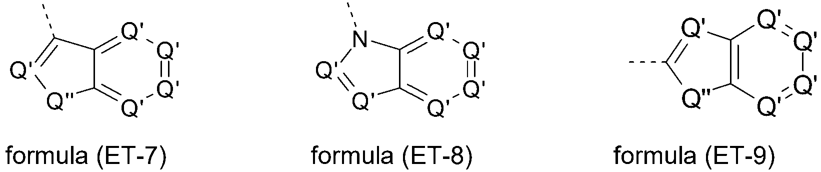 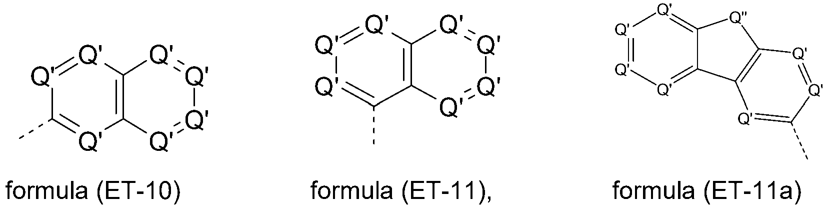 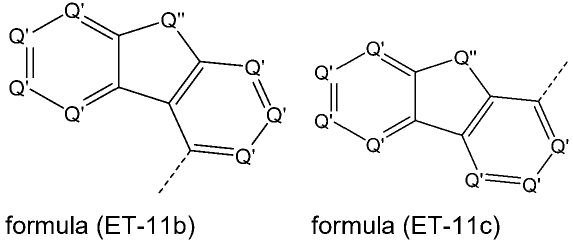 wherein
Q' is on each occurrence, identically or differently, selected from CAr² or N;
Q" is on each occurrence, identically or differently, selected from NR², O or S;
the dashed line in each case marks the position of attachment to the remainder of formula (I) and R² and Ar² are defined as in claim 1; and
wherein at least one Q' is equal to N.

6. Compound according to one or more of claims 1 to 5, **characterised in that** the group ET, is on each occurrence, identically or differently, selected from the groups of formulae (ET-12) to (ET-35) 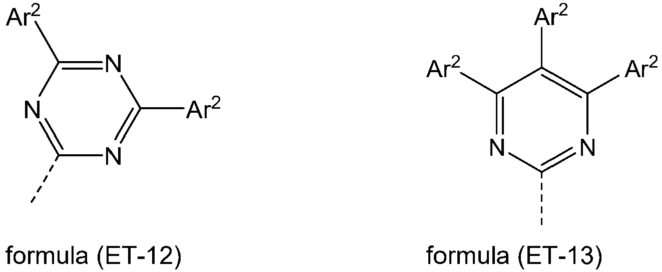 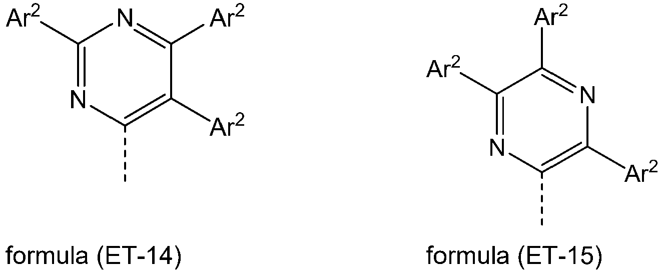 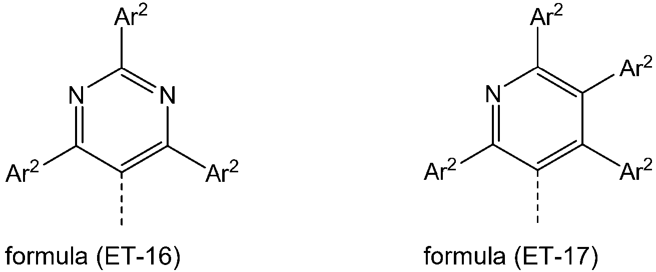 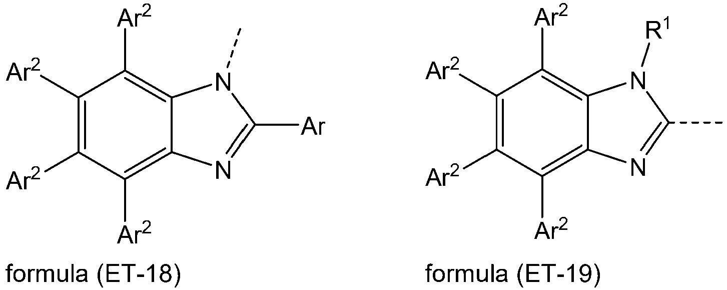 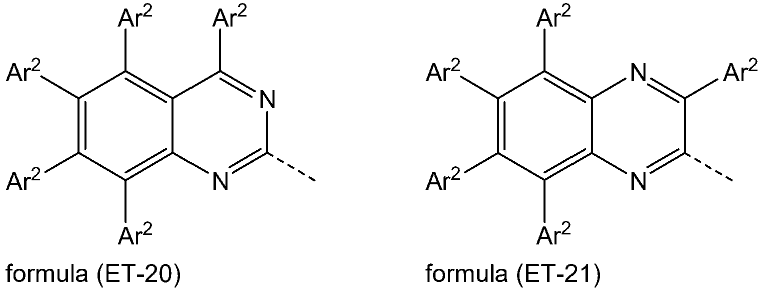 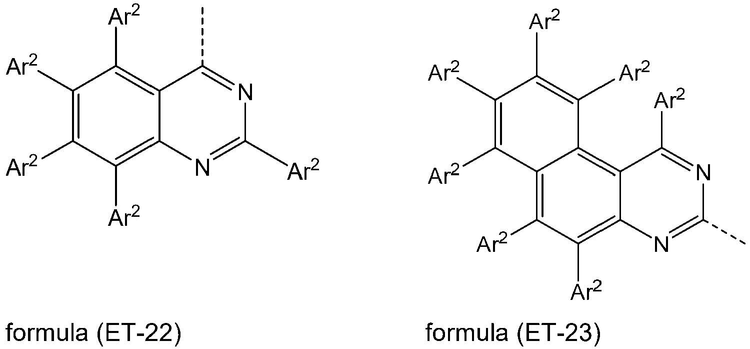 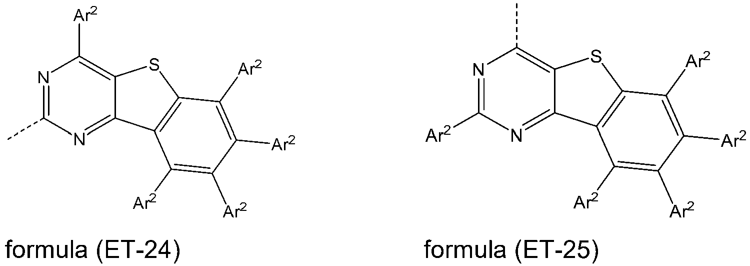 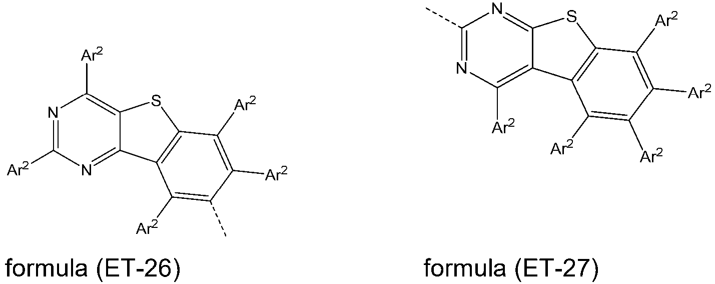 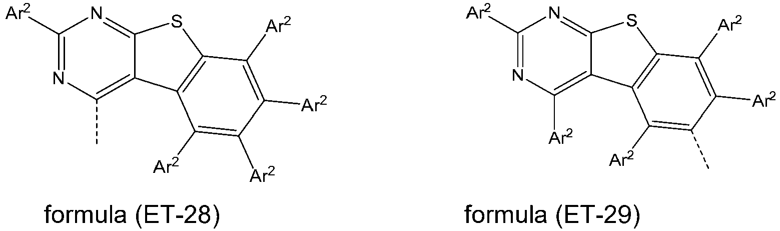 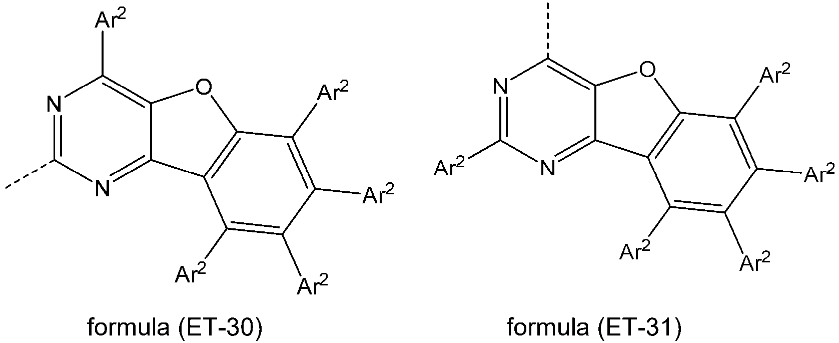 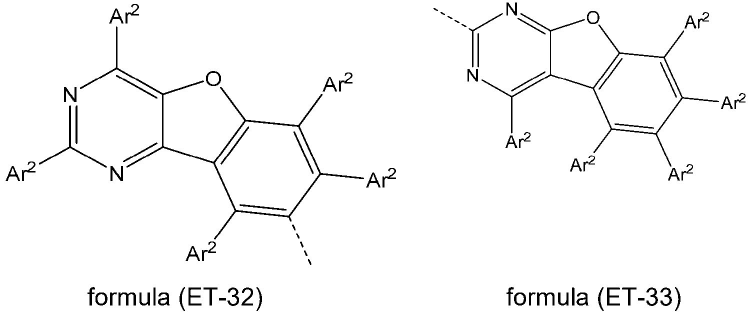 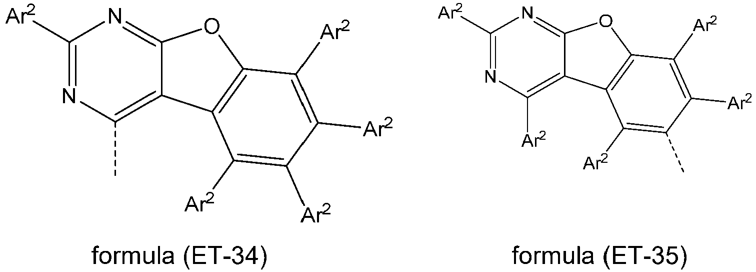 wherein the dashed lines represent the bonds to the remainder of formula (I) and Ar² is defined as in claim 1.

7. Compound according to one or more of claims 1 to 6, **characterised in that** it corresponds to the following formula (I-A) 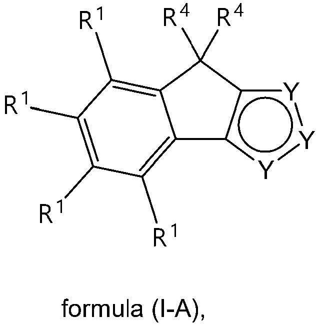
wherein the variables are as defined in one or more of claims 1 to 11 and there is at least one group A per formula; and
R⁴ is on each occurrence, identically or differently, selected from H, D, F, Cl, Br, I, C(=O)R¹, CN, Si(R¹)₃, N(R¹)₂, P(=O)(R¹)₂, OR¹, S(=O)R¹, S(=O)₂R¹, straight-chain alkyl, alkoxy or thioalkyl groups having 1 to 20 C atoms, branched or cyclic alkyl, alkoxy or thioalkyl groups having 3 to 20 C atoms, alkenyl or alkynyl groups having 2 to 20 C atoms, aromatic ring systems having 6 to 40 aromatic ring atoms, heteroaromatic ring systems having 5 to 40 aromatic ring atoms, aryloxy or heteroaryloxy groups having 5 to 40 aromatic ring atoms, and aralkyl groups having 5 to 40 aromatic ring atoms; wherein it is ruled out that the two radicals R⁴ are linked together and form an aliphatic or heteroaliphatic ring; wherein said alkyl, alkoxy, thioalkyl, alkenyl, alkynyl, aryloxy, heteroaryloxy and aralkyl groups and said aromatic ring systems and heteroaromatic ring systems are each substituted by radicals R¹; and wherein one or more CH₂ groups in said alkyl, alkoxy, thioalkyl, alkenyl, alkynyl, aryloxy, heteroaryloxy and aralkyl groups may be substituted by -R¹C=CR¹-, -C=C-, Si(R¹)₂, C=O, C=NR¹, -C(=O)O-, -C(=O)NR¹-, NR¹, P(=O)(R¹), -O-, -S-, SO or SO₂.

8. Compound according to one or more of claims 1 to 7, **characterised in that** it corresponds to one of the following formulae (II-A), (III-A) and (IV-A) 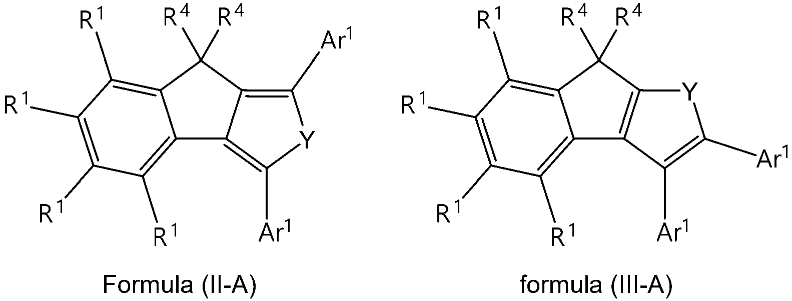 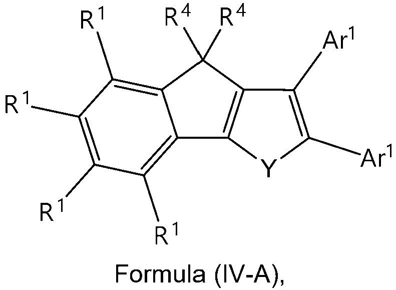
wherein the variables are defined as in one or more of claims 1 to 7 and there is at least one group A per formula; and
R⁴ is on each occurrence, identically or differently, selected from F, CN, Si(R¹)₃, straight-chain alkyl groups having 1 to 20 C atoms, branched or cyclic alkyl groups having 3 to 20 C atoms, aromatic ring systems having 6 to 40 aromatic ring atoms, and heteroaromatic ring systems having 5 to 40 aromatic ring atoms; wherein the said alkyl groups and the said aromatic ring systems and heteroaromatic ring systems are each substituted by radicals R¹ and wherein it is ruled out that the two radicals R⁴ are linked together and form an aliphatic or heteroaliphatic ring.

9. Compound according to one or more of claims 1 to 6, **characterised in that** it corresponds to the following formula (I-B): 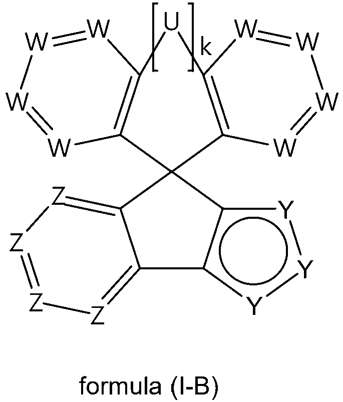 wherein
W is on each occurrence, identically or differently, selected from N and CR¹;
U is selected from O and S;
k is 0 or 1, wherein in the case k=0 the heteroatom U in question is omitted and U is instead a single bond connecting the corresponding two aromatic six-membered rings; and
at least one group A is present in formula (I-B), wherein the variables Z, Y and R¹ and the at least one group A are as defined in any one of claims 1 to 6.

10. Compound according to claim 9, **characterised in that** it corresponds to one of the following formulae (II-B) to (IV-B) and (II-C) to (IV-C):
| | |
|---|---|
| 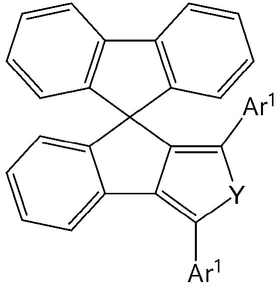 | 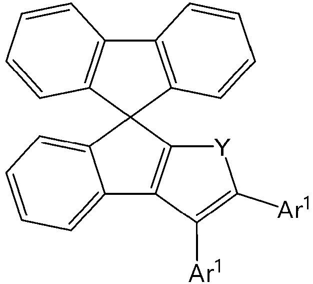 |
| formula (II-B) | formula (III-B) |
| 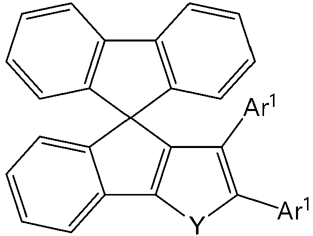 | 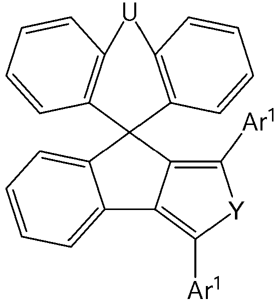 |
| formula (IV-B) | formula (II-C) |
| 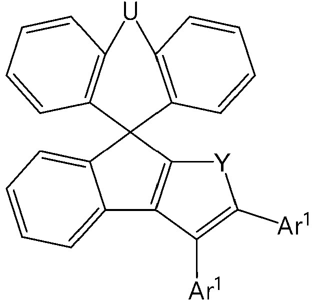 | 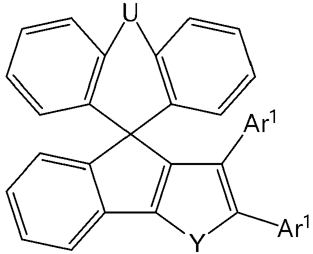 |
| formula (III-C) | formula (IV-C) |
wherein the variables are as defined in one or more of claims 9 and 1 to 6 and the free positions on the benzene rings are each substituted by radicals R¹, and wherein at least one group A is present per formula.

11. Oligomer, polymer or dendrimer containing one or more compounds according to one or more of claims 1 to 10, wherein the bond(s) to the polymer, oligomer or dendrimer may be located at any positions substituted with R⁰, R¹, R² or R³ in formula (I).

12. Formulation containing at least one compound according to one or more of claims 1 to 10 or at least one polymer, oligomer or dendrimer according to claim 11, and at least one solvent.

13. Composition containing at least one compound according to one or more of claims 1 to 10 or at least one polymer, oligomer or dendrimer according to claim 11, and at least one further compound selected from hole injection materials, hole transport materials, hole blocking materials, *wide-band-gap materials,* fluorescent emitters, phosphorescent emitters, delayed fluorescent materials, host materials, matrix materials, electron blocking materials, electron transport materials and electron injection materials, n-dotands and p-dotands.

14. Electronic device containing at least one compound according to one or more of claims 1 to 10, at least one polymer, oligomer or dendrimer according to claim 11, or a composition according to claim 13.

15. Electronic device according to claim 14, **characterised in that** it is an organic electroluminescent device and contains an anode, cathode and at least one emitting layer, and **in that** the compound is contained in an electron transporting layer or in an emitting layer of the device.

16. Electronic device according to claim 15, **characterised in that** the compound as electron transporting material is in the electron transport layer and one or more further electron transporting compounds are additionally contained in the electron transport layer or in the electron injection layer, wherein the further electron transporting compounds are preferably a hydroxyquinolinate, very preferably a Liq derivative and very particularly preferably Liq.

17. An electronic device according to claim 15, **characterised in that** the compound is contained as an electron transporting material in the emitting layer together with a hole transporting material.

18. Use of a connection according to one or more of claims 1 to 10 in an electronic device.

## Revendications

1. Composé selon la formule (I) 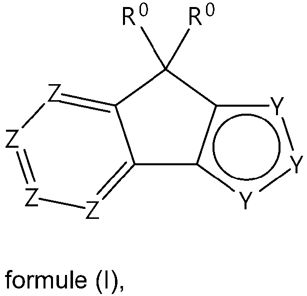 dans laquelle
Y est choisi à chaque occurrence, de manière identique ou différente, parmi O, S, NAr⁰ et CAr¹, dans lequel au moins un Y est choisi parmi O, S et NAr⁰;
Z est choisi à chaque occurrence, de manière identique ou différente, parmi N et CR¹;
Ar⁰ est choisi à chaque occurrence, de manière identique ou différente, parmi les systèmes cycliques aromatiques ayant de 6 à 40 atomes de cycle aromatique substitués par des résidus R² et les systèmes cycliques hétéroaromatiques ayant de 5 à 40 atomes de cycle aromatique substitués par des résidus R²;
Ar¹ est choisi à chaque occurrence, de manière identique ou différente, parmi H, D, F, CI, Br, I, C(=O)R², CN, Si(R²)₃, P(=O)(R²)₂, OR², S(=O)R², S(=O)₂R², des groupes alkyle ou alcoxy à chaîne linéare ayant de 1 à 20 atomes de C, des groupes alkyle ou alcoxy ramifiés ou cycliques ayant de 3 à 20 atomes de C, des groupes alcényle ou alcynyle ayant de 2 à 20 atomes de C, des systèmes cycliques aromatiques ayant de 6 à 40 atomes de cycle aromatiques, et des systèmes cycliques hétéroaromatiques ayant de 5 à 40 atomes de cycle aromatiques; dans lequel lesdits groupes alkyle, alcoxy, alcényle et alcynyle et lesdits systèmes cycliques aromatiques et systèmes cycliques hétéroaromatiques sont chacun substitués par des radicaux R²; et dans lequel un ou plusieurs groupes CH₂ dans lesdits groupes alkyle, alcoxy, alcényle et alcynyle peuvent être remplacés par -R²C=CR²-, -C≡C-, Si(R²)₂, C=O, C=NR², -C(=O)O-, -C(=O)NR²-, NR², P(=O)(R²), -O-, -S-, SO ou SO₂; dans lequel est exclu que deux groupes Ar¹ soient liés l'un à l'autre pour former un cycle mono- ou polycyclique, aliphatique ou aromatique système cyclique;
R⁰ est choisi à chaque occurrence, de manière identique ou différente, parmi H, D, F, Cl, Br, I, C(=O)R¹, CN, Si(R¹)₃, N(R¹)₂, P(=O)(R¹)₂, OR¹, S(=O)R¹, S(=O)₂R¹, des groupes alkyle, alcoxy ou thioalkyle à chaîne droite ayant de 1 à 20 atomes de C, des groupes alkyle, alcoxy ou thioalkyle ramifiés ou cycliques ayant de 3 à 20 atomes de C, des groupes alcényle ou alcynyle ayant de 2 à 20 atomes de C, des systèmes cycliques aromatiques ayant de 6 à 40 atomes de cycle aromatique, des systèmes cycliques hétéroaromatiques ayant de 5 à 40 atomes de cycle aromatique, des groupes aryloxy ou hétéroaryloxy ayant de 5 à 40 atomes de cycle aromatique, et des groupes aralkyle ayant de 5 à 40 atomes de cycle aromatique; dans laquelle les deux radicaux R⁰ peuvent être liés l'un à l'autre et former un cycle aliphatique ou hétéroaliphatique, de manière à former un composé spiro en position 8 du dérivé de fluorène, en particulier un dérivé de spirobifluorène; dans lequel lesdits groupes alkyle, alcoxy, thioalkyle, alcényle, alcynyle, aryloxy, hétéroaryloxy et aralkyle et lesdits systèmes cycliques aromatiques et systèmes cycliques hétéroaromatiques sont chacun substitués par des radicaux R¹; et dans lequel un ou plusieurs groupes CH₂ dans lesdits groupes alkyle, alcoxy, thioalkyle, alcényle, alcynyle, aryloxy, hétéroaryloxy et aralkyle peuvent être remplacés par -R¹C=CR¹-, -C=C-, Si(R¹)₂, C=O, C=NR¹, -C(=O)O-, -C(=O)NR¹-, NR¹, P(=O)(R¹), -O-, -S-, SO ou SO₂;
R¹ est choisi à chaque occurrence, de manière identique ou différente, parmi H, D, F, Cl, Br, I, C(=O)R⁵, CN, Si(R⁵)₃, P(=O)(R⁵)₂, OR⁵, S(=O)R⁵, S(=O)₂R⁵, des groupes alkyle ou alcoxy à chaîne droite ayant de 1 à 20 atomes de C, des groupes alkyle ou alcoxy ramifiés ou cycliques ayant de 3 à 20 atomes de C, des groupes alcényle ou alcynyle ayant de 2 à 20 atomes de C, des systèmes cycliques aromatiques ayant de 6 à 40 atomes de cycle aromatiques, et des systèmes cycliques hétéroaromatiques ayant de 5 à 40 atomes de cycle aromatiques; où deux ou plusieurs radicaux R¹ peuvent être liés ensemble et former un cycle aliphatique ou hétéroaliphatique; lesdits groupes alkyle, alcoxy, alcényle et alcynyle et lesdits systèmes cycliques aromatiques et systèmes cycliques hétéroaromatiques étant chacun substitués par des radicaux R⁵; et dans lequel un ou plusieurs groupes CH₂ dans lesdits groupes alkyle, alcoxy, alcényle et alcynyle peuvent être remplacés par -R⁵C=CR⁵-, -C≡C-, Si(R⁵)₂, C=O, C=NR⁵, - C(=O)O-, -C(=O)NR⁵-, NR⁵, P(=O)(R⁵), -O-, -S-, SO ou SO₂;
R² est choisi à chaque occurrence, de manière identique ou différente, parmi H, D, F, Cl, Br, I, C(=O)R⁵, CN, Si(R⁵)₃, P(=O)(R⁵)₂, OR⁵, S(=O)R⁵, S(=O)₂R⁵, des groupes alkyle ou alcoxy à chaîne droite ayant de 1 à 20 atomes de C, des groupes alkyle ou alcoxy ramifiés ou cycliques ayant de 3 à 20 atomes de C, des groupes alcényle ou alcynyle ayant de 2 à 20 atomes de C, des systèmes cycliques aromatiques ayant de 6 à 40 atomes de cycle aromatiques, et des systèmes cycliques hétéroaromatiques ayant de 5 à 40 atomes de cycle aromatiques; où deux ou plusieurs radicaux R² peuvent être liés ensemble et former un cycle; lesdits groupes alkyle, alcoxy, alcényle et alcynyle et lesdits systèmes cycliques aromatiques et systèmes cycliques hétéroaromatiques étant chacun substitués par des radicaux R⁵; et dans lequel un ou plusieurs groupes CH₂ dans lesdits groupes alkyle, alcoxy, alcényle et alcynyle peuvent être remplacés par -R⁵C=CR⁵-, -C≡C-, Si(R⁵)₂, C=O, C=NR⁵, -C(=O)O-, -C(=O)NR⁵-, NR⁵, P(=O)(R⁵), -O-, -S-, SO ou SO₂;
R⁵ est choisi à chaque occurrence, de manière identique ou différente, parmi H, D, F, Cl, Br, I, C(=O)R⁶, CN, Si(R⁶)₃, P(=O)(R⁶)₂, OR⁶, S(=O)R⁶, S(=O)₂R⁶, des groupes alkyle ou alcoxy à chaîne droite ayant de 1 à 20 atomes de C, des groupes alkyle ou alcoxy ramifiés ou cycliques ayant de 3 à 20 atomes de C, des groupes alcényle ou alcynyle ayant de 2 à 20 atomes de carbone, des systèmes cycliques aromatiques ayant de 6 à 40 atomes de cycle aromatiques, et des systèmes cycliques hétéroaromatiques ayant de 5 à 40 atomes de cycle aromatiques; où deux ou plusieurs radicaux R⁵ peuvent être liés ensemble et former un cycle; lesdits groupes alkyle, alcoxy, alcényle et alcynyle et lesdits systèmes cycliques aromatiques et systèmes cycliques hétéroaromatiques étant chacun substitués par des radicaux R⁶; et dans lequel un ou plusieurs groupes CH₂ dans lesdits groupes alkyle, alcoxy, alcényle et alcynyle peuvent être remplacés par -R⁶C=CR⁶-, -C≡C-, Si(R⁶)₂, C=O, C=NR⁶, -C(=O)O-, -C(=O)NR⁶-, NR⁶, P(=O)(R⁶), -O-, -S-, SO ou SO₂;
R⁶ est choisi à chaque occurrence, de manière identique ou différente, parmi H, D, F, CI, Br, I, CN, les groupes alkyle ou alcoxy ayant de 1 à 20 atomes de C, les groupes alcényle ou alcynyle ayant de 2 à 20 atomes de C, les systèmes cycliques aromatiques ayant de 6 à 40 atomes de cycle aromatique et les systèmes cycliques hétéroaromatiques ayant de 5 à 40 atomes de cycle aromatique; dans laquelle deux ou plusieurs radicaux R⁶ peuvent être liés ensemble et former un cycle; et dans laquelle lesdits groupes alkyle, alcoxy, alcényle et alcynyle, systèmes cycliques aromatiques et systèmes cycliques hétéroaromatiques peuvent être substitués par un ou plusieurs radicaux choisis parmi F et CN ; et
dans la formule (I), au moins un radical R¹, un groupe Ar⁰ ou un groupe Ar¹ est substitué par un groupe A correspondant à une formule (A) : 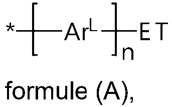
dans laquelle
* désigne la liaison à la structure de formule (I);
Ar^{L} est choisi à chaque occurrence, de manière identique ou différente, parmi les systèmes cycliques aromatiques ayant de 6 à 40 atomes cycliques aromatiques substitués par des résidus R² et les systèmes cycliques hétéroaromatiques ayant de 5 à 40 atomes cycliques aromatiques substitués par des résidus R² ;
ET est à chaque occurrence, de manière identique ou différente, un groupe transporteur d'électrons choisi parmi les groupes hétéroaromatiques pauvres en électrons auxquels sont liés un ou plusieurs groupes Ar²;
Ar² is on each occurrence, identically or differently, selected from H, D, F, CI, Br, I, C(=O)R³, CN, Si(R³)₃, P(=O)(R³)₂, OR³, S(=O)R³, S(=O)₂R³, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; wherein said alkyl, alkoxy, alkenyl and alkynyl groups and said aromatic ring systems and heteroaromatic ring systems are each substituted by radicals R³; wherein one or more CH₂ groups in said alkyl, alkoxy, alkenyl and alkynyl groups may be substituted by -R³C=CR³-, -C≡C-, Si(R³)₂, C=O, C=NR³, -C(=O)O-, -C(=O)NR³-, NR³, P(=O)(R³), -O-, -S-, SO or SO₂; and wherein two or more adjacent groups Ar² can form a mono- or polycyclic, aliphatic or aromatic ring system;
R³ est choisi à chaque occurrence, de manière identique ou différente, parmi H, D, F, Cl, Br, I, C(=O)R⁵, CN, Si(R⁵)₃, N(R⁵)₂, P(=O)(R⁵)₂, OR⁵, S(=O)R⁵, S(=O)₂R⁵, des groupes alkyle ou alcoxy à chaîne droite ayant de 1 à 20 atomes de C, des groupes alkyle ou alcoxy ramifiés ou cycliques ayant de 3 à 20 atomes de C, des groupes alcényle ou alcynyle ayant de 2 à 20 atomes de C, des systèmes cycliques aromatiques ayant de 6 à 40 atomes de cycle aromatiques, et des systèmes cycliques hétéroaromatiques ayant de 5 à 40 atomes de cycle aromatiques; où deux ou plusieurs radicaux R³ peuvent être liés ensemble et former un cycle; lesdits groupes alkyle, alcoxy, alcényle et alcynyle et lesdits systèmes cycliques aromatiques et systèmes cycliques hétéroaromatiques étant chacun substitués par des radicaux R⁵; et dans lequel un ou plusieurs groupes CH₂ dans lesdits groupes alkyle, alcoxy, alcényle et alcynyle peuvent être remplacés par -R⁵C=CR⁵-, -C=C-, Si(R⁵)₂, C=O, C=NR⁵, -C(=O)O-, - C(=O)NR⁵-, NR⁵, P(=O)(R⁵), -O-, -S-, SO ou SO₂; et
n est égal à 0 ou 1.

2. Composé selon la revendication 1, **caractérisé en ce qu'**exactement un Y est choisi parmi O, S et NAr⁰ et deux Y sont égaux à CAr¹ .

3. Composé selon la revendication 1 ou 2, **caractérisé en ce que** les groupes Z sont égaux à CR¹.

4. Composé selon une ou plusieurs des revendications 1 à 3, **caractérisé en ce que** le groupe ET, identique ou différent à chaque occurrence, est choisi parmi les groupes hétéroaryle ayant de 5 à 40 atomes de cycle aromatique substitués par des groupes Ar².

5. Composé selon une ou plusieurs des revendications 1 à 4, **caractérisé en ce que** le groupe ET, identique ou différent à chaque occurrence, est choisi parmi les groupes de formules (ET-1) à (ET-11c): 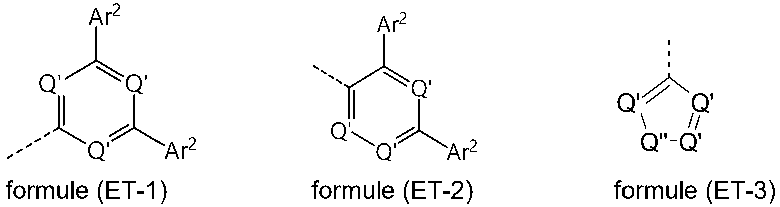 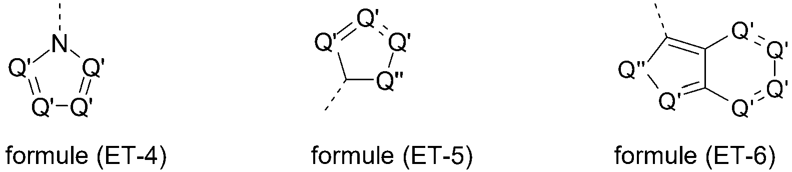 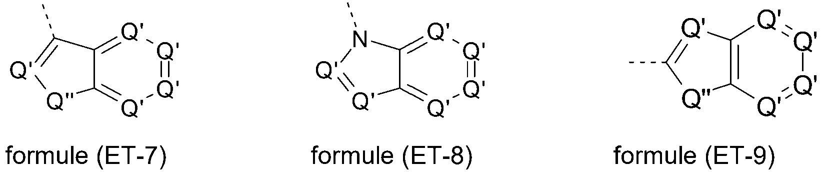 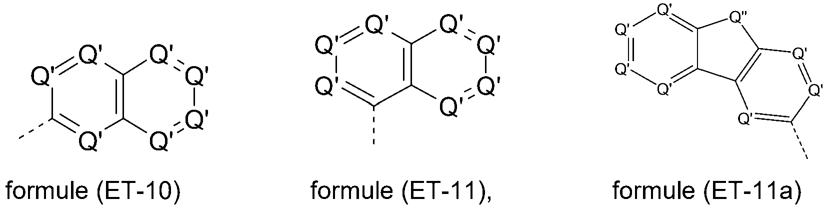 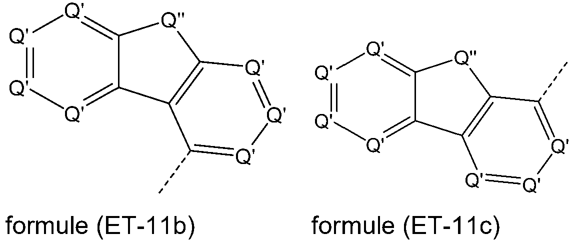 dans laquelle
Q' est choisi à chaque occurrence, de manière identique ou différente, parmi CAr² ou N ;
Q" à chaque occurrence est choisi de manière identique ou différente parmi NR², O ou S;
la ligne en pointillés marque chaque position de liaison au reste de la formule (I), et R² et Ar² sont tels que définis dans la revendication 1; et
dans laquelle au moins un Q' est égal à N.

6. Composé selon une ou plusieurs des revendications 1 à 5, **caractérisé en ce que** le groupe ET, identique ou différent à chaque occurrence, est choisi parmi les groupes de formules (ET-12) à (ET-35) 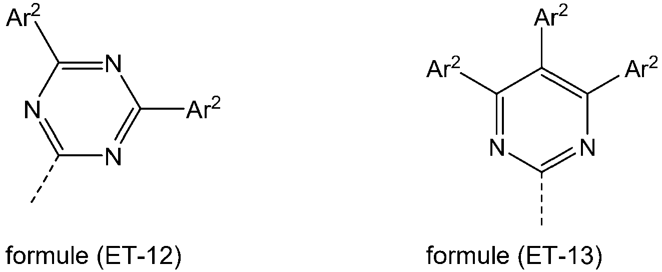 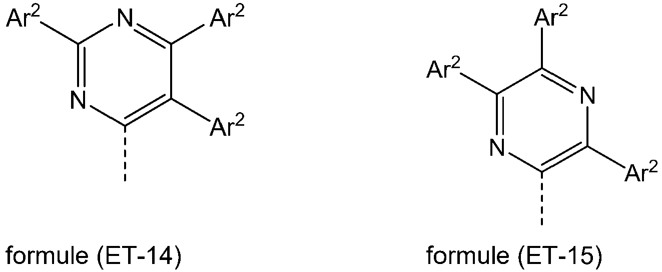 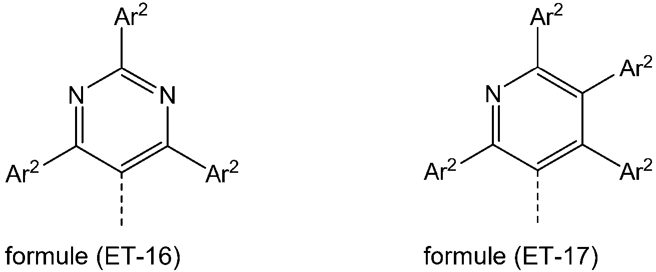 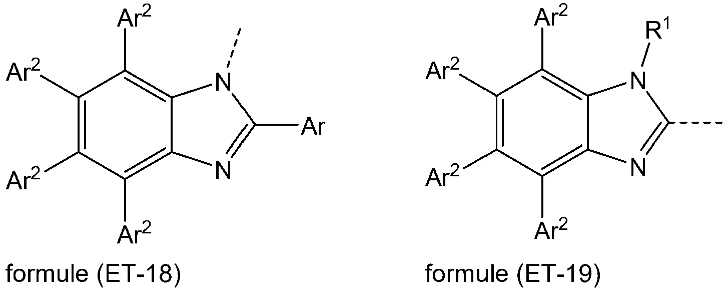 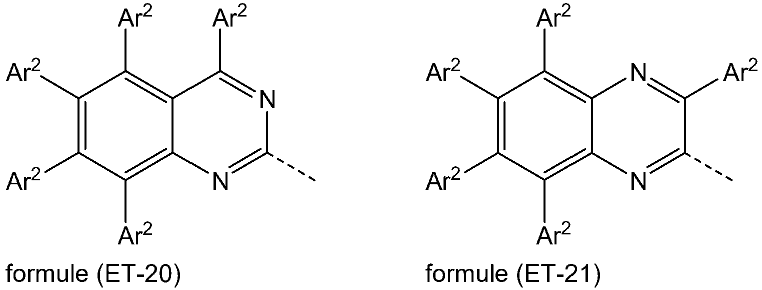 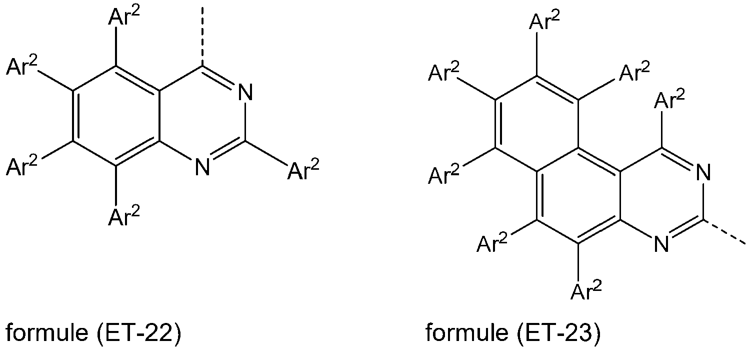 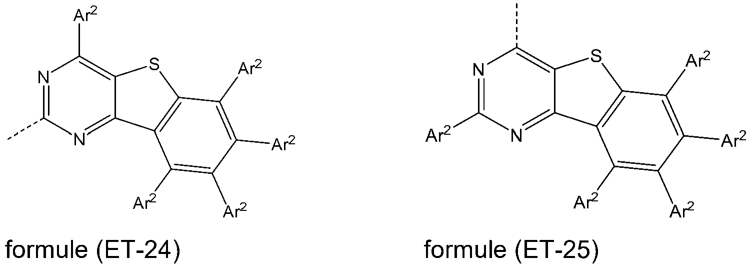 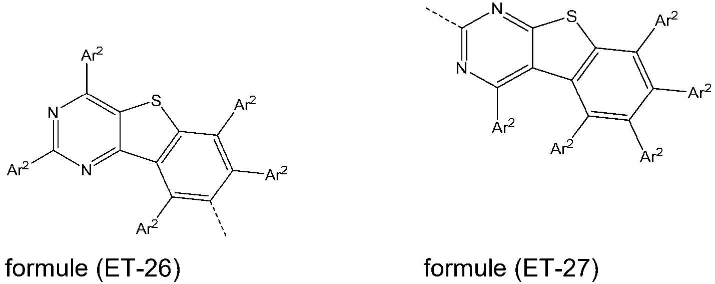 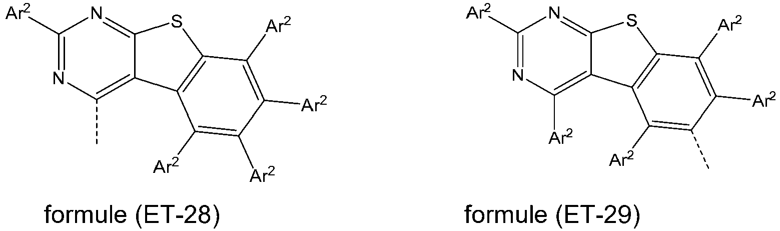 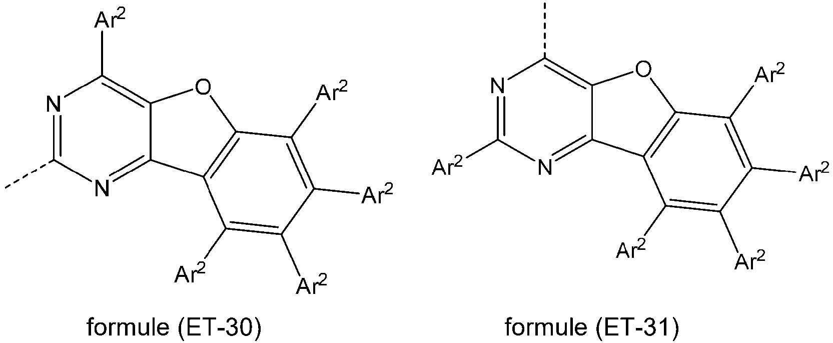 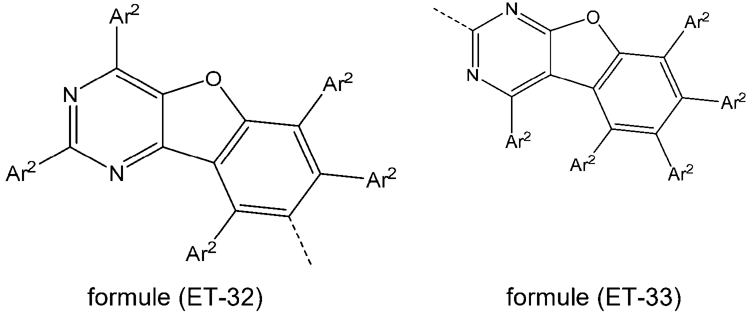 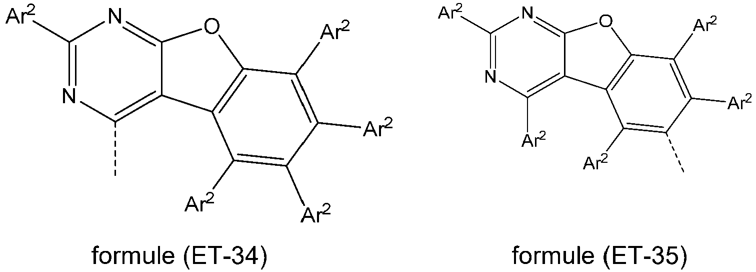 dans laquelle les lignes en pointillés représentent les liaisons avec le reste de la formule (I) et Ar² est tel que défini dans la revendication 1.

7. Composé selon l'une ou plusieurs des revendications 1 à 6, **caractérisé en ce qu'**il répond à la formule (I-A) suivante 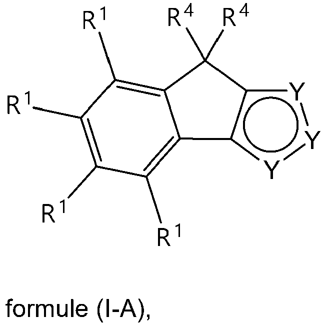
dans laquelle les variables sont définies comme dans une ou plusieurs des revendications 1 à 11 et au moins un groupe A est présent par formule; et
R⁴ est choisi à chaque occurrence, de manière identique ou différente, parmi H, D, F, CI, Br, I, C(=O)R¹, CN, Si(R¹)₃, N(R¹)₂, P(=O)(R¹)₂, OR¹, S(=O)R¹, S(=O)₂R¹, des groupes alkyle, alcoxy ou thioalkyle à chaîne droite ayant de 1 à 20 atomes de C, des groupes alkyle, alcoxy ou thioalkyle ramifiés ou cycliques ayant de 3 à 20 atomes de C, des groupes alcényle ou alcynyle ayant de 2 à 20 atomes de C, des systèmes cycliques aromatiques ayant de 6 à 40 atomes cycliques aromatiques, des systèmes cycliques hétéroaromatiques ayant de 5 à 40 atomes cycliques aromatiques, des groupes aryloxy ou hétéroaryloxy ayant de 5 à 40 atomes cycliques aromatiques, et des groupes aralkyle ayant de 5 à 40 atomes cycliques aromatiques; dans lequel il est exclu que les deux radicaux R⁴ soient liés l'un à l'autre et forment un cycle aliphatique ou hétéroaliphatique; lesdits groupes alkyle, alcoxy, thioalkyle, alcényle, alcynyle, aryloxy, hétéroaryloxy et aralkyle et lesdits systèmes cycliques aromatiques et systèmes cycliques hétéroaromatiques étant chacun substitués par des radicaux R¹; et dans lequel un ou plusieurs groupes CH₂ dans lesdits groupes alkyle, alcoxy, thioalkyle, alcényle, alcynyle, aryloxy, hétéroaryloxy et aralkyle peuvent être remplacés par -R¹C=CR¹-, - C≡C-, Si(R¹)₂, C=O, C=NR¹, -C(=O)O-, -C(=O)NR¹-, NR¹, P(=O)(R¹), - O-, -S-, SO ou SO₂.

8. Composé selon l'une ou plusieurs des revendications 1 à 7, **caractérisé en ce qu'**il répond à l'une des formules (II-A), (III-A) et (IV-A) suivantes 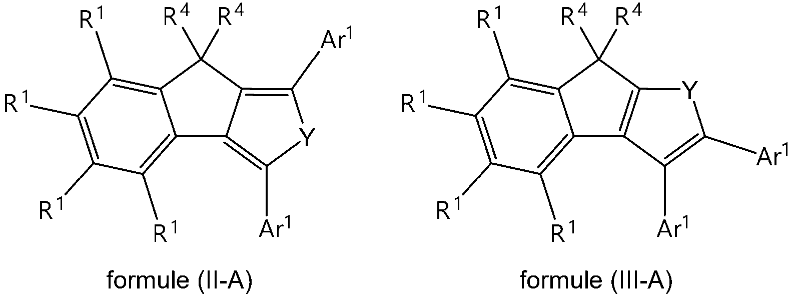 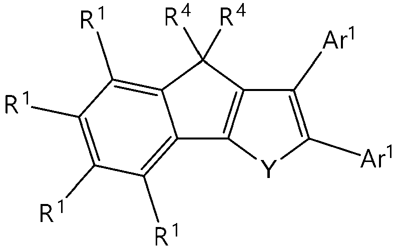 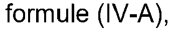
dans laquelle les variables sont définies comme dans une ou plusieurs des revendications 1 à 7 et au moins un groupe A est présent par formule ; et
R⁴ est choisi à chaque occurrence, de manière identique ou différente, parmi F, CN, Si(R¹)₃, des groupes alkyle à chaîne droite ayant de 1 à 20 atomes de C, des groupes alkyle ramifiés ou cycliques ayant de 3 à 20 atomes de C, des systèmes cycliques aromatiques ayant de 6 à 40 atomes de cycle aromatique, et des systèmes cycliques hétéroaromatiques ayant de 5 à 40 atomes de cycle aromatique; lesdits groupes alkyle et lesdits systèmes cycliques aromatiques et systèmes cycliques hétéroaromatiques étant chacun substitués par des radicaux R¹, et dans lequel **il** est exclu que les deux radicaux R⁴ soient reliés entre eux et forment un cycle aliphatique ou hétéroaliphatique.

9. Composé selon l'une ou plusieurs des revendications 1 à 6, **caractérisé en ce qu'**il répond à la formule (I-B) suivante: 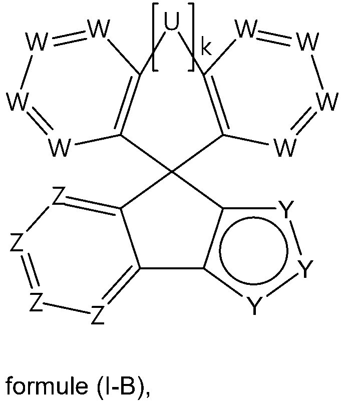 dans laquelle
W, identique ou différent à chaque occurrence, est choisi parmi N et CR¹;
U est choisi parmi O et S;
k est égal à 0 ou 1, dans le cas où k=0, l'hétéroatome U concerné est omis et U est à la place une liaison simple reliant les deux cycles aromatiques hexagonaux correspondants; et
au moins un groupe A est présent dans la formule (I-B), dans laquelle les variables Z, Y et R¹ et ledit au moins un groupe A sont tels que définis dans l'une quelconque des revendications 1 à 6.

10. Composé selon la revendication 9, **caractérisé en ce qu'**il répond à l'une des formules (II-B) à (IV-B) et (II-C) à (IV-C) suivantes:
| | |
|---|---|
| 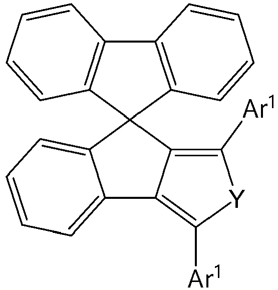 | 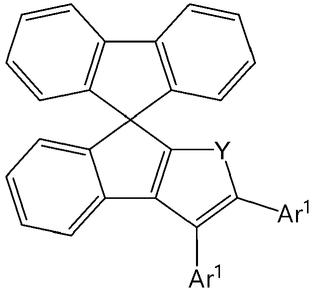 |
| formule (II-B) | formule (III-B) |
| 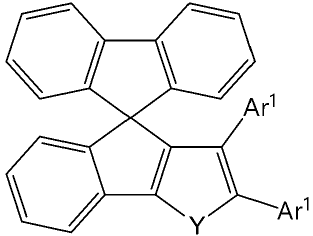 | 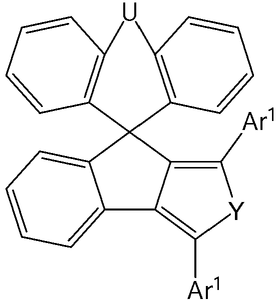 |
| formule (IV-B) | formule (II-C) |
| 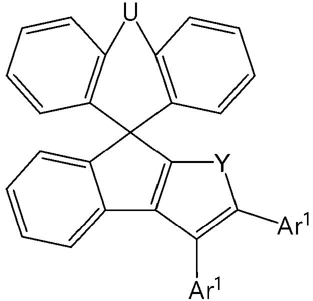 | 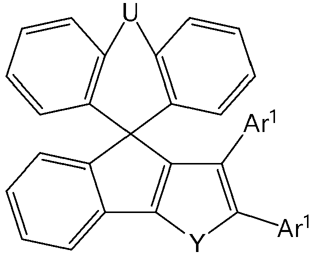 |
| formule (III-C) | formule (IV-C) |
dans laquelle les variables sont telles que définies dans une ou plusieurs des revendications 9 et 1 à 6, et les positions libres sur les cycles benzéniques sont respectivement substituées par des radicaux R¹, et dans laquelle au moins un groupe A est présent par formule.

11. Oligomère, polymère ou dendrimère contenant un ou plusieurs composés selon une ou plusieurs des revendications 1 à 10, dans lequel la ou les liaisons au polymère, à l'oligomère ou au dendrimère peuvent être localisées à n'importe quelle position substituée par R⁰, R¹, R² ou R³ dans la formule (I).

12. Formulation contenant au moins un composé selon l'une ou plusieurs des revendications 1 à 10 ou au moins un polymère, oligomère ou dendrimère selon la revendication 11, ainsi qu'au moins un solvant.

13. Composition contenant au moins un composé selon une ou plusieurs des revendications 1 à 10 ou au moins un polymère, oligomère ou dendrimère selon la revendication 11, ainsi qu'au moins un autre composé choisi parmi les matériaux d'injection de trous, les matériaux de transport de trous, les matériaux de blocage de trous, les *matériaux à large bande interdite,* les émetteurs fluorescents, les émetteurs phosphorescents, les matériaux à fluorescence différée, les matériaux hôtes, les matériaux de matrice, les matériaux de blocage d'électrons, les matériaux de transport d'électrons et les matériaux d'injection d'électrons, les n-dotands et les p-dotands.

14. Dispositif électronique contenant au moins un composé selon l'une ou plusieurs des revendications 1 à 10, au moins un polymère, oligomère ou dendrimère selon la revendication 11, ou une composition selon la revendication 13.

15. Dispositif électronique selon la revendication 14, **caractérisé en ce qu'**il est un dispositif organique électroluminescent et contient une anode, une cathode et au moins une couche émettrice, et **en ce que** le composé est contenu dans une couche de transport d'électrons ou dans une couche émettrice du dispositif.

16. Dispositif électronique selon la revendication 15, **caractérisé en ce que** le composé est un matériau de transport d'électrons dans la couche de transport d'électrons et un ou plusieurs autres composés de transport d'électrons sont en outre contenus dans la couche de transport d'électrons ou dans la couche d'injection d'électrons, les autres composés de transport d'électrons étant de préférence un hydroxyquinoléinate, de manière tout à fait préférée un dérivé de Liq et de manière tout à fait préférée Liq.

17. Dispositif électronique selon la revendication 15, **caractérisé en ce que** le composé est contenu en tant que matériau de transport d'électrons dans la couche émettrice conjointement avec un matériau de transport de trous.

18. Utilisation d'un composé selon une ou plusieurs des revendications 1 à 10 dans un dispositif électronique.
